# EUROPEAN PATENT APPLICATION

(11) **EP 2 048 150 A1**
(43) Date of publication of application: **15.04.2009**
(21) Application number: 07791905.8
(22) Date of filing: 03.08.2007
(51) Int. Cl.: C07H 15/203, A61K 31/7034, A61K 31/7036, A61P 1/16, A61P 3/00, A61P 3/04, A61P 3/06, A61P 3/10, A61P 7/10, A61P 9/04, A61P 9/10, A61P 9/12, A61P 13/12, A61P 19/06, A61P 25/02, A61P 27/02, A61P 27/12, A61P 31/00, A61P 43/00, C07H 17/02

(54) **BENZYL PHENYL GLUCOPYRANOSIDE DERIVATIVE**

(30) Priority: 04.08.2006 JP 2006213600
(71) Applicant: Daiichi Sankyo Company, Limited, Chuo-ku Tokyo 103-0023 (JP)
(72) Inventor: HONDA, Takeshi, Shinagawa-ku Tokyo 140-8710 (JP); OGUCHI, Minoru, Shinagawa-ku Tokyo 140-8710 (JP); YOSHIDA, Masao, Shinagawa-ku Tokyo 140-8710 (JP); OKUYAMA, Ryo, Shinagawa-ku Tokyo 140-8710 (JP); OGATA, Tsuneaki, Shinagawa-ku Tokyo 140-8710 (JP); ABE, Manabu, Shinagawa-ku Tokyo 140-8710 (JP); UEDA, Kenjiro, Shinagawa-ku Tokyo 140-8710 (JP); OHSUMI, Jun, Shinagawa-ku Tokyo 140-8710 (JP); IZUMI, Masanori, Shinagawa-ku Tokyo 140-8710 (JP)
(74) Representative: Fairbairn, Angus Chisholm
(86) International application number: PCT/JP2007/065231
(87) International publication number: WO 2008/016132

(57) **Abstract**

The present invention relates to a benzylphenyl glucopyranoside derivative having an excellent inhibitory effect on human SGLT1 and/or SGLT2 activity. There is provided a compound or a pharmacologically acceptable salt thereof represented by the following general formula (I): 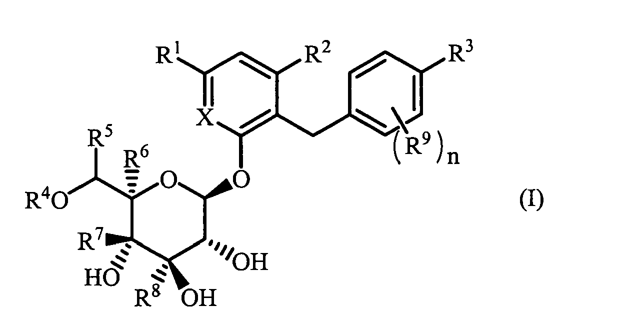 wherein
R¹ represents a hydrogen atom, an amino group, a hydroxy C₁-C₆ alkyl group, etc.; R² represents a hydrogen atom, etc.; R³ represents a C₁-C₆ alkyl group, a hydroxy C₁-C₆ alkyl group, etc.; R⁴ represents a hydrogen atom, a C₂-C₇ acyl group, etc.; R⁵, R⁶, R⁷, and R⁸ are the same or different and each represents a hydrogen atom or a C₁-C₆ alkyl group, provided that R⁵, R⁶, R⁷ and R⁸ are not hydrogen atoms at the same time; n is 0 to 4; and X is CH or N.

## Description

### Technical Field

The present invention relates to a compound having an inhibitory effect on human SGLT1 and/or SGLT2 activity.

### Background Art

Diabetes is a group of metabolic diseases that presents chronic high blood sugar levels due to insufficient insulin action as a primary characteristic. For the treatment of diabetes, drug therapy is performed along with diet therapy and exercise therapy, and biguanide drugs and thiazolidinedione drugs, which improve insulin resistance, sulfonylurea drugs and glinide drugs, which promote insulin secretion from pancreatic β cells, α-glucosidase inhibitors, which inhibit sugar absorption, and the like are used as diabetes remedies. However, it has been reported that biguanide drugs cause adverse drug reactions such as lactic acidosis, thiazolidinedione drugs cause weight gain and edema, sulfonylurea drugs and glinide drugs cause hypoglycemia and secondary nonresponse over long-term use, and α-glucosidase inhibitors cause diarrhea. Therefore, development of an antidiabetic drug having a novel mechanism of action that solves these problems has been awaited.

In recent years, research and development of a drug having a novel mechanism that increases sugar excretion in the urine by inhibiting sugar reabsorption in the kidneys to decrease blood sugar levels have been promoted (J. Clin. Invest., Vol. 79, pp. 1510-1515 [1987]). It has been shown that this drug suppresses sugar reabsorption from primitive urine by inhibiting sodium-dependent glucose cotransporter 2 (hereinafter referred to as SGLT2) present in the proximal renal tubules in the kidneys, thereby increasing sugar excretion out of the body to decrease blood sugar levels (J. Clin. Invest., Vol. 93, pp. 397-404 [1994]). Against this background, compounds that inhibit human SGLT2 are expected to normalize blood sugar levels by increasing sugar excretion to the urine and to be effective for type 1 and type 2 diabetes or various diseases associated with hyperglycemia. Furthermore, an anti-obesity effect is also expected because accumulation of sugar in the body is decreased by increasing the excretion of sugar.

Meanwhile, sodium-dependent glucose cotransporter 1 (hereinafter referred to as SGLT1), another subtype of SGLT, is expressed primarily in the small intestines and serves as a transporter for absorbing sugar (glucose and galactose) from food (Am. J. Clin. Nutr. Vol. 59 (3 Suppl.) pp. 690S-698S [1994]). It is known that sugar malabsorption occurs in humans who congenitally lack SGLT1 (Nature, Vol. 350, pp. 354-356 [1991]). These findings suggest that SGLT1-inhibiting drugs should exhibit an inhibitory effect on postprandial hyperglycemia by inhibiting and delaying sugar absorption from the small intestines. Furthermore, an anti-obesity effect can be expected by inhibiting the flow of sugar into the body.

Based on the above, drugs inhibiting human SGLT1 and/or SGLT2 activity that have both an effect of increasing sugar excretion to the urine and an effect of inhibiting sugar absorption from the small intestine can be expected to be used as potent type 1 and type 2 diabetes remedies, as anti-obesity drugs, and as drugs effective for various diseases associated with hyperglycemia.

O-Aryl glucoside compounds are known to have an inhibitory effect on human SGLT2 (refer to, for example, WO01/68660, WO02/28872, WO02/44192, WO02/64606, etc.). However, none of the above patent documents describes the compounds of the present invention, which have a substituent in a sugar moiety. Furthermore, it is not stated or indicated that such compounds have an inhibitory effect on human SGLT1.

### Disclosure of the Invention

The inventors of the present invention assiduously researched compounds inhibiting the human SGLT1 and/or SGLT2 activity. As a result, they found that the compounds of the present invention cause minimal adverse reactions, exhibit excellent human SGLT inhibiting activity, and are useful as therapeutic or preventive agents for type 1 diabetes, type 2 diabetes, gestational diabetes, hyperglycemia due to other causes, impaired glucose tolerance (IGT), diabetes-related diseases (for example, obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, and gout) or diabetic complications (for example, retinopathy, nephropathy, nervous disorder, cataract, foot gangrene, infections, and ketosis).

The present invention provides the following.
(1) A compound represented by the following general formula (I): 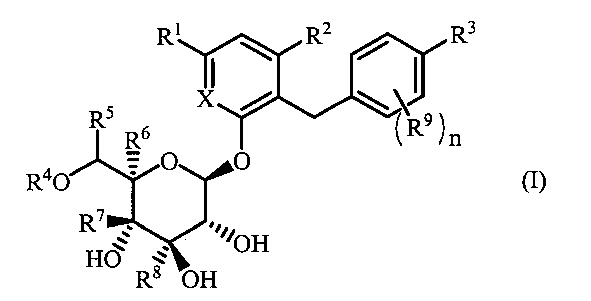 wherein
   R¹ represents a hydrogen atom, an amino group, a mono- or di-(C₁-C₆ alkyl)amino group, a C₁-C₆ alkyl group, a hydroxy C₁-C₆ alkyl group, a C₂-C₇ acyloxy C₁-C₆ alkyl group, a hydroxy C₂-C₇ acyloxy C₁-C₆ alkyl group, or an amino C₂-C₇ acylamino group;
   R² represents a hydrogen atom, a halogen atom, or a C₁-C₆ alkyl group;
   R³ represents a C₁-C₆ alkyl group, a hydroxy C₁-C₆ alkyl group, a C₁-C₆ alkoxy group, a hydroxy C₁-C₆ alkoxy group, a C₁-C₆ alkylthio group, or a halogenated C₁-C₆ alkoxy group;
   R⁴ represents a hydrogen atom, a C₁-C₆ alkyl group, a C₂-C₇ acyl group, a C₁-C₆ alkoxycarbonyl group, a hydroxy C₂-C₇ acyl group, a hydroxy C₁-C₆ alkoxycarbonyl group, a hydroxycarbonyl C₂-C₇ acyl group, a C₁-C₆ alkoxy C₂-C₇ acyl group, a C₁-C₆ alkoxy C₁-C₆ alkoxycarbonyl group, or a C₁-C₆ alkoxycarbonyl C₂-C₇ acyl group;
   R⁵, R⁶, R⁷ and R⁸ are the same or different and each represents a hydrogen atom or a C₁-C₆ alkyl group, provided that R⁵, R⁶, R⁷, and R⁸ are not hydrogen atoms at the same time;
   R⁹ represents a halogen atom;
   n is 0 to 4; and
   X is CH or N,
   or a pharmacologically acceptable salt thereof.
(2) The compound or a pharmacologically acceptable salt thereof according to the above (1), wherein R¹ represents an amino group, a mono- or di-(C₁-C₆ alkyl)amino group, a C₁-C₆ alkyl group, a hydroxy C₁-C₆ alkyl group, or a hydroxy C₂-C₇ acyloxy C₁-C₆ alkyl group.
(3) The compound or a pharmacologically acceptable salt thereof according to the above (1), wherein R¹ represents an amino group, a hydroxy C₁-C₆ alkyl group, or a hydroxy C₂-C₇ acyloxy C₁-C₆ alkyl group.
(4) The compound or a pharmacologically acceptable salt thereof according to the above (1), wherein R¹ represents an amino group, a hydroxymethyl group, a hydroxyethyl group, or a hydroxyacetyloxymethyl group.
(5) The compound or a pharmacologically acceptable salt thereof according to any one of the above (1) to (4), wherein R² represents a hydrogen atom, a fluorine atom, or a methyl group.
(6) The compound or a pharmacologically acceptable salt thereof according to any one of the above (1) to (5), wherein R³ represents a C₁-C₆ alkyl group, a C₁-C₆ alkoxy group, or a halogenated C₁-C₆ alkoxy group.
(7) The compound or a pharmacologically acceptable salt thereof according to any one of the above (1) to (5), wherein R³ represents a C₁-C₃ alkyl group, a C₁-C₃ alkoxy group, or a halogenated C₁-C₃ alkoxy group.
(8) The compound or a pharmacologically acceptable salt thereof according to any one of the above (1) to (5), wherein R³ represents a methyl group, an ethyl group, a methoxy group, an ethoxy group, an isopropoxy group, or a cyclopropyloxy group.
(9) The compound or a pharmacologically acceptable salt thereof according to any one of the above (1) to (8), wherein R⁴ represents a hydrogen atom, a C₂-C₇ acyl group, or a hydroxy C₂-C₇ acyl group.
(10) The compound or a pharmacologically acceptable salt thereof according to any one of the above (1) to (8), wherein R⁴ represents a hydrogen atom, a C₂-C₃ acyl group, or a hydroxy C₂-C₃ acyl group.
(11) The compound or a pharmacologically acceptable salt thereof according to any one of the above (1) to (8), wherein R⁴ represents a hydrogen atom.
(12) The compound or a pharmacologically acceptable salt thereof according to any one of the above (1) to (11), wherein R⁵ and R⁷ are the same or different and each represents a hydrogen atom or a C₁-C₆ alkyl group.
(13) The compound or a pharmacologically acceptable salt thereof according to any one of the above (1) to (11), wherein R⁵ and R⁷ are the same or different and each represents a hydrogen atom or a methyl group.
(14) The compound or a pharmacologically acceptable salt thereof according to any one of the above (1) to (13), wherein R⁶ and R⁸ represent a hydrogen atom.
(15) The compound or a pharmacologically acceptable salt thereof according to any one of the above (1) to (14), wherein n is 1.
(16) The compound or a pharmacologically acceptable salt thereof according to any one of the above (1) to (15), wherein R⁹ represents a fluorine atom.
(17) The compound or a pharmacologically acceptable salt thereof according to any one of the above (1) to (14), wherein n is 0.
(18) The compound or a pharmacologically acceptable salt thereof according to any one of the above (1) to (17), wherein X is CH.
(19) A compound represented by the following general formula (II): 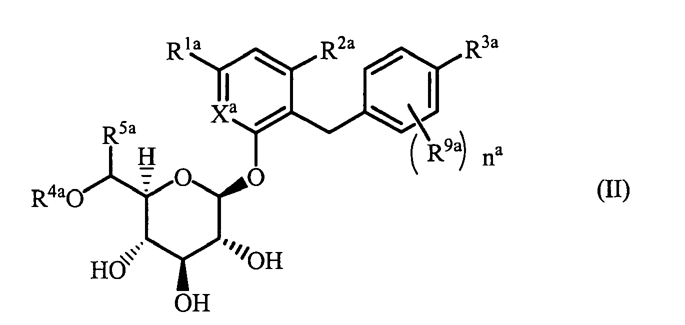 wherein
   R^{1a} represents a hydrogen atom, an amino group, a mono- or di-(C₁-C₆ alkyl)amino group, a C₁-C₆ alkyl group, a hydroxy C₁-C₆ alkyl group, a C₂-C₇ acyloxy C₁-C₆ alkyl group, a hydroxy C₂-C₇ acyloxy C₁-C₆ alkyl group, or an amino C₂-C₇ acylamino group;
   R^{2a} represents a hydrogen atom, a halogen atom, or a C₁-C₆ alkyl group;
   R^{3a} represents a C₁-C₆ alkyl group, a hydroxy C₁-C₆ alkyl group, a C₁-C₆ alkoxy group, a hydroxy C₁-C₆ alkoxy group, a C₁-C₆ alkylthio group, or a halogenated C₁-C₆ alkoxy group;
   R^{4a} represents a hydrogen atom, a C₁-C₆ alkyl group, a C₂-C₇ acyl group, a C₁-C₆ alkoxycarbonyl group, a hydroxy C₂-C₇ acyl group, a hydroxy C₁-C₆ alkoxycarbonyl group, a hydroxycarbonyl C₂-C₇ acyl group, a C₁-C₆ alkoxy C₂-C₇ acyl group, a C₁-C₆ alkoxy C₁-C₆ alkoxycarbonyl group, or a C₁-C₆ alkoxycarbonyl C₂-C₇ acyl group;
   R^{5a} represents a C₁-C₆ alkyl group;
   R^{9a} represents a halogen atom;
   n^{a} is 0 to 4; and
   X^{a} is CH or N,
   or a pharmacologically acceptable salt thereof.
(20) The compound or a pharmacologically acceptable salt thereof according to the above (19), wherein R^{1a} represents an amino group, a mono- or di-(C₁-C₆ alkyl)amino group, a C₁-C₆ alkyl group, a hydroxy C₁-C₆ alkyl group, or a hydroxy C₂-C₇ acyloxy C₁-C₆ alkyl group.
(21) The compound or a pharmacologically acceptable salt thereof according to the above (19), wherein R^{1a} represents an amino group, a hydroxy C₁-C₆ alkyl group, or a hydroxy C₂-C₇ acyloxy C₁-C₆ alkyl group.
(22) The compound or a pharmacologically acceptable salt thereof according to the above (19), wherein R^{1a} represents an amino group, a hydroxymethyl group, a hydroxyethyl group, or a hydroxyacetyloxymethyl group.
(23) The compound or a pharmacologically acceptable salt thereof according to any one of the above (19) to (22), wherein R^{2a} represents a hydrogen atom, a fluorine atom, or a methyl group.
(24) The compound or a pharmacologically acceptable salt thereof according to any one of the above (19) to (23), wherein R^{3a} represents a C₁-C₆ alkyl group, a C₁-C₆ alkoxy group, or a halogenated C₁-C₆ alkoxy group.
(25) The compound or a pharmacologically acceptable salt thereof according to any one of the above (19) to (23), wherein R^{3a} represents a C₁-C₃ alkyl group, a C₁-C₃ alkoxy group, or a halogenated C₁-C₃ alkoxy group.
(26) The compound or a pharmacologically acceptable salt thereof according to any one of the above (19) to (23), wherein R^{3a} represents a methyl group, an ethyl group, a methoxy group, an ethoxy group, an isopropoxy group, or a cyclopropyloxy group.
(27) The compound or a pharmacologically acceptable salt thereof according to any one of the above (19) to (26), wherein R^{4a} represents a hydrogen atom, a C₂-C₇ acyl group, or a hydroxy C₂-C₇ acyl group.
(28) The compound or a pharmacologically acceptable salt thereof according to any one of the above (19) to (26), wherein R^{4a} represents a hydrogen atom, a C₂-C₃ acyl group, or a hydroxy C₂-C₃ acyl group.
(29) The compound or a pharmacologically acceptable salt thereof according to any one of the above (19) to (26), wherein R^{4a} represents a hydrogen atom.
(30) The compound or a pharmacologically acceptable salt thereof according to any one of the above (19) to (29), wherein R^{5a} represents a methyl group.
(31) The compound or a pharmacologically acceptable salt thereof according to any one of the above (19) to (30), wherein n^{a} is 1.
(32) The compound or a pharmacologically acceptable salt thereof according to any one of the above (19) to (31), wherein R^{9a} represents a fluorine atom.
(33) The compound or a pharmacologically acceptable salt thereof according to any one of the above (19) to (30), wherein n^{a} is 0.
(34) The compound or a pharmacologically acceptable salt thereof according to any one of the above (19) to (33), wherein X^{a} is CH.
(35) A compound represented by the following general formula (III): 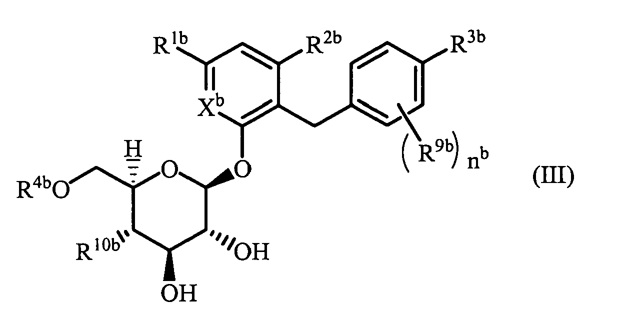 wherein
   R^{1b} represents a hydrogen atom, an amino group, a mono- or di-(C₁-C₆ alkyl)amino group, a C₁-C₆ alkyl group, a hydroxy C₁-C₆ alkyl group, a C₂-C₇ acyloxy C₁-C₆ alkyl group, a hydroxy C₂-C₇ acyloxy C₁-C₆ alkyl group, or an amino C₂-C₇ acylamino group;
   R^{2b} represents a hydrogen atom, a halogen atom, or a C₁-C₆ alkyl group;
   R^{3b} represents a C₁-C₆ alkyl group, a hydroxy C₁-C₆ alkyl group, a C₁-C₆ alkoxy group, a hydroxy C₁-C₆ alkoxy group, a C₁-C₆ alkylthio group, or a halogenated C₁-C₆ alkoxy group;
   R^{4b} represents a hydrogen atom, a C₁-C₆ alkyl group, a C₂-C₇ acyl group, a C₁-C₆ alkoxycarbonyl group, a hydroxy C₂-C₇ acyl group, a hydroxy C₁-C₆ alkoxycarbonyl group, a hydroxycarbonyl C₂-C₇ acyl group, a C₁-C₆ alkoxy C₂-C₇ acyl group, a C₁-C₆ alkoxy C₁-C₆ alkoxycarbonyl group, or a C₁-C₆ alkoxycarbonyl C₂-C₇ acyl group;
   R^{9b} represents a halogen atom;
   n^{b} is 0 to 4;
   R^{10b} represents a hydrogen atom, a C₁-C₆ alkyl group, a C₁-C₆ alkoxy group, or a hydroxyl group;
   X^{b} is CH or N; and
   provided that when R^{10b} represents a hydroxyl group, R^{4b} represents a C₁-C₆ alkyl group,
   or a pharmacologically acceptable salt thereof.
(36) The compound or a pharmacologically acceptable salt thereof according to the above (35), wherein R^{1b} represents an amino group, a mono- or di-(C₁-C₆ alkyl)amino group, a C₁-C₆ alkyl group, a hydroxy C₁-C₆ alkyl group, a C₂-C₃ acyloxy C₁-C₂ alkyl group, a hydroxy C₂-C₃ acyloxy C₁-C₂ alkyl group, or an amino C₂-C₃ acylamino group.
(37) The compound or a pharmacologically acceptable salt thereof according to the above (35), wherein R^{1b} represents an amino group, a mono- or di-(C₁-C₆ alkyl)amino group, a C₁-C₆ alkyl group, or a hydroxy C₁-C₆ alkyl group.
(38) The compound or a pharmacologically acceptable salt thereof according to the above (35), wherein R^{1b} represents an amino group, a mono or di-(C₁-C₂ alkyl)amino group, a C₁-C₂ alkyl group, or a hydroxy C₁-C₆ alkyl group.
(39) The compound or a pharmacologically acceptable salt thereof according to the above (35), wherein R^{1b} represents an amino group or a hydroxy C₁-C₆ alkyl group.
(40) The compound or a pharmacologically acceptable salt thereof according to the above (35), wherein R^{1b} represents an amino group or a hydroxy C₁-C₂ alkyl group.
(41) The compound or a pharmacologically acceptable salt thereof according to the above (35), wherein R^{1b} represents an amino group or a hydroxymethyl group.
(42) The compound or a pharmacologically acceptable salt thereof according to any one of the above (35) to (41), wherein R^{2b} represents a hydrogen atom or a C₁-C₆ alkyl group.
(43) The compound or a pharmacologically acceptable salt thereof according to any one of the above (35) to (41), wherein R^{2b} represents a hydrogen atom.
(44) The compound or a pharmacologically acceptable salt thereof according to any one of the above (35) to (43), wherein R^{3b} represents a C₁-C₆ alkyl group, a hydroxy C₁-C₂ alkyl group, a C₁-C₆ alkoxy group, a hydroxy C₁-C₂ alkoxy group, a C₁-C₂ alkylthio group, or a halogenated C₁-C₆ alkoxy group.
(45) The compound or a pharmacologically acceptable salt thereof according to any one of the above (35) to (43), wherein R^{3b} represents a C₁-C₆ alkyl group, a C₁-C₆ alkoxy group, or a halogenated C₁-C₆ alkoxy group.
(46) The compound or a pharmacologically acceptable salt thereof according to any one of the above (35) to (43), wherein R^{3b} represents a C₁-C₂ alkyl group, a C₁-C₂ alkoxy group, or a halogenated C₁-C₂ alkoxy group.
(47) The compound or a pharmacologically acceptable salt thereof according to any one of the above (35) to (43), wherein R^{3b} represents an ethyl group or a methoxy group.
(48) The compound or a pharmacologically acceptable salt thereof according to any one of the above (35) to (47), wherein R^{4b} represents a hydrogen atom, a C₁-C₂ alkyl group, a C₂-C₇ acyl group, a C₁-C₂ alkoxycarbonyl group, a hydroxy C₂-C₇ acyl group, a hydroxy C₁-C₂ alkoxycarbonyl group, a hydroxycarbonyl C₂-C₃ acyl group, a C₁-C₂ alkoxy C₂-C₃ acyl group, a C₁-C₂ alkoxy C₁-C₃ alkoxycarbonyl group, or a C₁-C₂ alkoxycarbonyl C₂-C₃ acyl group.
(49) The compound or a pharmacologically acceptable salt thereof according to any one of the above (35) to (47), wherein R^{4b} represents a hydrogen atom, a C₂-C₇ acyl group, or a hydroxy C₂-C₇ acyl group.
(50) The compound or a pharmacologically acceptable salt thereof according to any one of the above (35) to (47), wherein R^{4b} represents a hydrogen atom, a C₂-C₃ acyl group, or a hydroxy C₂-C₃ acyl group.
(51) The compound or a pharmacologically acceptable salt thereof according to any one of the above (35) to (47), wherein R^{4b} represents a hydrogen atom or a hydroxyacetyl group.
(52) The compound or a pharmacologically acceptable salt thereof according to any one of the above (35) to (51), wherein n^{b} is 1.
(53) The compound or a pharmacologically acceptable salt thereof according to any one of the above (35) to (52), wherein R^{9b} represents a fluorine atom.
(54) The compound or a pharmacologically acceptable salt thereof according to any one of the above (35) to (51), wherein n^{b} is 0.
(55) The compound or a pharmacologically acceptable salt thereof according to any one of the above (35) to (54), wherein X^{b} is CH.
(56) The compound or a pharmacologically acceptable salt thereof according to any one of the above (35) to (55), wherein R^{10b} represents a hydrogen atom, a C₁-C₂ alkyl group, a C₁-C₂ alkoxy group, or a hydroxyl group.
(57) The compound or a pharmacologically acceptable salt thereof according to any one of the above (35) to (55), wherein R^{10b} represents a hydrogen atom or a methoxy group.
(58) A compound or a pharmacologically acceptable salt thereof selected from the following:
   2-(4-methoxybenzyl)-5-hydroxymethylphenyl 7-deoxy-D-glycero-β-D-gluco-heptopyranoside, 5-amino-2-(4-ethylbenzyl)phenyl 7-deoxy-L-glycero-β-D-gluco-heptopyranoside, 5-amino-2-(4-ethylbenzyl)phenyl 4-C-methyl-β-D-glucopyranoside,
   5-amino-2-(4-ethylbenzyl)phenyl 7-deoxy-D-glycero-β-D-gluco-heptopyranoside, 5-amino-2-(4-methoxybenzyl)phenyl 7-deoxy-L-glycero-β-D-gluco-heptopyranoside,
   5-amino-2-(4-methoxybenzyl)phenyl 7-deoxy-D-glycero-β-D-gluco-heptopyranoside,
   3-fluoro-5-hydroxymethyl-2-(4-methoxybenzyl)phenyl 7-deoxy-D-glycero-β-D-gluco-heptopyranoside, 2-(4-ethylbenzyl)-3-fluoro-5-hydroxymethyl-phenyl 7-deoxy-D-glycero-β-D-gluco-heptopyranoside, 2-(4-ethoxybenzyl)-5-hydroxymethylphenyl 7-deoxy-D-glycero-β-D-gluco-heptapyranoside, 2-(4-ethylbenzyl)-5-(2-hydroxyethyl)phenyl 7-deoxy-D-glycero-β-D-gluco-heptopyranoside, 3-chloro-5-hydroxymethyl-2-(4-methoxybenzyl)phenyl 7-deoxy-D-glycero-β-D-gluco-heptopyranoside, 2-(2-fluoro-4-methoxybenzyl)-5-hydroxymethylphenyl 7-deoxy-D-glycero-β-D-gluco-heptopyranoside, 5-hydroxymethyl-2-(4-methoxybenzyl)-3-methylphenyl 7-deoxy-D-glycero-β-D-gluco-heptopyranoside, 2-(4-cyclopropyloxybenzyl)-5-hydroxymethylphenyl 7-deoxy-D-glycero-β-D-gluco-heptopyranoside, 2-(4-ethoxybenzyl)-3-fluoro-5-hydroxymethyl-phenyl 7-deoxy-D-glycero-β-D-gluco-heptopyranoside, 3-fluoro-5-hydroxymethyl-2-(4-isopropoxybenzyl)phenyl 7-deoxy-D-glycero-β-D-gluco-heptopyranoside, 5-hydroxyacetyloxymethyl-2-(4-ethoxybenzyl)phenyl 7-deoxy-D-glycero-β-D-gluco-heptopyranoside, 2-(4-cyclopropoxybenzyl)-5-hydroxymethyl-3-methyl-phenyl 7-deoxy-D-glycero-β-D-gluco-heptopyranoside, 2-(4-cyclopropylbenzyl)-5-hydroxymethylphenyl 7-deoxy-D-glycero-β-D-gluco-heptopyranoside, 3-chloro-2-(4-ethoxybenzyl)-5-hydroxymethyl-phenyl 7-deoxy-D-glycero-β-D-gluco-heptopyranoside, 3-fluoro-5-hydroxymethyl-2-(4-methylbenzyl)phenyl 7-deoxy-D-glycero-β-D-gluco-heptopyranoside, 2-(4-ethylbenzyl)-5-hydroxymethyl-3-methylphenyl 7-deoxy-D-glycero-β-D-gluco-heptopyranoside, and 2-(4-cyclopropylbenzyl)-3-fluoro-5-hydroxymethylphenyl 7-deoxy-D-glycero-β-D-gluco-heptopyranoside.
(59) A pharmaceutical composition comprising, as an active ingredient, the compound or a pharmacologically acceptable salt thereof according to any one selected from the above (1) to (58).
(60) The pharmaceutical composition according to the above (59) for inhibition of human SGLT1 and/or human SGLT2 activity.
(61) The pharmaceutical composition according to the above (60) for therapeutic or prophylactic treatment of type 1 diabetes, type 2 diabetes, gestational diabetes, hyperglycemia due to other causes, or impaired glucose tolerance.
(62) The pharmaceutical composition according to the above (60) for therapeutic or prophylactic treatment of type 1 diabetes, type 2 diabetes, or impaired glucose tolerance.
(63) The pharmaceutical composition according to the above (60) for therapeutic or prophylactic treatment of a diabetes-related disease.
(64) The pharmaceutical composition according to the above (63), wherein the diabetes-related disease is obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout.
(65) The pharmaceutical composition according to the above (63), wherein the diabetes-related disease is obesity.
(66) The pharmaceutical composition according to the above (60) for therapeutic or prophylactic treatment of a diabetic complication.
(67) The pharmaceutical composition according to the above (66), wherein the diabetic complication is retinopathy, nephropathy, nervous disorder, cataract, foot gangrene, infection, or ketosis.
(68) Use of the compound or a pharmacologically acceptable salt thereof according to any one selected from the above (1) to (58) for production of a pharmaceutical composition.
(69) The use according to the above (68), wherein the pharmaceutical composition is a composition for inhibition of human SGLT1 and/or human SGLT2 activity.
(70) The use according to the above (68), wherein the pharmaceutical composition is a composition for therapeutic or prophylactic treatment of type 1 diabetes, type 2 diabetes, gestational diabetes, hyperglycemia due to other causes, or impaired glucose tolerance.
(71) The use according to the above (68), wherein the pharmaceutical composition is a composition for therapeutic or prophylactic treatment of type 1 diabetes, type 2 diabetes, or impaired glucose tolerance.
(72) The use according to the above (68), wherein the pharmaceutical composition is a composition for therapeutic or prophylactic treatment of a diabetes-related disease.
(73) The use according to the above (72), wherein the diabetes-related disease is obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout.
(74) The use according to the above (72), wherein the diabetes-related disease is obesity.
(75) The use according to the above (68), wherein the pharmaceutical composition is a composition for therapeutic or prophylactic treatment of a diabetic complication.
(76) The use according to the above (75), wherein the diabetic complication is retinopathy, nephropathy, nervous disorder, cataract, foot gangrene, infection, or ketosis.
(77) A method for inhibiting human SGLT1 and/or human SGLT2 activity comprising administering a pharmacologically effective amount of the compound or a pharmacologically acceptable salt thereof according to any one selected from the above (1) to (58) to a warm-blooded animal.
(78) A method for therapeutic or prophylactic treatment of a disease comprising administering a pharmacologically effective amount of the compound or a pharmacologically acceptable salt thereof according to any one selected from the above (1) to (58) to a warm-blooded animal.
(79) The method according to the above (78), wherein the disease is type 1 diabetes, type 2 diabetes, gestational diabetes, hyperglycemia due to other causes, or impaired glucose tolerance.
(80) The method according to the above (78), wherein the disease is type 1 diabetes, type 2 diabetes, or impaired glucose tolerance.
(81) The method according to the above (78), wherein the disease is a diabetes-related disease.
(82) The method according to the above (81), wherein the diabetes-related disease is obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout.
(83) The method according to the above (81), wherein the diabetes-related disease is obesity.
(84) The method according to the above (78), wherein the disease is a diabetic complication.
(85) The method according to the above (84), wherein the diabetic complication is retinopathy, nephropathy, nervous disorder, cataract, foot gangrene, infection, or ketosis.
(86) The method according to any one selected from the above (77) to (85), wherein the warm-blooded animal is a human.

In the above formulas, the "C₁-C₆ alkyl group" in the definitions of R¹, R^{1a}, R^{1b}, R², R^{2a}, R^{2b}, R³, R^{3a}, R^{3b}, R⁴, R^{4a}, R^{4b}, R⁵, R^{5a}, R⁶, R⁷, R⁸, and R^{10b} is a straight, branched, or cyclic alkyl group having 1 to 6 carbon atoms, such as a methyl, ethyl, propyl, isopropyl, cyclopropyl, butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, or 2-ethylbutyl group. R¹, R^{1a}, R^{1b}, R², R^{2a}, R^{2b}, R⁴, R^{4a}, and R^{4b} are preferably a C₁-C₄ alkyl group, more preferably a C₁-C₃ alkyl group, most preferably a methyl group or an ethyl group. R³, R^{3a}, and R^{3b} are preferably a C₁-C₄ alkyl group, more preferably a C₁-C₂ alkyl group, most preferably an ethyl group. R⁵, R^{5a}, R⁶, R^{6b}, R⁷, R⁸, and R^{10b} are preferably a C₁-C₄ alkyl group, more preferably a C₁-C₂ alkyl group, most preferably a methyl group.

In the above formulas, the "halogen atom" in the definitions of R², R^{2a}, R^{2b}, R³, R^{3a}, R⁹, R^{9a}, and R^{9b} is a fluorine atom, a chlorine atom, a bromine atom, or an iodine atom, preferably a fluorine atom or a chlorine atom.

In the above formulas, R⁹ may be substituted at any of the 2nd, 3rd, 5th, or 6th positions. n represents the number of R⁹. When n is 0, it means that no substituent exists at the 2nd, 3rd, 5th, or 6th positions. When n is 2 or more and 4 or less, R⁹ may be the same halogen atoms or different halogen atoms.

In the above formulas, the "C₁-C₆ alkoxy group" in the definitions of R³, R^{3a}, R^{3b}, and R^{10b} is a straight or branched alkoxy group having 1 to 6 carbon atoms, such as a methoxy, ethoxy, propoxy, isopropoxy, butoxy, isobutoxy, s-butoxy, t-butoxy, pentoxy, isopentoxy, 2-methylbutoxy, neopentoxy, hexyloxy, 4-methylpentoxy, 3-methylpentoxy, or 2-methylpentoxy group, or a cyclic alkoxy group having 3 to 6 carbon atoms such as a cyclopropyloxy group, preferably a C₁-C₄ alkoxy group, more preferably a C₁-C₃ alkoxy group, most preferably a methoxy group, an ethoxy group, an isopropoxy group, or a cyclopropyloxy group.

In the above formulas, the "C₂-C₇ acyl group" in the definitions of R⁴, R^{4a}, and R^{4b} represents a group in which the above C₁-C₆ alkyl group binds to a carbonyl group and is, for example, an acetyl, propionyl, butyryl, isobutyryl, s-butyryl, t-butyryl, pentanoyl, isopentanoyl, 2-methylbutyryl, neopentanoyl, 1-ethylpropionyl, hexanoyl, 4-methylpentanoyl, 3-methylpentanoyl, 2-methylpentanoyl, or 1-methylpentanoyl group, preferably a C₂-C₅ acyl group, more preferably a C₂-C₃ acyl group, most preferably an acetyl group.

In the above formulas, the "mono- or di-(C₁-C₆ alkyl)amino group" in the definitions of R¹, R^{1a}, and R^{1b} represents a group in which 1 or 2 of the above "lower alkyl groups" bind to an amino group. Examples of the mono-(C₁-C₆ alkyl)amino group include methylamino, ethylamino, propylamino, isopropylamino, butylamino, isobutylamino, s-butylamino, t-butylamino, pentylamino, isopentylamino, 2-methylbutylamino, neopentylamino, 1-ethylpropylamino, hexylamino, isohexylamino, 4-methylpentylamino, 3-methylpentylamino, 2-methylpentylamino, or 1-methylpentylamino groups. Examples of the di-(C₁-C₆ alkyl)amino group include dimethylamino, diethylamino, N-ethyl-N-methylamino, dipropylamino, dibutylamino, dipentylamino, or dihexylamino groups. The "mono- or di-(C₁-C₆ alkyl)amino group" is preferably a mono- or di-(C₁-C₄ alkyl)amino group, more preferably a mono- or di-(C₁-C₂ alkyl)amino group, most preferably a methylamino group.

In the above formulas, the "hydroxy C₁-C₆ alkyl group" in the definitions of R¹, R^{1a}, R^{1b}, R³, R^{3a}, and R^{3b} represents a group in which a hydroxyl group is substituted on the above C₁-C₆ alkyl group and is, for example, a hydroxymethyl, 2-hydroxyethyl, 3-hydroxypropyl, 4-hydroxybutyl, 5-hydroxypentyl, 6-hydroxyhexyl, 1-hydroxyethyl, or 1-hydroxypropyl group, preferably a hydroxy C₁-C₄ alkyl group, more preferably a hydroxy C₁-C₂ alkyl group, most preferably a hydroxymethyl group or a hydroxyethyl group.

In the above formulas, the "hydroxy C₁-C₆ alkoxy group" in the definitions of R³, R^{3a}, and R^{3b} represents a group in which a hydroxyl group is substituted on the above C₁-C₆ alkoxy group and is, for example, a hydroxymethoxy, 2-hydroxyethoxy, 3-hydroxypropoxy, 4-hydroxybutoxy, 5-hydroxypentoxy, or 6-hydroxyhexyloxy group, preferably a hydroxy C₁-C₄ alkoxy group, more preferably a hydroxy C₁-C₂ alkoxy group, most preferably a 2-hydroxyethoxy group.

In the above formulas, the "C₁-C₆ alkylthio group" in the definitions of R³, R^{3a}, and R^{3b} is a straight or branched alkylthio group having 1 to 6 carbon atoms, such as a methylthio, ethylthio, propylthio, isopropylthio, butylthio, isobutylthio, s-butylthio, t-butylthio, pentylthio, isopentylthio, 2-methylbutylthio, neopentylthio, hexylthio, 4-methylpentylthio, 3-methylpentylthio, or 2-methylpentylthio group, preferably a C₁-C₄ alkylthio group, more preferably a C₁-C₂ alkylthio group, most preferably a methylthio group.

In the above formulas, "halogenated C₁-C₆ alkoxy group" in the definitions of R³, R^{3a}, and R^{3b} represents a group in which the above "halogen atom" is substituted on the above "lower alkyl group" and is, for example, a trifluoromethoxy, trichloromethoxy, difluoromethoxy, dichloromethoxy, dibromomethoxy, fluoromethoxy, 2,2,2-trifluoroethoxy, 2,2,2-trichloroethoxy, 2-bromoethoxy, 2-chloroethoxy, 2-fluoroethoxy, 2-iodoethoxy, 3-chloropropoxy, 4-fluorobutoxy, 6-iodohexyloxy, or 2,2-dibromoethoxy group, preferably a halogeno C₁-C₄ alkoxy group, more preferably a halogeno C₁-C₂ alkoxy group, most preferably a trifluoromethoxy group.

In the above formulas, the "C₂-C₇ acyloxy C₁-C₆ alkyl group" in the definitions of R¹, R^{1a}, and R^{1b} represents a group in which a "C₂-C₇ acyloxy group", in which the above "C₂-C₇ acyl group" binds to an oxygen atom, is substituted on the above "C₁-C₆ alkyl group" and is, for example, an acetyloxymethyl, 2-acetyloxyethyl, 3-acetyloxypropyl, 4-acetyloxybutyl, propionyloxymethyl, 2-propionyloxyethyl, or butyryloxymethyl group, preferably a C₂-C₅ acyloxy C₁-C₄ alkyl group, more preferably a C₂-C₃ acyloxy C₁-C₂ alkyl group, most preferably an acetyloxymethyl group.

In the above formulas, the "hydroxy C₂-C₇ acyloxy C₁-C₆ alkyl group" in the definitions of R¹, R^{1a}, and R^{1b} represents a group in which a hydroxyl group is substituted on the above "C₂-C₇ acyloxy C₁-C₆ alkyl group" and is, for example, a (hydroxyacetyloxy)methyl, 2-(hydroxyacetyloxy)ethyl, 3-(hydroxyacetyloxy)propyl, 4-(hydroxyacetyloxy)butyl, (hydroxypropionyloxy)methyl, 2-(3-hydroxypropionyloxy)ethyl, or (4-hydroxybutyryloxy)methyl group, preferably a hydroxy C₂-C₅ acyloxy C₁-C₄ alkyl group, more preferably a hydroxy C₂-C₃ acyloxy C₁-C₂ alkyl group, most preferably a (hydroxyacetyloxy)methyl group.

In the above formulas, the "amino C₂-C₇ acylamino group" in the definitions of R¹, R^{1a}, and R^{1b} represents a group in which an "amino C₂-C₇ acyl group," in which an amino group is substituted on the above "C₂-C₇ acyl group," is substituted on an amino group and is, for example, an aminoacetylamino, 3-aminopropionylamino, 4-aminobutyrylamino, 5-aminopentanoylamino, or 6-aminohexanoylamino group, preferably an amino C₂-C₅ acylamino group, more preferably an amino C₂-C₃ acylamino group, most preferably an aminoacetylamino group.

In the above formulas, the "C₁-C₆ alkoxycarbonyl group" in the definitions of R⁴, R^{4a}, and R^{4b} is a straight or branched alkoxy group having 1 to 6 carbon atoms that binds to a carbonyl group, such as a methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentoxycarbonyl, isopentoxycarbonyl, 2-methylbutoxycarbonyl, neopentoxycarbonyl, hexyloxycarbonyl, 4-methylpentoxycarbonyl, 3-methylpentoxycarbonyl, or 2-methylpentoxycarbonyl group, preferably a C₁-C₄ alkoxycarbonyl group, more preferably a C₁-C₂ alkoxycarbonyl group, most preferably an ethoxycarbonyl group.

In the above formulas, the "hydroxy C₂-C₇ acyl group" in the definitions of R⁴, R^{4a}, and R^{4b} represents a group in which a hydroxyl group is substituted on the above "C₂-C₇ acyl group" and is, for example, a hydroxyacetyl, 3-hydroxypropionyl, 4-hydroxybutyryl, 5-hydroxypentanoyl, or 6-hydroxyhexanoyl group, preferably a hydroxy C₂-C₅ acyl group, more preferably a hydroxy C₂-C₃ acyl group, most preferably a hydroxyacetyl group.

In the above formulas, the "hydroxy C₁-C₆ alkoxycarbonyl group" in the definitions of R⁴, R^{4a}, and R^{4b} represents a group in which a hydroxyl group is substituted on the above "C₁-C₆ alkoxycarbonyl group" and is, for example, a hydroxymethoxycarbonyl, 2-hydroxyethoxycarbonyl, 3-hydroxypropoxycarbonyl, 4-hydroxybutoxycarbonyl, 5-hydroxypentoxycarbonyl, or 6-hydroxyhexyloxycarbonyl group, preferably a hydroxy C₁-C₄ alkoxycarbonyl group, more preferably a hydroxy C₁-C₂ alkoxycarbonyl group, most preferably a hydroxymethoxycarbonyl group.

In the above formulas, the "hydroxycarbonyl C₂-C₇ acyl group" in the definitions of R⁴, R^{4a}, and R^{4b} is, for example, a hydroxycarbonylacetyl, 3-hydroxycarbonylpropionyl, 4-hydroxycarbonylbutyryl, 5-hydroxycarbonylpentanoyl, or 6-hydroxycarbonylhexanoyl group, preferably a hydroxycarbonyl C₂-C₅ acyl group, more preferably a hydroxycarbonyl C₂-C₃ acyl group, most preferably a hydroxycarbonylacetyl group.

In the above formulas, the "C₁-C₆ alkoxy C₂-C₇ acyl group" in the definitions of R⁴, R^{4a}, and R^{4b} represents a group in which the above "C₁-C₆ alkoxy group" is substituted on the above "C₂-C₇ acyl group" and is, for example, a methoxyacetyl, ethoxyacetyl, propoxyacetyl, butoxyacetyl, 3-methoxypropionyl, 3-ethoxypropionyl, 4-methoxybutyryl, 5-methoxypentanoyl, or 6-methoxyhexanoyl group, preferably a C₁-C₄ alkoxy C₂-C₅ acyl group, more preferably a C₁-C₂ alkoxy C₂-C₃ acyl group, most preferably a methoxyacetyl group.

In the above formulas, the "C₁-C₆ alkoxy C₁-C₆ alkoxycarbonyl group" in the definitions of R⁴, R^{4a}, and R^{4b} represents a group in which the above "C₁-C₆ alkoxy group" is substituted on the above "C₁-C₆ alkoxycarbonyl group" and is, for example, a methoxymethoxycarbonyl, ethoxymethoxycarbonyl, propoxymethoxycarbonyl, 2-methoxyethoxycarbonyl, 3-methoxypropoxycarbonyl, 4-methoxybutoxycarbonyl, 5-methoxypentoxycarbonyl, or 6- methoxyhexyloxycarbonyl group, preferably a C₁-C₄ alkoxy C₁-C₄ alkoxycarbonyl group, more preferably a C₁-C₂ alkoxy C₁-C₂ alkoxycarbonyl group, most preferably a methoxymethoxycarbonyl group.

In the above formulas, the "C₁-C₆ alkoxycarbonyl C₂-C₇ acyl group" in the definitions of R⁴, R^{4a}, and R^{4b} represents a group in which the above "C₁-C₆ alkoxycarbonyl" group is substituted on the above "C₂-C₇ acyl group" and is, for example, a methoxycarbonylacetyl, ethoxycarbonylacetyl, 3-methoxycarbonylpropionyl, 4-methoxycarbonylbutyryl, 5-methoxycarbonylpentanoyl, or 6-methoxycarbonylhexanoyl group, preferably a C₁-C₄ alkoxycarbonyl C₂-C₅ acyl group, more preferably a C₁-C₂ alkoxycarbonyl C₂-C₃ acyl group, most preferably a methoxycarbonylacetyl group.

Since a salt can be formed by reacting the compound represented by the general formula (I), (II), or (III) of the present invention with an acid when the compound has a basic group such as an amino group or with a base when the compound has an acidic group such as a carboxyl group, the term "a pharmacologically acceptable salt thereof" refers to such a salt.

Preferred examples of salts based on a basic group include hydrohalides such as hydrofluorides, hydrochlorides, hydrobromides, and hydroiodides; inorganic acid salts such as nitrates, perchlorates, sulfates, and phosphates; lower alkanesulfonates such as methanesulfonates, trifluoromethanesulfonates, and ethanesulfonates; aryl sulfonates such as benzenesulfonates and p-toluenesulfonates; organic acid salts such as acetates, malates, fumarates, succinates, citrates, ascorbates, tartrates, oxalates, and maleates; and amino acid salts such as glycine salts, lysine salts, arginine salts, ornithine salts, glutamates, and aspartates.

Preferred examples of salts based on an acidic group include alkali metal salts such as sodium salts, potassium salts, and lithium salts; alkaline earth metal salts such as calcium salts and magnesium salts; metal salts such as aluminium salts and iron salts; inorganic salts such as ammonium salts; amine salts of organic salts and the like such as t-octylamine salts, dibenzylamine salts, morpholine salts, glucosamine salts, phenyl glycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzylphenethylamine salts, piperazine salts, tetramethyl ammonium salts, tris(hydroxymethyl)aminomethane salts; and amino acid salts such as glycine salts, lysine salts, arginine salts, ornithine salts, glutamates, and aspartates.

The compound represented by the general formula (I), (II), or (III) or a pharmacologically acceptable salt thereof of the present invention may form a hydrate when the compound absorbs moisture or adsorbed water is attached by leaving it in an atmosphere or recrystallizing it. Such hydrates are also included in the salts of the present invention.

Since the compound represented by the general formula (I), (II), or (III) or a pharmacologically acceptable salt thereof of the present invention has an asymmetric carbon atom in the molecule, an optical isomer thereof exists. These isomers and mixtures of these isomers are all represented by one single formula, specifically, the general formula (I), (II), or (III) of the present invention. Therefore, the present invention includes all optical isomers and mixtures of optical isomers in arbitrary ratios.

Compounds listed in the following Table 1 can be mentioned as specific examples of the compound represented by the general formula (I) of the present invention, but the present invention is not limited to these compounds.

**[Table 1]**

| | | | | | | | | | | | |
|---|---|---|---|---|---|---|---|---|---|---|---|
| 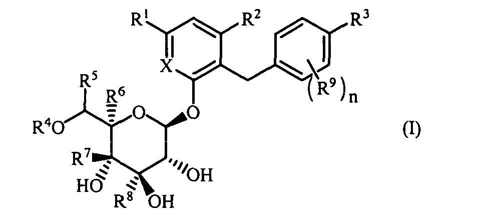 | | | | | | | | | | | |

| No. | R¹ | R² | R³ | R⁴ | R⁵ | R⁶ | R⁷ | R⁸ | R⁹ | n | X |
|---|---|---|---|---|---|---|---|---|---|---|---|
| 1-1 | H | CH₃ | CH₂CH₃ | H | CH₃ | H | H | H | - | 0 | N |
| 1-2 | H | H | OCH₃ | H | CH₃ | H | H | H | - | 0 | CH |
| 1-3 | NH₂ | H | CH₃ | H | CH₃ | H | H | H | - | 0 | CH |
| 1-4 | NH₂ | H | CH₃ | H | H | H | CH₃ | H | - | 0 | CH |
| 1-5 | NH₂ | H | CH₂CH₃ | H | CH₃ | H | H | H | - | 0 | CH |
| 1-6 | NH₂ | CH₃ | CH₂CH₃ | H | CH₃ | H | H | H | - | 0 | CH |
| 1-7 | NH₂ | Cl | CH₂CH₃ | H | CH₃ | H | H | H | - | 0 | CH |
| 1-8 | NH₂ | H | CH₂CH₃ | H | CH₃ | H | H | H | - | 0 | N |
| 1-9 | NH₂ | H | CH₂CH₃ | H | H | CH₃ | H | H | - | 0 | CH |
| 1-10 | NH₂ | H | CH₂CH₃ | H | CH₃ | H | CH₃ | H | - | 0 | CH |
| 1-11 | NH₂ | CH₃ | CH₂CH₃ | H | H | H | CH₃ | H | - | 0 | CH |
| 1-12 | NH₂ | Cl | CH₂CH₃ | H | H | H | CH₃ | H | - | 0 | CH |
| 1-13 | NH₂ | H | CH₂CH₃ | H | H | H | CH₃ | H | - | 0 | N |
| 1-14 | NH₂ | H | CH₂CH₃ | H | H | H | H | CH₃ | - | 0 | CH |
| 1-15 | NH₂ | H | CH₂CH₃ | C(O)CH₃ | CH₃ | H | H | H | - | 0 | CH |
| 1-16 | NH₂ | H | CH₂CH₃ | CO₂CH₂CH₃ | CH₃ | H | H | H | - | 0 | CH |
| 1-17 | NH₂ | H | CH₂CH₃ | COCH₂OH | CH₃ | H | H | H | - | 0 | CH |
| 1-18 | NH₂ | H | CH₂CH₃ | CO₂CH₂OH | CH₃ | H | H | H | - | 0 | CH |
| 1-19 | NH₂ | H | CH₂CH₃ | COCH₂CO₂H | CH₃ | H | H | H | - | 0 | CH |
| 1-20 | NH2 | H | CH₂CH₃ | COCH₂OCH₃ | CH₃ | H | H | H | - | 0 | CH |
| 1-21 | NH₂ | H | CH₂CH₃ | CO₂CH₂OCH₃ | CH₃ | H | H | H | - | 0 | CH |
| 1-22 | NH₂ | H | CH₂CH₃ | COCH₂CO₂CH₃ | CH₃ | H | H | H | - | 0 | CH |
| 1-23 | NH₂ | H | OCH₃ | H | CH₃ | H | H | H | - | 0 | CH |
| 1-24 | NH₂ | CH₃ | OCH₃ | H | CH₃ | H | H | H | - | 0 | CH |
| 1-25 | NH₂ | Cl | OCH₃ | H | CH₃ | H | H | H | - | 0 | CH |
| 1-26 | NH₂ | H | OCH₃ | H | CH₃ | H | H | H | - | 0 | N |
| 1-27 | NH₂ | H | OCH₃ | H | H | CH₃ | H | H | - | 0 | CH |
| 1-28 | NH₂ | H | OCH₃ | H | H | H | CH₃ | H | - | 0 | CH |
| 1-29 | NH₂ | CH₃ | OCH₃ | H | H | H | CH₃ | H | - | 0 | CH |
| 1-30 | NH₂ | Cl | OCH₃ | H | H | H | CH₃ | H | - | 0 | CH |
| 1-31 | NH₂ | H | OCH₃ | H | H | H | CH₃ | H | - | 0 | N |
| 1-32 | NH₂ | H | OCH₃ | H | H | H | H | CH₃ | - | 0 | CH |
| 1-33 | NH₂ | H | OCH₃ | C(O)CH₃ | CH₃ | H | H | H | - | 0 | CH |
| 1-34 | NH₂ | H | OCH₃ | CO₂CH₂CH₃ | CH₃ | H | H | H | - | 0 | CH |
| 1-35 | NH₂ | H | OCH₃ | COCH₂OH | CH₃ | H | H | H | - | 0 | CH |
| 1-36 | NH₂ | H | OCH₃ | CO₂CH₂OH | CH₃ | H | H | H | - | 0 | CH |
| 1-37 | NH₂ | H | OCH₃ | COCH₂CO₂H | CH₃ | H | H | H | - | 0 | CH |
| 1-38 | NH₂ | H | OCH₃ | COCH₂OCH₃ | CH₃ | H | H | H | - | 0 | CH |
| 1-39 | NH₂ | H | OCH₃ | CO₂CH₂OCH₃ | CH₃ | H | H | H | - | 0 | CH |
| 1-40 | NH₂ | H | OCH₃ | COCH₂CO₂CH₃ | CH₃ | H | H | H | - | 0 | CH |
| 1-41 | NH₂ | H | OCH₂CH₃ | H | CH₃ | H | H | H | - | 0 | CH |
| 1-42 | NH₂ | H | CH₂CH₂OH | H | CH₃ | H | H | H | - | 0 | CH |
| 1-43 | NH₂ | H | OCH₂CH₂OH | H | CH₃ | H | H | H | - | 0 | CH |
| 1-44 | NH₂ | H | SCH₃ | H | CH₃ | H | H | H | - | 0 | CH |
| 1-45 | NH₂ | H | OCF₃ | H | CH₃ | H | H | H | - | 0 | CH |
| 1-46 | NHCH₃ | H | CH₂CH₃ | H | CH₃ | H | H | H | - | 0 | CH |
| 1-47 | NHCH₃ | H | OCH₃ | H | CH₃ | H | H | H | - | 0 | CH |
| 1-48 | N(CH₃)₂ | H | CH₂CH₃ | H | CH₃ | H | H | H | - | 0 | CH |
| 1-49 | N(CH₃)₂ | H | OCH₃ | H | CH₃ | H | H | H | - | 0 | CH |
| 1-50 | CH₃ | H | CH₂CH₃ | H | CH₃ | H | H | H | - | 0 | N |
| 1-51 | CH₃ | H | OCH₃ | H | CH₃ | H | H | H | - | 0 | CH |
| 1-52 | CH₃ | H | OCH₃ | H | CH₃ | H | H | H | - | 0 | N |
| 1-53 | CH₃ | CH₃ | CH₂CH₃ | H | CH₃ | H | H | H | - | 0 | CH |
| 1-54 | CH₃ | CH₃ | OCH₃ | H | CH₃ | H | H | H | - | 0 | N |
| 1-55 | CH₂CH₃ | H | CH₂CH₃ | H | CH₃ | H | H | H | - | 0 | CH |
| 1-56 | CH₂CH₃ | H | OCH₃ | H | CH₃ | H | H | H | - | 0 | CH |
| 1-57 | CH₂OH | H | CH₃ | H | CH₃ | H | H | H | - | 0 | CH |
| 1-58 | CH₂OH | H | CH₃ | H | H | H | CH₃ | H | - | 0 | CH |
| 1-59 | CH₂OH | H | CH₂CH₃ | H | CH₃ | H | H | H | - | 0 | CH |
| 1-60 | CH₂OH | CH₃ | CH₂CH₃ | H | CH₃ | H | H | H | - | 0 | CH |
| 1-61 | CH₂OH | Cl | CH₂CH₃ | H | CH₃ | H | H | H | - | 0 | CH |
| 1-62 | CH₂OH | H | CH₂CH₃ | H | CH₃ | H | H | H | - | 0 | N |
| 1-63 | CH₂OH | H | CH₂CH₃ | H | H | CH₃ | H | H | - | 0 | CH |
| 1-64 | CH₂OH | H | CH₂CH₃ | H | H | H | CH₃ | H | - | 0 | CH |
| 1-65 | CH₂OH | CH₃ | CH₂CH₃ | H | H | H | CH₃ | H | - | 0 | CH |
| 1-66 | CH₂OH | Cl | CH₂CH₃ | H | H | H | CH₃ | H | - | 0 | CH |
| 1-67 | CH₂OH | H | CH₂CH₃ | H | H | H | CH₃ | H | - | 0 | N |
| 1-68 | CH₂OH | H | CH₂CH₃ | H | H | H | H | CH₃ | - | 0 | CH |
| 1-69 | CH₂OH | H | CH₂CH₃ | C(O)CH₃ | CH₃ | H | H | H | - | 0 | CH |
| 1-70 | CH₂OH | H | CH₂CH₃ | C(O)CH₃ | H | H | CH₃ | H | - | 0 | CH |
| 1-71 | CH₂OH | H | CH₂CH₃ | H | H | CH₃ | H | H | - | 0 | N |
| 1-72 | CH₂OH | H | CH₂CH₃ | COCH₂OH | CH₃ | H | H | H | - | 0 | CH |
| 1-73 | CH₂OH | H | CH₂CH₃ | C(O)CH₂OH | H | CH₃ | H | H | - | 0 | CH |
| 1-74 | CH₂OH | H | CH₂CH₃ | C(O)CH₂OH | H | CH₃ | H | H | - | 0 | CH |
| 1-75 | CH₂OH | H | CH₂CH₃ | CO₂CH₂OH | CH₃ | H | H | H | - | 0 | CH |
| 1-76 | CH₂OH | H | CH₂CH₃ | COCH₂CO₂H | CH₃ | H | H | H | - | 0 | CH |
| 1-77 | CH₂OH | H | CH₂CH₃ | COCH₂OCH₃ | CH₃ | H | H | H | - | 0 | CH |
| 1-78 | CH₂OH | H | CH₂CH₃ | CO₂CH₂OCH₃ | CH₃ | H | H | H | - | 0 | CH |
| 1-79 | CH₂OH | H | CH₂CH₃ | COCH₂CO₂CH₃ | CH₃ | H | H | H | - | 0 | CH |
| 1-80 | CH₂OH | H | OCH₃ | H | CH₃ | H | H | H | - | 0 | CH |
| 1-81 | CH₂OH | CH₃ | OCH₃ | H | CH₃ | H | H | H | - | 0 | CH |
| 1-82 | CH₂OH | Cl | OCH₃ | H | CH₃ | H | H | H | - | 0 | CH |
| 1-83 | CH₂OH | H | OCH₃ | H | CH₃ | H | H | H | - | 0 | N |
| 1-84 | CH₂OH | H | OCH₃ | H | H | CH₃ | H | H | - | 0 | CH |
| 1-85 | CH₂OH | H | OCH₃ | H | H | H | CH₃ | H | - | 0 | CH |
| 1-86 | CH₂OH | CH₃ | OCH₃ | H | H | H | CH₃ | H | - | 0 | CH |
| 1-87 | CH₂OH | Cl | OCH₃ | H | H | H | CH₃ | H | - | 0 | CH |
| 1-88 | CH₂OH | H | OCH₃ | H | H | H | CH₃ | H | - | 0 | N |
| 1-89 | CH₂OH | H | OCH₃ | H | H | H | H | CH₃ | - | 0 | CH |
| 1-90 | CH₂OH | H | OCH₃ | C(O)CH₃ | CH₃ | H | H | H | - | 0 | CH |
| 1-91 | CH₂OH | H | OCH₃ | CO₂CH₂CH₃ | CH₃ | H | H | H | - | 0 | CH |
| 1-92 | CH₂OH | H | OCH₃ | COCH₂OH | CH₃ | H | H | H | - | 0 | CH |
| 1-93 | CH₂OH | H | OCH₃ | C(O)CH₂OH | H | H | OCH₃ | H | - | 0 | CH |
| 1-94 | CH₂OH | H | OCH₃ | CO₂CH₂OH | CH₃ | H | H | H | - | 0 | CH |
| 1-95 | CH₂OH | H | OCH₃ | COCH₂CO₂H | CH₃ | H | H | H | - | 0 | CH |
| 1-96 | CH₂OH | H | OCH₃ | COCH₂OCH₃ | CH₃ | H | H | H | - | 0 | CH |
| 1-97 | CH₂OH | H | OCH₃ | CO₂CH₂OCH₃ | CH₃ | H | H | H | - | 0 | CH |
| 1-98 | CH₂OH | H | OCH₃ | COCH₂CO₂CH₃ | CH₃ | H | H | H | - | 0 | CH |
| 1-99 | CH₂OH | H | OCH₂CH₃ | H | CH₃ | H | H | H | - | 0 | CH |
| 1-100 | CH₂OH | H | CH₂CH₂OH | H | CH₃ | H | H | H | - | 0 | CH |
| 1-101 | CH₂OH | H | OCH₂CH₂OH | H | CH₃ | H | H | H | - | 0 | CH |
| 1-102 | CH₂OH | H | SCH₃ | H | CH₃ | H | H | H | - | 0 | CH |
| 1-103 | CH₂OH | H | OCF₃ | H | CH₃ | H | H | H | - | 0 | CH |
| 1-104 | CH₂CH₂OH | H | OCH₃ | H | CH₃ | H | H | H | - | 0 | CH |
| 1-105 | CH₂OC(O)CH₃ | H | CH₂CH₃ | H | CH₃ | H | H | H | - | 0 | CH |
| 1-106 | CH₂OC(O)CH₂OH | H | OCH₃ | H | CH₃ | H | H | H | - | 0 | CH |
| 1-107 | NHCH₂C(O)NH₂ | H | CH₂CH₃ | H | CH₃ | H | H | H | - | 0 | CH |
| 1-108 | H | H | OCH₃ | H | CH₃ | H | H | H | - | 0 | N |
| 1-109 | H | H | OCH₃ | H | CH₃ | H | H | H | - | 0 | N |
| 1-110 | CH₂OH | H | CH₂CH₃ | H | CH₃ | H | CH₃ | H | - | 0 | CH |
| 1-111 | CH₂OH | H | OCH₃ | H | CH₃ | H | CH₃ | H | - | 0 | CH |
| 1-112 | NH₂ | H | CH₂CH₃ | H | CH₃ | H | CH₃ | H | - | 0 | CH |
| 1-113 | H | Cl | OCH₃ | H | CH₃ | H | H | H | - | 0 | CH |
| 1-114 | CH₃ | Cl | OCH₃ | H | CH₃ | H | H | H | - | 0 | CH |
| 1-115 | CH₂OH | Cl | OCH₃ | H | CH₃ | H | H | H | - | 0 | CH |
| 1-116 | CH₂OH | Cl | OCH₂CH₃ | H | CH₃ | H | H | H | - | 0 | CH |
| 1-117 | CH₂OH | F | OCH₂CH₃ | H | CH₃ | H | H | H | - | 0 | CH |
| 1-118 | CH₂OH | F | CH₂CH₃ | H | CH₃ | H | H | H | - | 0 | CH |
| 1-119 | CH₂OH | F | CH₂CH₃ | H | CH₃ | H | CH₃ | H | - | 0 | CH |
| 1-120 | H | H | OCH₃ | H | H | H | CH₃ | H | - | 0 | CH |
| 1-121 | H | H | OCH₃ | H | H | CH₃ | H | H | - | 0 | CH |
| 1-122 | H | H | OCH₃ | H | CH₃ | H | H | H | - | 0 | CH |
| 1-123 | CH₂OH | H | CH₂CH₂OH | H | CH₃ | H | H | H | - | 0 | CH |
| 1-124 | H | H | Piperidine-OH | H | CH₃ | H | H | H | - | 0 | CH |
| 1-125 | CH₂OH | Cl | OCH₃ | H | H | CH₃ | H | H | - | 0 | CH |
| 1-126 | CH₂OH | Cl | OCH₃ | H | H | H | CH₃ | H | - | 0 | CH |
| 1-127 | CH₂OH | Cl | OCH₃ | H | CH₃ | H | H | H | - | 0 | CH |
| 1-128 | CH₂OH | H | -OCF₃ | H | CH₃ | H | H | H | - | 0 | CH |
| 1-129 | CH₂OH | H | CH₂OCH₃ | H | CH₃ | H | H | H | - | 0 | CH |
| 1-130 | CH₂OH | H | O-Cyclopropyl | H | CH₃ | H | H | H | - | 0 | CH |
| 1-131 | CH₂OH | H | C(O)CH₃ | H | CH₃ | H | H | H | - | 0 | CH |
| 1-132 | CH₂OH | H | Cyclopropyl | H | CH₃ | H | H | H | - | 0 | CH |
| 1-133 | CH₂OH | -F | O-Cyclopropyl | H | CH₃ | H | H | H | - | 0 | CH |
| 1-134 | NH₂ | H | OCH₃ | H | H | H | CH₃ | H | - | 0 | CH |
| 1-135 | CH₂OH | H | SCH₃ | H | CH₃ | H | H | H | - | 0 | CH |
| 1-136 | CH₂CH₂OH | H | CH₂CH₃ | H | CH₃ | H | H | H | - | 0 | CH |
| 1-137 | CH₂OH | H | CH₂CH₂CH₂OH | H | CH₃ | H | H | H | - | 0 | CH |
| 1-138 | CH₃ | CH₃ | OCH₃ | H | CH₃ | H | H | H | F | I | CH |
| 1-139 | CH₃ | CH₃ | CH₂CH₂OH | H | CH₃ | H | H | H | - | 0 | CH |
| 1-140 | CH₂CH₂OH | H | OCH₃ | H | CH₃ | H | H | H | - | 0 | CH |
| 1-141 | C=N-OH | H | OCH₃ | H | CH₃ | H | H | H | - | 0 | CH |
| 1-142 | CH(CH₃)OH | H | OCH₃ | H | CH₃ | H | H | H | - | 0 | CH |
| 1-143 | CH₂OH | F | OCH₂ CH₂CH₃ | H | CH₃ | H | H | H | - | 0 | CH |
| 1-144 | CH₂OH | F | OCH(CH₃)CH₃ | H | CH₃ | H | H | H | - | 0 | CH |
| 1-145 | CH₂OH | H | OCH₂CH₃ | H | CH₃ | H | H | H | - | 0 | CH |
| 1-146 | CH₃ | CH₃ | OCH₃ | H | CH₃ | H | H | H | - | 0 | CH |
| 1-147 | CH₂OH | H | OCH₃ | H | CH₃ | H | H | H | F | 1 | CH |
| 1-148 | CH₂OH | H | F | H | CH₃ | H | H | H | - | 0 | CH |
| 1-149 | CH₂OH | H | CH₂OH | H | CH₃ | H | H | H | - | 0 | CH |
| 1-150 | CH₂OH | H | OCH(CH₃)CH₃ | H | CH₃ | H | H | H | - | 0 | CH |
| 1-151 | CH₂OH | H | OCH₃ | H | CH₃ | H | H | H | F | I | CH |
| 1-152 | CH₂OH | OCH₃ | OCH₃ | H | CH₃ | H | H | H | - | 0 | CH |
| 1-153 | CH₂OH | F | OCH₃ | H | CH₃ | H | H | H | - | 0 | CH |
| 1-154 | CH₂O(O)CH₂OH | H | OCH(CH₃)CH₃ | H | CH₃ | H | H | H | - | 0 | CH |
| 1-155 | CH₂OH | F | Cyclopropyl | H | CH₃ | H | H | H | - | 0 | CH |
| 1-156 | CH₂OH | CH₃ | CH₂CH₃ | H | CH₃ | H | H | H | - | 0 | CH |
| 1-157 | CH₂OH | Cl | OCH₃ | H | CH₃ | H | CH₃ | H | - | 0 | CH |
| 1-158 | CH₂OH | F | OCH₃ | H | CH₃ | H | CH₃ | H | - | 0 | CH |
| 1-159 | CH₂OC(O)CH₂OH | F | OCH₃ | H | CH₃ | H | CH₃ | H | - | 0 | CH |
| 1-160 | CH₂OH | H | OCH₂CH₃ | H | CH₃ | H | H | H | - | 0 | CH |
| 1-161 | CH₂OH | CH₃ | OCH₃ | H | CH₃ | H | H | H | - | 0 | CH |
| 1-162 | CH₂OH | Cl | O-Cyclopropyl | H | CH₃ | H | H | H | - | 0 | CH |
| 1-163 | CH₂OH | CH₃ | O-Cyclopropyl | H | CH₃ | H | H | H | - | 0 | CH |
| 1-164 | CH₂OH | F | CH₃ | H | CH₃ | H | H | H | - | 0 | CH |
| 1-165 | CH₂OH | H | CH₃ | H | CH₃ | H | H | H | - | 0 | CH |
| 1-166 | CH₂OC(O)CH₂OH | H | OCH₂CH₃ | H | CH₃ | H | H | H | - | 0 | CH |
| 1-167 | CH₂OC(O)CH₂OH | H | OCH₃ | H | CH₃ | H | H | H | F | 1 | CH |
| 1-168 | CH₂OH | CH=CH₂ | OCH₃ | H | CH₃ | H | H | H | - | 0 | CH |

Among the example compounds listed above, compounds also represented by the general formula (II) are 1-1 to -3, -5 to -8, -15 to -26, -33 to -57, -59 to -62, -69, - 72, -75 to -83, -90 to -92, -94 to -109, -113 to -118, -122 to -124, -127 to -133, -135 to -156, and -160 to -168.

In the above Table 1, Example Compound Nos. 1-2, -5, -9, -10, -23, -27, -46, -50 to -54, -59, -63, -64, -70, -73, -74, -80, -93, -103, -105 to -119, -122 to -124, -127 to -133, and -135 to -168 are preferred, and Example Compound Nos. 1-5, -10, -23, - 80, -115, -117, -118, -130, -136, -144, -151, -153, -160, -161, and -166 are most preferred.

Compounds listed in the following Table 2 can be mentioned as specific examples of the compound represented by the general formula (III) of the present invention, but the present invention is not limited to these compounds.

**[Table 2]**

| | | | | | | | | |
|---|---|---|---|---|---|---|---|---|
| 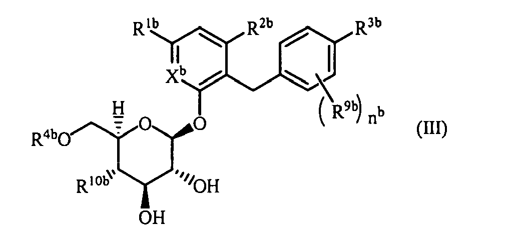 | | | | | | | | |

| Compd. | R^{1b} | R^{2b} | R^{3b} | R^{4b} | R^{9b} | n^{b} | R^{10b} | X^{b} |
|---|---|---|---|---|---|---|---|---|
| 2-1 | CH₂OH | H | CH₂CH₃ | H | - | 0 | H | CH |
| 2-2 | CH₂OH | H | CH₂CH₃ | H | - | 0 | CH₃ | CH |
| 2-3 | CH₂OH | H | CH₂CH₃ | H | - | 0 | OCH₃ | CH |
| 2-4 | CH₂OH | H | CH₂CH₃ | CH₃ | - | 0 | OH | CH |
| 2-5 | CH₂OH | H | CH₂CH₃ | COCH₂OH | - | 0 | H | CH |
| 2-6 | CH₂OH | H | CH₂CH₃ | COCH₂OH | - | 0 | OCH₃ | CH |
| 2-7 | CH₂OH | H | CH₂CH₃ | COCH₂OH | - | 0 | OCH₃ | N |
| 2-8 | CH₂OH | H | OCH₃ | COCH₂OH | - | 0 | OCH₃ | CH |
| 2-9 | NH₂ | H | CH₂CH₃ | H | - | 0 | H | CH |
| 2-10 | NH₂ | H | CH₂CH₃ | H | - | 0 | CH₃ | CH |
| 2-11 | NH₂ | H | CH₂CH₃ | H | - | 0 | OCH₃ | CH |
| 2-12 | NH₂ | H | CH₂CH₃ | CH₃ | - | 0 | OH | CH |
| 2-13 | NH₂ | H | CH₂CH₃ | COCH₂OH | - | 0 | H | CH |
| 2-14 | NH₂ | H | CH₂CH₃ | COCH₂OH | - | 0 | OCH₃ | CH |
| 2-15 | NH₂ | H | CH₂CH₃ | COCH₂OH | - | 0 | OCH₃ | N |
| 2-16 | NH₂ | H | OCH₃ | COCH₂OH | - | 0 | OCH₃ | CH |
| 2-17 | H | H | OCH₃ | H | - | 0 | H | CH |
| 2-18 | NH₂ | H | OCH₃ | H | - | 0 | H | CH |
| 2-19 | CH₂OH | F | OCH₃ | H | - | 0 | H | CH |
| 2-20 | CH₂OH | Cl | OCH₃ | H | - | 0 | H | CH |

Among the above Table 2, Example Compound Nos. 2-1, -2, -4 to -6, -9, and -17 to -20 are preferred, and Example Compound No. 2-1, -6, and -17 to -20 are most preferred.

The compound represented by the general formula (I), (II), or (III) or a pharmacologically acceptable salt thereof of the present invention causes minimal adverse reactions and exhibits excellent human SGLT1 and/or SGLT2 inhibiting activity, is useful as a therapeutic or preventive agent of a disease (preferably, type 1 diabetes, type 2 diabetes, gestational diabetes, hyperglycemia due to other causes, impaired glucose tolerance [IGT], a diabetes-related disease [for example, obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout, preferably obesity] or a diabetic complication [for example, retinopathy, nephropathy, nervous disorder, cataract, foot gangrene, infections, or ketosis], most preferably type 1 diabetes, type 2 diabetes, hyperglycemia due to other causes, impaired glucose tolerance, or obesity), and is useful as a pharmaceutical composition for prophylactic or therapeutic treatment of a warm-blooded animal (for example, a human, an equine, a bovine, or a swine, preferably a human).

### Best Mode for Carrying Out the Invention

The compound represented by the general formula (I), (II), or (III) of the present invention can be produced as described below.

Solvents used in reactions in each step of Methods A to D are not particularly limited, so long as they do not inhibit the reaction and dissolve a starting material to some extent, and are selected from the following group of solvents:
aliphatic hydrocarbons such as hexane, heptane, ligroin, and petroleum ether; aromatic hydrocarbons such as benzene, toluene, and xylene; halogenated hydrocarbons such as chloroform, methylene chloride, 1,2-dichloroethane, and carbon tetrachloride; esters such as methyl acetate, ethyl acetate, propyl acetate, butyl acetate, and diethyl carbonate; ethers such as diethyl ether, diisopropyl ether, tetrahydrofuran, dioxane, dimethoxyethane, and diethylene glycol dimethyl ether; nitriles such as acetonitrile, propionitrile, butyronitrile, and isobutyronitrile; carboxylic acids such as acetic acid and propionic acid; alcohols such as methanol, ethanol, n-propanol, isopropanol, n-butanol, isobutanol, t-butanol, isoamyl alcohol, diethylene glycol, glycerine, octanol, cyclohexanol, and methyl cellosolve; amides such as formamide, dimethylformamide, dimethylacetamide, and hexamethyl phosphoric acid triamide; sulfoxides such as dimethyl sulfoxide; sulfones such as sulfolane; water; and mixtures thereof.

Examples of bases used in reactions in each step of Methods A to D include alkali metal carbonate salts such as lithium carbonate, sodium carbonate, and potassium carbonate; alkali metal bicarbonate salts such as lithium hydrogencarbonate, sodium hydrogencarbonate, and potassium hydrogencarbonate; alkali metal hydrides such as lithium hydride, sodium hydride, and potassium hydride; alkali metal hydroxides such as lithium hydroxide, sodium hydroxide, and potassium hydroxide; alkali metal alkoxides such as lithium methoxide, sodium methoxide, sodium ethoxide, and potassium t-butoxide; organic amines such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), and 1,8-diazabicyclo[5.4.0]-7-undecene (DBU).

Examples of acid catalysts used in reactions in each step of Methods A to D include Brønsted acids such as inorganic acids such as hydrochloric acid, hydrobromic acid, sulfuric acid, perchloric acid, and phosphoric acid or organic acids such as acetic acid, formic acid, oxalic acid, methanesulfonic acid, p-toluenesulfonic acid, camphorsulfonic acid, trifluoroacetic acid, and trifluoromethanesulfonic acid, Lewis acids such as zinc chloride, tin tetrachloride, boron trichloride, boron trifluoride, and boron tribromide, or acidic ion exchange resins.

When a compound used as a reactive substrate in reactions in each step of Methods A to D has a group that inhibits the target reaction, such as an amino group, a hydroxyl group, or a carboxyl group, a protective group may be introduced into the group, and the introduced protective group may be removed suitably, if necessary. Such protective groups are not particularly limited so long as they are commonly used protective groups, and can be, for example, protective groups described in Greene T.H. and Wuts P.G., Protective Groups in Organic Synthesis. Third Edition, 1999, John Wiley & Sons, Inc. and the like. These protective groups can be introduced or removed according to usual methods such as the methods described in the above-mentioned documents.

### [Method A]

The compound represented by the general formula (I) can be produced by Method A. Substituent R⁹ can be inserted into an aglycone moiety by methods known to those skilled in the art.

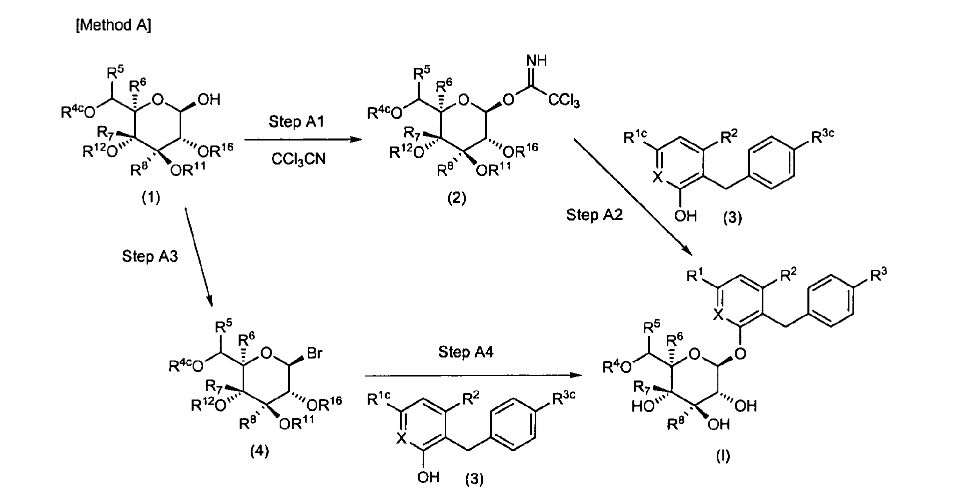

In the formulas, R¹, R², R³, R⁴, R⁵, R⁶, R⁷, R⁸, and X have the same meaning as defmed above, R^{1c}, R^{3c}, and R^{4c} represent the same groups as those in the definitions of groups of R^{1c}, R^{3c} and R^{4c} except that an amino group and/or a hydroxyl group included as a substituent in each group is an amino group and/or a hydroxyl group that may be protected, and R¹¹, R¹², and R¹⁶ are the same or different and each represent a hydrogen atom or a protective group of a hydroxyl group.

Step A1 is the step of producing compound (2), which is achieved by reacting compound (1) with trichloroacetonitrile in the presence of a base in an inert solvent.

The inert solvent used in the above-mentioned reaction is, for example, a halogenated hydrocarbon or an ether, preferably a halogenated hydrocarbon (most preferably methylene chloride).

The base used in the above-mentioned reaction is, for example, an organic amine, preferably 1,8-diazabicyclo[5.4.0]-7-undecene.

The reaction temperature varies depending on the starting compound, the base, the solvent, and the like, but is usually -20°C to reflux temperature (preferably 0°C to room temperature).

The reaction time varies depending on the starting compound, the base, the solvent, the reaction temperature, and the like, but is usually 15 min to 48 h (preferably 30 min to 5 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and then dried over anhydrous sodium sulfate or the like, and then the solvents are evaporated to obtain the target compound.

Step A2 is the step of producing compound (I), which is achieved by reacting compound (2) with compound (3) in the presence of a Lewis acid in an inert solvent and then removing protective groups of an amino group and/or a hydroxyl group in R^{1c}, R^{3c}, R^{4c}, R¹¹, R¹² and R¹⁶ as required.

The inert solvent used to react compound (2) with compound (3) is, for example, a halogenated hydrocarbon, an aromatic hydrocarbon, an ether, or a nitrile, preferably a halogenated hydrocarbon (most preferably methylene chloride).

The Lewis acid used in the above-mentioned reaction is, for example, a boron trifluoride-diethyl ether complex or trimethylsilyl trifluoromethanesulfonate, preferably, a boron trifluoride-diethyl ether complex.

The reaction temperature varies depending on the starting compound, the Lewis acid, the solvent, and the like, but is usually -30°C to reflux temperature (preferably 0°C to room temperature).

The reaction time varies depending on the starting compound, the Lewis acid, the solvent, the reaction temperature, and the like, but is usually 5 min to 24 h (preferably 10 min to 12 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and then dried over anhydrous sodium sulfate or the like, and then the solvents are evaporated to obtain the target compound. The resulting compound can be isolated or purified by a usual method, for example, silica gel column chromatography, if necessary.

Removal of a protective group from an amino group and/or a hydroxyl group varies depending on the type of protective group, but, as described above, can be done by methods generally known in synthetic organic chemistry techniques, for example, usual methods such as the methods described in Greene T.H. and Wuts P.G., Protective Groups in Organic Synthesis. Third Edition, 1999, John Wiley & Sons, Inc. and the like.

Step A3 is the step of producing compound (4), which is achieved by reacting compound (1) with hydrogen bromide-acetic acid in an inert solvent.

The inert solvent used in the above-mentioned reaction is, for example, a halogenated hydrocarbon, preferably methylene chloride.

The reaction temperature varies depending on the starting compound and the solvent, but is usually 0°C to reflux temperature (preferably room temperature).

The reaction time varies depending on the starting compound, the solvent, the reaction temperature, and the like, but is usually 5 to 50 h (preferably 15 to 35 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step A4 is the step of producing compound (I), which is achieved by reacting compound (4) with compound (3) in the presence of silver carbonate in an inert solvent and then removing protective groups of an amino group and/or a hydroxyl group in R^{1c}, R^{3c}, R^{4c}, R¹¹, R¹², and R¹⁶ as required.

The inert solvent used in the above-mentioned reaction is, for example, a halogenated hydrocarbon, an aromatic hydrocarbon, an ether, or a nitrile, preferably a halogenated hydrocarbon (most preferably methylene chloride).

The reaction temperature varies depending on the starting compound, the solvent, and the like, but is usually 0°C to reflux temperature (preferably room temperature).

The reaction time varies depending on the starting compound, the solvent, the reaction temperature, and the like, but is usually 5 to 150 h (preferably 10 to 50 h).

Removal of a protective group from an amino group and/or a hydroxyl group varies depending on the type of protective group, but, as described above, can be done by methods generally known in synthetic organic chemistry techniques, for example, usual methods such as the methods described in Greene T.H. and Wuts P.G., Protective Groups in Organic Synthesis. Third Edition, 1999, John Wiley & Sons, Inc. and the like.

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound. The resulting compound can be isolated or purified by a usual method, for example, silica gel column chromatography, if necessary.

For example, compounds (1) and (3), starting compounds of Method A, can be produced by the following Methods B and C, respectively.

### [Method B]

Compound (1), a starting compound of Method A, can be produced according to Method B. 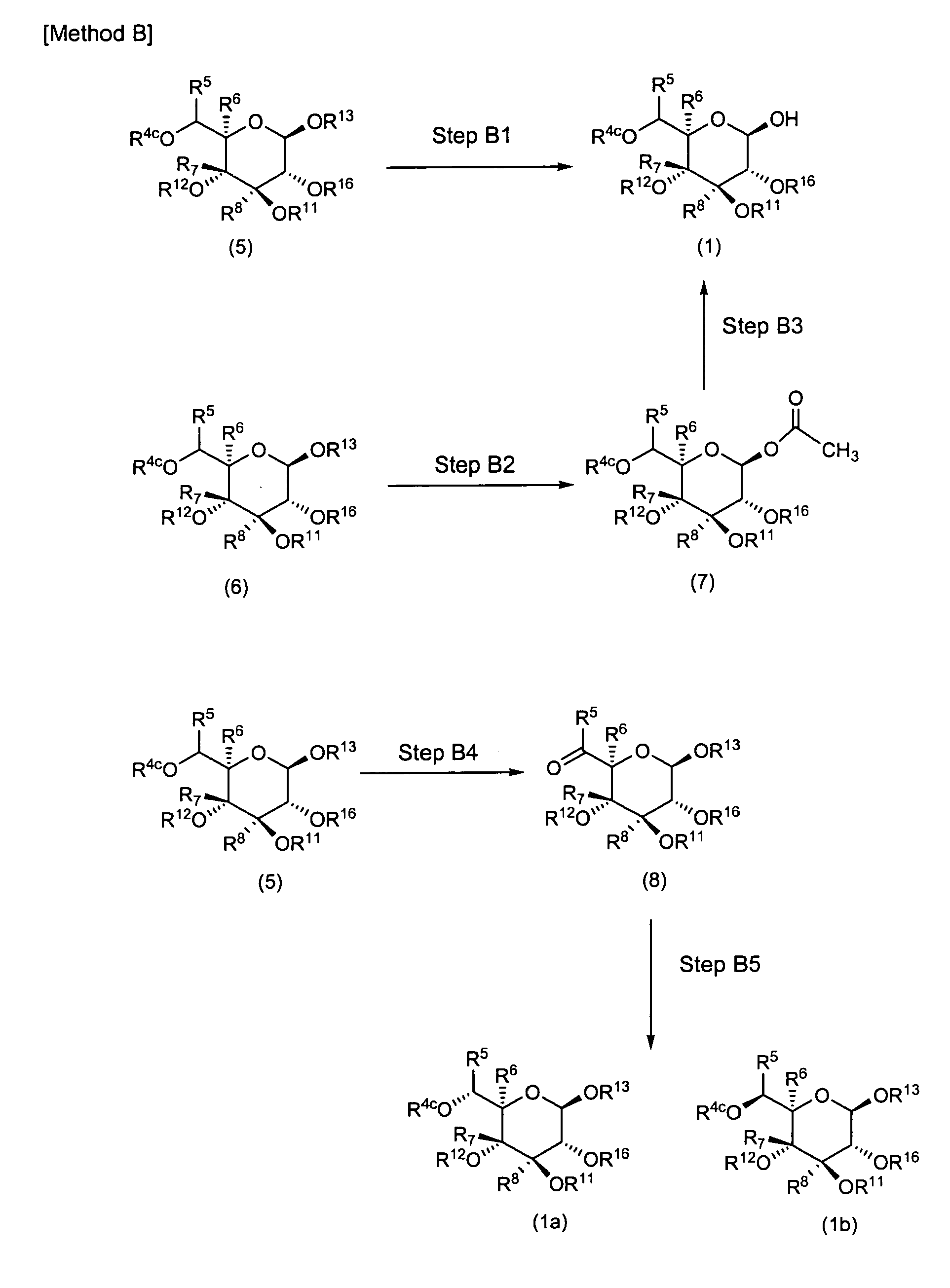 In the formulas, R^{4c}, R⁵, R⁶, R⁷, R⁸, R¹¹, R¹², and R¹⁶ have the same meaning as defined above, and R¹³ represents a protective group of a hydroxyl group.

Step B1 is the step of producing compound (1), which is achieved by removing the protective group of a hydroxyl group R¹³.

Removal of a protective group from a hydroxyl group varies depending on the type of protective group, but, as described above, can be done by methods generally known in synthetic organic chemistry techniques, for example, usual methods such as the methods described in Greene T.H. and Wuts P.G., Protective Groups in Organic Synthesis. Third Edition, 1999, John Wiley & Sons, Inc. and the like.

For example, when the protective group of a hydroxyl group is a benzoyl group, hydrazine acetate is allowed to act in an inert solvent.

The inert solvent used in the above-mentioned reaction is, for example, an amide, most preferably dimethylformamide.

The reaction temperature varies depending on the starting compound, the solvent, and the like, but is usually 0 to 50°C (preferably room temperature).

The reaction time varies depending on the starting compound, the solvent, the reaction temperature, and the like, but is usually 30 min to 35 h (preferably 1 to 24 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound. The resulting compound can be isolated or purified by a usual method, for example, silica gel column chromatography, if necessary.

Step B2 is the step of producing compound (7), which is achieved by acetylating the R¹³ group of compound (6) in the presence of an acid catalyst in an inert solvent.

The inert solvent used in the above-mentioned reaction is, for example, a carboxylic acid, preferably acetic acid.

The acid catalyst used in the above-mentioned reaction is preferably an inorganic acid (most preferably sulfuric acid).

The reaction temperature varies depending on the starting compound, the acid catalyst, the solvent, and the like, but is usually 0 to 50°C (preferably room temperature).

The reaction time varies depending on the starting compound, the acid catalyst, the solvent, the reaction temperature, and the like, but is usually 3 to 48 h (preferably 6 to 24 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step B3 is the step of producing compound (1), which is achieved by allowing hydrazine acetate to act on compound (7) in an inert solvent. This step is performed in the same manner as in the method for removing a protective group when R¹³ of compound (1) is a benzoyl group in Step B1.

Step B4 is the step of producing compound (8), which is achieved by reacting compound (5) with an oxidizing agent in an inert solvent.

The inert solvent used in the above-mentioned reaction is, for example, a halogenated hydrocarbon, preferably methylene chloride.

The oxidizing agent used in the above-mentioned reaction is, for example, dimethyl sulfoxide, chromic acid, or Dess-Martin reagent, preferably dimethyl sulfoxide.

The reaction temperature varies depending on the starting compound, the oxidizing agent, the solvent, and the like, but is usually -100 to 0°C (preferably -70 to 0°C).

The reaction time varies depending on the starting compound, the oxidizing agent, the solvent, the reaction temperature, and the like, but is usually 15 min to 10 h (preferably 1 to 5 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound. The resulting compound can be isolated or purified by a usual method, for example, silica gel column chromatography, if necessary.

Step B5 is the step of producing compounds (1a) and (1b), which is achieved by reacting compound (VIII) with a reducing agent in an inert solvent.

The inert solvent used in the above-mentioned reaction is, for example, an ether, an alcohol, or a mixed solvent of these solvents, preferably a mixed solvent of an ether and an alcohol (most preferably a mixed solvent of tetrahydrofuran and ethanol).

The reducing agent used in the above-mentioned reaction is, for example, an alkali metal borohydride such as sodium borohydride or lithium borohydride, an aluminium hydride compound such as lithium aluminium hydride or lithium hydride triethoxide aluminium, a hydride reagent such as sodium hydrogen tellurate, preferably an alkali metal borohydride (most preferably sodium borohydride).

The reaction temperature varies depending on the starting compound, the solvent, the reducing agent, and the like, but is usually -50 to 50°C (preferably -20°C to room temperature).

The reaction time varies depending on the starting compound, the solvent, the reducing agent, the reaction temperature, and the like, but is usually 15 min to 20 h (preferably 30 min to 5 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

### [Method C]

Compound (3), a starting compound of Method A, can be produced according to Method C. 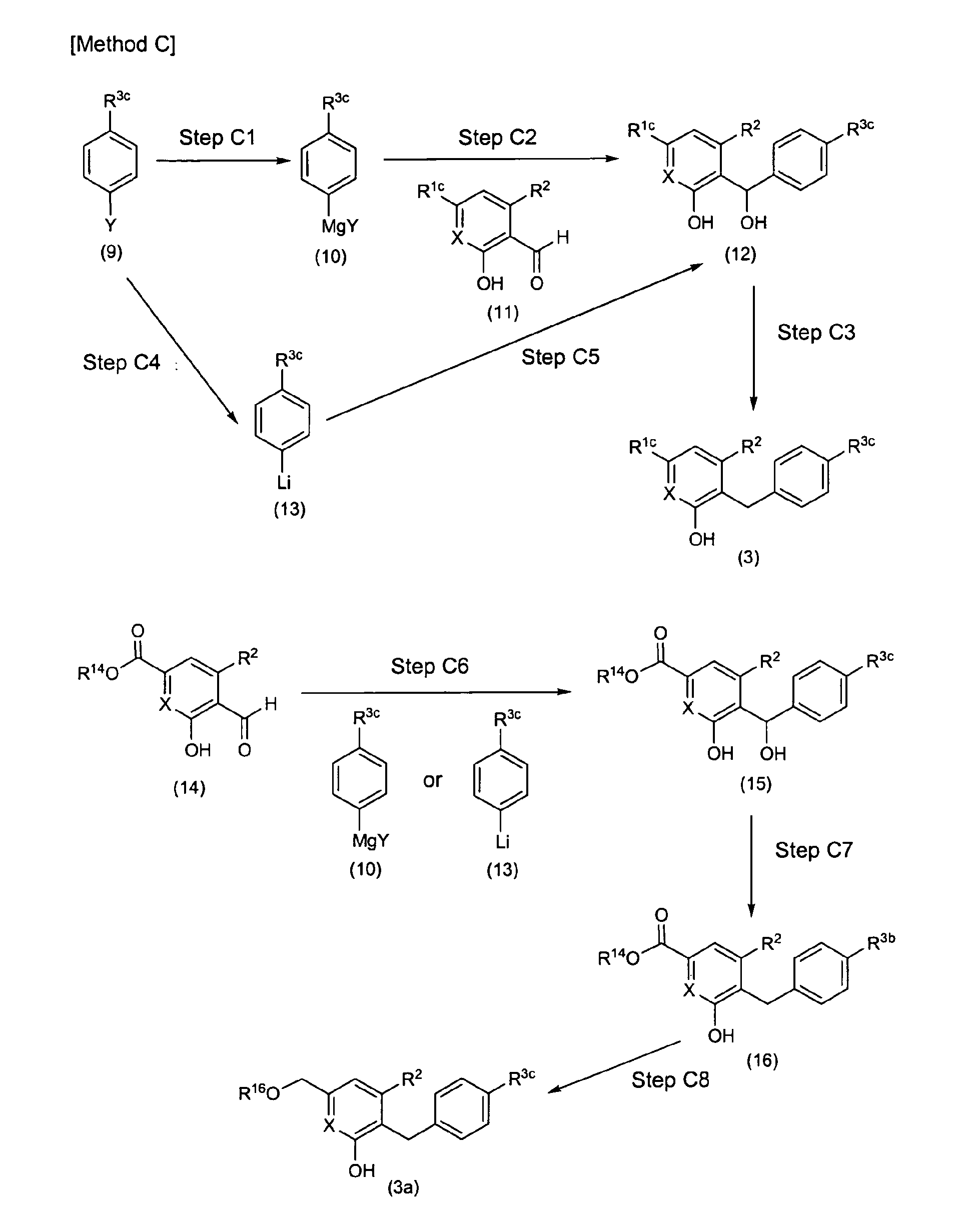 In the formulas, R^{1c}, R², R^{3c}, R¹⁶, and X have the same meaning as defined above, R¹⁴ represents a C₁-C₆ alkyl group, and Y represents a halogen atom.

Step C1 is the step of producing compound (10), which is achieved by reacting metal magnesium with compound (9) in the presence of an activator (preferably a catalytic amount of iodine) in an inert solvent to prepare Grignard reagent.

The inert solvent used in the above-mentioned reaction is, for example, an ether, preferably tetrahydrofuran.

The reaction temperature varies depending on the starting compounds, the activator, the solvent, and the like, but is usually 0°C to reflux temperature (preferably room temperature to reflux temperature).

The reaction time varies depending on the starting compound, the activator, the solvent, the reaction temperature, and the like but is usually 15 min to 10 h (preferably 30 min to 5 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step C2 is the step of producing compound (12), which is achieved by condensing compound (10) with compound (11) in an inert solvent.

The inert solvent used in the above-mentioned reaction is, for example, an ether, preferably tetrahydrofuran.

The reaction temperature varies depending on the starting compound, the solvent, and the like, but is usually -100 to 20°C(preferably -70 to 0°C).

The reaction time varies depending on the starting compound, the solvent, the reaction temperature, and the like, but is usually 5 min to 12 h (preferably 10 min to 6 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step C3 is the step of producing compound (3), which is achieved by reducing compound (12) in an inert solvent (preferably by catalytic reduction in the presence of an acid catalyst under a hydrogen atmosphere at room temperature).

The inert solvent used in the above-mentioned reaction is, for example, an alcohol or an ether, preferably an alcohol (most preferably methanol).

The acid catalyst used in removal by catalytic reduction is, for example, an inorganic acid, preferably hydrochloric acid.

Catalysts used in removal by catalytic reduction are not particularly limited so long as they are usually used for a catalytic reduction reaction. Examples thereof include palladium on carbon, palladium hydroxide on carbon, Raney nickel, platinum oxide, platinum black, rhodium-aluminium oxide, triphenylphosphine-rhodium chloride, and palladium-barium sulfate, and palladium on carbon is preferred.

The reaction temperature varies depending on the starting compound, the catalyst, the solvent, and the like, but is usually 0 to 50°C (preferably room temperature).

The reaction time varies depending on the starting compound, catalyst, the solvent, the reaction temperature, and the like, but is usually 5 min to 24 h (preferably 15 min to 12 h).

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step C4 is the step of producing compound (13), which is achieved by reacting compound (9) with an alkyl lithium compound in an inert solvent.

The inert solvent used in the above-mentioned reaction is, for example, an ether or an aliphatic hydrocarbon, preferably an ether (most preferably tetrahydrofuran).

The alkyl lithium compound used in the above-mentioned reaction is, for example, methyl lithium, n-butyllithium, or t-butyllithium, preferably t-butyllithium.

The reaction temperature varies depending on the starting compound, the alkyl lithium compound, the solvent, and the like, but is usually -120 to 20°C (preferably -90 to 0°C).

The reaction time varies depending on the starting compound, the alkyl lithium compound, the solvent, the reaction temperature, and the like, but is usually 5 min to 12 h (preferably 10 min to 6 h).

Step C5 is the step of producing compound (12), which is achieved by condensing compound (13) with compound (11) in an inert solvent in the same manner as in the above Step C2.

Step C6 is the step of producing compound (15), which is achieved by condensing compound (14) with compound (10) or compound (13) in an inert solvent in the same manner as in the above Step C2.

Step C7 is the step of producing compound (16), which is achieved by reducing compound (15) in an inert solvent (preferably by catalytic reduction in the presence of an acid catalyst under a hydrogen atmosphere at room temperature) in the same manner as in the above Step C3.

Step C8 is the step of producing compound (3a), in which R^{1c} in compound (3) is a -OR¹⁶ group, in an inert solvent, and is achieved by converting a -CO₂R¹⁴ group (C₁-C₆ alkoxycarbonyl group) in compound (16) to a hydroxymethyl group and then introducing a protective group into the hydroxyl group as required.

The inert solvent used in the above-mentioned reduction reaction is, for example, an ether, preferably tetrahydrofuran.

The reducing agent used in the above-mentioned reduction reaction is, for example, an alkali metal borohydride such as sodium borohydride or lithium borohydride, an aluminium hydride compound such as lithium aluminium hydride or lithium triethoxide aluminium hydride, or a hydride reagent such as sodium hydrogen tellurate, preferably an aluminium hydride compound (most preferably lithium aluminium hydride).

The reaction temperature in the above-mentioned reduction reaction varies depending on the starting compound, the reducing agent, the solvent, and the like, but is usually 0 to 50°C (preferably room temperature).

The reaction time in the above-mentioned reduction reaction varies depending on the starting compound, the reducing agent, the solvent, the reaction temperature, and the like, but is usually 10 min to 10 h (preferably 20 min to 5 h).

Introduction of a protective group into a hydroxyl group varies depending on the type of protective group, but, as described above, can be done by a method generally known in synthetic organic chemistry techniques, for example, usual methods such as the methods described in Greene T.H. and Wuts P.G., Protective Groups in Organic Synthesis. Third Edition, 1999, John Wiley & Sons, Inc. and the like.

### [Method D]

Compound (3b), in which R^{1c} in compound (3) is an amino group, and compound (3c), in which it is a NHR¹⁵ group (R¹⁵ is an alkyl group or an amino C₂-C₇ acyl group) can be produced by Method D. 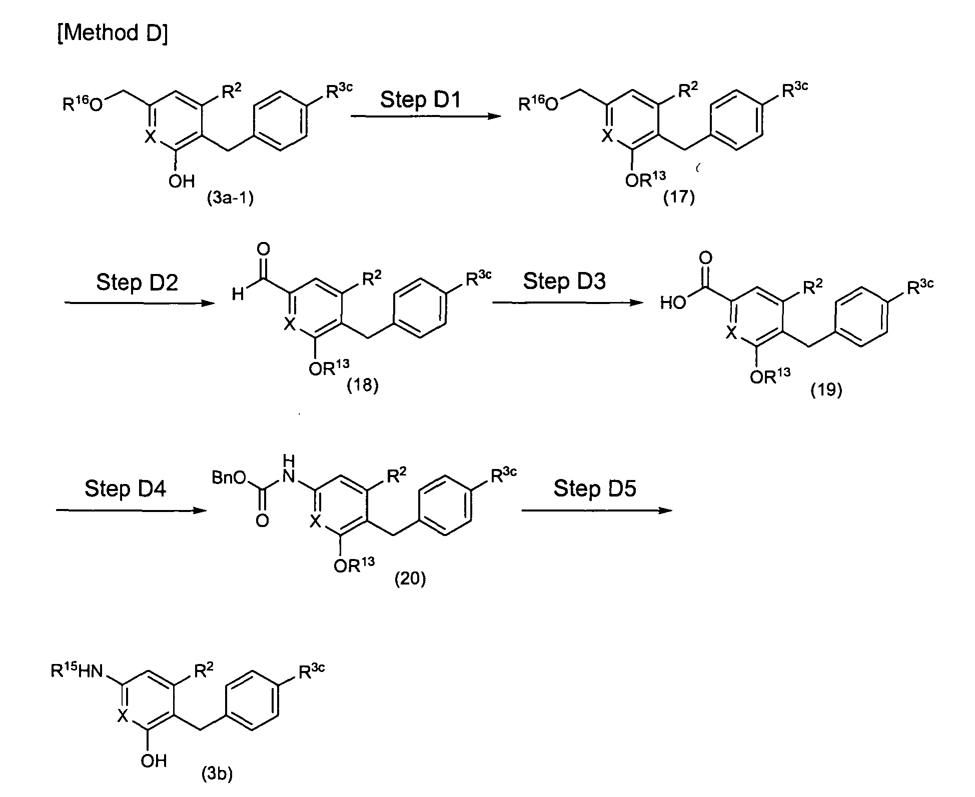 In the formulas, R², R^{3c}, R¹³, R¹⁴, R¹⁶, and X have the same meaning as defined above, R¹⁵ represents a hydrogen atom or a protective group of an amino group, and Bn represents a benzyl group.

Step D1 is the step of producing compound (17), which is achieved by protecting a hydroxyl group of compound (3a-1).

Introduction of a protective group into a hydroxyl group varies depending on the type of protective group, but, as described above, can be done by a method generally known in synthetic organic chemistry techniques, for example, usual methods such as the methods described in Greene T.H. and Wuts P.G., Protective Groups in Organic Synthesis. Third Edition, 1999, John Wiley & Sons, Inc. and the like.

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound. The resulting compound can be isolated or purified by a usual method, for example, silica gel column chromatography, if necessary.

Step D2 is the step of producing compound (18), which is achieved by removing the protective group from the hydroxyl group of the R¹⁶ group in compound (17) as desired and then reacting compound (17) with an oxidizing agent in an inert solvent.

Removal of a protective group from a hydroxyl group varies depending on the type of protective group, but, as described above, can be done by a method generally known in synthetic organic chemistry techniques, for example, usual methods such as the methods described in Greene T.H. and Wuts P.G., Protective Groups in Organic Synthesis. Third Edition, 1999, John Wiley & Sons, Inc. and the like.

The inert solvent used in the reaction of compound (17) with an oxidizing agent is, for example, a halogenated hydrocarbon, preferably methylene chloride.

The oxidizing agent reacted with compound (17) is, for example, potassium permanganate or a manganese oxide such as manganese dioxide, preferably manganese dioxide.

The reaction temperature varies depending on the starting compound, the oxidizing agent, the solvent, and the like, but is usually 0°C to reflux temperature (preferably room temperature).

The reaction time varies depending on the starting compound, the oxidizing agent, the solvent, the reaction temperature, and the like, but is usually 30 min to 48 h (preferably 1 to 10 h).

For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step D3 is the step of producing compound (19), which is achieved by reacting compound (18) with an oxidizing agent in an inert solvent.

The inert solvent used in the above-mentioned reaction is, for example, an ether, an alcohol, water, or a mixed solvent of these solvents, preferably a mixed solvent of an ether, an alcohol, and water (most preferably a mixed solvent of tetrahydrofuran, t-butanol, and water).

The oxidizing agent used in the above-mentioned reaction is, for example, a chlorite compound such as potassium chlorite or sodium chlorite, preferably sodium chlorite.

The reaction temperature varies depending on the starting compound, the oxidizing agent, the solvent, and the like, but is usually 0 to 50°C (preferably room temperature).

The reaction time varies depending on the starting compound, the oxidizing agent, the solvent, the reaction temperature, and the like, but is usually 5 min to 12 h (preferably 15 min to 6 h). For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step D4 is the step of producing compound (20), which is achieved by reacting compound (19) with an azide-inducing reagent in an inert solvent in the presence of a base, and then reacting with a benzyl alcohol.

The inert solvent used in the above-mentioned reaction is, for example, an ether or an aromatic hydrocarbon, preferably an ether (most preferably dioxane).

The base used in the above-mentioned reaction is, for example, an organic amine, preferably triethylamine.

The azide-inducing reagent used in the above-mentioned reaction is, for example, a diaryl phosphate azide derivative such as diphenylphosphate azide; a trialkylsilyl azide such as trimethylsilyl azide or triethyl silyl azide or an alkali metal azide salt such as sodium azide or potassium azide, preferably a diaryl phosphate azide derivative (diphenylphosphate azide).

The reaction temperature varies depending on the starting compound, the base, the azide-inducing reagent, the solvent, and the like, but is usually -10 to 150°C (preferably 50 to 100°C).

The reaction time varies depending on the starting compound, the base, the azide-inducing reagent, the solvent, the reaction temperature, and the like, but is usually 30 min to 15 h (preferably 1 to 10 h). For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound.

Step D5 is the step of producing compound (3b), which is achieved by reducing compound (20) (preferably by catalytic reduction under a hydrogen atmosphere at room temperature) to remove the protective group from the hydroxyl group and then protecting the amino group as required.

The inert solvent used in the catalytic reduction reaction is, for example, an alcohol, an ether, or a mixed solvent of these solvents, preferably a mixed solvent of an alcohol and an ether (most preferably a mixed solvent of methanol and tetrahydrofuran).

Catalysts used in the removal by catalytic reduction are not particularly limited so long as they are usually used for a catalytic reduction reaction. Examples thereof include palladium on carbon, palladium hydroxide on carbon, Raney nickel, platinum oxide, platinum black, rhodium-aluminium oxide, triphenylphosphine-rhodium chloride, palladium-barium sulfate, preferably palladium on carbon.

The reaction temperature varies depending on the starting compound, the catalyst, the solvent, and the like, but is usually 0 to 50°C (preferably room temperature).

The reaction time varies depending on the starting compound, the catalyst, the solvent, the reaction temperature, and the like, but is usually 5 min to 24 h (preferably 15 min to 12 h).

Removal of a protective group from a hydroxyl group and introduction of a protective group into an amino group vary depending on the type of protective group, but, as described above, can be done by a method generally known in the synthetic organic chemistry techniques, for example, usual methods such as the methods described in Greene T.H. and Wuts P.G., Protective Groups in Organic Synthesis. Third Edition, 1999, John Wiley & Sons, Inc. and the like.

After completion of the reaction, the target compound of the reaction is collected from the reaction mixture according to a usual method. For example, the reaction mixture is suitably neutralized, or insoluble matters are removed therefrom by filtration if they exist, then organic solvents that are not miscible with each other, such as water and ethyl acetate, are added to separate an organic layer containing the target compound, the organic layer is washed with water or the like and dried over anhydrous sodium sulfate or the like, and then the solvents are evaporated to obtain the target compound. The resulting compound can be isolated or purified by a usual method, for example, silica gel column chromatography, if necessary.

The compound represented by the general formula (II) can be produced in the same manner as in the above-described Methods A to D.

The compound represented by the general formula (III) can be produced by using the following compounds (21), (22), and (23) instead of starting compounds (1), (5), and (6), respectively, in the above-described Methods A to D. 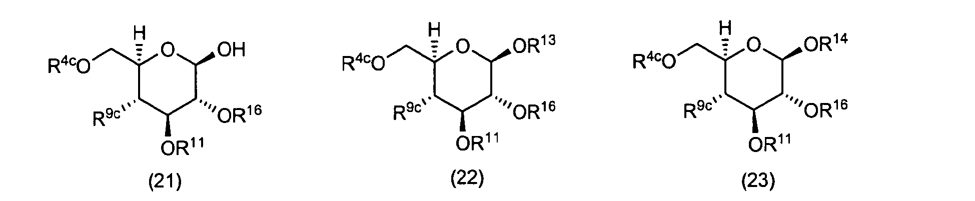 In the formulas, R^{4c}, R¹¹, R¹³, R¹⁴, and R¹⁶ have the same meaning as defined above, and R^{9c} has the same meaning as definition of the R^{10b} group except that the hydroxyl group is a hydroxyl group that may be protected.

The starting compounds (1), (3), (5), (6), (9), (11), (14), (21), (22), and (23) in the above-described Methods A to D can easily be produced from known compounds according to the examples described later or known methods (for example, the following list of documents).
Chem. Ber, 71, 1938, 1843-1849; Carbohydr. Res. 1995, 273, 249-254 Bull. Chem. Soc. Jpn., 1982, 55, 938-942; Bull. Chem. Soc. Jpn., 1976, 49, 788-790; Org. Lett., 2003, 5, 3419-3421; Org. Biomol. Chem, 2003, 1, 767-771; J. Chem. Soc., 1956, 2124-2126; WO02/064606; and Liebigs Ann. Chem. GE. 1992, 7, 747-758.

The compound represented by the general formula (I), (II), or (III) or a pharmacologically acceptable salt thereof of the present invention causes minimal adverse reactions, exhibits excellent human SGLT1 and/or SGLT2 inhibiting activity, is useful as a therapeutic or preventive drug for type 1 diabetes, type 2 diabetes, gestational diabetes, hyperglycemia due to other causes, impaired glucose tolerance (IGT), a diabetes-related disease (for example, obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout), or a diabetic complication (for example, retinopathy, nephropathy, nervous disorder, cataract, foot gangrene, infections, or ketosis), and can be administered to a warm-blooded animal (for example, a human, an equine, bovine, or a swine, preferably a human). For example, the compound can be administered orally by tablet, capsule, granule, powder, syrup, or the like or parenterally by injection, suppository, or the like.

These formulations can be produced by known methods using additives such as excipients (examples thereof include organic excipients such as sugar derivatives such as lactose, sucrose, glucose, mannitol, and sorbitol; starch derivatives such as corn starch, potato starch, α-starch, and dextrin; cellulose derivatives such as crystalline cellulose; gum arabic; dextran; pullulan; and inorganic excipients such as silicate derivatives such as light anhydrous silicic acids, synthetic silicic acid aluminium, silicic acid calcium, and magnesium aluminometasilicate; phosphates such as calcium hydrogen phosphate; carbonates such as calcium carbonate; and sulfates such as calcium sulfate), lubricants (examples thereof include stearic acid metal salts such as stearic acid, calcium stearate, and magnesium stearate; talc; colloidal silica; waxes such as veegum and spermaceti; fluoboric acid; adipic acid; sulfates such as sodium sulfate; glycol; fumaric acid; sodium benzoate; DL leucine; fatty acid sodium salts; lauryl sulfates such as lauryl sodium sulfate and lauryl magnesium sulfate; silicic acids such as anhydrous silicic acid and silicic acid hydrate; and the above-mentioned starch derivatives), binders (examples thereof include hydroxypropylcellulose, hydroxypropylmethylcellulose, polyvinylpyrrolidone, macrogol, and the same compounds as the above excipients), disintegrants (examples thereof include cellulose derivatives such as low-substituted hydroxypropyl cellulose, carboxymethylcellulose, carboxymethylcellulose calcium, and internally crosslinked carboxymethylcellulose sodium; chemically modified starches and celluloses such as carboxymethyl starch, carboxymethyl starch sodium, crosslinked polyvinylpyrrolidone), stabilizers (examples thereof include p-hydroxybenzoates such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid), flavoring agents (examples thereof include commonly used sweeteners, acidifiers, flavors, and so forth), and diluting agents.

Doses of the compound represented by the general formula (I), (II), or (III) or a pharmacologically acceptable salt thereof of the present invention vary depending on symptoms, age, and the like. The desirable dosages for adults are from 1 mg, as the minimum daily dosage, (preferably 10 mg) to 2000 mg, as the maximum daily dosage, (preferably 400 mg) in the case of oral administration and from 0.1 mg, as the minimum daily dosage, (preferably 1 mg) to 500 mg, as the maximum daily dosage, (preferably 300 mg) in the case of intravenous administration, which are administered as one dose or divided into several doses depending on symptoms.

### Examples

Hereafter, the present invention will be explained in more detail with reference to examples, test examples, and formulation examples. However, the scope of the present invention is not limited to these examples.

### (Example 1) 2-(4-Ethylbenzyl)-5-hydroxymethylphenyl 6-O-methyl-β-D-glucopyranoside (Example Compound No. 2-4)

### (1a) 6-O-Methyl-2,3,4-tri-O-benzoyl-D-glucopyranoside

6-O-Methyl-1,2,3,4-tetra-O-benzoyl-D-glucose (Chem. Ber, 71, 1938, 1843-1849) (13.6 g, 22.3 mmol) was dissolved in N,N-dimethylformamide (100 mL), followed by addition of hydrazine acetate (3.10 g, 33.5 mmol) at room temperature, and the mixture was stirred at room temperature for 6 h. The reaction mixture was poured into water (200 mL) and extracted 3 times with ethyl acetate (a total of 350 mL). The organic layer was washed with saturated brine (20 mL), dried over anhydrous sodium sulfate, and the solvent was evaporated under reduced pressure. The residue was purified by silica gel flash column chromatography (hexane:ethyl acetate, 3:1, v/v) to obtain the title compound (9.36 g, yield 83%) as a colorless oil. ¹H NMR (400MHz, CDCl₃): δ3.35 (3H, s), 3.62-3.53 (2H, m), 4.55-4.50 (1H, m), 5.29 (1H, dd, J=10.0 and 3.6 Hz), 5.52 (1H, t, J=10.0 Hz), 5.75 (1H, d, J=3.6 Hz), 6.23 (1H, t, J=10.0 Hz), 8.00-7.27 (15H, m)
MS (FAB) m/z:507 (M + H)⁺.

### (1b) 5-Acetoxymethyl-2-(4-ethylbenzyl)phenyl 6-O-methyl-2,3,4-tri-O-benzoyl-β-D-glucopyranoside

The compound synthesized in (1a) (200 mg, 0.39 mmol) was dissolved in methylene chloride (5 mL), followed by addition of trichloroacetonitrile (0.20 ml, 1.98 mmol) and 1,8-diazabicyclo[5.4.0]-7-undecene (6 µL, 0.04 mmol), and the mixture was stirred with ice cooling for 1 h. The reaction mixture was diluted with ethyl acetate (10 mL) and washed with saturated aqueous ammonium chloride (10 mL) and saturated brine (10 mL). The organic layer was dried over anhydrous sodium sulfate, and then the solvent was evaporated under reduced pressure. Toluene (2 mL) was added to the residue, and the mixture was dehydrated azeotropically under reduced pressure to obtain an imidate (250 mg) as a yellow amorphous compound. The resulting imidate was used in the subsequent step without purification.

5-Acetoxymethyl-2-(4-ethylbenzyl)phenol (WO2002/064606) (100 mg, 0.35 mmol) and imidate (250 mg, 0.39 mmol) were dissolved in methylene chloride (5 mL), followed by addition of MS4A, a boron trifluoride-diethyl ether complex (50 µL, 0.39 mmol) was added dropwise to the reaction mixture with ice cooling, and the mixture was stirred with ice cooling at room temperature for 15 min. Saturated sodium hydrogencarbonate (3 mL) was added to the reaction mixture, and the mixture was diluted with ethyl acetate (10 mL) and washed with saturated aqueous sodium hydrogencarbonate (10 mL) and saturated brine (10 mL). The organic layer was dried over anhydrous sodium sulfate, and then the solvent was evaporated under reduced pressure. The residue was purified by silica gel flash column chromatography (hexane:ethyl acetate, 10:1 1 to 5:1, v/v) to isolate the title compound (200 mg, yield 63%) as a colorless amorphous compound.
¹H NMR (400MHz, CDCl₃):δ1.16 (3H, t, J=7.5 Hz), 2.10 (3H, s), 2.52 (2H, q, J=7.5 Hz), 3.36 (3H, s), 3.61-3.70 (2H, m), 3.69(1H, d, J=15.0 Hz), 3.80 (1H, d, J=15.0 Hz), 4.12-4.16 (1H, m), 5.05 (2H, s), 5.41 (1H, d, J=7.8 Hz), 5.63 (1H, t, J=9.6 Hz), 5.87 (1H, dd, J=9.8 and 7.8 Hz), 5.99 (1H, t, J=9.6 Hz), 6.92-6.97 (5H, m), 7.15 (1H, s), 7.28-7.56 (10H, m), 7.86 (2H, d, J=8.2 Hz), 7.90 (2H, d, J=7.4 Hz), 7.96 (2H, d, J=8.6 Hz).

### (1c) 2-(4-Ethylbenzyl)-5-hydroxymethylphenyl 6-O-methyl-β-D-glucopyranoside

The compound synthesized in (1b) (200 mg, 0.24 mmol) was dissolved in methanol-methylene chloride (8 mL/2 mL), followed by addition of potassium carbonate (338 mg, 2.4 mmol), and the mixture was stirred overnight at room temperature. The reaction mixture was filtered using Celite to remove excess potassium carbonate and neutralized by adding an appropriate amount of acetic acid to remove methanol under reduced pressure. The residue was dissolved in ethyl acetate (10 mL) and washed with saturated aqueous sodium hydrogencarbonate (10 mL) and saturated brine (10 mL). The organic layer was dried over anhydrous sodium sulfate, and then the solvent was evaporated under reduced pressure. The residue was crystallized from hexane-ethyl acetate to obtain the title compound (79 mg, yield 77%) as a colorless solid.
¹H NMR (500MHz, CD₃OD):δ 1.19 (3H, t, J=7.7 Hz), 2.58 (2H, q, J=7.7 Hz), 3.36 (3H, s), 3.38-3.61 (5H, m), 3.73 (1H, dd, J=10.8 and 2.0 Hz), 3.94 (1H, d, J=14.7 Hz), 4.03 (1H, d, J=14.7 Hz), 4.54 (2H, s), 4.91 (1H, d, J=7.9 Hz), 6.92 (1H, d, J=7.8 Hz), 7.01 (1H, d, J=7.8 Hz), 7.06 (2H, d, J=7.8 Hz), 7.12 (2H, d, J=7.8 Hz), 7.15 (1H, s) MS (FAB) m/z: 457 (M+K)⁺.

### (Example 2) 2-(4-Ethylbenzyl)-5-hydroxymethylphenyl 4-O-methyl-β-D-glucopyranoside (Example Compound No. 2-3)

### (2a) 4-O-Methyl-2,3,6-tri-O-benzoyl-α,β-D-glucopyranoside

Methyl 2,3,6-tri-O-benzoyl-4-O-methyl-α-D-glucopyranoside (Carbohydr. Res. 1995, 273, 249-254.) (139 g, 0.267 mol) was dissolved in acetic anhydride (505 mL) and acetic acid (250 mL), followed by addition of concentrated sulfuric acid (1.6 mL, 30 mmol) with ice cooling, and the mixture was stirred at room temperature for 24 h. Concentrated sulfuric acid (3.2 mL, 60 mmol) was added to the reaction mixture, the mixture was further stirred at room temperature for 18 h, followed by addition of sodium acetate (43.8 g, 0.534 mol) and saturated aqueous sodium hydrogencarbonate (50 mL) with ice cooling, and the solvent was evaporated under reduced pressure. The resulting residue was diluted with methylene chloride (1 L) and washed with saturated aqueous sodium hydrogencarbonate (150 mL) and 10% brine (150 mL × 2). The solvent was concentrated under reduced pressure to obtain acetyl 2,3,6-tri-O-benzoyl-4-O-methyl-α,β-D-glucopyranoside (160 g) as a yellow oil. This yellow oily residue (160 g) was dissolved in N,N-dimethylformamide (750 mL), followed by addition of hydrazine acetate (29.0 g, 0.315 mol), and the mixture was stirred at room temperature for 7 h. The reaction mixture was diluted with ethyl acetate (1 L) with ice cooling and washed with 10% brine (1.2 L × 1, 800 mL × 1) and water (800 mL × 3), and then the solvent was evaporated under reduced pressure. The residue was purified by silica gel flash column chromatography (hexane:ethyl acetate, 3:2 to 1:1, v/v) to obtain the title compound (119 g, 87.7%) as a white solid.
¹H NMR (400 MHz, CDCl₃):δ 3.13 (0.8H, d, J=3.5 Hz), 3.45 (0.6H, s), 3.47 (2.4H, s), 3.68 (0.8H, t, J=9.6 Hz), 3.69 (0.2H, t, J=9.4 Hz), 3.84-3.89 (0.4H, m), 4.37-4.41 (0.8H, m), 4.59-4.72 (2H, m), 4.95 (0.2H, t, J=7.8 Hz), 5.18-5.22 (1H, m), 5.65 (0.8H, t, J=3.5 Hz), 5.76 (0.2H, t, J=9.6 Hz), 6.06 (0.8H, t, J=9.8 Hz), 7.35-7.42 (4H, m), 7.48-7.54 (4H, m), 7.59-7.63 (1H, m), 7.96-8.04 (4H, m), 8.11-8.13 (2H, m);
MS (FAB) m/z: 507 (M+H)⁺.

### (2b) 5-Acetoxymethyl-2-(4-ethylbenzyl)phenyl 4-O-methyl-2,3,6-tri-O-benzoyl-β-D-glucopyranoside

The compound synthesized in (2a) (235 mg, 0.74 mmol), trichloroacetonitrile (0.23 mL, 2.28 mmol), 1,8-diazabicyclo[5.4.0]-7-undecene (7 µL, 0.05 mmol), and methylene chloride (5 mL) were used to prepare an imidate by the same technique as in (1b), and subsequently 5-acetoxymethyl-2-(4-ethylbenzyl)phenol (56 mg, 0.20 mmol), a boron trifluoride-diethyl ether complex (26 µL, 0.21 mmol), and methylene chloride (7 mL) were used to obtain a crude product of the title compound (150 mg) by the same technique as in (1b).

### (2c) 2-(4-Ethylbenzyl)-5-hydroxymethylphenyl 4-O-methyl-β-D-glucopyranoside

The compound synthesized in (2b) (250 mg, 0.27 mmol), potassium carbonate (14 mg, 0.10 mmol), methanol (5 mL), and methylene chloride (5 mL) were used to obtain the title compound (42 mg, yield 31 %) as a colorless solid by the same technique as in (1c). However, purification was performed by silica gel flash column chromatography (methylene chloride:methanol, 40:1 to 10:1, v/v).
¹H NMR (400MHz, CD₃OD):δ1.19 (3H, t, J=7.7 Hz), 2.58 (2H, q, J=7.7 Hz), 3.20 (1H, t, J=9.1 Hz), 3.40-3.44 (1H, m), 3.49-3.55 (2H, m), 3.59 (3H, s), 3.70 (1H, dd, J=12.2 and 4.7 Hz), 3.85 (1H, dd, J=12.2 and 2.4 Hz), 3.93 (1H, d, J=14.8 Hz), 4.04 (1H, d, J=14.8 Hz), 4.54 (2H, s), 4.91 (1H, d, J=7.5 Hz), 6.92 (1H, d, J=7.8 Hz), 7.02 (1H, d, J=7.8 Hz), 7.06 (2H, d, J=7.8 Hz), 7.13 (2H, d, J=7.8 Hz), 7.14 (1H, s)
MS (FAB) m/z: 457 (M+K)⁺.

### (Example 3) 2-(4-Ethylbenzyl)-5-hydroxymethylphenyl 4-C-methyl-β-D-glucopyranoside (Example Compound No. 1-64)

### (3a) Methyl 2,3,6-tri-O-benzoyl-4-C-methyl-α-D-glucopyranoside

Methyl 2,3-di-O-benzyl-4-C-methyl-6-O-triphenylmethyl-α-D-glucopyranoside (Bull. Chem. Soc. Jpn., 1982, 55, 938-942.) (40.0 g, 63.4 mmol) was dissolved in methanol (300 mL), followed by addition of 36% hydrochloric acid (1.6 mL) and 10% palladium on carbon (12 g), and the mixture was stirred under a hydrogen atmosphere at room temperature for 5 h. Then, ethyl acetate (30 mL), 36% hydrochloric acid (0.5 mL), and 10% palladium on carbon (4.0 g) were added into the reaction system, and the mixture was stirred under a hydrogen atmosphere at room temperature for 3 h. Triethylamine (7.0 mL) was added, then the reaction mixture was filtered using Celite, and the solvent was evaporated from the filtrate under reduced pressure. Dichloromethane (300 mL) and triethylamine (180 mL) were added to the resulting residue, the reaction mixture was stirred with ice cooling, followed by addition of benzoyl chloride (74 mL, 0.63 mol), and stirred at 40°C for 16 h. Methanol (30 mL) was added with ice cooling to terminate the reaction, and then the reaction mixture was diluted with ethyl acetate and washed successively with water, saturated aqueous sodium hydrogencarbonate, and saturated brine. The organic layer was dried over anhydrous sodium sulfate, then the solvent was evaporated under reduced pressure, and the residue was purified by silica gel flash column chromatography (hexane:ethyl acetate, 5:1 to 4:1, v/v) to obtain the title compound (24.1 g, 73%) as a yellow amorphous compound.
¹H NMR (400MHz, CDCl₃):δ1.44 (3H, s), 3.45 (3H, s), 4.27(1H, dd, J=8.6 and 2.0 Hz), 4.54 (1H, dd, J=11.8 and 8.6 Hz), 4.76 (1H, dd, J=11.8 and 2.0 Hz), 5.16 (1H, d, J=3.9 Hz), 5.27 (1H, dd, J=10.6 and 3.9 Hz), 5.70 (1H, d, J=10.6 Hz), 7.61-7.33 (9H, m), 8.03-7.94 (4H, m), 8.07 (2H, d, J=8.6 Hz)
MS (FAB) m/z:521 (M + H)⁺.

### (3b) Methyl 4-O-acetyl-2,3,6-tri-O-benzoyl-4-C-methyl-α-D-glucopyranoside

The compound synthesized in (3a) (24.1 g, 46.3 mmol) was dissolved in pyridine (100 mL), followed by addition of acetic anhydride (34 mL, 0.46 mmol) and N,N-dimethylaminopyridine (570 mg, 4.63 mmol), and the mixture was stirred at 50°C for 3 h. The reaction mixture was diluted with ethyl acetate, washed with 1 N hydrochloric acid solution and saturated brine, and dried over anhydrous sodium sulfate, and then the solvent was evaporated under reduced pressure. The residue was purified by silica gel flash column chromatography (hexane:ethyl acetate, 6:1 to 4:1, v/v) to obtain the title compound (21.7 g, yield 79%) as a yellow white amorphous compound.
¹H NMR (400MHz,CDCl₃):δ1.66(3H, s), 1.97 (3H, s), 3.47(3H, s), 4.49 (1H, dd, J=11.7 and 8.2 Hz), 4.60 (1H, dd, J=11.7 and 2.7 Hz), 5.16 (1H, d, J=4.3 Hz), 5.26 (1H, dd, J=10.1 and 4.3 Hz), 5.37 (1H, dd, J=8.2 and 2.7 Hz), 6.58 (1H, d, J=10.1 Hz), 7.61-7.33 (9H, m), 8.02-7.93 (4H, m), 8.06 (2H, dd, J=8.4 and 1.4 Hz)
MS (FAB) m/z:585 (M + Na)⁺.

### (3c) 4-O-Acetyl-4-C-methyl-2,3,6-tri-O-benzoyl-α,β-D-glucopyranoside

The compound synthesized in (3b) (21.7 g, 38.6 mmol) was dissolved in acetic acid (66 mL) and acetic anhydride (110 mL), 97% concentrated sulfuric acid (10.6 mL, 0.19 mol) was added dropwise at room temperature, and the mixture was stirred at room temperature for 18 h. Ammonium acetate (47 g, 0.58 mol) was added with ice cooling to terminate the reaction, then the solvent was evaporated under reduced pressure, and the residue was diluted with diethyl ether and washed successively with water, saturated aqueous sodium hydrogencarbonate, and saturated brine. The organic layer was dried over anhydrous sodium sulfate, then the solvent was evaporated under reduced pressure, and the residue was dried under reduced pressure. The resulting residue was dissolved in N,N-dimethylformamide (180 mL), followed by addition of hydrazine acetate (5.33 g, 57.9 mmol), and the mixture was stirred at room temperature for 1 h. The reaction mixture was diluted with ethyl acetate, then washed successively with saturated aqueous sodium hydrogencarbonate, saturated aqueous ammonium chloride, and saturated brine, and dried over anhydrous sodium sulfate, and then the solvent was evaporated under reduced pressure. The residue was purified by silica gel flash column chromatography (hexane:ethyl acetate, 4:1 to 3:1, v/v) to obtain the title compound (18.9 g, yield 89%) as a white amorphous compound.
¹H NMR (400MHz,CDCl₃):δ1.66(3H, s), 1.96(3H, s), 3.00(2/3H, brd, J=4.3 Hz), 3.72(1/3H, d, J=7.8 Hz), 4.51-4.41 (1H, m), 4.68-4.61 (1H, m), 5.09 (1/3H, t like, J=7.8 Hz), 5.36-5.23 (4/3H, m), 5.69-5.62 (4/3H, m), 6.51 (1/3H, d, J=10.2 Hz), 6.67 (2/3H, d, J=10.6 Hz), 7.61-7.32 (9H, m), 8.02-7.93 (4H, m), 8.07 (2H, brd, J=7.0 Hz) MS (FAB) m/z:587 (M + K)⁺.

### (3d) 5-Acetoxymethyl-2-(4-ethylbenzyl)phenyl 4-O-acetyl-4-C-methyl-2,3,6-tri-O-benzoyl-β-D-glucopyranoside

The compound synthesized in (3c) (233 mg, 0.42 mmol), trichloroacetonitrile (0.21 mL, 2.08 mmol), 1,8-diazabicyclo[5.4.0]-7-undecene (6 µL, 0.05 mmol), and methylene chloride (5 mL) were used to prepare an imidate by the same technique as in (1b), and subsequently 5-acetoxymethyl-2-(4-ethylbenzyl)phenol (110 mg, 0.39 mmol), a boron trifluoride-diethyl ether complex (53 µL, 0.42 mmol), and methylene chloride (5 mL) were used to obtain a crude product of the title compound (227 mg) by the same technique as in (1b).

### (3e) 2-(4-Ethylbenzyl)-5-hydroxymethylphenyl 4-C-methyl-β-D-glucopyranoside

The compound synthesized in (3d) (227 mg, 0.28 mmol), potassium carbonate (385 mg, 2.79 mmol), methanol (10 mL), and methylene chloride (2 mL) were used to obtain the title compound (79 mg, yield 68%) as a colorless solid by the same technique as in (1c).
¹H NMR (400MHz, CD₃OD):δ1.13 (3H, s), 1.19 (3H, t, J=7.6 Hz), 2.58 (2H, q, J=7.6 Hz), 3.35(1H, s), 3.44-3.47 (2H, m), 3.62 (1H, dd, J=11.8 and 8.2 Hz), 3.91 (1H, dd, J=11.8 and 2.4 Hz), 3.94 (1H, d, J=15.2 Hz), 4.05 (1H, d, J=15.2 Hz), 4.54 (2H, s), 4.93 (1H, dd, J=5.5 and 1.9 Hz), 6.93 (1H, d, J=7.9 Hz), 7.03 (1H, d, J=7.9 Hz), 7.07 (2H, d, J=8.2 Hz), 7.14 (2H, d, J=8.2 Hz), 7.19 (1H, s)
MS (FAB) m/z: 419 (M+H)⁺.

### (Example 4) 2-(4-Ethylbenzyl)-5-hydroxymethylphenyl 5-C-methyl-β-D-glucopyranoside (Example Compound No. 1-63)

### (4a) Benzoyl 3-O-benzyl-2,4,6-tri-O-benzoyl-5-C-methyl-β-D-glucopyranoside

3-O-Benzyl-1,2-O-isopropylidene-5-C-methyl-6-O-trityl-α-D-glucofuranose (Bull. Chem. Soc. Jpn., 1976, 49, 788-790.) (47.0 g, 82.9 mmol) was dissolved in 1,4-dioxane (375 mL) and water (125 mL), followed by addition of 97% concentrated sulfuric acid (2.25 mL, 41.2 mmol), and the mixture was stirred at 80°C for 17 h. Triethylamine (34 mL) was added with ice cooling, then the solvent was evaporated under reduced pressure, and the residue was dried under reduced pressure. Dichloromethane (400 mL) and triethylamine (170 mL) were added to the resulting residue, then the mixture was stirred with ice cooling, followed by addition of benzoyl chloride (76 mL, 0.66 mol) and N,N-dimethylaminopyridine (1.0 g, 8.2 mmol), and stirred at 40°C for 18 h. Methanol (30 mL) was added with ice cooling to terminate the reaction, the solvent was evaporated under reduced pressure, and the residue was diluted with ethyl acetate and washed successively with water, saturated aqueous sodium hydrogencarbonate, and saturated brine. The organic layer was dried over anhydrous sodium sulfate, then the solvent was evaporated under reduced pressure, and the residue was dried under reduced pressure. The resulting residue was dissolved in pyridine (200 mL), followed by addition of benzoyl chloride (38 mL, 0.33 mol) and N,N-dimethylaminopyridine (1.0 g, 8.2 mmol) with ice cooling, and the mixture was stirred at 60°C for 18 h. Methanol (30 mL) was added with ice cooling to terminate the reaction, the solvent was evaporated under reduced pressure, and then the residue was diluted with ethyl acetate and washed successively with water, 1 N hydrochloric acid, saturated aqueous sodium hydrogencarbonate, and saturated brine. The organic layer was dried over anhydrous sodium sulfate, then the solvent was evaporated under reduced pressure, and the residue was purified by silica gel flash column chromatography (hexane:ethyl acetate, 6:1 to 2:1, v/v) to obtain the title compound (40.2 g, 69%) as a pale yellow solid.
¹H NMR (400MHz,CDCl₃):δ1.66(3H, s), 4.26(1H, dd, J=8.2 and 7.3Hz), 4.33(1H, d, J=11.8Hz), 4.51(1H, d, J=11.8Hz), 4.67(1H, d, J=11.3Hz), 4.71(1H, d, J=11.3Hz), 5.67(1H, dd, J=7.3 and 6.6Hz), 5.87(1H, d, J=8.2Hz), 6.43(1H, d, J=6.6Hz), 7.14-7.03(5H, m), 7.63-7.32(12H, m), 8.04-7.96(8H, m)
MS (FAB) m/z:723 (M + Na)⁺.

### (4b) Benzoyl 2,3,4,6-tetra-O-benzoyl-5-C-methyl-β-D-glucopyranoside

The compound synthesized in (4a) (40.2 g, 57.4 mmol) was dissolved in tetrahydrofuran (150 mL) and methanol (150 mL), followed by addition of 2 N hydrochloric acid (5.7 mL, 11.5 mmol) and 20% palladium hydroxide (6.0 g), and the mixture was stirred under a hydrogen atmosphere at room temperature for 2 h. The reaction mixture was filtered using Celite, followed by addition of triethylamine (5.0 mL), the solvent was evaporated under reduced pressure, and the residue was dried under reduced pressure. The resulting residue was dissolved in pyridine (200 mL), followed by addition of benzoyl chloride (13 mL, 0.12 mol) and N,N-dimethylaminopyridine (700 mg, 5.74 mmol) with ice cooling, and the mixture was stirred at 60°C for 20 h. Methanol (5.0 mL) was added with ice cooling to terminate the reaction, the solvent was evaporated under reduced pressure, and then the residue was diluted with dichloromethane and washed successively with water, 1 N hydrochloric acid, saturated aqueous sodium hydrogencarbonate, and saturated brine. The organic layer was dried over anhydrous sodium sulfate, then the solvent was evaporated under reduced pressure, and the resulting residue was purified by washing with a hexane-ethyl acetate mixed solvent to obtain the title compound (34.4 g, 84%) as a colorless solid.
¹H NMR (400MHz,CDCl₃):δ1.83(3H, s), 4.33(1H, d, J=11.8Hz), 4.49(1H, d, J=11.8Hz), 5.83(1H, dd, J=9.0 and 7.8Hz), 6.01(1H, d, J=9.8Hz), 6.13(1H, dd, J=9.8 and 9.0Hz), 6.51(1H, d, J=7.8Hz), 7.58-7.28(15H, m), 7.98-7.85(6H, m), 8.03(4H, t, J=6.9Hz)
MS (FAB) m/z:737 (M + Na)⁺.

### (4c) 2,3,4,6-Tetra-O-benzoyl-5-C-methyl-α,β-D-glucopyranoside

The compound synthesized in (4b) (7.56 g, 10.6 mmol) was dissolved in N,N-dimethylformamide (100 mL), followed by addition of hydrazine acetate (1.46 g, 15.9 mmol), and the mixture was stirred at room temperature for 20 h. The reaction mixture was diluted with ethyl acetate, then washed successively with saturated aqueous sodium hydrogencarbonate, saturated aqueous ammonium chloride, and saturated brine, and dried over anhydrous sodium sulfate, and then the solvent was evaporated under reduced pressure. The residue was purified by silica gel flash column chromatography (hexane:ethyl acetate, 6:1 to 2:1, v/v) to obtain the title compound (1.96 g, yield 30%) as a white amorphous compound.
¹H NMR (400MHz,CDCl₃):δ1.69(2H, s), 1.80(1H, s), 3.03(1/4H, brd, J=3.5Hz), 3.57(3/4H, d, J=8.6Hz), 4.14(1/4H, d, J=12.2Hz), 4.32(3/4H, d, J=11.8Hz), 4.47(3/4H, d, J=11.8Hz), 4.50(1/4H, d, J=12.2Hz), 5.36-5.25(7/4H, m), 5.83(1/4H, t like, J=3.5Hz), 5.93(1H, t like, J=9.2Hz), 6.07(3/4H, t like, J=9.8Hz), 6.37(1/4H, t like, J=10.2Hz), 7.60-7.25(12H, m), 8.12-7.81(8H, m)
MS (FAB) m/z:611 (M + H)⁺.

### (4d) 5-Acetoxymethyl-2-(4-ethylbenzyl)phenyl 5-C-methyl-2,3,4,6-tetra-O-benzoyl-β-D-glucopyranoside

The compound synthesized in (4c) (100 mg, 0.16 mmol), trichloroacetonitrile (97 µL, 0.96 mmol), 1,8-diazabicyclo[5.4.0]-7-undecene (3 µL, 0.03 mmol), and methylene chloride (4 mL) were used to prepare an imidate by the same technique as in (1b), and subsequently 5-acetoxymethyl-2-(4-ethylbenzyl)phenol (41 mg, 0.14 mmol), a boron trifluoride-diethyl ether complex (20 µL, 0.16 mmol), and methylene chloride (4 mL) were used to obtain the title compound (100 mg, yield 70%) by the same technique as in (1b).
¹H NMR (400MHz,CDCl₃):δ1.15 (3H, t, J=7.6Hz), 1.83 (3H, s), 2.06 (3H, s), 2.51 (2H, q, J=7.6 Hz), 3.75 (1H, d, J=15.2 Hz), 3.82 (1H, d, J=15.2 Hz), 4.35 (1H, d, J=11.9 Hz), 4.52 (1H, d, J=11.9 Hz), 5.00 (2H, s), 5.73 (1H, d, J=7.0 Hz), 5.80- 5.84 (1H, m), 6.03 (1H, d, J=10.2 Hz), 6.18-6.13 (1H, m), 6.88-6.94 (5H, m), 7.21 (1H, s), 7.28-7.55 (12H, m), 7.85-8.05 (9H, m)
MS (FAB) m/z: 877 (M+K)⁺.

### (4e) 2-(4-Ethylbenzyl)-5-hydroxymethylphenyl 5-C-methyl-β-D-glucopyranoside

The compound synthesized in (4d) (100 mg, 0.11 mmol), potassium carbonate (158 mg, 1.14 mmol), methanol (6 mL), and methylene chloride (1.5 mL) were used to obtain the title compound (28 mg, yield 59%) by the same technique as in (1c) as a colorless solid.
¹H NMR (400MHz, CD₃OD):δ1.19 (3H, t, J=7.7 Hz), 1.28 (3H, s), 2.58 (2H, q, J=7.7 Hz), 3.47 (1H, t, J=8.0 Hz), 3.53 (2H, d, J=3.2 Hz), 3.56-3.67 (2H, m), 3.93(1H, d, J=14.9 Hz), 4.03 (1H, d, J=14.9 Hz), 4.55(2H, s), 5.17 (1H, d, J=7.8 Hz), 6.89 (1H, d, J=7.8 Hz), 7.01 (1H, d, J=7.8 Hz), 7.06 (2H, d, J=7.8 Hz), 7.12 (2H, d, J=7.8 Hz), 7.20 (1H, s)
MS (FAB) m/z: 457 (M+K)⁺.

### (Example 5) 2-(4-Ethylbenzyl)-5-hydroxymethylphenyl 4-deoxy-4-C-methyl-β-D-glucopyranoside (Example Compound No. 2-2)

### (5a) Methyl 2,3,6-tri-O-benzoyl-4-deoxy-4-C-methyl-α-D-glucopyranoside

Methyl 2,3,6-tri-O-benzyl-4-deoxy-4-C-methyl-α-D-glucopyranoside (Org. Lett., 2003, 5, 3419-3421.) (266 mg, 0.575 mmol) was dissolved in methanol (4.0 mL) and ethyl acetate (1.0 mL), followed by addition of 2 N hydrochloric acid (85 µL, 0.17 mmol) and 20% palladium hydroxide (110 mg), and the mixture was stirred under a hydrogen atmosphere at room temperature for 4 h. The reaction mixture was filtered using Celite, followed by addition of triethylamine (240 µL), the solvent was evaporated under reduced pressure, and the residue was dried under reduced pressure. Dichloromethane (7.0 mL) and triethylamine (640 µL) were added to the resulting residue, then the mixture was stirred with ice cooling, followed by addition of benzoyl chloride (400 µL, 3.45 mmol) and N,N-dimethylaminopyridine (7.0 mg, 0.058 mmol), and stirred at 40°C for 3 h. Triethylamine (320 µL) was added to the reaction mixture, followed by addition of benzoyl chloride (200 µL, 1.73 mmol) and N,N-dimethylaminopyridine (a catalytic amount, about 3 mg), and the mixture was stirred at 40°C for 16 h. The mixture was diluted with ethyl acetate and washed successively with saturated aqueous sodium hydrogencarbonate and saturated brine. The organic layer was dried over anhydrous sodium sulfate, then the solvent was evaporated under reduced pressure, and the residue was purified by silica gel flash column chromatography (hexane:ethyl acetate, 10:1 to 2:1, v/v) to obtain the title compound (272 mg, 94%) as a white amorphous compound.
¹H NMR (400MHz,CDCl₃):δ1.08 (3H, d, J=6.6 Hz), 2.31-2.19 (1H, m), 3.43 (3H, s), 4.05-3.98 (1H, m), 4.62-4.48 (2H, m), 5.24-5.18 (2H, m), 5.72 (1H, dd, J=10.5 and 9.4 Hz), 7.41-7.32 (4H, m), 7.53-7.46 (4H, m), 7.64-7.56 (1H, m), 8.02-7.93 (4H, m), 8.13-8.08 (2H, m)
MS (FAB) m/z:505 (M + H)⁺.

### (5b) 2,3,6-Tri-O-benzoyl-4-deoxy-4-C-methyl-α,β-D-glucopyranoside

The compound synthesized in (5a) (268 mg, 0.531 mmol) was dissolved in acetic acid (1.3 mL) and acetic anhydride (2.6 mL), 95% concentrated sulfuric acid (15 µL, 0.27 mmol) was added dropwise at room temperature, and the mixture was stirred at room temperature for 3 h. Ammonium acetate (218 mg, 2.66 mmol) was added with ice cooling to terminate the reaction, then the solvent was evaporated under reduced pressure, and the residue was diluted with diethyl ether and washed successively with water, saturated aqueous sodium hydrogencarbonate, and saturated brine. The organic layer was dried over anhydrous sodium sulfate, then the solvent was evaporated under reduced pressure, and the residue was dried under reduced pressure. The resulting residue was dissolved in N,N-dimethylformamide (4.0 mL), followed by addition of hydrazine acetate (63 mg, 0.68 mmol), and the mixture was stirred at room temperature for 18 h. The reaction mixture was diluted with ethyl acetate, then washed successively with saturated aqueous sodium hydrogencarbonate, saturated aqueous ammonium chloride, and saturated brine, and dried over anhydrous sodium sulfate, and then the solvent was evaporated under reduced pressure. The residue was purified by silica gel flash column chromatography (hexane:ethyl acetate, 5.5:1 to 1.5:1, v/v) to obtain the title compound (218 mg, 84%) as a white amorphous compound.
¹H NMR (400MHz,CDCl₃):δ1.08(2H, d, J=6.6Hz), 1.09(1H, d, J=6.3Hz), 2.36-2.23(1H, m), 2.91(2/3H, d, J=3.3Hz), 3.78-3.72(1/3H, m), 3.80(1/3H, d, J=9.0Hz), 4.29(2/3H, dt, J=10.6 and 3.3Hz), 4.68-4.50(2H, m), 4.91(1/3H, t, J=8.2Hz), 5.17(1/3H, dd, J=9.8 and 8.2Hz), 5.24(2/3H, dd, J=10.6 and 3.3Hz), 5.47(1/3H, dd, J=10.5 and 9.8Hz), 5.72(2/3H, t, J=3.3Hz), 5.79(2/3H, t, J=10.6Hz), 7.42-7.34(4H, m), 7.54-7.47(4H, m), 7.65-7.59(1H, m), 8.03-7.93(4H, m), 8.15-8.10(2H, m)
MS (FAB) m/z:491 (M + H)⁺.

### (5c) 5-Acetoxy-2-(4-ethylbenzyl)phenyl 4-deoxy-4-C-methyl-2,3,6-tri-O-benzoyl-β-D-glucopyranoside

The compound synthesized in (5b) (214 mg, 0.44 mmol), trichloroacetonitrile (0.22 mL, 2.18 mmol), 1,8-diazabicyclo[5.4.0]-7-undecene (6 µL, 0.05 mmol), and methylene chloride (4 mL) were used to prepare an imidate by the same technique as in (1b), and subsequently 5-acetoxymethyl-2-(4-ethylbenzyl)phenol (110 mg, 0.39 mmol), a boron trifluoride-diethyl ether complex (50 µL, 0.39 mmol), and methylene chloride (4 mL) were used to obtain the title compound (290 m, yield 91 %) by the same technique as in (1b) as a colorless amorphous compound.
¹H NMR (400MHz,CDCl₃):δ 1.11-1.16 (6H, m), 2.04 (3H, s), 2.32-2.39 (1H, m), 2.50 (2H, q, J=7.6 Hz), 3.68 (1H, d, J=15.7 Hz), 3.80 (1H, d, J=15.7 Hz), 3.93-3.97 (1H, m), 4.54 (1H, dd, J=12.1 and 6.3 Hz), 4.71(1H, dd, J=12.1 and 2.0 Hz), 4.82(1H, d, J=12.1 Hz), 4.88 (1H, d, J=12.1 Hz), 5.34 (1H, d, J=7.7 Hz), 5.51 (1H, t, J=10.2 Hz), 5.75 (1H, dd, J=9.6 and 7.7 Hz), 6.89-6.93 (6H, m), 7.07 (1H, s), 7.32 (2H, t, J=7.7 Hz), 7.39 (2H, t, J=7.7 Hz), 7.46-7.54 (4H, m), 7.61 (1H, t, J=7.5 Hz), 7.88 (2H, d, J=7.1 Hz), 7.99 (2H, d, J=7.5 Hz), 8.09 (2H, d, J=7.5 Hz)
MS (FAB) m/z: 795 (M+K)⁺.

### (5d) 2-(4-Ethylbenzyl)-5-hydroxymethylphenyl 4-deoxy-4-C-methyl-β-D-glucopyranoside

The compound synthesized in (5c) (290 mg, 0.38 mmol), potassium carbonate (530 mg, 3.83 mmol), methanol (8 mL), and methylene chloride (2 mL) were used to obtain the title compound (110 mg, yield 71 %) by the same technique as in (1c) as a colorless solid.
¹H NMR (400MHz, CD₃OD):δ 1.04 (3H, d, J=6.2 Hz), 1.19 (3H, t, J=7.5 Hz), 1.67-1.73 (1H, m),2.58 (2H, q, J=7.5 Hz), 3.21 (1H, dd, J=10.2 and 9.0 Hz), 3.46 (1H, d, J=7.8 Hz), 3.65 (1H, d, J=5.5Hz), 3.80 (1H, dd, J=12.1 and 2.3 Hz), 3.94 (1H, d, J=14.9 Hz), 4.05 (1H, d, J=14.9 Hz), 4.54 (2H, s), 4.89 (1H, d, J=7.8 Hz), 6.91 (1H, d, J=7.7 Hz), 7.02 (1H, d, J=7.7 Hz), 7.06 (2H, d, J=8.6 Hz), 7.13(2H, d, J=8.6 Hz), 7.15(1H, s)
MS (FAB) m/z: 457 (M+K)⁺.

### (Example 6) 2-(4-Ethylbenzyl)-5-hydroxymethylphenyl 7-deoxy-L-glycero-β-D-gluco-heptopyranoside (Example Compound No. 1-59)

### (6a) Benzyl 2,3,4-tri-O-benzyl-7-deoxy-L-glycero-β-D-gluco-heptopyranoside

A solution of dimethyl sulfoxide (9.80 mL, 138 mmol) in methylene chloride (50 mL) was added dropwise to a solution of oxalyl chloride (6.05 mL, 70.5 mmol) in methylene chloride (200 mL) at -70°C. The mixture was stirred at the same temperature for 10 min, and a solution of benzyl 2,3,4-tri-O-benzyl-β-D-glucopyranoside (Org. Biomol. Chem., 2003, 1, 767-771) (25.0 g, 46.2 mmol) in methylene chloride (200 mL) was added dropwise at -70°C over 1 h. The mixture was heated to -45°C, then triethylamine (38.7 mL, 278 mmol) was added dropwise, and the mixture was heated to room temperature. Water (200 mL) was added to terminate the reaction, and the organic layer was washed successively with hydrochloric acid (1 M, 200 mL), water (200 mL), and saturated aqueous sodium hydrogencarbonate (100 mL). The organic layer was dried over anhydrous magnesium sulfate, and then the solvent was evaporated under reduced pressure to obtain an aldehyde as a yellow oil. The resulting crude product of the aldehyde was dissolved in tetrahydrofuran (250 mL), and methylmagnesium bromide (100 mL, 0.96 M tetrahydrofuran solution) was added dropwise at -70°C. The mixture was heated to room temperature, then aqueous ammonium chloride (100 mL) was added to terminate the reaction, and the mixture was extracted with ethyl acetate (250 mL). The organic layer was washed with saturated brine (50 mL) and dried over anhydrous magnesium sulfate, and then the solvent was evaporated under reduced pressure. The residue containing a diastereomer mixture was purified by silica gel flash column chromatography (hexane:ethyl acetate, 3:1 to 2:1, v/v) to isolate the title compound (8.23 g, 32.1 %) as a colorless oil. Furthermore, the diastereomer mixture (approx. 1:1, 17.4 g) was obtained as a white solid.
Rf 0.56 (hexane:ethyl acetate = 2:1);
¹H NMR (400 MHz, CDCl₃):δ 1.31 (3H, d, J=6.7 Hz), 1.76 (1H, d, J=10.6 Hz), 3.11 (1H, dd, J=9.0 and 1.5 Hz), 3.47-3.51 (1H, m), 3.66 (1H, t, J=9.0 Hz), 3.73 (1H, t, J=9.0 Hz), 4.02-4.10 (1H, m), 4.54 (1H, d, J=7.8 Hz), 4.69-4.75 (3H, m), 4.82 (1H, d, J=11.0 Hz), 4.89-4.97 (4H, m), 7.27-7.40 (20H, m);
MS (FAB) m/z: 577 (M+Na)⁺.

### (6b) Benzoyl 2,3,4,6-tetra-O-benzoyl-7-deoxy-L-glycero-α,β-D-gluco-heptopyranoside

A low-polarity diastereomer compound of the alcohol synthesized in (6a) (8.70 g, 15.7 mmol) was dissolved in ethyl acetate (17 mL) and methanol (174 mL), dilute hydrochloric acid (2 M, 0.40 mL, 0.80 mmol) and palladium hydroxide on carbon (20% by weight Pd, wet, 1.74 g) were successively added with ice cooling, and the mixture was stirred under a hydrogen atmosphere at room temperature for 2 h. Triethylamine (0.30 mL, 2.1 mmol) was added, then the mixture was filtered using Celite, and the solvent was evaporated under reduced pressure. The resulting residue was dissolved in pyridine (87 mL), followed by addition of benzoyl chloride (10.9 mL, 93.8 mmol) at room temperature, and the mixture was stirred overnight at room temperature. Water (0.57 mL, 32 mmol) was added with ice cooling, the mixture was stirred at room temperature for 2 h and then diluted with ethyl acetate (100 mL), and the resulting insoluble matters were removed by filtration. The mother liquor was concentrated under reduced pressure, followed by addition of water (50 mL), and the mixture was extracted with ethyl acetate (100 mL). The organic layer was washed successively with hydrochloric acid (1 M, 20 mL), saturated aqueous sodium hydrogencarbonate (20 mL), and saturated brine (20 mL) and dried over anhydrous magnesium sulfate. The solvent was evaporated under reduced pressure, and the residue was purified by silica gel flash column chromatography (n-hexane:ethyl acetate, 2:1, v/v) to obtain the title compound (9.07 g, 81.0%) as a white solid.
¹H NMR (400 MHz, CDCl₃):δ 1.46 (1.5H, d, J=6.7 Hz), 1.52 (1.5H, d, J=6.7 Hz), 4.17 (0.5H, dd, J=10.0 and 2.0 Hz), 4.39 (0.5H, dd, J=10.0 and 2.0 Hz), 5.33-5.37 (1H, m), 5.68 (0.5H, dd, J=10.0 and 3.5 Hz), 5.79-5.89 (1.5H, m), 6.01 (0.5H, t, J=10.0 Hz), 6.24 (0.5H, d, J=7.8 Hz), 6.30 (0.5H, t, J=10.0 Hz), 6.91 (0.5H, d, J=3.5 Hz), 7.24-7.70 (15H, m), 7.80-8.20 (10H, m);
MS (FAB) m/z: 737 (M+Na)⁺.

### (6c) 2,3,4,6-Tetra-O-benzoyl-7-deoxy-L-glycero-α,β-D-gluco-heptopyranoside

The compound synthesized in (6b) (13.7 g, 19.2 mmol), hydrazine acetate (3.53 g, 38.3 mmol), and N,N-dimethylformamide (140 mL) were stirred at room temperature for 7 h. The mixture was diluted with ethyl acetate (200 mL) with ice cooling and washed with 10% brine (150 mL × 3), water (100 mL × 2), and saturated brine (50 mL), and then the solvent was evaporated under reduced pressure. The residue was purified by silica gel flash column chromatography (hexane:ethyl acetate, 2:1 to 3:2, v/v) to obtain the title compound (9.82 g, 83.9%) as a white solid.
¹H NMR (500 MHz, CDCl₃):δ 1.49 (2.7H, d, J=6.8 Hz), 1.54 (0.3H, d, J=6.8 Hz), 3.10 (0.9H, brs), 3.94-3.96 (0.1H, m), 4.00 (0.1H, dd, J=9.8 and 2.5 Hz), 4.49 (0.9H, dd, J=10.3 and 2.0 Hz), 5.04-5.08 (0.1H, m), 5.32-5.38 (2H, m), 5.70-5.76 (1H, m), 5.83-5.84 (0.9H, m), 5.94 (0.1H, t, J=9.8 Hz), 6.22 (0.9H, t, J=9.8 Hz), 7.24-7.41 (9H, m), 7.45-7.55 (3H, m), 7.80-7.88 (4H, m), 7.98-8.02 (4H, m);
MS (FAB) m/z: 611 (M+H)⁺.

### (6d) 5-Acetoxymethyl-2-(4-ethylbenzyl)phenyl 2,3,4,6-tetra-O-benzoyl-7-deoxy-L-glycero-β-D-gluco-heptopyranoside

The compound synthesized in (6c) (3.00 g, 4.91 mmol), trichloroacetonitrile (0.98 mL, 9.80 mmol), 1,8-diazabicyclo[5.4.0]-7-undecene (75 µL, 0.50 mmol), and methylene chloride (30 mL) were used to prepare an imidate by the same technique as in (1b), and subsequently 5-acetoxymethyl-2-(4-ethylbenzyl)phenol (1.40 g, 4.92 mmol), a boron trifluoride-diethyl ether complex (0.62 mL, 4.90 mmol), and methylene chloride (30 mL) were used to obtain a crude product of the title compound (3.15 g) by the same technique as in (1b). The product was used in the subsequent reaction as it was.

### (6e) 2-(4-Ethylbenzyl)-5-hydroxymethylphenyl 7-deoxy-L-glycero-β-D-gluco-heptopyranoside

The compound synthesized in (6c) (2.12 g, 2.42 mmol) was dissolved in tetrahydrofuran (12 mL) and methanol (12 mL), followed by addition of aqueous sodium hydroxide (2 M, 7.3 mL, 14.6 mmol) with ice cooling, and the mixture was allowed to stand overnight at room temperature. Hydrochloric acid (2 M, 0.85 mL) was added with ice cooling, the solvent was evaporated under reduced pressure, followed by addition of water (50 mL), and the mixture was extracted with ethyl acetate (100 mL × 3). The organic layer was washed with saturated aqueous sodium hydrogencarbonate (30 mL) and then dried over anhydrous sodium sulfate, and the solvent was evaporated under reduced pressure. The residue was purified by silica gel flash column chromatography (methylene chloride:methanol, 7:1 to 6:1, v/v) to obtain the title compound (870 mg, 86.1 %) as a white solid.
¹H NMR (500MHz, MeOH-d4):δ1.19 (3H, t, J=7.6 Hz), 1.31 (3H, d, J=6.9 Hz), 2.58 (2H, q, J=7.6 Hz), 3.21 (1H, dd, J=9.4 and 1.8 Hz), 3.43-3.53 (2H, m), 3.62 (1H, t, J=9.3 Hz), 3.94 (1H, d, J=14.9 Hz), 4.03 (1H, d, J=14.9 Hz), 4.09-4.13 (1H, m), 4.54 (2H, s), 4.89 (1H, d, J=7.8 Hz), 6.91 (1H, d, J=7.8 Hz), 7.02 (1H, d, J=7.8 Hz), 7.06 (2H, d, J=7.8 Hz), 7.13 (2H, d, J=7.8 Hz), 7.14 (1H, s)
MS (FAB) m/z: 457 (M+K)⁺.

### (Example 7) 2-(4-Ethylbenzyl)-5-hydroxymethylphenyl 7-deoxy-D-glycero-β-D-gluco-heptopyranoside (Example Compound No. 1-59)

### (7a) Benzyl 2,3,4-tri-O-benzyl-7-deoxy-β-D-glucoheptopyranosid-6-ulose

A solution of dimethyl sulfoxide (7.50 mL, 106 mmol) in methylene chloride (20 mL) was added dropwise to a solution of oxalyl chloride (4.60 mL, 53.6 mmol) in methylene chloride (180 mL) at -70°C. The mixture was stirred at the same temperature for 20 min, and then the solution of diastereomer mixture (19.6 g, 35.3 mmol) in methylene chloride (190 mL) obtained in (6a) was added dropwise at - 70°C. The mixture was heated to -45°C, triethylamine (29.6 mL, 212 mmol) was added dropwise, and the mixture was heated to room temperature. Water (200 mL) was added with ice cooling to terminate the reaction, and the reaction mixture was washed successively with hydrochloric acid (1 M, 200 mL) and saturated aqueous sodium hydrogencarbonate (100 mL). The organic layer was dried over anhydrous magnesium sulfate, then the solvent was evaporated under reduced pressure, and the residue was purified by silica gel flash column chromatography (hexane:ethyl acetate, 6:1 to 4:1, v/v) to obtain the title compound (16.6 g, 85.1%) as a white solid.
¹H NMR (400 MHz, CDCl₃):δ 2.15 (3H, s), 3.50-3.57 (1H, m), 3.66-3.72 (2H, m), 3.75-3.81 (1H, m), 4.58 (1H, d, J=7.4 Hz), 4.62 (1H, d, J=10.6 Hz), 4.67 (1H, d, J=12.1 Hz), 4.72 (1H, d, J=11.0 Hz), 4.76 (1H, d, J=10.6 Hz), 4.79 (1H, d, J=11.0 Hz), 4.89-4.95 (3H, m), 7.22-7.37 (20H, m);
MS (FAB) m/z: 575 (M+Na)⁺.

### (7b) Benzyl 2,3,4-tri-O-benzyl-7-deoxy-D-glycero-β-D-gluco-heptopyranoside

The ketone synthesized in (7a) (16.6 g, 30.0 mmol) was dissolved in tetrahydrofuran (170 mL) and ethanol (32 mL), sodium borohydride (1.70 g, 44.9 mmol) was added at -70°C, and the mixture was heated to room temperature and then allowed to stand for 18 h. Water (100 mL) was added with ice cooling to terminate the reaction, and the mixture was extracted with ethyl acetate (200 mL). The organic layer was washed with saturated aqueous sodium hydrogencarbonate (50 mL) and saturated brine (50 mL) and dried over anhydrous magnesium sulfate, and then the solvent was evaporated under reduced pressure. The residue was recrystallized from ethyl acetate (32 mL) and hexane (170 mL) to obtain the title compound (5.83 g, 35.1%) as a white solid.
Rf 0.47 (hexane:ethyl acetate = 2:1);
¹H NMR (400 MHz, CDCl₃):δ 1.15 (3H, d, J=6.2 Hz), 2.55 (1H, d, J=5.8 Hz), 3.26 (1H, dd, J=9.8 and 4.7 Hz), 3.47-3.51 (2H, m), 3.69 (1H, t, J=9.0 Hz), 3.94-4.02 (1H, m), 4.53 (1H, d, J=7.4 Hz), 4.64 (1H, d, J=11.0 Hz), 4.71 (1H, d, J=12.1 Hz), 4.72 (1H, d, J=10.9 Hz), 4.77 (1H, d, J=10.9 Hz), 4.89 (1H, d, J=12.1 Hz), 4.92-4.98 (3H, m), 7.24-7.39 (20H, m);
MS (FAB) m/z: 577 (M+Na)⁺.

### (7c) Benzoyl 2,3,4,6-tetra-O-benzoyl-7-deoxy-D-glycero-α,β-D-gluco-heptopyranoside

The compound synthesized in (7b) (5.83 g, 10.5 mmol) was dissolved in ethyl acetate (24 mL) and methanol (116 mL), dilute hydrochloric acid (2 M, 0.26 mL, 0.52 mmol) and palladium hydroxide on carbon (20% by weight Pd, wet, 1.20 g) were successively added with ice cooling, and the mixture was stirred under a hydrogen atmosphere at room temperature for 2 h. Triethylamine (0.30 mL, 2.1 mmol) was added, then the mixture was filtered using Celite, and the solvent was evaporated under reduced pressure. The resulting residue was dissolved in pyridine (58 mL), benzoyl chloride (7.30 mL, 62.8 mmol) was added at room temperature, and the mixture was stirred overnight at room temperature. Water (0.57 mL, 32 mmol) was added with ice cooling, the mixture was stirred at room temperature for 2 h and then diluted with ethyl acetate (100 mL), and the resulting insoluble matters were removed by filtration. The mother liquor was concentrated under reduced pressure, followed by addition of water (50 mL), and the mixture was extracted with ethyl acetate (100 mL). The organic layer was washed successively with hydrochloric acid (1 M, 20 mL), saturated aqueous sodium hydrogencarbonate (20 mL), and saturated brine (20 mL) and dried over anhydrous magnesium sulfate. The solvent was evaporated under reduced pressure, and the residue was purified by silica gel flash column chromatography (hexane:ethyl acetate, 4:1 to 3:2, v/v) to obtain the title compound (7.50 g, 100%) as a white solid.
¹H NMR (400 MHz, CDCl₃):δ 1.51 (1.8H, d, J=6.3 Hz), 1.52 (1.2H, d, J=6.3 Hz), 4.34 (0.6H, dd, J=9.8 and 3.1 Hz), 4.58 (0.4H, dd, J=10.4 and 2.5 Hz), 5.32-5.42 (1H, m), 5.64 (0.4H, dd, J=10.4 and 3.9 Hz), 5.72-5.77 (1H, m), 5.82 (0.6H, dd, J=9.8 and 8.0 Hz), 6.01 (0.6H, t, J=9.8 Hz), 6.26 (0.6H, d, J=8.0 Hz), 6.28 (0.4H, t, J=10.4 Hz), 6.85 (0.4H, d, J=3.9 Hz), 7.24-7.63 (15H, m), 7.84-8.10 (10H, m);
MS (FAB) m/z: 737 (M+Na)⁺.

### (7d) 2,3,4,6-Tetra-O-benzoyl-7-deoxy-D-glycero-α,β-D-gluco-heptopyranoside

The compound synthesized in (7c) (5.83 g, 10.5 mmol) was dissolved in N,N-dimethylformamide (47 mL), followed by addition of hydrazine acetate (1.20 g, 13.0 mmol), and the mixture was stirred at room temperature for 7 h. The mixture was diluted with ethyl acetate (200 mL) with ice cooling and washed with 10% brine (150 mL × 3), water (100 mL × 2), and saturated brine (50 mL), and then the solvent was evaporated under reduced pressure. The residue was purified by silica gel flash column chromatography (hexane:ethyl acetate, 2:1 to 3:2, v/v) to obtain the title compound (3.51 g, 88.2%) as a white solid.
¹H NMR (400 MHz, CDCl₃):δ 1.50 (2.4H, d, J=6.7 Hz), 1.55 (0.6H, d, J=6.2 Hz), 2.96 (0.8H, d, J=4.0 Hz), 3.83 (0.2H, d, J=8.6 Hz), 4.17 (0.2H, dd, J=10.0 and 2.5 Hz), 4.66 (0.8H, dd, J=10.0 and 2.6 Hz), 5.04 (0.2H, t, J=8.6 Hz), 5.26-5.30 (1H, m), 5.32-5.41 (1H, m), 5.62 (0.2H, t, J=10.0 Hz), 5.65 (0.8H, t, J=10.0 Hz), 5.77 (0.8H, t, J=4.0 Hz), 5.94 (0.2H, t, J=10.0 Hz), 6.22 (0.8H, t, J=10.0 Hz), 7.24-7.45 (9H, m), 7.48-7.56 (3H, m), 7.83-7.88 (2H, m), 7.92-8.00 (4H, m), 8.04-8.06 (2H, m);
MS (FAB) m/z: 611 (M+H)⁺.

### (7e) 5-Acetoxymethyl-2-(4-ethylbenzyl)phenyl 2,3,4,6-tetra-O-benzoyl-7-deoxy-D-glycero-β-D-gluco-heptopyranoside

The compound synthesized in (7d) (2.49 g, 4.08 mmol), trichloroacetonitrile (0.82 mL, 8.18 mmol), 1,8-diazabicyclo[5.4.0]-7-undecene (60 µL, 0.50 mmol), and methylene chloride (50 mL) were used to prepare an imidate by the same technique as in (1b), and subsequently 5-acetoxymethyl-2-(4-ethylbenzyl)phenol (1.05 g, 3.71 mmol), a boron trifluoride-diethyl ether complex (0.52 mL, 4.10 mmol), and methylene chloride (25 mL) were used to obtain a crude product of the title compound (2.64 g) by the same technique as in (1b). The product was used in the subsequent reaction as it was.

### (7f) 2-(4-Ethylbenzyl)-5-hydroxymethylphenyl-7-deoxy-D-glycero-β-D-gluco-heptopyranoside

The compound synthesized in (7e) (2.64 g, 3.01 mmol) was dissolved in tetrahydrofuran (5.2 mL) and methanol (26 mL), followed by addition of sodium methoxide (120 mg, 0.621 mmol) with ice cooling, and the mixture was allowed to stand overnight at room temperature. Acetic acid (72 mg, 1.2 mmol) was added with ice cooling to terminate the reaction, and the solvent was evaporated under reduced pressure. The residue was purified by silica gel flash column chromatography (methylene chloride:methanol, 7:1 to 6:1, v/v) to obtain the title compound (850 mg, 67.4%) as a white solid.
¹H NMR (400MHz, CD₃OD):δ1.19 (3H, t, J=7.7 Hz), 1.22 (3H, d, J=6.2 Hz), 2.58 (2H, q, J=7.7 Hz), 3.38-3.36 (2H, m), 3.43-3.50 (2H, m), 3.95 (1H, d, J=15.1 Hz), 4.03 (1H, d, J=15.1 Hz), 4.04-4.07 (1H, m), 4.54 (2H, s), 4.91 (1H, d, J=7.9 Hz), 6.92 (1H, d, J=7.5 Hz), 7.02 (1H, d, J=7.5 Hz), 7.06 (2H, d, J=7.8 Hz), 7.13 (2H, d, J=7.8 Hz), 7.14 (1H, s)
MS (FAB) m/z: 457 (M+K)⁺.

### (Example 8) 2-(4-Methoxybenzyl)-5-hydroxymethylphenyl 7-deoxy-L-glycero-β-D-gluco-heptopyranoside (Example Compound No. 1-80)

### (8a) 5-Hydroxymethyl-2-(4-methoxybenzyl)phenol

1-Bromo-4-methoxybenzene (2.60 mL, 20.7 mmol), metal magnesium (500 mg, 20.5 mmol), a catalytic amount of iodine, and tetrahydrofuran (12 mL) were used to prepare Grignard reagent by a usual method. The obtained Grignard reagent was added to a solution of ethyl 4-formyl-3-hydroxybenzoate (1.0 g, 5.15 mmol) in tetrahydrofuran (12 mL), and the mixture was stirred at -50°C for 20 min. Saturated aqueous ammonium chloride (50 mL) was added to the reaction mixture, and the mixture was extracted with ethyl acetate (40 mL) and then washed with saturated brine (50 mL). The organic layer was dried over anhydrous sodium sulfate, and then the solvent was evaporated under reduced pressure. The resulting crude product was used in the subsequent reaction as it was.

The crude product was dissolved in methanol (20 mL), followed by addition of concentrated hydrochloric acid (0.46 mL) and 10% palladium on carbon (320 mg), and the mixture was stirred under a hydrogen atmosphere at room temperature for 30 h. Methylene chloride (2 mL) was added to the reaction mixture, the mixture was stirred for 10 min, 10% palladium on carbon was removed by filtration, and the solvent was evaporated under reduced pressure. The residue was diluted with ethyl acetate (20 mL) and washed with saturated aqueous sodium hydrogencarbonate (20 mL) and saturated brine (20 mL). The organic layer was dried over anhydrous sodium sulfate, and then the solvent was evaporated under reduced pressure. The resulting crude product was used in the subsequent reaction as it was. The crude product was dissolved in tetrahydrofuran (16 mL), followed by addition of lithium aluminium hydride (490 mg) with ice cooling, and the mixture was stirred at room temperature for 30 min. Subsequently, 2 mol/L hydrochloric acid (30 mL) was added with ice cooling, and the mixture was extracted with ethyl acetate (40 mL) and washed with saturated aqueous sodium hydrogencarbonate (50 mL) and saturated brine (50 mL). The organic layer was dried over anhydrous sodium sulfate, and then the solvent was evaporated under reduced pressure. The residue was purified by silica gel flash column chromatography (methylene chloride:methanol, 30:1, v/v) to obtain the title compound (930 mg, yield 74%) as a colorless solid.
¹H NMR (400MHz, DMSO-d6):δ3.70 (3H, s), 3.76 (2H, s), 4.37 (2H, d, J=5.5 Hz), 5.03 (1H, t, J=5.5Hz), 6.64 (1H, d, J=7.6 Hz), 6.79 (1H, s), 6.82 (2H, d, J=8.4 Hz), 6.93 (1H, d, J=7.6 Hz), 7.11 (2H, d, J=8.4 Hz), 9.28 (1H, s)
MS (EI⁺) m/z: 244 (M)⁺.

### (8b) 5-Acetoxymethyl-2-(4-methoxybenzyl)phenol

The compound synthesized in (8a) (908 mg, 3.02 mmol), tetrahydrofuran (9 m), vinyl acetate (9 mL), and bis(dibutylchlorotin)oxide (83 mg, 0.15 mmol) were stirred at 30°C for 16 h. The solvent was evaporated under reduced pressure, and then the residue was purified by silica gel flash column chromatography (hexane:ethyl acetate, 30:1 to 5:1, v/v) to obtain the title compound (760 mg, yield 70%) as a colorless solid.
¹H NMR (400MHz, CDCl₃):δ2.09 (3H, s), 3.78 (3H, s), 3.92 (2H, s), 5.03 (2H, s), 6.80 (1H, d, J=1.5 Hz), 6.83 (2H, d, J=8.6 Hz), 6.87 (1H, dd, J=7.8 and 1.5 Hz), 7.09 (1H, d, J=7.8 Hz), 7.14 (2H, d, J=8.6 Hz)
MS (EI⁺) m/z: 286 (M)⁺.

### (8c) 5-Acetoxymethyl-2-(4-methoxybenzyl)phenyl 2,3,4,6-tetra-O-benzoyl-7-deoxy-L-glycero-β-D-gluco-heptopyranoside

The compound synthesized in (6c) (2.00 g, 3.28 mmol), trichloroacetonitrile (0.66 mL, 6.58 mmol), 1,8-diazabicyclo[5.4.0]-7-undecene (50 µL, 0.33 mmol), and methylene chloride (40 mL) were used to prepare an imidate by the same technique as in (1b), and subsequently 5-acetoxymethyl-2-(4-methoxybenzyl)phenol synthesized in (8b) (850 mg, 2.97 mmol), a boron trifluoride-diethyl ether complex (0.42 mL, 3.31 mmol), and methylene chloride (20 mL) were used to obtain a crude product of the title compound (1.93 g) by the same technique as in (1a). The obtained product was used in the subsequent reaction as it was.

### (8d) 2-(4-Methoxybenzyl)-5-hydroxymethylphenyl 7-deoxy-L-glycero-β-D-gluco-heptopyranoside

The compound synthesized in (8c) (1.93 g, 2.20 mmol), sodium methoxide (170 mg, 0.881 mmol), tetrahydrofuran (3.8 mL), methanol (19 mL), and acetic acid (75 mg, 1.2 mmol) were used to obtain the title compound (780 mg, 84.5%) by the same method as in (7f). However, purification was performed by silica gel flash column chromatography (methylene chloride:methanol, 7:1 to 6:1, v/v).
¹H NMR (400MHz, CD₃OD):δ1.32 (3H, d, J=6.6 Hz), 3.22 (1H, dd, J=9.6 and 1.7 Hz), 3.43-3.54 (2H, m), 3.63 (1H, t, J=9.1 Hz), 3.74 (3H, s), 3.92 (1H, d, J=14.3 Hz), 4.01 (1H, d, J=14.3 Hz), 4.12 (1H, dd, J=6.6 and 1.7 Hz), 4.54 (2H, s), 4.90 (1H, d, J=7.4 Hz), 6.80 (2H, d, J=8.8 Hz), 6.92 (1H, d, J=7.8 Hz), 7.03 (1H, d, J=7.8 Hz), 7.14 (1H, s), 7.15 (2H, d, J=8.8 Hz)
MS (FAB) m/z: 459 (M+K)⁺.

### (Example 9) 2-(4-Methoxybenzyl)-5-hydroxymethylphenyl 7-deoxy-D-glycero-β-D-gluco-heptopyranoside (Example Compound No. 1-80)

### (9a) 5-Acetoxymethyl-2-(4-methoxybenzyl)phenyl 2,3,4,6-tetra-O-benzoyl-7-deoxy-D-glycero-β-D-gluco-heptopyranoside

The compound synthesized in (7d) (1.30 g, 2.13 mmol), trichloroacetonitrile (0.43 mL, 4.29 mmol), 1,8-diazabicyclo[5.4.0]-7-undecene (30 µL, 0.20 mmol), and methylene chloride (26 mL) were used to prepare an imidate by the same technique as in (1b), and subsequently 5-acetoxymethyl-2-(4-methoxybenzyl)phenol synthesized in (8b) (550 mg, 1.92 mmol), a boron trifluoride-diethyl ether complex (0.25 mL, 1.97 mmol), and methylene chloride (13 mL) were used to obtain a crude product of the title compound (1.54 g) by the same technique as in (1b). The obtained product was used in the subsequent reaction as it was.

### (9b) 2-(4-Methoxybenzyl)-5-hydroxymethylphenyl 7-deoxy-D-glycero-β-D-gluco-heptopyranoside

The compound synthesized in (9a) (1.54 g, 1.75 mmol), sodium methoxide (68 mg, 0.35 mmol), tetrahydrofuran (3 mL), methanol (26.5 mL), and acetic acid (40 mg, 0.67 mmol) were used to obtain the title compound (600 mg, 81.4%) by the same method as in (7f). However, purification was performed by silica gel flash column chromatography (methylene chloride:methanol, 7:1 to 6:1, v/v).
¹H NMR (400MHz, CD₃OD):δ1.22 (3H, d, J=6.7 Hz), 3.36-3.38 (2H, m), 3.45-3.48(2H, m), 3.74 (3H, s), 3.92 (1H, d, J=15.1 Hz), 4.00 (1H, d, J=15.1 Hz), 4.04-4.07 (1H, m), 4.54 (2H, s), 4.91(1H, d, J=7.5 Hz), 6.79 (2H, d, J=8.8 Hz), 6.92 (1H, d, J=7.8 Hz), 7.01 (1H, d, J=7.8 Hz), 7.13 (1H, s), 7.14 (2H, d, J=8.8 Hz)
MS (FAB) m/z: 459 (M+K)⁺.

### (Example 10) 2-(4-Trifluoromethoxybenzyl)-5-hydroxymethylphenyl 7-deoxy-D-glycero-β-D-gluco-heptopyranoside (Example Compound No. 1-103)

### (10a) 5-Hydroxymethyl-2-(4-trifluoromethoxybenzyl)phenol

1-Bromo-4-trifluoromethoxybenzene (3.00 mL, 20.4 mmol), metal magnesium (500 mg, 20.5 mmol), a catalytic amount of iodine, and tetrahydrofuran (12 mL) were used to prepare Grignard reagent by a usual method. The resulting Grignard reagent was added to a solution of ethyl 4-formyl-3-hydroxybenzoate (1.0 g, 5.15 mmol) in tetrahydrofuran (12 mL), and the mixture was stirred at -50°C for 20 min. Saturated aqueous ammonium chloride (50 mL) was added to the reaction mixture, and the mixture was extracted with ethyl acetate (40 mL) and then washed with saturated brine (50 mL). The organic layer was dried over anhydrous sodium sulfate, and then the solvent was evaporated under reduced pressure. The resulting crude product was used in the subsequent reaction as it was.

The crude product was dissolved in methanol (34 mL), followed by addition of concentrated hydrochloric acid (0.40 mL) and 10% palladium on carbon (170 mg), and the mixture was stirred under a hydrogen atmosphere at room temperature for 30 h. Methylene chloride (2 mL) was added to the reaction mixture, the mixture was stirred for 10 min, 10% palladium on carbon was removed by filtration, and the solvent was evaporated under reduced pressure. The mixture was diluted with ethyl acetate (20 mL) and washed with saturated aqueous sodium hydrogencarbonate (20 mL) and saturated brine (20 mL). The organic layer was dried over anhydrous sodium sulfate, and then the solvent was evaporated under reduced pressure. The resulting crude product was used in the subsequent reaction as it was.

The crude product was dissolved in tetrahydrofuran (16 mL), followed by addition of lithium aluminium hydride (540 mg) with ice cooling, and the mixture was stirred at room temperature for 30 min. Subsequently, pure water (0.54 mL), 15% aqueous sodium hydroxide (0.54 mL), and pure water(1.62 mL) were added with ice cooling, and the mixture was stirred at room temperature for 1 h and allowed to stand overnight at room temperature. Solids were removed by Celite filtration, and the solvent was evaporated under reduced pressure. The residue was purified by silica gel flash column chromatography (methylene chloride:methanol, 30:1, v/v) to obtain the title compound (908 mg, yield 59%) as a colorless solid.
¹H NMR (400MHz, CD₃OD):δ4.50 (2H, s), 4.50 (2H, s), 6.76 (1H, d, J=7.9 Hz), 6.82 (1H, s), 7.01 (1H, d, J=7.9 Hz), 7.12 (2H, d, J=8.3 Hz), 7.29 (2H, d, J=8.3 Hz) MS (FAB⁺) m/z: 298 (M)⁺.

### (10b) 5-Acetoxymethyl-2-(4-trifluoromethoxybenzyl)phenol

The compound synthesized in (10a) (908 mg, 3.02 mmol) was dissolved in tetrahydrofuran (9 mL), followed by addition of vinyl acetate (9 mL) and bis(dibutylchlorotin)oxide (83 mg, 0.15 mmol), and the mixture was stirred at 30°C for 16 h. The solvent was evaporated under reduced pressure, and then the residue was purified by silica gel flash column chromatography (hexane:ethyl acetate, 30:1 to 5:1, v/v) to obtain the title compound (990 mg, yield 96%) as a colorless solid.
¹H NMR (400MHz, CDCl₃):δ2.10 (3H, s), 3.98 (2H, s), 4.95 (1H, s), 5.04 (2H, s), 6.80 (1H, d, J=1.7 Hz), 6.89 (1H, dd, J=7.7 and 1.7 Hz), 7.10 (1H, d, J=7.7 Hz), 7.13 (2H, d, J=8.3 Hz), 7.24 (2H, d, J=8.3 Hz)
MS (FAB⁺) m/z: 298 (M)⁺.

### (10c) 5-Acetoxymethyl-2-(4-trifluoromethoxybenzyl)phenyl 2,3,4,6-tetra-O-benzoyl-7-deoxy-D-glycero-β-D-gluco-heptopyranoside

The compound synthesized in (7d) (640 mg, 1.04 mmol), trichloroacetonitrile (0.52 mL, 3.60 mmol), 1,8-diazabicyclo[5.4.0]-7-undecene (16 µL, 0.33 mmol), and methylene chloride (10 mL) were used to prepare an imidate by the same technique as in (1b), and subsequently the compound synthesized in (10b) (340 mg, 1.00 mmol), a boron trifluoride-diethyl ether complex (0.13 mL, 1.03 mmol), and methylene chloride (10 mL) were used to obtain the title compound (250 mg, yield 26%) by the same technique as in (1b) as a colorless amorphous compound.
¹H NMR (400MHz,CDCl₃):δ1.57 (3H, d, J=6.7 Hz), 1.99 (3H, s), 3.77 (1H, d, J=15.3 Hz), 3.84 (1H, d, J=15.3 Hz), 4.37 (1H, dd, J=9.8 and 2.8 Hz), 4.70 (1H, d, J=12.5 Hz), 4.84 (1H, d, J=12.5 Hz), 5.40 (1H, dd, J=6.7 and 2.8 Hz), 5.45 (1H, d, J=7.9 Hz), 5.70 (1H, t, J=9.8 Hz), 5.86 (1H, dd, J=9.8 and 7.9 Hz), 6.00 (1H, t, J=9.8 Hz), 6.90-7.06 (7H, m), 7.27-7.56 (12H, m), 7.82-8.01 (8H, m)
MS (FAB⁺) m/z: 955 (M+Na)⁺.

### (10d) 2-(4-Trifluoromethoxybenzyl)-5-hydroxymethylphenyl 7-deoxy-D-glycero-β-D-gluco-heptopyranoside

The compound synthesized in (10c) (250 mg, 0.27 mmol), potassium carbonate (370 mg, 2.68 mmol), methanol (8 mL), and methylene chloride (2 mL) were used to obtain the title compound (65 mg, yield 51%) by the same technique as in (1c) as a colorless solid.
¹H NMR (400MHz, CD₃OD):δ1.21 (3H, d, J=6.2 Hz), 3.36-3.37 (2H, m), 3.46-3.47 (2H, m), 4.00 (1H, d, J=14.9 Hz), 4.05 (1H, dd, J=6.5 and 3.7 Hz), 4.11 (1H, d, J=14.9 Hz), 4.54 (2H, s), 4.93 (1H, d, J=7.8 Hz), 6.95 (1H, d, J=7.6 Hz), 7.08 (1H, d, J=7.6 Hz), 7.11 (2H, d, J=8.8 Hz), 7.16 (1H, s), 7.34 (2H, d, J=8.8 Hz)
MS (FAB) m/z: 513 (M+K)⁺.

### (Example 11) 3-(4-Ethylbenzyl)-4,6-dimethylpyridin-2-yl 7-deoxy-D-glycero-β-D-gluco-heptopyranoside (Example Compound No. 1-53)

### (11a) 3-(4-Ethylbenzyl)-4,6-dimethylpyridin-2-yl 2,3,4,6-tetra-O-benzoyl-7-deoxy-D-glycero-β-D-gluco-heptopyranoside

The compound synthesized in (7d) (200 mg, 0.28 mmol) was dissolved in methylene chloride (6 mL), followed by addition of 30% hydrobromide-acetic acid solution (0.40 mL) with ice cooling, and the mixture was stirred at room temperature for 22 h. The mixture was diluted with toluene (6 mL), and the solvent was evaporated under reduced pressure. Subsequently, the residue was diluted with ethyl acetate (10 mL) and washed with saturated aqueous sodium hydrogencarbonate (10 mL) and saturated brine (10 mL). The organic layer was dried over anhydrous sodium sulfate, and then the solvent was evaporated under reduced pressure. Toluene (2 mL) was added, and the mixture was dehydrated azeotropically under reduced pressure to obtain a bromo sugar (140 mg) as a yellow amorphous compound. The resulting bromo sugar was used in the subsequent reaction without purification.

3-(4-Ethylbenzyl)-4,6-dimethyl-2-hydroxypyridine (EP1405859A1) (50 mg, 0.21 mmol) and bromo sugar (140 mg, 0.21 mmol) were dissolved in methylene chloride (2 mL), followed by addition of silver carbonate (57 mg, 0.21 mmol), and the mixture was stirred with light shielding at room temperature for 12 days. Solids were removed from the reaction mixture by Celite filtration, the filtrate was washed with ethyl acetate, and then the solvent was evaporated under reduced pressure. The starting material was removed from the residue by silica gel flash column chromatography (hexane:ethyl acetate, 3:1, v/v), and the resulting crude product of the title compound (130 mg) was used in the subsequent reaction as it was.

### (11b) 3-(4-Ethylbenzyl)-4,6-dimethylpyridin-2-yl 7-deoxy-D-glycero-β-D-gluco-heptopyranoside

The crude product of the compound synthesized in (11a) (130 mg, 0.16 mmol) was dissolved in methanol-methylene chloride (6 mL/1.5 mL), followed by addition of potassium carbonate (210 mg, 1.51 mmol), and the mixture was stirred overnight at room temperature. Excess potassium carbonate was removed by Celite filtration, then the mixture was neutralized by adding an appropriate amount of acetic acid, and the solvent was removed under reduced pressure. The residue was dissolved in ethyl acetate (10 mL) and washed with saturated aqueous sodium hydrogencarbonate (10 mL) and saturated brine (10 mL). The organic layer was dried over anhydrous sodium sulfate, and then the solvent was evaporated under reduced pressure. The residue was crystallized from hexane-methylene chloride to obtain the title compound (18 mg, yield 18%) as a colorless solid.
¹H NMR (400MHz, CD₃OD):δ1.16 (3H, d, J=7.4 Hz), 1.17 (3H, t, J=7.6 Hz), 2.18 (3H, s), 2.35 (3H, s), 2.56 (2H, q, J=7.6 Hz), 3.33-3.39 (2H, m), 3.43-3.50 (2H, m), 3.89(1H, d, J=15.3 Hz), 3.96-4.02 (1H, m), 4.09 (1H, d, J=15.3 Hz), 5.89 (1H, d, J=7.8 Hz), 6.72 (1H, s), 7.03 (2H, d, J=7.8 Hz), 7.10 (2H, d, J=7.8 Hz)
MS (FAB) m/z: 418 (M+H)⁺.

### (Example 12) 3-(4-Ethylbenzyl)-6-methylpyridin-2-yl 7-deoxy-D-glycero-β-D-gluco-heptopyranoside (Example Compound No. 1-50)

### (12a) 2-Benzyloxy-6-methylnicotinaldehyde

2-Benzyloxy-6-methylnicotinonitrile (4.0 g, 17.8 mmol) was dissolved in tetrahydrofuran (8 mL), and the mixture was added dropwise to 1.01 mol/L diisobutylaluminium hydride in toluene (44 mL, 44 mmol) with ice cooling, and stirred with ice cooling for 6 h. MeOH (2 ml) was added to the reaction mixture, the mixture was stirred for 10 min, followed by addition of 2 N hydrochloric acid (50 mL), the mixture was diluted with ethyl acetate (100 mL) and separated, and the resulting oil layer was washed with saturated aqueous sodium hydrogencarbonate (100 mL) and saturated brine (100 mL). The organic layer was dried over anhydrous sodium sulfate, and then the solvent was evaporated under reduced pressure. The residue was purified by silica gel flash column chromatography (hexane:ethyl acetate, 20:1, v/v) to obtain an oily title compound (2.33 g, yield 58%). ¹H NMR (400MHz,CDCl₃):δ2.53 (3H, s), 5.54 (2H, s), 6.87 (1H, d, J=7.8 Hz), 7.33 - 7.51 (5H, m), 8.03 (1H, d, J=7.8 Hz), 10.4 (1H, s)
MS (FAB) m/z: 228 (M+H)⁺.

### (12b) 2-Benzyloxy-6-methylpyridin-3-yl-4-ethylphenyl methanol

1-Bromo-4-ethylbenzene (0.54 mL, 3.90 mmol) was dissolved in tetrahydrofuran (20 mL), the mixture was cooled to -78°C, followed by addition of a solution of 1.42 mol/L t-butyllithium in n-pentane (5.54 mL, 7.87 mmol), and stirred for 30 min. The reaction mixture was added to a solution of the compound synthesized in (12a) (688 mg, 3.03 mmol) in tetrahydrofuran (10 mL), and the mixture was heated to 0°C and stirred for 30 min. Saturated aqueous ammonium chloride (20 mL) was added to the reaction mixture, the aqueous layer was extracted with ethyl acetate (20 mL), and the oil layer was washed with saturated brine (20 mL). The organic layer was dried over anhydrous sodium sulfate, and then the solvent was evaporated under reduced pressure. The residue was purified by silica gel flash column chromatography (hexane:ethyl acetate, 30:1 to 10:1, v/v) to obtain the title compound (900 mg, yield 89%) as a colorless solid.
¹H NMR (400MHz,CDCl₃):δ1.23 (3H, t, J=7.6 Hz), 2.43 (3H, s), 2.64 (2H, q, J=7.8 Hz), 2.81 (1H, d, J=5.3 Hz), 5.38 (2H, s), 5.93 (1H, d, J=5.3 Hz), 6.74 (1H, d, J=7.5 Hz), 7.14 (2H, d, J=8.2 Hz), 7.24-7.32 (7H, m), 7.48 (1H, d, J=7.5 Hz)
MS (FAB) m/z: 334 (M+H)⁺.

### (12c) 2-Benzyloxy-6-methylpyridin-3-yl-4-ethylphenylmethyl acetate

The compound synthesized in (12b) (900 mg, 2.70 mmol) was dissolved in pyridine (10 mL), followed by addition of acetic anhydride (0.92 mL, 8.14 mmol) and 4-dimethylaminopyridine (32 mg, 0.26 mmol), and the mixture was stirred at room temperature for 1 h. The solvent was evaporated under reduced pressure, and the residue was diluted with ethyl acetate (20 mL) and washed with saturated aqueous ammonium chloride (20 mL) and saturated brine (20 mL). The organic layer was dried over anhydrous sodium sulfate, and then the solvent was evaporated under reduced pressure. The residue was purified by silica gel flash column chromatography (hexane:ethyl acetate, 30:1, v/v) to obtain an oily title compound (990 mg, yield 86%).
¹H NMR (400MHz,CDCl₃):δ1.22 (3H, t, J=7.7 Hz), 2.09 (3H, s), 2.42 (3H, s), 2.62 (2H, q, J=7.7 Hz), 5.37 (2H, d, J=2.0 Hz), 6.73 (1H, d, J=7.4 Hz), 7.04 (1H, s), 7.12 (2H, d, J=8.2 Hz), 7.23 (2H, d, J=8.2 Hz), 7.34-7.26 (5H, m), 7.52 (1H, d, J=7.4 Hz) MS (FAB) m/z: 376 (M+H)⁺.

### (12d) 3-(4-Ethylbenzyl)-6 methylpyridin-2-ol

The compound synthesized in (12c) (990 mg, 2.64 mmol) was dissolved in methanol-tetrahydrofuran (6 mL/1.5 mL), followed by addition of 10% palladium on carbon (99 mg), and the mixture was stirred under a hydrogen atmosphere at room temperature for 4 h. The methylene chloride (2 mL) was added to the reaction mixture, the mixture was stirred for 10 min, then 10% palladium on carbon was removed by filtration, and the solvent was evaporated under reduced pressure. The residue was diluted with ethyl acetate (20 mL) and washed with saturated aqueous sodium hydrogencarbonate (20 mL) and saturated brine (20 mL). The organic layer was dried over anhydrous sodium sulfate, and then the solvent was evaporated under reduced pressure to obtain the title compound (520 mg, yield 88%) as a colorless solid.
¹H NMR (400MHz,CDCl₃):δ1.22 (3H, t, J=7.7 Hz), 2.25 (3H, s), 2.62 (2H, q, J=7.7 Hz), 3.80 (2H, s), 5.92 (1H, d, J=7.1 Hz), 7.03 (1H, d, J=7.1 Hz), 7.13 (2H, d, J=8.0 Hz), 7.19 (2H, d, J=8.0 Hz), 11.7 (1H, brs)
MS (EI⁺) m/z: 227 (M)⁺.

### (12e) 3-(4-Ethylbenzyl)-6-methylpyridin-2-yl 2,3,4,6-tetra-O-benzoyl-7-deoxy-D-glycero-β-D-gluco-heptopyranoside

The compound synthesized in (7d) (200 mg, 0.28 mmol), methylene chloride (6 mL), and 30% hydrobromide in acetic acid (0.40 mL) were used to prepare a bromo sugar by the same method as in (11a), and subsequently 3-(4-ethylbenzyl)-2-hydroxy-4-methylpyridine (58 mg, 0.26 mmol), methylene chloride (2 mL), and silver carbonate (74 mg, 0.27 mmol) were used to obtain a crude product of the title compound (140 mg) by the same method as in (11a).

### (12f) 3-(4-Ethylbenzyl)-6-methylpyridin-2-yl 7-deoxy-D-glycero-β-D-gluco-heptopyranoside

The crude product of the compound synthesized in (12e) (140 mg, 0.17 mmol), methanol (6 mL), methylene chloride (1.5 mL), and potassium carbonate (240 mg, 1.74 mmol) were used to obtain the title compound (27 mg, yield 26%) as a colorless solid by the same method as in (11a).
¹H NMR (400MHz, CD₃OD):δ1.16 (3H, d, J=6.7 Hz), 1.19 (3H, t, J=7.7 Hz), 2.37(3H, s), 2.59 (2H, q, J=7.7 Hz), 3.36-3.37 (2H, m), 3.49-3.50 (2H, m), 3.84(1H, d, J=15.2 Hz), 3.94 (1H, d, J=15.2 Hz), 3.97-4.01 (1H, m), 5.91 (1H, d, J=7.8 Hz), 6.78 (1H, d, J=7.4 Hz), 7.09 (2H, d, J=8.0 Hz), 7.15 (2H, d, J=8.0 Hz), 7.27 (1H, d, J=7.4 Hz)
MS (FAB) m/z: 404 (M+H)⁺.

### (Example 13) 3-(4-Ethylbenzyl)pyridin-2-yl 7-deoxy-D-glycero-β-D-gluco-heptopyranoside (Example Compound No. 1-1)

### (13a) 2-Benzyloxypyridin-3-yl-4-ethylphenyl methanol

1-Bromo-4-ethylbenzene (0.78 mL, 5.05 mmol), tetrahydrofuran (25 mL), a solution of 1.42 mol/L t-butyllithium in n-pentane (7.90 mL, 11.2 mmol), and 2-benzyloxypyridine-3-carbaldehyde (1.0 g, 4.09 mmol) were used to obtain an oily title compound (520 mg, yield 35%) by the same technique as in (12b).
¹H NMR (500MHz, CDCl₃):δ1.23 (3H, t, J=7.8 Hz), 2.64 (2H, q, J=7.8 Hz), 2.75 (1H, d, J=4.9 Hz), 5.38 (2H, s), 5.96 (1H, d, J=4.9 Hz), 6.92 (1H, dd, J=7.3 and 4.9 Hz), 7.15 (2H, d, J=7.8 Hz), 7.24-7.33 (7H, m), 7.67 (1H, dd, J=7.3 and 2.0 Hz), 8.08(1H, dd, J=4.9 and 2.0 Hz)
MS (EI⁺) m/z: 319 (M)⁺.

### (13b) 2-Benzyloxypyridin-3-yl-4-ethylphenylmethyl acetate

The compound synthesized in (13a) (520 mg, 1.03 mmol), pyridine (5 mL), acetic anhydride (0.55 mL, 4.07 mmol), and 4-dimethylaminopyridine (19 mg, 0.16 mmol) were used to obtain an oily title compound (510 mg, yield 86%) by the same technique as in (12c).
¹H NMR (400MHz,CDCl₃):δ1.22 (3H, t, J=7.5 Hz), 2.10 (3H, s), 2.63 (2H, q, J=7.5 Hz), 5.37 (2H, d, J=3.1 Hz), 6.92 (1H, dd, J=7.4 and 5.0 Hz), 7.06 (1H, s), 7.13 (2H, d, J=7.8 Hz), 7.23-7.34 (7H, m), 7.68(1H, dd, J=7.4 and 1.8 Hz), 8.10 (1H, dd, J=5.0 and 1.8 Hz)
MS (FAB) m/z: 362 (M+H)⁺.

### (13c) 3-(4-Ethylbenzyl)pyridin-2-ol

The compound synthesized in (12b) (510 mg, 1.41 mmol), 10% palladium on carbon (51 mg), methanol (4 mL), and tetrahydrofuran (1 mL) were used to obtain the title compound (300 mg, yield 100%) as a colorless solid by the same technique as in (12d).
¹H NMR (400MHz,CDCl₃):δ1.23 (3H, t, J=7.4 Hz), 2.64 (2H, q, J=7.4 Hz), 3.85 (2H, s), 6.17 (1H, t, J=6.6 Hz), 7.08 (1H, dd, J=6.6 and 1.8 Hz), 7.13-7.19 (4H, m), 7.23 (1H, dd, J=6.6 and 1.8 Hz), 11.9(1H, brs)
MS (FAB) m/z: 214 (M+H)⁺.

### (13d) 3-(4-Ethylbenzyl)pyridin-2-yl 2,3,4,6-tetra-O-benzoyl-7-deoxy-D-glycero-β-D-gluco-heptopyranoside

The compound synthesized in (7d) (200 mg, 0.28 mmol), methylene chloride (6 mL), and 30% hydrobromide-acetic acid solution (0.40 mL) were used to prepare a bromo sugar by the same method as in (11a), and subsequently 3-(4-ethylbenzyl)-2-hydroxypyridine (60 mg, 0.26 mmol), methylene chloride (2 mL), and silver carbonate (92 mg, 0.33 mmol) were used to obtain a crude product of the title compound (130 mg) by the same method as in (11a).

### (13e) 3-(4-Ethylbenzyl)pyridin-2-yl 7-deoxy-D-glycero-β-D-gluco-heptopyranoside

The crude product of the compound synthesized in (13d) (130 mg, 0.16 mmol), methanol (6 mL), methylene chloride (1.5 mL), and potassium carbonate (220 mg, 1.59 mmol) were used to obtain the title compound (31 mg, yield 28%) as a colorless solid by the same method as in (11a).
¹H NMR (400MHz, MeOH-d4):δ1.15 (3H, d, J=6.3 Hz), 1.20 (3H, t, J=7.6 Hz), 2.59 (3H, q, J=7.6 Hz), 3.35-3.43 (2H, m), 3.46-3.53 (1H, m), 3.90 (1H, d, J=15.2 Hz), 4.00 (1H, d, J=15.2 Hz), 3.99-4.03 (1H, m), 5.86(1H, d, J=7.8 Hz), 6.94 (1H, dd, J=7.4 and 5.1 Hz), 7.10 (2H, d, J=8.2 Hz), 7.16 (2H, d, J=8.2Hz), 7.41 (1H, dd, J=7.4 and 1.9 Hz), 7.98 (1H, dd, J=5.1 and 1.9 Hz)
MS (FAB) m/z: 390 (M+H)⁺.

### (Example 14) 3-(4-Methoxybenzyl)-4,6-dimethylpyridin-2-yl 7-deoxy-D-glycero-β-D-gluco-heptopyranoside (Example Compound No. 1-54)

### (14a) 3-(4-Methoxybenzyl)-4,6-dimethylpyridin-2-yl 2,3,4,6-tetra-O-benzoyl-7-deoxy-D-glycero-β-D-gluco-heptopyranoside

The compound synthesized in (7d) (200 mg, 0.28 mmol), methylene chloride (6 mL), and 30% hydrobromide-acetic acid solution (0.40 mL) were used to prepare a bromo sugar by the same method as in (11a), and subsequently 3-(4-methoxybenzyl)-4,6-dimethyl-2-hydroxypyridine (EP1405859A1) (60 mg, 0.26 mmol), methylene chloride (2 mL), and silver carbonate (92 mg, 0.33 mmol) were used to obtain a crude product of the title compound (118 mg) by the same method as in (11a).

### (14b) 3-(4-Methoxybenzyl)-4,6-dimethylpyridin-2-yl 7-deoxy-D-glycero-β-D-gluco-heptopyranoside

The crude product of the compound synthesized in (14a) (118 mg, 0.14 mmol), methanol (6 mL), methylene chloride (1.5 mL), and potassium carbonate (195 mg, 1.41 mmol) were used to obtain the title compound (47 mg, yield 40%) as a colorless solid by the same method as in (11a).
¹H NMR (400MHz, CD₃OD):δ1.17 (3H, d, J=6.2 Hz), 2.18 (3H, s), 2.35 (3H, s), 3.34-3.39 (2H, m), 3.43-3.51 (2H, m), 3.72 (3H, s), 3.87 (1H, d, J=15.0 Hz), 3.96-4.02 (1H, m), 4.05 (1H, d, J=15.0Hz), 5.90 (1H, d, J=7.8 Hz), 6.72 (1H, s), 6.76 (2H, d, J=8.6 Hz), 7.12 (2H, d, J=8.6 Hz)
MS (FAB) m/z: 420 (M+H)⁺.

### (Example 15) 3-(4-Methoxybenzyl)-6-methylpyridin-2-yl 7-deoxy-D-glycero-β-D-gluco-heptopyranoside (Example Compound No. 1-52)

### (15a) 2-Benzyloxy-6-methylpyridin-3-yl-4-methoxyphenyl methanol

4-Bromoanisole (0.82 mL, 4.45 mmol), a solution of 2.64 mol/L n-butyllithium in n-hexane (1.68 mL, 4.44 mmol), tetrahydrofuran (20 mL), and the compound synthesized in (12a) (720 mg, 3.17 mmol) were used to obtain the title compound (940 mg, yield 85%) as a colorless solid by the same technique as in (12b). ¹H NMR (400MHz,CDCl₃):δ2.44 (3H, s), 2.79 (1H, d, J=5.1 Hz), 3.80 (3H, s), 5.39 (2H, s), 5.92 (1H, d, J=5.1 Hz), 6.75 (1H, d, J=7.5 Hz), 6.85 (2H, d, J=8.6 Hz), 7.23-7.35 (7H, m), 7.47 (1H, d, J=7.5 Hz)
MS (FAB) m/z: 336 (M+H)⁺.

### (15b) 2-Benzyloxy-6-methylpyridin-3-yl-4-methoxyphenylmethyl acetate

The compound synthesized in (15a) (940 mg, 2.71 mmol), pyridine (10 mL), acetic anhydride (0.92 mL, 8.14 mmol), and 4-dimethylaminopyridine (33 mg, 0.26 mmol) were used to obtain an oily title compound (1.00 g, yield 98%) by the same technique as in (12c).
¹H NMR (400MHz,CDCl₃):δ2.08 (3H, s), 2.42 (3H, s), 3.78 (3H, s), 5.36 (2H, d, J=3.5 Hz), 6.74 (1H, d, J=7.4 Hz), 6.81 (2H, d, J=8.8 Hz), 7.01(1H, s), 7.23 (2H, d, J=8.8 Hz), 7.26-7.32 (5H, m), 7.53 (1H, d, J=7.4 Hz)
MS (FAB) m/z: 378 (M+H)⁺.

### (15c) 3-(4-Methoxybenzyl)-6-methylpyridin-2-ol

The compound synthesized in (15b) (1.00 g, 2.65 mmol), 10% palladium on carbon (100 mg), methanol (6 mL), and tetrahydrofuran (1.5 mL) were used to obtain the title compound (600 mg, yield 98%) as a colorless solid by the same technique as in (12d).
¹H NMR (400MHz,CDCl₃):δ2.26 (3H, s), 3.77 (2H, s), 3.78 (3H, s), 5.92 (1H, d, J=6.9 Hz), 6.83 (2H, d, J=9.0 Hz), 7.01 (1H, d, J=6.9 Hz), 7.19 (2H, d, J=9.0 Hz), 11.9 (1H, brs)
MS (EI⁺) m/z: 229 (M)⁺.

### (15d) 3-(4-Methoxybenzyl)-6-methylpyridin-2-yl 2,3,4,6-tetra-O-benzoyl-7-deoxy-D-glycero-β-D-gluco-heptopyranoside

The compound synthesized in (7d) (200 mg, 0.28 mmol), methylene chloride (6 mL), and 30% hydrobromide-acetic acid solution (0.40 mL) were used to prepare a bromo sugar by the same method as in (11a), and subsequently 3-(4-methoxybenzyl)-2-hydroxy-4-methylpyridine (64 mg, 0.28 mmol), methylene chloride (2 mL), and silver carbonate (92 mg, 0.33 mmol) were used to obtain a crude product of the title compound (120 mg) by the same method as in (11a).

### (15e) 3-(4-Methoxybenzyl)-6-methylpyridin-2-yl 7-deoxy-D-glycero-β-D-gluco-heptopyranoside

The crude product of the compound synthesized in (15d) (120 mg, 0.15 mmol), methanol (6 mL), methylene chloride (1.5 mL), and potassium carbonate (200 mg, 1.45 mmol) were used to obtain the title compound (42 mg, yield 37%) as a colorless solid by the same method as in (11a).
¹H NMR (400MHz, CD₃OD):δ1.16 (3H, d, J=6.3 Hz), 2.37 (3H, s), 3.36-3.37 (2H, m), 3.49-3.51 (2H, m), 3.75(3H, s), 3.82(1H, d, J=15.3 Hz), 3.91 (1H, d, J=15.3 Hz), 3.96- 4.01 (1H, m), 5.91 (1H, d, J=8.2 Hz), 6.77 (1H, d, J=7.4 Hz), 6.81 (2H, d, J=8.8 Hz), 7.16 (2H, d, J=8.8 Hz), 7.26 (1H, d, J=7.4 Hz)
MS (FAB) m/z: 406 (M+H)⁺.

### (Example 16) 5-Amino-2-(4-ethylbenzyl)phenyl 7-deoxy-L-glycero-β-D-gluco-heptopyranoside (Example Compound No. 1-5)

The compound synthesized in (6c) (203 mg, 0.33 mmol) was dissolved in methylene chloride (4 mL), followed by addition of trichloroacetonitrile (168 µL, 1.66 mmol) and 1,8-diazabicyclo[5.4.0]-7-undecene (10 µL, 0.067 mmol), and the mixture was stirred at room temperature for 30 min. The reaction mixture was diluted with ethyl acetate and washed with saturated ammonium chloride and saturated brine. The organic layer was dried over anhydrous sodium sulfate, and the solvent was evaporated under reduced pressure to obtain an imidate (0.25 g) as a yellow oil. The resulting imidate (0.25 g) and benzyl N-{4-(4-ethylbenzyl)-3-hydroxyphenyl}carbamate (WO2002/064606) (0.10 g, 0.28 mmol) were dissolved in methylene chloride (5 mL), followed by addition of a small amount of molecular sieve (4Å), a boron trifluoride-diethyl ether complex (35 µL, 0.28 mmol) was added dropwise with ice cooling, and the mixture was stirred at room temperature for 1 h. The reaction mixture was diluted with ethyl acetate and washed with saturated aqueous sodium hydrogencarbonate and saturated brine. The organic layer was dried over anhydrous sodium sulfate, then the solvent was evaporated under reduced pressure, and the resulting residue was purified by silica gel column chromatography (hexane:ethyl acetate, 4:1 to 2:1, v/v) to obtain 5-benzyloxycarbonylamino-2-(4-ethylbenzyl)phenyl 2,3,4,6-tetra-O-benzoyl-7-deoxy-L-glycero-β-D-gluco-heptopyranoside (0.30 g) as a colorless oily crude product. The resulting glycoside compound (0.30 g) was dissolved in methanol (3 mL), followed by addition of 10% palladium on carbon (0.10 g), and the mixture was stirred under a hydrogen atmosphere at room temperature for 2 h. Tetrahydrofuran (1 mL) was added to the reaction mixture, and the mixture was further stirred at room temperature for 2 h. Insoluble matters were removed by filtration, and then the solvent was evaporated under reduced pressure to obtain 5-amino-2-(4-ethylbenzyl)phenyl 2,3,4,6-tetra-O-benzoyl-7-deoxy-L-glycero-β-D-gluco-heptopyranoside (0.28 g) as a pale brown oily crude product. The resulting amino compound (0.28 g) was dissolved in a mixed solvent of methylene chloride (3 mL) and methanol (15 mL), followed by addition of potassium carbonate (0.38 g, 2.75 mmol), 5 drops of water were added dropwise, and the mixture was stirred at room temperature for 3 h. The mixture was allowed to stand overnight at room temperature, then insoluble matters were removed by filtration, and the mixture was neutralized with acetic acid. The solvent was evaporated under reduced pressure, and the resulting residue was diluted with ethyl acetate and washed with saturated aqueous sodium hydrogencarbonate and saturated brine. The organic layer was dried over anhydrous sodium sulfate, and then the solvent was evaporated under reduced pressure. The residue was purified by silica gel column chromatography (ethyl acetate to ethyl acetate:methanol = 10:1, v/v), ethyl acetate and hexane were added to the resulting pale yellow oil for crystallization, and the crystals were collected by filtration to obtain 5-amino-2-(4-ethylbenzyl)phenyl 7-deoxy-L-glycero-β-D-gluco-heptopyranoside (32 mg, yield 29%) as a pale yellow powder.
¹H NMR (400 MHz, CD₃OD): δ1.19 (3H, t, J=7.7 Hz), 1.32 (3H, d, J=6.6 Hz), 2.57 (2H, q, J=7.7 Hz), 3.18 (1H, m), 3.40-3.49 (2H, m), 3.61 (1H, t, J=9.0 Hz), 3.83 (1H, d, J=15.1 Hz), 3.91 (1H, d, J=15.1 Hz), 4.06-4.12 (1H, m), 4.83 (1H, d, J=7.5 Hz), 6.35 (1H, dd, J=8.0 and 2.3 Hz), 6.53 (1H, d, J=2.3 Hz), 6.79 (1H, d, J=8.0 Hz), 7.05 (2H, d, J=8.2 Hz), 7.11 (2H, d, J=8.2 Hz);
MS (FAB) m/z: 403 (M)⁺.

### (Example 17) 5-Amino-2-(4-ethylbenzyl)phenyl 4-C-methyl-β-D-glucopyranoside (Example Compound No. 1-10)

The compound synthesized in (3c) (0.20 g, 0.36 mmol), methylene chloride (4 mL), trichloroacetonitrile (0.18 mL, 1.78 mmol), and 1,8-diazabicyclo[5.4.0]-7-undecene (11 µL, 0.074 mmol) were used to synthesize an imidate (0.26 g) by the same method as in (16). The resulting imidate (0.26 g), benzyl N-{4-(4-ethylbenzyl)-3-hydroxyphenyl}carbamate (0.11 g, 0.30 mmol), methylene chloride (5 mL), and a boron trifluoride-diethyl ether complex (39 µL, 0.31 mmol) were used to synthesize 5-benzyloxycarbonylamino-2-(4-ethylbenzyl)phenyl 4-O-acetyl-4-C-methyl-2,3,6-tri-O-benzoyl-β-D-glucopyranoside (0.30 g) as a colorless oily crude product by the same method as in (16). The resulting glycoside compound (0.30 g), methanol (3 mL), tetrahydrofuran (1 mL), and 10% palladium on carbon (0.10 g) were used to synthesize 5-amino-2-(4-ethylbenzyl)phenyl 4-O-acetyl-4-C-methyl-2,3,6-tri-O-benzoyl-β-D-glucopyranoside (0.26 g) as a crude product by the same method as in (16). The resulting amino compound (0.26 g), methylene chloride (3 mL), methanol (15 mL), and potassium carbonate (0.42 g, 3.04 mmol) were used to obtain 5-amino-2-(4-ethylbenzyl)phenyl 4-C-methyl-β-D-glucopyranoside (44 mg, yield 36%) as a pale yellow powder by the same method as in (16).
¹H NMR (400 MHz, CD₃OD): δ 1.12 (3H, s), 1.18 (3H, t, J=7.7 Hz), 2.57 (2H, q, J=7.7 Hz), 3.41-3.44 (3H, m), 3.61-3.66 (1H, m), 3.81 (1H, d, J=15.3 Hz), 3.89-3.94 (2H, m), 4.83 (1H, d, J=7.4 Hz), 6.33 (1H, dd, J=8.1 and 2.2 Hz), 6.64 (1H, d, J=2.2 Hz), 6.78 (1H, d, J=8.1Hz), 7.04 (2H, d, J=7.9 Hz), 7.11 (2H, d, J=7.9 Hz);
MS (FAB) m/z: 403 (M)⁺.

### (Example 18) 5-Amino-2-(4-ethylbenzyl)phenyl 5-C-methyl-β-D-glucopyranoside (Example Compound No. 1-9)

The compound synthesized in (4c) (203 mg, 0.33 mmol), methylene chloride (4 mL), trichloroacetonitrile (168 µL, 1.66 mmol), and 1,8-diazabicyclo[5.4.0]-7-undecene (10 µL, 0.067 mmol) were used to synthesize an imidate (0.28 g) by the same method as in (16). The resulting imidate (0.28 g), benzyl N-{4-(4-ethylbenzyl)-3-hydroxyphenyl}carbamate (0.10 g, 0.28 mmol), methylene chloride (4 mL), and a boron trifluoride-diethyl ether complex (35 µL, 0.28 mmol) were used to synthesize 5-benzyloxycarbonylamino-2-(4-ethylbenzyl)phenyl 2,3,4,6-tetra-O-benzoyl-5-C-methyl-µ-D-glucopyranoside (0.37 g) as a pale yellow viscous oily crude product by the same method as in (16). The resulting glycoside compound (0.37 g), methanol (3 mL), tetrahydrofuran (3 mL), and 10% palladium on carbon (0.10 g) were used to synthesize 5-amino-2-(4-ethylbenzyl)phenyl 2,3,4,6-tetra-O-benzoyl-5-C-methyl-β-D-glucopyranoside (85 mg) as a crude product by the same method as in (16). The resulting amino compound (85 mg), methylene chloride (2 mL), methanol (5 mL), and potassium carbonate (143 mg, 1.03 mmol) were used to obtain 5-amino-2-(4-ethylbenzyl)phenyl 5-C-methyl-β-D-glucopyranoside (18 mg, yield 16%) as a white powder by the same method as in (16).
¹H NMR (400 MHz, CD₃OD): δ 1.19 (3H, t, J=7.6 Hz), 1.28 (3H, s), 2.57 (2H, q, J=7.6 Hz), 3.41-3.65 (5H, m), 3.81 (1H, d, J=15.3 Hz), 3.92 (1H, d, J=15.3 Hz), 5.09 (1H, d, J=7.9 Hz), 6.34 (1H, dd, J=7.9 and 2.3 Hz), 6.61 (1H, d, J=2.3 Hz), 6.79 (1H, d, J=7.9 Hz), 7.05 (2H, d, J=8.1 Hz), 7.10 (2H, d, J=8.1 Hz);
MS (FAB) m/z: 404 (M+H)⁺.

### (Example 19) 5-Amino-2-(4-ethylbenzyl)phenyl 7-deoxy-D-glycero-β-D-gluco-heptopyranoside (Example Compound No. 1-5)

2,3,4,6-Tetra-O-benzoyl-7-deoxy-D-glycero-α,β-D-gluco-heptopyranoside synthesized in (7d) (600 mg, 0.98 mmol), methylene chloride (12 mL), trichloroacetonitrile (496 µL, 4.91 mmol), and 1,8-diazabicyclo[5.4.0]-7-undecene (29 µL, 0.19 mmol) were used to synthesize an imidate (0.75 g) by the same method as in (16). The resulting imidate (0.75 g), benzyl N-{4-(4-ethylbenzyl)-3-hydroxyphenyl}carbamate (361 mg, 1.00 mmol), methylene chloride (15 mL), and a boron trifluoride-diethyl ether complex (127 µL, 1.00 mmol) were used to synthesize 5-benzyloxycarbonylamino-2-(4-ethylbenzyl)phenyl 2,3,4,6-tetra-O-benzoyl-7-deoxy-D-glycero-β-D-gluco-heptopyranoside (0.80 g) as a pale yellow oily crude product by the same method as in (16). The resulting glycoside compound (0.40 g), methanol (4 mL), tetrahydrofuran (2 mL), and 10% palladium on carbon (0.10 g) were used to synthesize 5-amino-2-(4-ethylbenzyl)phenyl 2,3,4,6-tetra-O-benzoyl-7-deoxy-D-glycero-β-D-gluco-heptopyranoside (143 mg) as a crude product by the same method as in (16). The resulting amino compound (40 mg), methylene chloride (0.5 mL), methanol (2.5 mL), and potassium carbonate (67 mg, 0.48 mmol) were used to obtain 5-amino-2-(4-ethylbenzyl)phenyl 7-deoxy-D-glycero-β-D-gluco-heptopyranoside (5 mg, yield 9%) as a white powder by the same method as in (16). ¹H NMR (400 MHz, CD₃OD): δ 1.19 (3H, t, J=7.6 Hz), 1.23 (3H, d, J=6.3 Hz), 2.57 (2H, q, J=7.6 Hz), 3.31-3.44 (4H, m), 3.83 (1H, d, J=15.1 Hz), 3.91 (1H, d, J=15.1 Hz), 4.06-4.09 (1H, m), 4.82 (1H, d, J=7.4 Hz), 6.34 (1H, dd, J=8.1 and 2.2 Hz), 6.58 (1H, d, J=2.2 Hz), 6.78 (1H, d, J=8.1 Hz), 7.05 (2H, d, J=8.2 Hz), 7.11 (2H, d, J=8.2 Hz);
MS (FAB) m/z: 404 (M+H)⁺.

### (Example 20) 5-Amino-2-(4-ethylbenzyl)phenyl 4-deoxy-β-D-glucopyranoside (Example Compound No. 2-9)

2,3,6-Tri-O-benzoyl-4-deoxy-α,β-D-glucopyranoside (Liebigs Ann. Chem., GE, 1992, 7, 747-758) (0.52 g, 1.09 mmol), methylene chloride (10 mL), trichloroacetonitrile (0.55 mL, 5.45 mmol), and 1,8-diazabicyclo[5.4.0]-7-undecene (33 µL, 0.22 mmol) were used to synthesize an imidate (0.69 g) by the same method as in (16). The resulting imidate (0.69 g), benzyl N-{4-(4-ethylbenzyl)-3-hydroxyphenyl}carbamate (0.39 g, 1.08 mmol), methylene chloride (10 mL), and a boron trifluoride-diethyl ether complex (0.28 mL, 2.21 mmol) were used to synthesize 5-benzyloxycarbonylamino-2-(4-ethylbenzyl)phenyl 2,3,6-tri-O-benzoyl-4-deoxy-β-D-glucopyranoside (0.88 g) as a white powder crude product by the same method as in (16). The resulting glycoside compound (0.56 g), methanol (6 mL), tetrahydrofuran (6 mL), and 10% palladium on carbon (0.30 g) were used to synthesize 5-amino-2-(4-ethylbenzyl)phenyl 2,3,6-tri-O-benzoyl-4-deoxy-β-D-glucopyranoside (0.46 g) as a crude product by the same method as in (16). The resulting amino compound (0.46 g), methylene chloride (5 mL), methanol (25 mL), and potassium carbonate (0.93 g, 6.73 mmol) were used to obtain 5-amino-2-(4-ethylbenzyl)phenyl 4-deoxy-β-D-glucopyranoside (0.17 g, yield 66%) as a white powder by the same method as in (16).
¹H NMR (400 MHz, CD₃OD): δ 1.19 (3H, t, J=7.6 Hz), 1.41-1.50 (1H, m), 1.95-2.00 (1H, m), 2.57 (2H, q, J=7.6 Hz), 3.30-3.36 (2H, m), 3.60 (1H, d, J=5.1 Hz), 3.66-3.71 (2H, m), 3.82 (1H, d, J=15.1 Hz), 3.92 (1H, d, J=15.1 Hz), 4.79 (1H, d, J=7.5 Hz), 6.33 (1H, dd, J=8.2 and 2.3 Hz), 6.59 (1H, d, J=2.3 Hz), 6.78 (1H, d, J=8.2 Hz), 7.05 (2H, d, J=8.2 Hz), 7.10 (2H, d, J=8.2 Hz);
MS (FAB) m/z: 374 (M+H)⁺.

### (Example 21) 5-Amino-2-(4-methoxybenzyl)phenyl 7-deoxy-L-glycero-β-D-gluco-heptopyranoside (Example Compound No. 1-23)

### (21a) 3-Benzyloxy-4-(4-methoxybenzyl)benzyl acetate

5-Acetoxymethyl-2-(4-methoxybenzyl)phenol (1.00 g, 3.49 mmol) was dissolved in N,N-dimethylformamide (20 mL), followed by addition of benzyl bromide (0.46 mL, 3.87 mmol) and potassium carbonate (0.72 g, 5.21 mmol), and the mixture was stirred at room temperature for 5 h. The reaction mixture was poured into water and extracted with ethyl acetate. The organic layer was washed with saturated brine and dried over anhydrous sodium sulfate, and then the solvent was evaporated under reduced pressure. The resulting residue was purified by silica gel column chromatography (hexane:ethyl acetate, 5:1, v/v) to obtain the title compound (1.32 g, yield 100%) as a colorless oil.
¹H NMR (400 MHz, CDCl₃): δ 2.08 (3H, s), 3.78 (3H, s), 3.95 (2H, s), 5.05 (2H, s), 5.05 (2H, s), 6.80 (2H, d, J=8.6 Hz), 6.88-6.92 (2H, m), 7.07-7.11 (3H, m), 7.32-7.39 (5H, m);
MS (FAB) m/z: 376 (M)⁺.

### (21b) 3-Benzyloxy-4-(4-methoxybenzyl)benzyl alcohol

The compound obtained in (21a) (1.32 g, 3.51 mmol) was dissolved in methanol (10 mL)-tetrahydrofuran (10 mL), followed by addition of 2 N aqueous potassium hydroxide (10 mL), and the mixture was stirred at room temperature for 1 h. The reaction mixture was poured into ice water, neutralized with 2 N hydrochloric acid, and then extracted with ethyl acetate. The organic layer was washed with saturated brine and dried over anhydrous sodium sulfate, and then the solvent was evaporated under reduced pressure to obtain the title compound (1.25 g) as a colorless oily crude product.
¹H NMR (400 MHz, CDCl₃): δ 3.78 (3H, s), 3.95 (2H, s), 4.65 (2H, s), 5.08 (2H, s), 6.80 (2H, d, J=8.6 Hz), 6.88 (1H, d, J=7.5 Hz), 6.97 (1H, s), 7.07-7.12 (3H, m), 7.31-7.39 (5H, m);
MS (FAB) m/z: 334 (M)⁺.

### (21c) 3-Benzyloxy-4-(4-methoxybenzyl)benzaldehyde

The compound obtained in (21b) (1.25 g) was dissolved in methylene chloride (20 mL), followed by addition of manganese dioxide (3.25 g, 37.4 mmol), and the mixture was stirred at room temperature for 4 h. The mixture was allowed to stand overnight at room temperature and further stirred at room temperature for 10 h. The mixture was allowed to stand at room temperature for 2 days, then insoluble matters were removed by filtration, and the solvent was evaporated under reduced pressure to obtain the title compound (1.23 g) as a white powder crude product.
¹H NMR (400MHz, CDCl₃): δ 3.79 (3H, s), 4.02 (2H, s), 5.14 (2H, s), 6.82 (2H, d, J=8.6 Hz), 7.10 (2H, d, J=8.6 Hz), 7.25-7.26 (1H, m), 7.33-7.41 (6H, m), 7.44 (1H, s), 9.93 (1H, s).

### (21d) 3-Benzyloxy-4-(4-methoxybenzyl)phenyl carboxylate

The compound obtained in (21c) (1.20 g) was dissolved in a mixed solvent of tertiary butyl alcohol-tetrahydrofuran-water (5:2:1) (32 mL), followed by addition of 2-methyl-2-butene (1.53 mL, 14.4 mmol), sodium dihydrogenphosphate dihydrate (0.84 g, 5.38 mmol), and sodium chlorite (0.98 g, 10.8 mmol), and the mixture was stirred at room temperature for 1 h. Following addition of water, the reaction mixture was neutralized with 2 N hydrochloric acid and then extracted with ethyl acetate. The organic layer was washed with saturated brine and dried over anhydrous sodium sulfate, and then the solvent was evaporated under reduced pressure. Ethyl acetate and hexane were added to the resulting residue for crystallization, and insoluble matters were collected by filtration to isolate the title compound (1.07 g, yield 88%) as a white powder.
¹H NMR (400 MHz, CDCl₃): δ 3.79 (3H, s), 4.01 (2H, s), 5.13 (2H, s), 6.81 (2H, d, J=8.6 Hz), 7.10 (2H, d, J=8.6 Hz), 7.19 (1H, d, J=7.8 Hz), 7.35-7.38 (5H, m), 7.65-7.67 (2H, m);
MS (FAB) m/z: 348 (M)⁺.

### (21e) Benzyl N-{3-benzyloxy-4-(4-methoxybenzyl)phenyl}carbamate

The compound obtained in (21d) (1.05 g, 3.01 mmol) was dissolved in dioxane (10 mL), followed by addition of triethylamine (1.01 mL, 7.25 mmol) and a solution of diphenylphosphate azide (1.00 g, 3.63 mmol) in dioxane (10 mL), and the mixture was heated to reflux for 1 h. Benzyl alcohol (1.24 mL, 12.0 mmol) was added, the mixture was further heated to reflux for 1 h, then the reaction mixture was cooled to room temperature, and the solvent was evaporated under reduced pressure. The resulting residue was purified by silica gel column chromatography (hexane:ethyl acetate, 4:1, v/v) to isolate the title compound (1.56 g) as a colorless oily crude product.
¹H NMR (400 MHz, CDCl₃): δ 3.78 (3H, s), 3.90 (2H, s), 5.05 (2H, s), 5.20 (2H, s), 6.61 (1H, s), 6.70 (1H, d, J=7.8 Hz), 6.79 (2H, d, J=8.8 Hz), 6.99 (1H, d, J=7.8 Hz), 7.09 (2H, d, J=8.8 Hz), 7.26-7.40 (11H, m).

### (21f) 5-Amino-2-(4-methoxybenzyl)phenol

The compound obtained in (21e) (1.56 g), methanol (20 mL), tetrahydrofuran (5 mL), and 10% palladium on carbon (0.50 g) were stirred under a hydrogen atmosphere at room temperature for 3 h. Insoluble matters were removed by filtration, and then the solvent was evaporated under reduced pressure. The resulting residue was purified by silica gel column chromatography (hexane:ethyl acetate, 4:1 to 2:1, v/v) to synthesize the title compound (0.43 g, yield 62%).
¹H NMR (400 MHz, CDCl₃): δ 3.57 (2H, brs), 3.78 (3H, s), 3.83 (2H, s), 4.52 (1H, s), 6.18 (1H, d, J=2.3 Hz), 6.25 (1H, dd, J=8.0 and 2.3 Hz), 6.83 (2H, d, J=8.6 Hz), 6.89 (1H, d, J=8.0 Hz), 7.14 (2H, d, J=8.6 Hz);
MS (FAB) m/z: 229 (M)⁺.

### (21g) Benzyl N-{3-hydroxy-4-(4-methoxybenzyl)phenyl}carbamate

The compound obtained in (21f) (0.42 g, 1.83 mmol) was dissolved in tetrahydrofuran (20 mL), followed by addition of N-(benzyloxycarbonyloxy)succinimide (0.68 g, 2.73 mmol), and the mixture was stirred at room temperature for 1 h. The reaction mixture was allowed to stand overnight at room temperature, and then the solvent was evaporated under reduced pressure. The resulting residue was purified by silica gel column chromatography (hexane:ethyl acetate , 5:1 to 3:1, v/v) to isolate the title compound (0.62 g, yield 93%) as a pale brown powder.
¹H NMR (400 MHz, CDCl₃): δ 3.78 (3H, s), 3.88 (2H, s), 4.98 (1H, s), 5.19 (2H, s), 6.60 (1H, s), 6.71 (1H, d, J=8.2 Hz), 6.83 (2H, d, J=8.6 Hz), 7.00 (1H, d, J=8.2 Hz), 7.12-7.14 (3H, m), 7.34-7.40 (5H, m);
MS (FAB) m/z: 363 (M)⁺.

### (21h) 5-Amino-2-(4-ethylbenzyl)phenyl 7-deoxy-L-glycero-β-D-glucopyranoside

2,3,4,6-Tetra-O-benzoyl-7-deoxy-L-glycero-α,β-D-gluco-heptopyranoside synthesized in (6c) (203 mg, 0.33 mmol), methylene chloride (4 mL), trichloroacetonitrile (168 µL, 1.66 mmol), and 1,8-diazabicyclo[5.4.0]-7-undecene (10 µL, 0.067 mmol) were used to synthesize an imidate (0.26 g) by the same method as in (16). The resulting imidate (0.26 g), benzyl N-{3-hydroxy-4-(4-methoxybenzyl)phenyl} carbamate synthesized in (21 g) (0.10 g, 0.28 mmol), methylene chloride (4 mL), and a boron trifluoride-diethyl ether complex (35 µL, 0.28 mmol) were used to synthesize 5-benzyloxycarbonylamino-2-(4-methoxybenzyl)phenyl 2,3,4,6-tetra-O-benzoyl-7-deoxy-L-glycero-β-D-gluco-heptopyranoside (0.17 g) as a colorless viscous oily crude product by the same method as in (16). The resulting glycoside compound (157 mg), methanol (2 mL), tetrahydrofuran (2 mL), and 10% palladium on carbon (0.10 g) were used to synthesize 5-amino-2-(4-methoxybenzyl)phenyl 2,3,4,6-tetra-O-benzoyl-7-deoxy-L-glycero-β-D-gluco-heptopyranoside (153 mg) as a crude product by the same method as in (16). The resulting amino compound (151 mg), methylene chloride (1 mL), methanol (5 mL), and potassium carbonate (0.23 g, 1.66 mmol) were used to obtain 5-amino-2-(4-methoxybenzyl)phenyl 7-deoxy-L-glycero-β-D-gluco-heptopyranoside (28 mg, yield 27%) as a white powder by the same method as in (16).
¹H NMR (400 MHz, CD₃OD): δ 1.32 (3H, d, J=6.6 Hz), 3.17-3.20 (1H, m), 3.41-3.49 (2H, m), 3.58-3.63 (1H, m), 3.74 (3H, s), 3.81 (1H, d, J=14.9 Hz), 3.88 (1H, d, J=14.9 Hz), 4.08-4.10 (1H, m), 4.83 (1H, d, J=7.5 Hz), 6.34 (1H, dd, J=8.0 and 2.2 Hz), 6.53 (1H, d, J=2.2 Hz), 6.77-6.80 (3H, m), 7.12 (2H, d, J=8.6 Hz);
MS (FAB) m/z: 405 (M)⁺.

### (Example 22) 5-Amino-2-(4-methoxybenzyl)phenyl 5-C-methyl-β-D-glucopyranoside (Example Compound No. 1-27)

2,3,4,6-Tetra-O-benzoyl-5-C-methyl-α,β-D-glucopyranoside synthesized in (4c) (203 mg, 0.33 mmol), methylene chloride (4 mL), trichloroacetonitrile (168 µL, 1.66 mmol), and 1,8-diazabicyclo[5.4.0]-7-undecene (10 µL, 0.067 mmol) were used to synthesize an imidate (0.25 g) by the same method as in (16). The resulting imidate (0.25 g), benzyl N-{3-hydroxy-4-(4-methoxybenzyl)phenyl}carbamate synthesized in (21 g) (0.10 g, 0.28 mmol), methylene chloride (4 mL), and a boron trifluoride-diethyl ether complex (35 µL, 0.28 mmol) were used to synthesize 5-benzyloxycarbonylamino-2-(4-methoxybenzyl)phenyl 2,3,4,6-tetra-O-benzoyl-5-C-methyl-β-D-glucopyranoside (0.18 g) as a pale yellow oily crude product by the same method as in (16). The resulting glycoside compound (0.18 g), methanol (2 mL), tetrahydrofuran (2 mL), and 10% palladium on carbon (0.10 g) were used to synthesize 5-amino-2-(4-methoxybenzyl)phenyl 2,3,4,6-tetra-O-benzoyl-5-C-methyl-β-D-glucopyranoside (0.15 g) as a crude product by the same method as in (16). The resulting amino compound (0.15 g), methylene chloride (2 mL), methanol (5 mL), and potassium carbonate (0.25 g, 1.80 mmol) were used to obtain the title compound (17 mg, yield 15%) as a pale yellow powder by the same method as in (16).
¹H NMR (400 MHz, CD₃OD): δ 1.28 (3H, s), 3.40-3.65 (5H, m), 3.74 (3H, s), 3.78 (1H, d, J=15.1 Hz), 3.88 (1H, d, J=15.1 Hz), 5.09 (1H, d, J=7.8 Hz), 6.33 (1H, dd, J=7.8 and 2.4 Hz), 6.60 (1H, d, J=2.4 Hz), 6.76-6.79 (1H, m), 6.77 (2H, d, J=8.6 Hz), 7.11 (2H, d, J=8.6 Hz):
MS (FAB) m/z: 405 (M)⁺.

### (Example 23) 5-Aminoacetylamino-2-(4-ethylbenzyl)phenyl 7-deoxy-D-glycero-β-D-gluco-heptopyranoside (Example Compound No. 1-107)

5-Amino-2-(4-ethylbenzyl)phenyl 2,3,4,6-tetra-O-benzoyl-7-deoxy-D-glycero-β-D-gluco-heptopyranoside obtained as an intermediate of Example 19 (60 mg, 0.073 mmol) was dissolved in methylene chloride (1 mL), followed by addition of N-(tertiary butoxycarbonyl)glycine (15 mg, 0.086 mmol), triethylamine (20 µL, 0.14 mmol), and 1-ethyl-3-(3'-dimethylaminopropyl)carbodiimide (17 mg, 0.089 mmol), and the mixture was stirred at room temperature for 1 h. The mixture was allowed to stand overnight at room temperature, and the reaction mixture was poured into water and extracted with ethyl acetate. The organic layer was washed with saturated brine and dried over anhydrous sodium sulfate, and then the solvent was evaporated under reduced pressure. The resulting residue was purified by silica gel column chromatography (hexane:ethyl acetate, 3:1 to 2:1, v/v) to synthesize 5-tertiary butoxycarbonylaminoacetylamino-2-(4-ethylbenzyl)phenyl 2,3,4,6-tetra-O-benzoyl-7-deoxy-D-glycero-β-D-gluco-heptopyranoside (35 mg) as a colorless oily crude product. The resulting 5-tertiary butoxycarbonylaminoacetylamino-2-(4-ethylbenzyl)phenyl 2,3,4,6-tetra-O-benzoyl-7-deoxy-D-glycero-β-D-gluco-heptopyranoside (34 mg) was dissolved in dioxane (1 mL), followed by addition of 4 N hydrochloric acid in dioxane (1 mL). The mixture was stirred at room temperature for 2 h, and the solvent was evaporated under reduced pressure to synthesize 5-aminoacetylamino-2-(4-ethylbenzyl)phenyl 2,3,4,6-tetra-O-benzoyl-7-deoxy-D-glycero-β-D-gluco-heptopyranoside (35 mg) as a colorless oily crude product. The resulting amine hydrochloride (35 mg), methylene chloride (0.5 mL), and methanol (2.5 mL) were used to obtain 5-aminoacetylamino-2-(4-ethylbenzyl)phenyl 7-deoxy-D-glycero-β-D-gluco-heptopyranoside (7 mg, yield 21%) as a white powder by the same method as in (16).
¹H NMR (400 MHz, CD₃OD): δ 1.19 (3H, t, J=7.6 Hz), 1.23 (3H, d, J=6.6 Hz), 2.58 (2H, q, J=7.6 Hz), 3.31-3.39 (4H, m), 3.45-3.47 (2H, m), 3.92 (1H, d, J=15.4 Hz), 4.01 (1H, d, J=15.4 Hz), 4.06-4.09 (1H, m), 4.88 (1H, d, J=7.4 Hz), 6.99 (1H, d, J=8.2 Hz), 7.08 (2H, d, J=8.3 Hz), 7.11-7.15 (1H, m), 7.14 (2H, d, J=8.3 Hz), 7.50 (1H, d, J=1.9 Hz);
MS (FAB) m/z: 460 (M)⁺.

### (Example 24) 5-Methylamino-2-(4-ethylbenzyl)phenyl 7-deoxy-D-glycero-β-D-gluco-heptopyranoside (Example Compound No. 1-46)

5-Benzyloxycarbonylamino-2-(4-ethylbenzyl)phenyl 2,3,4,6-tetra-O-benzoyl-7-deoxy-D-glycero-β-D-gluco-heptopyranoside obtained as an intermediate of Example 19 (0.20 g, 0.21 mmol) was dissolved in tetrahydrofuran (4 mL), followed by addition of methyl iodide (26 µL, 0.42 mmol) and sodium hydride (55% by weight) (14 mg, 0.32 mmol), and the mixture was stirred at room temperature for 3 h. Methyl iodide (26 µL, 0.42 mmol) and sodium hydride (55% by weight) (14 mg, 0.32 mmol) were added, the mixture was further stirred at room temperature for 1 h. The reaction mixture was poured into water and extracted with ethyl acetate. The organic layer was washed with saturated brine and dried over anhydrous sodium sulfate, and then the solvent was evaporated under reduced pressure. The resulting residue was purified by silica gel column chromatography (hexane:ethyl acetate, 2:1, v/v) to synthesize 5-(N-benzyloxycarbonyl-N-methyl)amino-2-(4-ethylbenzyl)phenyl 2,3,4,6-tetra-O-benzoyl-7-deoxy-D-glycero-β-D-gluco-heptopyranoside (0.12 g) as a pale yellow oily crude product. The resulting glycoside compound (0.12 g) was dissolved in methanol (1 mL) and tetrahydrofuran (1 mL), followed by addition of 10% palladium on carbon (60 mg), and the mixture was stirred under a hydrogen atmosphere at room temperature for 3 h. Insoluble matters were removed by filtration, and then the solvent was evaporated under reduced pressure. The resulting residue was purified by silica gel column chromatography (hexane:ethyl acetate, 4:1 to 2:1, v/v) to synthesize 2-(4-ethylbenzyl)-5-methylaminophenyl 2,3,4,6-tetra-O-benzoyl-7-deoxy-D-glycero-β-D-gluco-heptopyranoside (18 mg) as a pale yellow oily crude product. The resulting methylamino compound (18 mg), methylene chloride (0.2 mL), methanol (1 mL), and potassium carbonate (28 mg, 0.20 mmol) were used to obtain 2-(4-ethylbenzyl)-5-methylaminophenyl 7-deoxy-D-glycero-β-D-gluco-heptopyranoside (5 mg, yield 6%) as a pale yellow powder by the same method as in (16).
¹H NMR (400 MHz, CD₃OD): δ 1.19 (3H, t, J=7.7 Hz), 1.23 (3H, d, J=6.6 Hz), 2.57 (2H, q, J=7.7 Hz), 2.74 (3H, s), 3.30-3.49 (4H, m), 3.83 (1H, d, J=14.9 Hz), 3.91 (1H, d, J=14.9 Hz), 4.02-4.07 (1H, m), 4.83 (1H, d, J=7.9 Hz), 6.26 (1H, dd, J=8.2 and 2.1 Hz), 6.48 (1H, d, J=2.1 Hz), 6.81 (1H, d, J=8.2 Hz), 7.04 (2H, d, J=8.2H z), 7.10 (2H, d, J=8.2 Hz);
MS (FAB) m/z: 417 (M)⁺.

### (Example 25) 5-Amino-2-(4-methoxybenzyl)phenyl 7-deoxy-D-glycero-β-D-gluco-heptopyranoside (Example Compound No. 1-23)

2,3,4,6-Tetra-O-benzoyl-7-deoxy-D-glycero-α,β-D-gluco-heptopyranoside synthesized in (7d) (202 mg, 0.33 mmol), methylene chloride (4 mL), trichloroacetonitrile (0.17 mL, 1.68 mmol), and 1,8-diazabicyclo[5.4.0]-7-undecene (10 µL, 0.067 mmol) were used to synthesize an imidate (0.25 g) by the same method as in (16). The resulting imidate (0.25 g), benzyl N-{3-hydroxy-4-(4-methoxybenzyl)phenyl}carbamate synthesized in (21g) (0.10 g, 0.28 mmol), methylene chloride (4 mL), and a boron trifluoride-diethyl ether complex (35 µL, 0.28 mmol) were used to synthesize 5-benzyloxycarbonylamino-2-(4-methoxybenzyl)phenyl 2,3,4,6-tetra-O-benzoyl-7-deoxy-D-glycero-β-D-gluco-heptopyranoside (0.20 g) as a colorless oily crude product by the same method as in (16). The resulting glycoside compound (0.19 g), methanol (2 mL), tetrahydrofuran (2 mL), and 10% palladium on carbon (0.10 g) were used to synthesize 5-amino-2-(4-methoxybenzyl)phenyl 2,3,4,6-tetra-O-benzoyl-7-deoxy-D-glycero-α,β-D-gluco-heptopyranoside (0.17 g) as a crude product by the same method as in (16). The resulting amino compound (0.16 g), methylene chloride (2 mL), methanol (10 mL), and potassium carbonate (0.27 g, 1.95 mmol) were used to obtain 5-amino-2-(4-methoxybenzyl)phenyl 7-deoxy-D-glycero-β-D-gluco-heptopyranoside (26 mg, yield 25%) as a white powder by the same method as in (16).
¹H NMR (400 MHz, CD₃OD): δ 1.23 (3H, d, J=6.3 Hz), 3.34-3.36 (2H, m), 3.41-3.44 (2H, m), 3.74 (3H, s), 3.80 (1H, d, J=15.0 Hz), 3.88 (1H, d, J=15.0 Hz), 4.04-4.11 (1H, m), 4.81 (1H, d, J=7.8 Hz), 6.34 (1H, dd, J=7.9 and 2.2 Hz), 6.58 (1H, d, J=2.2 Hz), 6.76-6.79 (3H, m), 7.11 (2H, d, J=8.6 Hz);
MS (FAB) m/z: 405 (M)⁺.

### (Example 26) 2-(4-Ethylbenzyl)-5-hydroxymethylphenyl 4-deoxy-β-D-glucopyranoside (Example Compound No. 2-1)

### (26a) 5-Acetoxymethyl-2-(4-ethylbenzyl)phenyl 2,3,6-tri-O-benzoyl-4-deoxy-β-D-glucopyranoside

2,3,6-Tri-4-O-benzoyl-4-deoxy-D-glucopyranoside (Liebigs Ann. Chem. GE, 1992, 7, 747-758) (1.20 g, 2.52 mmol) was dissolved in methylene chloride (8 mL), followed by addition of trichloroacetonitrile (750 µL, 7.48 mmol) and 1,8-diazabicyclo[5.4.0]-7-undecene (40 µL, 0.27 mmol), and the mixture was stirred at room temperature for 1 h. The solvent was evaporated under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was evaporated under reduced pressure to obtain an imidate (1.61 g, crude) as a brown amorphous compound. 5-Acetoxymethyl-2-(4-ethylbenzyl)phenol (W02002/064606) (655 mg, 2.29 mmol) was dissolved in methylene chloride (8 mL), followed by addition of an imidate (1.61 g, crude), a solution of trimethylsilyl trifluoromethanesulfonate (45 µL, 0.36 mmol) in methylene chloride (2 mL) was added dropwise, and the mixture was stirred at 0°C for 2 h. Triethylamine (95 µL) was added to the reaction mixture, the solvent was evaporated under reduced pressure, and then the residue was diluted with ethyl acetate (20 mL) and washed with saturated aqueous sodium hydrogencarbonate (20 mL) and saturated brine (10 mL). The organic layer was dried over anhydrous sodium sulfate, then the solvent was evaporated under reduced pressure, and the residue was purified by silica gel flash column chromatography (hexane:ethyl acetate, 5:1 to 3:1, v/v) to isolate the title compound (1.56 g, 91.2%) as a pale yellow amorphous compound.
¹H NMR (400 MHz, CDCl₃) δ 1.15 (3H, t, J=7.7 Hz), 1.99 (1H, dd, J=24.8, 12.7 Hz), 2.03 (3H, s), 2.51 (2H, q, J=7.6 Hz), 2.56 (1H, m), 3.68 (1H, d, J=15.3 Hz), 3.81 (1H, d, J=15.6 Hz), 4.27-4.31 (1H, m), 4.52 (1H, d, J=5.0 Hz), 4.85 (1H, d, J=12.6 Hz), 4.90 (1H, d, J=12.6 Hz), 5.34 (1H, d, J=7.8 Hz), 5.49-5.56 (1H, m), 5.80 (1H, dd, J=9.2, 8.0 Hz), 6.91 (6H, s), 7.08-7.61 (10H, m), 7.95 (2H, d, J=8.2 Hz), 7.99 (2H, d, J=8.2 Hz), 8.08 (2H, d, J=8.2 Hz);
MS (FAB) m/z: 743 (M+H)⁺.

### (26b) 2-(4-Ethylbenzyl)-5-hydroxymethylphenyl 4-deoxy-β-D-glucopyranoside

Methanol (10 mL) and potassium carbonate (2.91 g, 21.05 mmol) were added to the compound synthesized in (26a) (1.56 g, 2.10 mmol), and the mixture was stirred at room temperature for 14 h. The solvent was evaporated under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was evaporated under reduced pressure. The residue was purified by silica gel flash column chromatography (2-propanol:methylene chloride, 1:15 to 1:10 to 1:5, v/v) to obtain the title compound (666 mg, 81.6%) as a colorless solid.
¹H NMR (400 MHz, CD₃OD): δ 1.19 (3H, t, J=7.6 Hz), 1.47 (1H, dd, J=24.3, 11.8 Hz), 1.99 (1H, ddd, J=12.7, 5.3, 1.6 Hz), 2.58 (2H, q, J=7.6 Hz), 3.39 (1H, t, J=8.2 Hz), 3.59 (2H, d, J=5.1 Hz), 3.66-3.73 (2H, m), 3.94 (1H, d, J=15.2 Hz), 4.04 (1H, d, J=14.9 Hz), 4.54 (2H, s), 4.88 (1H, d, J=7.4 Hz), 6.91 (1H, d, J=7.9Hz), 7.02 (1H, d, J=7.4 Hz), 7.06 (2H, d, J=8.2 Hz), 7.11 (1H, s), 7.14 (2H, d, J=4.0 Hz);
MS (FAB) m/z: 389 (M+H)⁺, 411 (M+Na)⁺.

### (Example 27) 2-(4-Ethylbenzyl)-5-hydroxymethylphenyl 4-O-methyl-6-O-hydroxyacetyl-β-D-glucopyranoside (Example Compound No. 2-6)

### (27a) 2-(4-Ethylbenzyl)-5-hydroxymethylphenyl 4-O-methyl-2,3,6-tri-O-benzoyl-β-D-glucopyranoside

The compound synthesized in (2b) (35.5 g, 45.9 mmol) was dissolved in 1,4-dioxane (175 mL) and methanol (175 mL), 2 M aqueous sodium hydroxide (11.4 mL, 22.8 mmol) was added dropwise with ice cooling, and the mixture was stirred at 0°C for 1 h and 30 min. The reaction mixture was neutralized with 2 M hydrochloric acid (9.1 mL, 18.3 mmol) and diluted with toluene (100 mL), and the solvent was evaporated under reduced pressure. The residue was diluted with ethyl acetate (450 mL), washed 3 times with saturated brine (50 mL), and then dried over anhydrous sodium sulfate, and the solvent was evaporated under reduced pressure. The resulting residue was purified by silica gel flash column chromatography (hexane:ethyl acetate, 3:1 1 to 1:1, v/v) to obtain the title compound (19.8 g, yield 59%) as a colorless amorphous compound.
¹H NMR (400MHz,CDCl₃):δ(ppm)=1.14 (3H, t, J=7.6 Hz), 2.50(2H, q, J=7.6 Hz), 3.48 (3H, s), 3.68(1H, d, J=15.2 Hz), 3.77 (1H, dd, J=10.6 and 7.8 Hz), 3.79 (1H, d, J=15.7 Hz), 4.06-4.00 (1H, m), 4.48 (2H, d, J=3.9 Hz), 4.59 (1H, dd, J=11.9 and 5.7 Hz), 4.78 (1H, dd, J=12.2 and 2.3 Hz), 5.37 (1H, d, J=7.4 Hz), 5.81-5.72 (2H, m), 6.93-6.87 (7H, m), 7.62-7.09 (12H, m), 7.90 (1H, d, J=7.1 Hz), 8.02 (1H, d, J=7.4 Hz), 8.10 (1H, d, J=7.0 Hz)
MS (FAB) m/z:731 (M + H)⁺.

### (27b) 2-(4-Ethylbenzyl)-5-(tetrahydrofuran-2-yl)oxymethylphenyl 4-O-methyl-β-D-glucopyranoside

The compound synthesized in (27a) (26.7 g, 36.6 mmol) was dissolved in methylene chloride (270 mL), followed by addition of p-toluenesulfonic acid monohydrate (0.34 g, 1.8 mmol) and 3, 4-dihydro-2H-pyran (3.97 mL, 43.9 mmol), the mixture was stirred at room temperature for 1 h. After the reaction was completed, triethylamine (0.5 mL, 3.59 mmol) was added, the reaction solvent was evaporated to approximately half of the volume under reduced pressure and diluted with ethyl acetate (300 mL). The mixture was washed with saturated brine (50 mL) and dried over anhydrous sodium sulfate, and then the solvent was evaporated under reduced pressure.

The resulting crude product was dissolved in 1,4-dioxane (200 mL) and methanol (100 mL), then 2 M aqueous sodium hydroxide (145 mL, 290 mmol) was added dropwise, and the mixture was heated to 40°C and stirred for 1 h. After the reaction was completed, the solvent was evaporated under reduced pressure, ethyl acetate (400 mL) and 15% brine (100 mL) were poured to the residue, and the organic layer was washed 3 times with saturated brine (50 mL). This organic layer was dried over anhydrous sodium sulfate, the solvent was evaporated under reduced pressure, and then the residue was purified by silica gel flash column chromatography (hexane:ethyl acetate, 1:1, v/v) to obtain the title compound (17.2 g, yield 95%) as a colorless amorphous compound.
¹H NMR (500MHz, CDCl₃): δ1.20 (3H, t, J=7.7 Hz), 1.88-1.50 (6H, m), 2.58 (2H, q, J=7.7 Hz), 3.22 (1H, t, J=9.3 Hz), 3.32-3.31 (1H, m), 3.42-3.39 (1H, m), 3.52 (1H, d, J=7.9 Hz), 3.56 (1H, d, J=8.8 Hz), 3.59 (3H, s), 3.71 (1H, dd, J=12.0 and 4.6 Hz), 3.83-3.85 (1H, m), 3.92-3.88 (1H, m), 3.95 (1H, d, J=15.1 Hz), 4.05 (1H, d, J=14.7 Hz), 4.45 (1H, d, J=11.8 Hz), 4.69 (1H, d, J=11.7 Hz), 4.70-4.68 (1H, m), 4.91 (1H, dd, J=7.3 and 4.4 Hz), 6.92 (1H, d, J=7.4 Hz), 7.02 (1H, d, J=7.8 Hz), 7.07 (2H, d, J=8.3 Hz), 7.14 (2H, d, J=7.8 Hz), 7.15 (1H, s)
MS (FAB) m/z: 503 (M + H)⁺.

### (27c) 2-(4-Ethylbenzyl)-5-(tetrahydrofuran-2-yl)oxymethylphenyl 4-O-methyl-6-O-(tetrahydrofuran-2-yl)oxyacetyl-β-D-glucopyranoside

Ethyl tetrahydrofuran-2-yloxyacetate (J. Chem. Soc., 1956, 2124-2126) (11.7 g, 62.0 mmol) was dissolved in ethanol (120 mL), followed by addition of 2 M aqueous sodium hydroxide (31 mg, 62.0 mmol), and the mixture was stirred at 40°C for 1 h. After the reaction was completed, the solvent was evaporated under reduced pressure, and the residue was dehydrated azeotropically twice with toluene (50 mL). 2,4,6-Trimethylpyridine (60 mL, 0.46 mol) and N,N-dimethylformamide (40 mL) were added to the resulting residue, followed by addition of the compound synthesized in (27b) (12.5 g, 24.9 mmol) and N-hydroxybenzotriazole (8.4 g, 62.0 mmol). This suspension was cooled to 0°C, followed by addition of 1-ethyl-3-(3-dimethylaminopropyl)carbodiimide hydrochloride (11.9 g, 62.0 mmol), and the mixture was stirred for 4 h as it was. After the reaction was completed, water (30 mL) was poured, the mixture was diluted with ethyl acetate (300 mL), and then the organic layer was washed successively with 2 M hydrochloric acid (230 mL, 0.46 mol), saturated aqueous sodium hydrogencarbonate (50 mL), and saturated brine (50 mL). The organic layer was dried over anhydrous sodium sulfate, the solvent was evaporated under reduced pressure, and then the residue was purified by silica gel flash column chromatography (hexane:ethyl acetate, 2:3, v/v) to obtain the title compound (7.54 g, yield 47%) as a colorless amorphous compound.
¹H NMR (400MHz, CD₃OD): δ1.19 (3H, t, J=7.7 Hz), 1.86-1.49 (12H, m), 2.58 (2H, q, J=7.7 Hz), 3.16 (1H, t, J=9.4 Hz), 3.44-3.41 (1H, m), 3.56-3.47 (2H, m), 3.58 (3H, s), 3.65-3.59 (1H, m), 3.83-3.78 (1H, m), 3.93-3.88 (1H, m), 3.94 (1H, d, J=14.5 Hz), 4.04 (1H, d, J=15.3 Hz), 4.23-4.21 (2H, m), 4.34-4.28 (1H, m), 4.44 (1H, s), 4.48 (1H, s), 4.72-4.64 (3H, m), 4.91-4.87 (1H, m), 6.93 (1H, d, J=8.6 Hz), 7.01 (1H, d, J=7.4 Hz), 7.07 (2H, d, J=8.2 Hz), 7.13 (2H, d, J=7.8 Hz), 7.14 (1H, s)
MS (FAB) m/z: 683 (M + K)⁺.

### (27d) 2-(4-Ethylbenzyl)-5-hydroxymethylphenyl 4-O-methyl-6-O-hydroxyacetyl-β-D-glucopyranoside

The compound synthesized in (27c) (5.98 g, 9.27 mmol) was dissolved in methanol (60 mL), followed by addition of 2 M hydrochloric acid (9.3 mL, 18.6 mmol) with ice cooling, and the mixture was stirred at room temperature for 30 min. After the reaction was completed, the mixture was neutralized with sodium hydrogencarbonate (1.56 g, 18.6 mmol), and the solvent was evaporated under reduced pressure to approximately half of the volume. The residue was diluted with ethyl acetate (200 mL) and washed twice with saturated brine (50 mL). The organic layer was dried over anhydrous sodium sulfate, the solvent was evaporated under reduced pressure, and then the residue was purified by silica gel flash column chromatography (2-propanol:methylene chloride, 7:93, v/v) to obtain the title compound (2.89 g, yield 65%) as a colorless amorphous compound.
¹H NMR (400MHz, CD₃OD): δ1.19 (3H, t, J=7.7 Hz), 2.58 (2H, q, J=7.7 Hz), 3.14 (1H, t, J=9.2 Hz), 3.49 (1H, dd, J=9.2 and 7.6 Hz), 3.57 (3H, s), 3.60-3.55 (1H, m), 3.68-3.64 (1H, m), 3.93 (1H, d, J=14.8 Hz), 4.02 (1H, d, J=15.3 Hz), 4.14 (2H, s), 4.32 (1H, dd, J=11.9 and 6.1 Hz), 4.45 (1H, dd, J=11.7 and 2.3 Hz), 4.55 (2H, s), 4.88 (1H, d, J=7.9 Hz), 6.92 (1H, d, J=7.8 Hz), 7.01 (1H, d, J=7.8 Hz), 7.13-7.05 (5H, m)
MS (FAB) m/z: 515 (M + K)⁺.

### (Example 28) 2-(4-Ethylbenzyl)-5-hydroxymethylphenyl 4-deoxy-6-O-hydroxyacetyl-β-D-glucopyranoside (Example Compound No. 2-5)

### (28a) 2-(4-Ethylbenzyl)-5-hydroxymethylphenyl 2,3,6-tri-O-benzoyl-4-deoxy-β-D-glucopyranoside

The compound synthesized in (26a) (6.42 g, 5.91 mmol) was dissolved in methanol (30 mL) and 1,4-dioxane (30 mL), followed by addition of 2 M aqueous sodium hydroxide (1.5 mL, 3.0 mmol) with ice cooling, and the mixture was stirred at 0°C for 15 min. The reaction mixture was neutralized with 2 M aqueous hydrochloric acid (1.4 mL, 2.8 mmol), and the solvent was evaporated under reduced pressure. The residue was diluted with ethyl acetate (30 mL) and washed with saturated brine (20 mL). The residue was dried over anhydrous sodium sulfate, and then the solvent was evaporated under reduced pressure. The residue was purified by silica gel flash column chromatography (hexane:ethyl acetate, 3:1 to 2:1 to 1:1, v/v) to obtain the title compound (1.30 g, yield 31.4%) as a pale yellow amorphous compound.
¹H NMR (400 MHz, CDCl₃):δ 1.14 (3H, t, J=7.6 Hz), 1.54-1.61 (1H, br, d, J=27.0 Hz), 1.96 (1H. dd, J=23.8, 11.8 Hz), 2.50 (2H, q, J=7,8 Hz), 2.54 (1H, m), 3.69 (1H, d, J=15.3 Hz), 3.80 (1H, d, J=15.2 Hz), 4.26-4.30 (1H, m), 4.43 (2H, s), 4.50 (2H, m), 5.34 (1H, d, J=7.8 Hz), 5.47-5.54 (1H, m), 5.79 (1H, dd, J=9.6, 7.6 Hz), 6.90 (6H, s), 7.08-7.61 (10H, m), 7.94 (2H, d, J=7.0 Hz), 7.99 (2H, d, J=7.1 Hz), 8.08(2H, d, J=7.0 Hz);
MS (FAB) m/z: 699 (M-H)⁺.

### (28b) 2-(4-Ethylbenzyl)-5-(tetrahydrofuran-2-yl)oxymethylphenyl 2,3,6-tri-O-benzoyl-4-deoxy-β-D-glucopyranoside

The compound synthesized in (28a) (2.53 g, 3.61 mmol) was dissolved in methylene chloride (25 mL), followed by addition of p-toluenesulfonic acid monohydrate (34 mg, 0.18 mmol) and 3,4-dihydro-2H-pyran (390 µL, 4.31 mmol) with ice cooling, and the mixture was stirred at room temperature for 2 h. The mixture was neutralized with triethylamine (50 µL) with ice cooling, then diluted with ethyl acetate (20 mL), and washed with saturated brine (10 mL). The mixture was dried over anhydrous sodium sulfate, and then the solvent was evaporated under reduced pressure. The residue was purified by silica gel flash column chromatography (hexane:ethyl acetate, 6:1 to 4:1, v/v) to obtain the title compound (2.60 g, yield 91.9%) as a pale yellow amorphous compound.
¹H NMR (400 MHz, CDCl₃): δ 1.15 (3H, t, J=7.7 Hz), 1.48-1.85 (8H, m), 2.96-3.13 (1H, m), 2.51 (2H, q, J=7.1 Hz), 2.56 (1H, m), 3.42-4.52 (1H, m), 3.68 (1H, d, J=15.2 Hz), 3.77-3.89 (2H, m), 4.25 (1H, d, J=12.1 Hz), 4.44-4.55 (2H, m), 4.61 (1H, d, J=11.7 Hz), 5.32-5.35 (1H, m), 5.48-5.54 (1H, m), 5.79 (1H, dd, J=9.8, 7.8 Hz), 6.90 (6H, s), 7.09 (1H, d, J=7.1 Hz), 7.32 -7.41 (4H, m), 7.44-7.53 (4H, m), 7.56-7.60 (1H, m), 7.94 (2H, d, J=8.2 Hz), 7.99 (2H, d, J= 8.2 Hz), 8.07 (2H, d, J=7.1 Hz); MS (FAB) m/z: 783 (M-H)⁺, 807 (M+Na)⁺.

### (28c) 2-(4-Ethylbenzyl)-5-(tetrahydrofuran-2-yl)oxymethylphenyl 4-deoxy-β-D-glucopyranoside

The compound synthesized in (28b) (2.60 g, 3.31 mmol) was dissolved in methanol (20 mL) and 1,4-dioxane (20 mL), followed by addition of 2 M aqueous sodium hydroxide (16.5 mL, 33.0 mmol), and the mixture was stirred at 40°C for 2 h. The solvent was evaporated under reduced pressure, and then the residue was diluted with ethyl acetate (20 mL) and washed with saturated aqueous ammonium chloride (10 mL) and saturated brine (10 mL). The mixture was dried over anhydrous sodium sulfate, and then the solvent was evaporated under reduced pressure. The residue was purified by silica gel flash column chromatography (hexane:ethyl acetate, 1:1 to 1:3, v/v) to obtain the title compound (1.52 g, yield 97.4%) as a colorless amorphous compound.
¹H NMR (400 MHz, CDCl₃):δ 1.20 (3H, t, J=7.4 Hz), 1.49-1.93 (9H, m), 2.23 (1H, br, d, J=23.0 Hz), 2.54 (1H, br, s), 2.60 (2H, q, J=7.5 Hz), 3.32 (1H, t, J=8.0 Hz), 3.48-3.58 (1H, m), 3.58-3.73 (4H, m), 3.82-3.92 (2H, m), 4.08 (1H, d, J=15.6 Hz), 4.48 (1H, dd, J=12.1, 3.1 Hz), 4.65-4.75 (3H, m), 7.00 (1H, s), 7.00-7.01 (1H, m), 7.1 (4H, dd, 16.0, 8.2 Hz), 7.21 (1H, d, J=7.8 Hz);
MS (FAB) m/z: 495 (M+Na)⁺.

### (28d) 2-(4-Ethylbenzyl)-5-(tetrahydrofuran-2-yl)oxymethylphenyl 4-deoxy-6-O-(tetrahydrofuran-2-yl)oxyacetyl-β-D-glucopyranoside

2,4,6-Trimethylpyridine (7.6 mL) and 1-hydroxybenzotriazole (566 mg, 4.19 mmol) was added to ethyl tetrahydrofuran-2-yloxyacetate (J. Chem. Soc., 1956, 2124-2126) (704 mg, 3.86 mmol), and the mixture was stirred at 0°C for 10 min. The compound synthesized in (28c) (1.52 mg, 3.22 mmol) and 1-ethyl-3-(3-dimethylaminopropyl)carbodiimide hydrochloride (802 mg, 4.18 mmol) were added to the reaction mixture, and the mixture was stirred at room temperature for 6 h. Distilled water (5 mL) was added with ice cooling, and the mixture was diluted with ethyl acetate (30 mL) and washed with 2 M aqueous hydrochloric acid (30 mL), saturated aqueous sodium hydrogencarbonate (20 mL), and saturated brine (10 mL). The mixture was dried over anhydrous sodium sulfate, and then the solvent was evaporated under reduced pressure. The residue was purified by silica gel flash column chromatography (hexane:ethyl acetate, 2:1 to 1:1 to 1:2, v/v) to obtain the title compound (1.26 g, yield 63.9%) as a colorless amorphous compound.
¹H NMR (400 MHz, CDCl₃): δ 1.20 (3H, t, J=7.6 Hz), 1.42 (1H, br, s), 1.51-1.88 (15H, m), 2.01 (1H, ddd, J=13.0, 5.0, 1.5 Hz), 2.50 (1H, br, s), 2.60 (2H, q, J=7.7 Hz), 3.32 (1H, t, J=8.2 Hz), 3.47-3.56 (2H, m), 3.62-3.70 (1H, m), 3.81-3.94 (4H, m), 4.08 (1H, d, J=15.2 Hz), 4.19-4.28 (3H, m), 4.49 (1H, dd, J=12.1, 3.1 Hz), 4.64-4.77 (3H, m), 6.95 (1H, s), 7.02 (1H, d, J=7.8 Hz), 7.08 (2H, d, J=8.2 Hz), 7.12 (1H, d, J=8.2 Hz), 7.20 (1H, d, J=7.8 Hz);
MS (FAB) m/z: 645 (M-H)⁺, 669 (M+Na)⁺.

### (28e) 2-(4-Ethylbenzyl)-5-hydroxymethylphenyl 4-deoxy-6-O-hydroxyacetyl-β-D-glucopyranoside

The compound synthesized in (28d) (1.26 g, 2.05 mmol) was dissolved in methanol (12 mL), followed by addition of macroporous polystyrene-bound p-toluenesulfonic acid (MP-TsOH, manufactured by Argonaut) (100 mg, 0.41 mmol), and the mixture was stirred at room temperature for 4 h. The mixture was filtered, and then the solvent was evaporated under reduced pressure. The resulting residue was purified by silica gel flash column chromatography (2-propanol:methylene chloride, 1:20 to 1:10, v/v) to obtain the title compound (492 mg, 53.8%) as a colorless solid.
¹H NMR (400 MHz, CD₃OD): δ 1.18 (3H, t, J=7.8 Hz), 1.49 (1H, dd, J=24.3, 11.7 Hz), 2.00 (1H, ddd, J=12.8, 5.2, 1.9 Hz), 2.58 (2H, q, J=7.8 Hz), 3.39 (1H, t, J=8.2 Hz), 3.71 (2H, m), 3.90-3.96 (1H, m), 3.94 (1H, d, J=14.9 Hz), 4.03 (1H, d, J=14.9 Hz), 4.12 (2H, s), 4.22-4.23 (2H, m), 4.55 (2H, s), 6.91 (1H, d, J=8.6 Hz), 7.01 (1H, d, J=7.9 Hz), 7.06 (2H, d, J=7.8 Hz), 7.13 (3H, d, J=8.2 Hz);
MS (FAB) m/z: 469 (M+Na)⁺.

### (Example 29) 2-(4-Ethylbenzyl)-5-hydroxymethylphenyl 4-C-methyl-6-O-hydroxyacetyl-β-D-glucopyranoside (Example Compound No. 1-74)

### (29a) 2-(4-Ethylbenzyl)-5-hydroxymethylphenyl 4-O-acetyl-2,3,6-tri-O-benzoyl-4-C-methyl-α-D-glucopyranoside

The compound synthesized in (3c) (2.5 g, 4.4 mmol) and 5-acetoxymethyl-2-(4-ethylbenzyl)phenol (1.3 g, 4.4 mmol) as an aglycone site were glycosylated by the same method in (3d), and the glycoside compound was subjected to the same step as in (28a) to obtain the title compound (0.47 g, yield 20%) as a colorless amorphous compound.
¹H NMR (500MHz, CDCl₃):δ1.14 (3H, t, J=7.7 Hz), 2.02 (3H, s), 2.50 (2H, q, J=7.7 Hz), 3.67 (1H, d, J=15.6 Hz), 3.78 (1H, d, J=15.1 Hz), 4.39 (2H, d, J=5.9 Hz), 4.51 (1H, dd, J=11.9 and 8.0 Hz), 4.68 (1H, dd, J=12.2 and 2.5 Hz), 5.46 (1H, dd, J=8.3 and 2.4 Hz), 5.49 (1H, d, J=8.3 Hz), 5.84 (1H, dd, J=9.8 and 7.8 Hz), 6.52 (2H, d, J=9.8 Hz), 6.92-6.87 (4H, m), 7.09 (1H, s), 7.31 (2H, t, J=7.8 Hz), 7.41 (3H, t, J=7.8 Hz), 7.49 (2H, t, J=7.4 Hz), 7.54 (1H, t, J=7.6 Hz), 7.61 (1H, t, J=7.3 Hz), 7.87 (2H, d, J=8.3 Hz), 8.00 (2H, d, J=7.3 Hz), 8.09 (2H, d, J=8.3 Hz).

### (29b) 2-(4-Ethylbenzyl)-5-(tetrahydrofuran-2-yl)oxymethylphenyl 4-C-methyl-β-D-glucopyranoside

The compound synthesized in (29a) (0.47 g, 0.60 mmol) was subjected to the same 2 steps as in (28b) and (28c) to obtain a crude product of the title compound (0.30 g).
¹H NMR (500MHz, CD₃OD): δ1.18 (3H, t, J=7.8 Hz), 1.87-1.51 (6H, m), 2.57 (2H, q, J=7.8 Hz), 3.30 (1H, s), 3.47-3.43 (3H, m), 3.53-3.49 (1H, m), 3.62 (1H, dd, J=11.7 and 8.3 Hz), 3.90-3.87 (2H, m), 3.94 (1H, d, J=15.0 Hz), 4.04 (1H, d, J=15.0 Hz), 4.44 (1H, dd, J=12.0 and 6.6 Hz), 4.70-4.67 (2H, m), 4.93-4.92 (1H, m), 6.92 (1H, d, J=7.3 Hz), 7.02 (1H, d, J=7.8 Hz), 7.06 (2H, d, J=7.8 Hz), 7.13 (2H, d, J=7.8 Hz), 7.19 (1H, s)
MS (FAB) m/z: 525 (M + Na)⁺.

### (29c) 2-(4-Ethylbenzyl)-5-(tetrahydrofuran-2-yl)oxymethylphenyl 4-C-methyl-6-O-(tetrahydrofuran-2-yl)oxyacetyl-β-D-glucopyranoside

The compound synthesized in (29b) (1.0 g, 2.0 mmol) was used to obtain the title compound (0.34 g, yield 26% in 2 steps) as a colorless amorphous compound by the same technique as in (28d).
¹H NMR (500MHz, CD₃OD): δ1.19 (6H, m, J=7.6 Hz), 1.89-1.46 (12H, m), 2.58 (2H, q, J=7.6 Hz), 3.47-3.45 (3H, m), 3.54-3.51 (1H, m), 3.60 (1H, d, J=7.4 Hz), 3.78 (1H, dd, J=9.0 and 2.8 Hz), 3.82 (1H, dd, J=9.5 and 3.1 Hz), 3.92-3.88 (1H, m), 3.94 (1H, d, J=15.1 Hz), 4.03 (1H, d, J=15.1 Hz), 4.30-4.18 (3H, m), 4.50-4.47 (2H, m), 4.63 (1H, dd, J=6.6 and 3.1 Hz), 4.68-4.66 (1H, m), 4.68 (1H, d, J=7.3 Hz), 4.72 (1H, d, J=11.7 Hz), 4.90-4.87 (1H, m), 6.95-6.93 (1H, m), 7.02 (1H, d, J=7.8 Hz), 7.07 (2H, d, J=7.8 Hz), 7.13 (2H, d, J=8.3 Hz), 7.13 (1H, s)
MS (FAB) m/z: 645 (M + H)⁺.

### (29d) 2-(4-Ethylbenzyl)-5-hydroxymethylphenyl 4-C-methyl-6-O-hydroxyacetyl-β-D-glucopyranoside

The compound synthesized in (29c) (0.79 g, 1.2 mmol) was dissolved in methanol (8 mL), followed by addition of macroporous polystyrene-bound p-toluenesulfonic acid (MP-TsOH, manufactured by Argonaut) (90 mg, 0.37 mmol), and the mixture was stirred at room temperature for 5 h. After the reaction was completed, the catalyst was removed by filtration, then the solvent was evaporated under reduced pressure, and the residue was purified by silica gel flash column chromatography (ethanol:methylene chloride, 7:93, v/v) to obtain the title compound (0.45 g, yield 79%) as a colorless amorphous compound.
¹H NMR (500MHz, CD₃OD): δ1.18 (3H, t, J=7.4 Hz), 2.57 (2H, q, J=7.4 Hz), 3.47-3.46 (2H, m), 3.64 (1H, d, J=9.3 Hz), 3.93 (1H, d, J=15.1 Hz), 4.03 (1H, d, J=15.1 Hz), 4.14 (2H, s), 4.27 (1H, dd, J=11.8 and 8.8 Hz), 4.50 (1H, d, J=11.8 Hz), 4.56 (2H, s), 4.89 (1H, dd, J=4.9 and 2.5 Hz), 6.92 (1H, d, J=7.8 Hz), 7.01 (1H, d, J=7.4 Hz), 7.06 (2H, d, J=7.8 Hz), 7.12 (2H, d, J=7.8 Hz), 7.14 (1H, s)
MS (FAB) m/z: 515 (M + K)⁺.

### (Example 30) 2-(4-Ethylbenzyl)-5-hydroxymethylphenyl 4-C-methyl-6-O-acetyl-β-D-glucopyranoside (Example Compound No. 1-70)

### (30a) 2-(4-Ethylbenzyl)-5-(tetrahydrofuran-2-yl)oxymethylphenyl 4-C-methyl-6-O-acetyl-β-D-glucopyranoside

The compound synthesized in (29b) (0.14 g, 0.28 mmol) was dissolved in 2,4,6-trimethylpyridine (1.4 mL, 11 mmol), followed by addition of acetyl chloride (46 µmL, 0.64 mmol) with ice cooling, and the mixture was stirred for 4 h as it was. After the reaction was completed, methanol (0.1 mL) was added, the mixture was diluted with ethyl acetate (50 mL), and the organic layer was washed successively with 2 M hydrochloric acid (5 mL, 10 mmol), saturated aqueous sodium hydrogencarbonate (5 mL), and saturated brine (5 mL). The organic layer was dried over anhydrous sodium sulfate, the solvent was evaporated under reduced pressure, and then the residue was purified by silica gel flash column chromatography (2-propanol:methylene chloride, 5:95, v/v) to obtain the title compound (90 mg, yield 59%) as a colorless amorphous compound.
¹H NMR (400MHz, CD₃OD): δ1.17 (3H, t, J=7.4 Hz), 1.18 (3H, s), 1.84-1.48 (6H, m), 2.05 (3H, s), 2.55 (2H, q, J=7.4 Hz), 3.31-3.30 (1H, m), 3.61 (1H, d, J=8.6 Hz), 3.90-3.83 (1H, m), 3.95 (1H, d, J=15.1 Hz), 4.03 (1H, d, J=15.1 Hz), 4.17 (1H, t, J=10.3 Hz), 4.47-4.41 (2H, m), 4.70-4.63 (2H, m), 4.90-4.87 (1H, m), 7.16-6.92 (7H, m)
MS (FAB) m/z: 567 (M + Na)⁺.

### (30b) 2-(4-Ethylbenzyl)-5-hydroxymethylphenyl 4-C-methyl-6-O-acetyl-β-D-glucopyranoside

The compound synthesized in (30a) (0.61 g, 1.1 mmol) was used to obtain the title compound (0.40 g, yield 79%) as a colorless amorphous compound by the same technique as in (28e).
¹H NMR (500MHz, CD₃OD): δ1.20-1.18 (6H, m), 2.06 (3H, s), 2.57 (2H, q, J=7.8 Hz), 3.47-3.46 (2H, m), 3.62 (1H, d, J=8.8 Hz), 3.94 (1H, d, J=15.1 Hz), 4.03 (1H, d, J=15.1 Hz), 4.16 (1H, dd, J=11.5 and 8.8 Hz), 4.42 (1H, d, J=11.5 Hz), 4.55 (2H, s), 4.89 (1H, dd, J=5.4 and 2.5 Hz), 6.93 (1H, d, J=7.8 Hz), 7.01 (1H, d, J=7.8 Hz), 7.06 (2H, d, J=7.8 Hz), 7.12 (2H, d, J=7.8 Hz), 7.16 (1H, s)
MS (FAB) m/z:483 (M + Na)⁺.

### (Example 31) 2-(4-(Methyloxy)benzyl)-5-hydroxymethylphenyl 4-C-methyl-6-O-hydroxyacetyl-β-D-glucopyranoside (Example Compound No. 1-93)

### (31a) 2-(4-(Methyloxy)benzyl)-5-hydroxymethylphenyl 2,3,6-O-tribenzoyl-4-O-acetyl-4-C-methyl-β-D-glucopyranoside

The compound synthesized in (3c) (10.0 g, 17.8 mmol), trichloroacetonitrile (7.10 mL, 71.1 mmol), 1,8-diazabicyclo[5.4.0]-7-undecene (0.54 mL, 3.6 mmol), and methylene chloride (60 mL) were used to prepare an imidate by the same technique as in (1b), and subsequently 5-acetoxymethyl-2-(4-methoxybenzyl)phenol synthesized in (8b) (5.10 g, 17.8 mmol), a boron trifluoride-diethyl ether complex (0.34 mL, 2.7 mmol), and methylene chloride (30 mL) were converted in the same manner as in (1b). The resulting crude product was converted in the same manner as in (28a) to obtain the title compound (3.65 g, yield 26%) as a colorless amorphous compound.
¹H NMR (500MHz, CDCl₃): δ1.69 (3H, s), 2.02 (3H, s), 3.66 (1H, d, J=15.1 Hz), 3.68 (3H, s), 3.74 (1H, d, J=15.1 Hz), 4.39 (2H, s), 4.51 (1H, dd, J=12.0 and 8.0 Hz), 4.68 (1H, dd, J=12.0 and 2.2 Hz), 5.46 (1H, dd, J=9.0 and 3.0 Hz), 5.48 (1H, d, J=8.3 Hz), 5.84 (1H, t, J=9.0 Hz), 6.52 (1H, d, J=9.8 Hz), 6.60 (2H, d, J=8.3 Hz), 6.87 (2H, d, J=8.3 Hz), 6.89 (2H, s), 7.09 (2H, s), 7.31 (2H, t, J=7.8 Hz), 7.41 (2H, t, J=7.8 Hz), 7.50-7.46 (3H, m), 7.54 (1H, t, J=7.3 Hz), 7.61 (1H, t, J=7.3 Hz), 7.86 (2H, d, J=7.3 Hz), 8.00 (2H, d, J=7.3 Hz), 8.09 (2H, d, J=7.3 Hz)
MS (FAB) m/z: 797 (M + Na)⁺.

### (31b) 2-(4-(Methyloxy)benzyl)-5-(tetrahydrofuran-2-yl)oxymethylphenyl 4-C-methyl-β-D-glucopyranoside

The compound synthesized in (31a) (5.7 g, 7.4 mmol) was used to obtain a crude product of the title compound (3.73 g) by the same technique as in (28c).
¹H NMR (400MHz, CD₃OD): δ1.14 (3H, s), 1.88-1.51 (6H, m), 3.48-3.44 (2H, m), 3.56-3.50 (1H, m), 3.63 (1H, dd, J=12.0 and 8.2 Hz), 3.74 (3H, s), 3.91-3.85 (2H, m), 3.92 (1H, d, J=14.9 Hz), 4.02 (1H, d, J=14.9 Hz), 4.45 (1H, dd, J=12.0 and 4.9 Hz), 4.71-4.67 (2H, m), 4.94-4.91 (1H, m), 6.80 (2H, d, J=8.8 Hz), 6.94 (1H, d, J=7.6 Hz), 7.03 (1H, d, J=7.6 Hz), 7.15 (2H, d, J=8.8 Hz), 7.20 (1H, s)
MS (FAB) m/z: 505 (M + H)⁺.

### (31c) 2-(4-(Methyloxy)benzyl)-5-(tetrahydrofuran-2-yl)oxymethylphenyl 4-C-methyl-6-O-(tetrahydrofuran-2-yl)oxyacetyl-β-D-glucopyranoside

The compound synthesized in (31b) (1.1 g, 2.2 mmol) was converted in the same manner as in (28d). The resulting crude product was purified by silica gel flash column chromatography (2-propanol:methylene chloride, 5:95, v/v) to obtain the title compound (0.54 g, yield 38% in 2 steps) as a colorless amorphous compound.
¹H NMR (500MHz, CDCl₃): δ1.19 (3H, s), 1.89-1.46 (12H, m), 3.48-3.42 (3H, m), 3.54-3.50 (1H, m), 3.60 (1H, d, J=9.3 Hz), 3.74 (3H, s), 3.82-3.79 (1H, m), 3.92-3.88 (1H, m), 3.90 (1H, d, J=17.1 Hz), 3.92 (1H, dd, J=15.1 and 2.2 Hz), 4.01 (1H, d, J=15.1 Hz), 4.30-4.18 (3H, m), 4.51-4.46 (2H, m), 4.73-4.58 (4H, m), 6.79 (2H, d, J=8.8 Hz), 6.96-6.93 (1H, m), 7.01 (1H, d, J=7.8 Hz), 7.11 (1H, s), 7.14 (2H, d, J=8.3 Hz)
MS (FAB) m/z: 647 (M + H)⁺.

### (31d) 2-(4-(Methyloxy)benzyl)-5-hydroxymethylphenyl 4-C-methyl-6-O-hydroxyacetyl-β-D-glucopyranoside

The compound synthesized in (31c) (2.1 g, 3.2 mmol) was used to obtain the title compound (0.59 g, yield 39%) as a colorless amorphous compound by the same technique as in (28e).
¹H NMR (400MHz, CD₃OD): δ1.19 (3H, s), 3.31-3.30 (3H, m), 3.48-3.47 (2H, m), 3.64 (1H, dd, J=8.8 and 1.8 Hz), 3.74 (3H, s), 3.91 (1H, d, J=14.9 Hz), 4.00 (1H, d, J=14.9 Hz), 4.14 (2H, s), 4.27 (1H, dd, J=11.6 and 8.8 Hz), 4.50 (1H, dd, J=11.6 and 2.0 Hz), 4.56 (2H, s), 4.89 (1H, dd, J=5.1 and 2.7 Hz), 6.79 (1H, d, J=8.7 Hz), 6.92 (1H, d, J=7.5 Hz), 7.01 (2H, d, J=7.5 Hz), 7.13 (2H, d, J=8.7 Hz), 7.14 (1H, s)
MS (FAB) m/z: 517 (M + K)⁺.

### (Example 32) 5-Methyl-2-(4-methoxybenzyl)phenyl 7-deoxy-D-glycero-β-D-gluco-heptopyranoside (Example Compound No. 1-51)

### (32a) 5-(Acetyloxy)methyl-2-(4-methoxybenzyl)phenyl 7-deoxy-D-glycero-β-D-gluco-heptopyranoside

The compound synthesized in (9b) (670 mg, 1.59 mmol) was dissolved in 2,4,6-trimethylpyridine (6.7 mL) and methylene chloride (6.7 mL), a solution of acetyl chloride (190 mg, 2.42 mmol) in methylene chloride (0.8 mL) was added at - 45°C, and the mixture was gradually heated to -25°C. Methanol (1 mL) was added to terminate the reaction, followed by addition of ethyl acetate (30 mL) and water (30 mL), and the mixture was stirred at room temperature. The mixture was filtered, and the resulting white solid was washed successively with dilute hydrochloric acid (2 M, 25 mL), water (10 mL x 2), methanol (10 mL), and ethyl acetate (10 mL) and dried under reduced pressure to obtain the title compound (406 mg, 55.2%) as a white solid.
¹H NMR (400 MHz, DMSO-d₆):δ 1.07 (3H, d, J=6.7 Hz), 2.04 (3H, s), 3.15-3.21 (1H, m), 3.25-3.34 (3H, m), 3.70 (3H, s), 3.83 (1H, d, J=14.5 Hz), 3.89-3.96 (1H, m), 3.96 (1H, d, J=14.5 Hz), 4.67 (1H, d, J=4.3 Hz), 4.77 (1H, d, J=7.0 Hz), 4.95 (1H, d, J=12.5 Hz), 4.99 (1H, d, J=12.5 Hz), 5.12-5.16 (2H, m), 5.33 (1H, d, J=5.1 Hz), 6.79-6.83 (2H, m), 6.90-6.92 (1H, m), 7.05-7.10 (2H, m), 7.18-7.22 (2H, m);
MS (FAB) m/z: 463 (M+H)⁺.

### (32b) 5-Methyl-2-(4-methoxybenzyl)phenyl 7-deoxy-D-glycero-β-D-gluco-heptopyranoside

The compound synthesized in (32a) (100 mg, 0.216 mmol) was suspended in methanol (7 mL) and ethyl acetate (3 mL), followed by addition of palladium on carbon (10% by weight Pd, wet, 32 mg), and the mixture was stirred under a hydrogen atmosphere at room temperature for 2 h. Insoluble matters were removed by Celite filtration, and the solvent was evaporated under reduced pressure. The residue was purified by silica gel flash column chromatography (ethyl acetate:methanol, 19:1, v/v) to obtain the title compound (78.3 mg, 89.6%) as a white solid. ¹H NMR (400 MHz, CD₃OD):δ 1.22 (3H, d, J=6.7 Hz), 2.28 (3H, s), 3.33-3.40 (2H, m), 3.42-3.49 (2H, m), 3.74 (3H, s), 3.89 (1H, d, J=14.9 Hz), 3.96 (1H, d, J=14.9 Hz), 4.02-4.08 (1H, m), 4.87-4.88 (1H, m), 6.73-6.76 (1H, m), 6.77-6.81 (2H, m), 6.90 (1H, d, J=7.8 Hz), 6.97 (1H, brs), 7.11-7.15 (2H, m);
MS (FAB) m/z: 405 (M+H)⁺.

### (Example 33) 5-(Acetyloxy)methyl-2-(4-ethylbenzyl)phenyl 7-deoxy-L-glycero-β-D-gluco-heptopyranoside (Example Compound No. 1-105)

The compound synthesized in (6e) (870 mg, 2.08 mmol) was dissolved in 2,4,6-trimethylpyridine (8.7 mL) and methylene chloride (8.7 mL), a solution of acetyl chloride (240 mg, 3.06 mmol) in methylene chloride (0.5 mL) was added at - 45°C, and the mixture was gradually heated to -25°C. Methanol (1 mL) was added to terminate the reaction, followed by addition of dilute hydrochloric acid (2 M, 33 3 mL), and the mixture was extracted with ethyl acetate (100 mL x 2). The organic layer was washed successively with dilute hydrochloric acid (2 M, 15 mL) and saturated aqueous sodium hydrogencarbonate (15 mL) and dried over anhydrous sodium sulfate. The solvent was evaporated under reduced pressure, and the residue was purified by silica gel flash column chromatography (methylene chloride:methanol, 12:1 to 9:1, v/v) to obtain the title compound (820 mg, 85.6%) as a white solid.
¹H NMR (400 MHz, CD₃OD):δ 1.19 (3H, t, J=7.7 Hz), 1.32 (3H, d, J=6.6 Hz), 2.04 (3H, s), 2.58 (2H, q, J=7.7 Hz), 3.22 (1H, dd, J=9.2 and 1.7 Hz), 3.43-3.54 (2H, m), 3.63 (1H, t, J=9.2 Hz), 3.95 (1H, d, J=14.8 Hz), 4.05 (1H, d, J=14.8 Hz), 4.09-4.14 (1H, m), 4.89 (1H, d, J=7.7 Hz), 5.03 (2H, s), 6.92-6.94 (1H, m), 7.03-7.08 (3H, m), 7.13-7.15 (3H, m);
MS (FAB) m/z: 461 (M+H)⁺.

### (Example 34) 5-(Hydroxyacetyloxy)methyl-2-(4-methoxybenzyl)phenyl 7-deoxy-D-glycero-β-D-gluco-heptopyranoside (Example Compound No. 1-106)

### (34a) 5-[(Allyloxycarbonyloxy)acetyloxy]methyl-2-(4-methoxybenzyl)phenyl 7-deoxy-D-glycero-β-D-gluco-heptopyranoside

The compound synthesized in (9b) (100 mg, 0.238 mmol) was dissolved in 2,4,6-trimethylpyridine (1.0 mL) and methylene chloride (1.0 mL), a solution of 2-(allyloxycarbonyloxy)acetyl chloride (EP1362856A1) (64.0 mg, 0.358 mmol) in methylene chloride (1.0 mL) was added at -45°C, and the mixture was gradually heated to -25°C. Ethanol (1 mL) was added to terminate the reaction, followed by addition of dilute hydrochloric acid (2 M, 3.8 mL), and the mixture was extracted with ethyl acetate (20 mL x 2). The organic layer was washed with saturated aqueous sodium hydrogencarbonate (10 mL) and dried over anhydrous magnesium sulfate. The solvent was evaporated under reduced pressure, and the residue was purified by silica gel flash column chromatography (methylene chloride:ethanol, 12:1 to 9:1, v/v) to obtain the title compound (102 mg, 76.1%) as a white solid.
¹H NMR (400 MHz, CD₃OD):δ 1.23 (3H, d, J=6.7 Hz), 3.36-3.42 (2H, m), 3.44-3.51 (2H, m), 3.74 (3H, s), 3.93 (1H, d, J=14.8 Hz), 4.01 (1H, d, J=14.8 Hz), 4.02-4.08 (1H, m), 4.63 (2H, dt, J=5.6 and 1.5 Hz), 4.70 (2H, s), 4.90-4.95 (1H, m), 5.14 (1H, d, J=12.1 Hz), 5.17-5.24 (2H, m), 5.31-5.37 (1H, m), 5.87-5.97 (1H, m), 6.78-6.82 (2H, m), 6.92-6.95 (1H, m), 7.04 (1H, d, J=7.4 Hz), 7.13-7.16 (3H, m);
MS (FAB) m/z: 563 (M+H)⁺.

### (34b) 5-(Hydroxyacetyloxy)methyl-2-(4-methoxybenzyl)phenyl 7-deoxy-D-glycero-β-D-gluco-heptopyranoside

The compound synthesized in (34a) (98.1 mg, 0.174 mmol) was dissolved in methylene chloride (3.0 mL) and tetrahydrofuran (1.0 mL), bis(triphenylphosphine)palladium(II) dichloride (12 mg, 0.017 mmol), and tri-n-butyltin hydride (50 µL, 0.19 mmol) were successively added, and the mixture was stirred at room temperature for 25 min. The mixture was diluted with methylene chloride (10 mL) and purified as it was by silica gel flash column chromatography (methylene chloride:ethanol, 7:1 to 6:1, v/v) to obtain the title compound (59.3 mg, 71.2%) as a white solid.
¹H NMR (400 MHz, CD₃OD):δ 1.22 (3H, d, J=6.7 Hz), 3.36-3.40 (2H, m), 3.44-3.51 (2H, m), 3.74 (3H, s), 3.93 (1H, d, J=14.8 Hz), 4.01 (1H, d, J=14.8 Hz), 4.03-4.09 (1H, m), 4.14 (2H, s), 4.90 (1H, d, J=7.4 Hz), 5.14 (2H, s), 6.78-6.82 (2H, m), 6.94-6.96 (1H, m), 7.03-7.05 (1H, m), 7.13-7.17 (3H, m);
MS (FAB) m/z: 501 (M+Na)⁺.

### (Example 35) 2-(4-Ethylbenzyl)-5-hydroxymethylphenyl 6-O-hydroxyacetyl-5-C-methyl-β-D-glucopyranoside (Example Compound No. 1-73)

### (35a) 2-(4-Ethylbenzyl)-5-hydroxymethylphenyl 2,3,4,6-tetra-O-benzoyl-5-C-methyl-β-D-glucopyranoside

The compound synthesized in (4d) (3.46 g, 3.95 mmol) was dissolved in methanol (20 mL) and 1,4-dioxane (20 mL), followed by addition of 2 N aqueous sodium hydroxide (990 µL, 1.97 mmol) with ice cooling, and the mixture was stirred with ice cooling for 1 h. Subsequently, 2 N aqueous sodium hydroxide (395 µL, 0.79 mmol) was added to the reaction mixture, the mixture was stirred with ice cooling for 1 h, followed by addition of 2 N hydrochloric acid (1.39 mL, 2.76 mmol), the solvent was evaporated under reduced pressure, and the residue was diluted with ethyl acetate and washed successively with water and saturated brine. The organic layer was dried over anhydrous sodium sulfate, then the solvent was evaporated under reduced pressure, and the resulting residue was purified by silica gel flash column chromatography (hexane:ethyl acetate, 4:1 1 to 1.5:1, v/v) to obtain the title compound (1.33 g, 40%) as a white amorphous compound.
¹H NMR (400MHz,CDCl₃):δ1.15(3H, t, J=7.6Hz), 1.80(3H, s), 2.51(2H, q, J=7.6Hz), 3.80(2H, s), 4.29(1H, d, J=11.7Hz), 4.62-4.54(3H, m), 5.71(1H, d, J=6.6Hz), 5.81-5.75(1H, m), 6.08(2H, d, J=4.3Hz), 6.97-6.82(6H, m), 7.20(1H, s), 7.32-7.24(2H, m), 7.41-7.33(6H, m), 7.56-7.41(4H, m), 7.87(2H, d, J=8.2Hz), 8.02-7.90(6H, m)
MS (FAB) m/z:941 (M + H)⁺.

### (35b) 2-(4-Ethylbenzyl)-5-(tetrahydrofuran-2-yl)oxymethylphenyl 5-C-methyl-β-D-glucopyranoside

The compound synthesized in (35a) (3.85 g, 4.61 mmol) was dissolved in dichloromethane (40 mL), followed by addition of a molecular sieve 4A (approx. 1 g) and paratoluenesulfonic acid monohydrate (44 mg, 0.23 mmol), the mixture was stirred, then dihydropyran (460 µL, 5.07 mmol) was added dropwise, and the mixture was stirred at room temperature for 40 min. Subsequently, dihydropyran (40 µL, 0.46 mmol) was added to the reaction mixture, and the mixture was stirred at room temperature for 20 min. Triethylamine (130 µL, 0.92 mmol) was added to terminate the reaction, the mixture was diluted with ethyl acetate, washed with saturated brine, dried over anhydrous sodium sulfate, and filtered using a silica gel pad for simplified purification, and the solvent was evaporated under reduced pressure. The resulting residue was dissolved in ethanol (40 mL) and tetrahydrofuran (40 mL), 2 N aqueous sodium hydroxide (22.5 mL, 45.0 mmol) was added dropwise, and the mixture was stirred at 45°C for 1 h. The solvent was evaporated under reduced pressure, and the residue was diluted with ethyl acetate and washed successively with saturated aqueous sodium hydrogencarbonate and saturated brine. The organic layer was dried over anhydrous sodium sulfate, then the solvent was evaporated under reduced pressure, and the residue was dried under reduced pressure to obtain the title compound (2.31 g, quantitative) as a brown white amorphous compound. The product was used in the subsequent reaction without further purification.
¹H NMR (400MHz,CDCl₃):δ1.20(3H, t, J=7.6Hz), 1.31(3H, s), 1.70-1.49(5H, m), 1.80-1.70(1H, m), 1.92-1.81(1H, m), 2.24-2.15(1H, m), 2.60(2H, q, J=7.6Hz), 2.86(2H, brd, J=14.1Hz), 3.40(1H, brt, J=8.4Hz), 3.71-3.52(4H, m), 3.86-3.77(2H, m), 3.95-3.87(1H, m), 4.05(1H, d, J=15.2Hz), 4.48(1H, d, J=12.1Hz), 4.73(1H, dd, J=6.9 and 3.3Hz), 4.78(1H, d, J=12.1Hz), 5.02(1H, dd, J=7.6 and 4.1Hz), 6.98(1H, d, J=7.6Hz), 7.08-7.04(3H, m), 7.12(2H, d, J=8.2Hz), 7.20(1H, d, J=7.6Hz)
MS (FAB) m/z:525 (M + Na)⁺.

### (35c) 2-(4-Ethylbenzyl)-5-(tetrahydrofuran-2-yl)oxymethylphenyl 5-C-methyl-6-O-(tetrahydrofuran-2-yl)oxyacetyl-β-D-glucopyranoside

Tetrahydropyran-2-yloxyethyl acetate ester (J. Chem. Soc., 1956, 2124-2126.) (1.13 g, 6.00 mmol) was dissolved in ethanol (15 mL), followed by addition of 2 N aqueous sodium hydroxide (2.3 ml, 4.58 mmol), and the mixture was stirred at room temperature for 2 h. The solvent was evaporated under reduced pressure, the residue was dried under reduced pressure, followed by addition of the compound synthesized in (35b) (2.30 g, 4.58 mmol), and the mixture was dissolved in collidine (11.5 mL, 87.3 mmol) and dichloromethane (4.6 mL). 1-Hydroxybenzotriazole (928 mg, 6.87 mmol) and 1-ethyl-3-(3-dimethylaminopropyl)carbodiimide hydrochloride (966 mg, 5.04 mmol) were added with ice cooling, and the mixture was stirred at room temperature for 3 days. The reaction mixture was diluted with ethyl acetate, washed successively with 1 N hydrochloric acid (87 mL, 87 mmol), saturated brine, saturated aqueous sodium hydrogencarbonate, and saturated brine, and dried over anhydrous sodium sulfate, and then the solvent was evaporate under reduced pressure. The residue was purified by silica gel flash column chromatography (dichloromethane:isopropyl alcohol, 19:1 to 5.5:1, v/v) to obtain the title compound (1.54 g, 52%) as a pale yellow amorphous compound.
¹H NMR (400MHz, CDCl₃):δ1.20(3H, t, J=7.6Hz), 1.33-1.24(1H, m), 1.37(3H, s), 1.44-1.39(1H, m), 1.92-1.49(11H, m), 2.60(2H, q, J=7.6Hz), 2.68(1H, brs), 2.96(1H, dt, J=25.7 and 3.5Hz), 3.41(1H, brt, J=8.4Hz), 3.63-3.47(3H, m), 3.68(1H, brt, J=9.4Hz), 3.96-3.78(3H, m), 4.14-4.05(2H, m), 4.28(2H, brt, J=1.9Hz), 4.40-4.32(1H, m), 4.48(1H, dd, J=12.2 and 2.4Hz), 4.75-4.69(2H, m), 4.78(1H, d, J=12.2Hz), 4.99(1H, brd, J=7.8Hz), 6.98(1H, d, J=7.4Hz), 7.10-7.04(3H, m), 7.13(2H, d, J=8.2Hz), 7.19(1H, d, J=7.4Hz)
MS (FAB) m/z:645 (M + H)⁺.

### (35d) 2-(4-Ethylbenzyl)-5-hydroxymethylphenyl 6-O-hydroxyacetyl-5-C-methyl-β-D-glucopyranoside

The compound synthesized in (35c) (2.32 g, 3.60 mmol) was dissolved in methanol (25 mL), followed by addition of macroporous polystyrene-bound paratoluenesulfonic acid (MP-TsOH, manufactured by Argonaut) (4.07 mmol/g; 265 mg, 1.08 mmol), and the mixture was stirred at room temperature for 4 h. The reaction mixture was filtered using a filter paper, the solvent was evaporated under reduced pressure, and then the resulting residue was purified by silica gel flash column chromatography (dichloromethane:isopropyl alcohol, 13:1 to 5.5:1, v/v). The resulting product was washed with hexane to obtain the title compound (450 mg, 26%) as a white amorphous compound.
¹H NMR (400MHz, CD₃OD):δ1.19(3H, t, J=7.4Hz), 1.34(3H, s), 2.58(2H, q, J=7.4Hz), 3.47(1H, dd, J=9.2 and 7.9Hz), 3.53(1H, d, J=9.2Hz), 3.65(1H, t, J=9.2Hz), 3.95(1H, d, J=14.8Hz), 4.01(1H, d, J=14.8Hz), 4.12(2H, s), 4.14(1H, d, J=11.4Hz), 4.25(1H, d, J=11.4Hz), 4.56(2H, s), 5.13(1H, d, J=7.9Hz), 6.91(1H, brd, J=7.8Hz), 7.00(1H, d, J=7.8Hz), 7.07(2H, d, J=8.2Hz), 7.12(2H, d, J=8.2Hz), 7.20(1H, brs)
MS (FAB) m/z:499 (M + Na)⁺.

### (Example 36) 2-(4-Methoxybenzyl)phenyl 7-deoxy-D-glycero-β-D-gluco-heptopyranoside (Example Compound No. 1-2)

2,3,4,6-Tetra-O-benzoyl-7-deoxy-D-glycero-α,β-D-gluco-heptopyranoside synthesized in (7d) (0.43 g, 0.70 mmol), methylene chloride (10 mL), trichloroacetonitrile (0.36 mL, 3.57 mmol), and 1,8-diazabicyclo[5.4.0]-7-undecene (22 µL, 0.15 mmol) were used to synthesize an imidate (0.67 g) by the same method as in (1b). The resulting imidate (0.67 g), 2-(4-methoxybenzyl)phenol (0.10 g, 0.47 mmol), methylene chloride (10 mL), and a boron trifluoride-diethyl ether complex (59 µL, 0.47 mmol) were used to synthesize 2-(4-methoxybenzyl)phenyl 2,3,4,6-tetra-O-benzoyl-7-deoxy-D-glycero-β-D-gluco-heptopyranoside (0.39 g) as a colorless viscous oily crude product by the same method as in (1b). The resulting glycoside compound (0.39 g), methanol (5 mL), tetrahydrofuran (5 mL), and 2 N aqueous sodium hydroxide (5 mL) were used to obtain 2-(4-methoxybenzyl)phenyl 7-deoxy-D-glycero-β-D-gluco-heptopyranoside (100 mg, yield 55%) as a white powder by the same method as in (6e).
¹H NMR (400 MHz, CD₃OD): δ 1.21 (3H, d, J=6.3 Hz), 3.50-3.35 (4H, m), 3.74 (3H, s), 4.02 (1H, d, J=14.4 Hz), 4.02 (1H, d, J=14.4 Hz), 4.06-4.04 (1H, m), 4.88 (1H, d, J=7.5 Hz), 6.80 (2H, d, J=8.6 Hz), 6.93-6.89 (1H, m), 7.03 (1H, d, J=7.8 Hz), 7.16-7.13 (4H, m);
MS (FAB) m/z: 390 (M)⁺.

### (Example 37) 3-(4-Methoxybenzyl)pyridin-2-yl 7-deoxy-D-glycero-β-D-gluco-heptopyranoside (Example Compound No. 1-108)

### (37a) 2-Benzyloxypyridin-3-yl-4-methoxyphenyl methanol

1-Bromo-4-methoxybenzene (3.40 g, 18.5 mmol), tetrahydrofuran (80 mL), a solution of 2.64 mol/L n-butyllithium in n-hexane (6.90 mL, 18.2 mmol), and 2-benzyloxypyridine-3-carbaldehyde (3.0 g, 14.1 mmol) were used to obtain the title compound (3.30 g, yield 73%) as an oil by the same method as in (12b).
¹H NMR (500 MHz, CDCl₃): δ 2.75 (1H, d, J = 4.5 Hz), 3.80 (3H, s), 5.38 (2H, s), 5.95 (1H, d, J = 4.5 Hz), 6.85 (2H, d, J = 8.6 Hz), 6.93 (1H, dd, J = 7.3 and 5.0 Hz), 7.24 - 7.33 (7H, m), 7.67 (1H, dd, J = 7.3 and 1.4 Hz), 8.09 (1H, dd, J = 5.0 and 1.9 Hz);
MS ((FAB)m/z: 322 (M+H)⁺.

### (37b) 2-Benzyloxypyridin-3-yl-4-methoxyphenylmethyl acetate

The compound synthesized in (37a) (3.30 g, 9.90 mmol), pyridine (20 mL), acetic anhydride (2.80 mL, 29.6 mmol), and 4-dimethylaminopyridine (120 mg, 0.98 mmol) were used to obtain the title compound (3.50 g, yield 97%) as an oil by the same method as in (12c). ¹H NMR (400 MHz, CDCl₃):δ2.10 (3H, s), 3.79 (3H, s), 5.37 (2H, d, J=4.0 Hz), 6.83 (2H, d, J = 8.6 Hz), 6.93 (1H, dd, J = 7.4 and 5.1 Hz), 7.04 (1H, s), 7.24 - 7.33 (7H, m), 7.69 - 7.72 (1H, m), 8.11 (1H, dd, J = 5.1 and 2.0 Hz);
MS (FAB)m/z: 364 (M+H)⁺.

### (37c) 3-(4-Methoxybenzyl)pyridin-2-ol

The compound synthesized in (37b) (3.50 g, 9.60 mmol), 10% palladium on carbon (350 mg), methanol (32 mL), and tetrahydrofuran (8 mL) were used to obtain the title compound (2.06 g, yield 99%) as a colorless solid by the same method as in (12d).
¹H NMR (400 MHz, CDCl₃): δ 3.80 (3H, s), 3.83 (2H, s), 6.18 (1H, t, J = 6.6 Hz), 6.87 (2H, d, J = 8.6 Hz), 7.07 - 7.09 (1H, m), 7.19 (2H, d, J = 8.6 Hz), 7.25 (1H, dd, J = 6.2 and 2.0 Hz);
MS (EI)m/z: 215 (M)⁺.

### (37d) 3-(4-Methoxybenzyl)pyridin-2-yl 2,3,4,6-tetra-O-benzoyl-7-deoxy-D-glycero-β-D-gluco-heptopyranoside

The compound obtained in (7d) (200 mg, 0.28 mmol), methylene chloride (6 mL), and 30% hydrobromide-acetic acid solution (0.40 mL) were used to prepare a bromo sugar by the same method as in (11a). Subsequently, 3-(4-methoxybenzyl)-2-hydroxypyridine (60 mg, 0.26 mmol), methylene chloride (2 mL), and silver carbonate (92 mg, 0.33 mmol) were used to obtain a crude product of the title compound (170 mg) by the same method as in (11a).

### (37e) 3-(4-Methoxybenzyl)pyridin-2-yl 7-deoxy-D-glycero-β-D-gluco-heptopyranoside

The crude product obtained in (37d) (170 mg, 0.21 mmol), methanol (6 mL), methylene chloride (1.5 mL), and potassium carbonate (290 mg, 2.10 mmol) were used to obtain the title compound (31 mg, yield 28%) as a colorless solid by the same method as in (11b).
¹H NMR (400 MHz, CD₃OD): δ 1.15 (3H, d, J = 6.6 Hz), 3.36 - 3.44 (2H, m), 3.46-3.54 (2H, m), 3.76 (3H, s), 3.88 (1H, d, J = 15.0 Hz), 3.97 (1H, d, J = 15.0 Hz), 4.00 - 4.02 (1H, m), 5.87 (1H, d, J = 7.8 Hz), 6.83 (2H, d, J = 8.6 Hz), 6.94 (1H, dd, J = 7.6 and 5.0 Hz), 7.18 (2H, d, J = 8.6 Hz), 7.41 (1H, d, J = 7.6 Hz), 7.98 (1H, d, J = 5.0 Hz);
MS (FAB)m/z: 430 (M+K)⁺.

### (Example 38) 3-(4-Methoxybenzyl)-5-hydroxymethylpyridin-2-yl 7-deoxy-D-glycero-β-D-gluco-heptopyranoside (Example Compound No. 1-109)

### (38a) Methyl 5-(4-methoxybenzyl)pyridine-2-carboxylate

2-Hydroxymethyl-5-(4-methoxybenzyl)pyridine (US4109000A1) (880 mg, 4.05 mmol) was dissolved in chloroform (20 mL), followed by addition of manganese dioxide (4.20 g, 48.3 mmol), and the mixture was stirred at 50°C for 1 h. The reaction mixture was cooled to room temperature and filtered using Celite, and then the solvent was removed under reduced pressure to obtain a crude product (550 mg) as an oil.

The resulting crude product (550 mg, 2.56 mmol) was dissolved in methyl alcohol (5 mL), the mixture was cooled to 0°C, followed by addition of a solution of potassium hydroxide (370 mg, 6.59 mmol) in methyl alcohol (3 mL) and iodine (840 mg, 3.31 mmol), and the mixture was stirred at 0°C for 1 h. Subsequently, saturated aqueous sodium sulfite (30 mL) was added, the mixture was extracted with ethyl acetate (50 mL), and then the organic layer was washed with saturated brine (20 mL). The organic layer was dried over anhydrous sodium sulfate, and then the solvent was removed under reduced pressure. The residue was passed through a short column to obtain the title compound (610 mg, yield 62%) as an oil.
¹H NMR (400 MHz, CDCl₃): δ 3.80 (3H, s), 4.00 (2H + 3H, s), 6.86 (2H, d, J = 8.8 Hz), 7.09 (2H, d, J = 8.8 Hz), 7.59 (1H, dd, J = 8.1 and 2.1 Hz), 8.05 (1H, d, J = 8.1 Hz), 8.63 (1H, d, J = 2.1 Hz);
MS (EI)m/z: 257 (M)⁺.

### (38b) Methyl 6-hydroxy-5-(4-methoxybenzyl)pyridine-2-carboxylate

The compound obtained in (38a) (610 mg, 2.49 mmol) was dissolved in chloroform (10 mL), followed by addition of 75% m-chloroperbenzoic acid (1.14 g, 4.95 mmol), and the mixture was stirred at room temperature for 30 h. Chloroform (20 mL) was further added, and the mixture was washed with saturated aqueous sodium hydrogencarbonate (30 mL). The organic layer was dried over anhydrous sodium sulfate, and then the solvent was removed under reduced pressure. The residue was passed through a short column to obtain a crude product (590 mg) as an oil.

The resulting crude product (590 mg) was dissolved in acetic anhydride (6.0 mL), and the mixture was heated to reflux for 7 h. The mixture was cooled to room temperature, and then the solvent was removed under reduced pressure. Methylene chloride (20 mL) was further added, and the mixture was washed with saturated sodium hydrogencarbonate (20 mL). The organic layer was dried over anhydrous sodium sulfate, and then the solvent was removed under reduced pressure. The residue was passed through a short column to obtain a crude product (280 mg) as an oil.

The resulting crude product (280 mg, 0.89 mmol) was dissolved in methyl alcohol (3 mL), followed by addition of potassium carbonate (60 mg, 0.43 mmol), and the mixture was stirred at room temperature for 1 h. The reaction mixture was filtered using Celite, and then the solvent was removed under reduced pressure. Ethyl acetate (15 mL) was added, and the mixture was washed with saturated aqueous ammonium chloride (10 mL) and saturated brine (10 mL). The organic layer was dried over anhydrous sodium sulfate, and then the solvent was removed under reduced pressure to obtain a crude product of the title compound (240 mg, yield 35%). The resulting crude product was dehydrated azeotropically with toluene and used in the subsequent reaction as it was.
¹H NMR (400 MHz, CD₃OD): δ 3.77 (3H, s), 3.80 (2H, s), 3.92 (3H, s), 6.86 (2H, d, J = 9.0 Hz), 7.02 (1H, d, J = 7.2 Hz), 7.16 (2H, d, J = 9.0 Hz), 7.28 (1H, d, J = 7.2 Hz);
MS (EI)m/z: 273 (M)⁺.

### (38c) 6-Hydroxymethyl-3-(4-methoxybenzyl)pyridin-2-ol

The crude product obtained in (38b) (240 mg, 0.84 mmol) was dissolved in tetrahydrofuran (4 mL), the mixture was cooled to -20°C, followed by addition of lithium aluminium hydride (67 mg, 1.76 mmol), and the mixture was stirred at 0°C for 10 min. Subsequently, 2 N hydrochloric acid (10 mL) and saturated brine (10 mL) were added, the mixture was extracted with ethyl acetate (20 mL), and the organic layer was washed with saturated brine (10 mL). The organic layer was dried over anhydrous sodium sulfate, and then the solvent was removed under reduced pressure to obtain a solid crude product (170 mg, yield 78%). The resulting crude product was used in the subsequent reaction as it was.

### (38d) 6-Acetoxymethyl-3-(4-methoxybenzyl)pyridin-2-ol

The crude product obtained in (38c) (170 mg, 0.69 mmol) was dissolved in tetrahydrofuran (4.0 mL), followed by addition of vinyl acetate (2.0 mL) and bis(dibutylchlorotin)oxide (380 mg, 0.69 mmol), and the mixture was stirred at 30°C for 16 h. The solvent was removed under reduced pressure. The residue was purified by silica gel flash chromatography (methylene chloride:methyl alcohol 100:0 to 20:1, v/v) to obtain the title compound (150 mg, yield 75%) as a pale yellow solid.
¹H NMR (400 MHz, CD₃OD):δ 2.13 (3H, s), 3.78 (2H, s), 3.79 (3H, s), 4.93 (2H, s), 6.13 (1H, d, J = 6.8 Hz), 6.85 (2H, d, J = 8.6 Hz), 7.06 (1H, d, J = 6.8 Hz), 7.18 (2H, d, J = 8.6 Hz);
MS (EI)m/z: 287 (M)⁺.

### (38e) 3-(4-Methoxybenzyl)-5-acetoxymethylpyridin-2-yl 2,3,4,6-tetra-O-benzoyl-7-deoxy-D-glycero-β-D-gluco-heptopyranoside

The bromo sugar prepared by the same method as in (11a) (394 mg, 0.59 mmol), 6-acetoxymethyl-3-(4-methoxybenzyl)pyridin-2-ol (84 mg, 0.29 mmol), methylene chloride (4 mL), and silver carbonate (160 mg, 0.58 mmol) were used to obtain a crude product of the title compound (212 mg) by the same method as in (11a).

### (38f) 3-(4-Methoxybenzyl)-5-hydroxymethylpyridin-2-yl 7-deoxy-D-glycero-β-D-gluco-heptopyranoside

The crude product obtained in (38e) (212 mg), methanol (8.0 mL), methylene chloride (2.0 mL), and potassium carbonate (333 mg, 2.41 mmol) were used to obtain the title compound (67 mg, yield 66%) as a white solid by the same method as in (11b).
¹H NMR (400 MHz, CD₃OD): δ 1.44 (3H, d, J = 6.3 Hz), 4.21 - 4.23 (2H, m), 4.36-4.39 (2H, m), 4.68 (3H, s), 4.81 (1H, d, J = 15.3 Hz), 4.93 (1H, d, J = 15.3 Hz), 4.99 (1H, dd, J = 6.5 and 3.7 Hz), 5.67 (2H, s), 7.44 (1H, d, J = 7.8 Hz), 8.52 (2H, d, J = 8.8 Hz), 8.76 (1H, d, J = 7.4 Hz), 8.96 (2H, d, J = 8.8 Hz), 9.23 (1H, d, J = 7.4 Hz); MS (FAB)m/z: 422 (M+H)⁺.

### (Example 39) 2-(4-Ethylbenzyl)-5-hydroxymethylphenyl 4-C-methyl-7-deoxy-glycero-β-D-gluco-heptapyranoside (Example Compound No. 1-110)

### (39a) Methyl-2,3,4-tri-O-benzyl-4-C-methyl-7-deoxy-glycero-α-D-gluco-heptapyranoside

Oxalyl chloride (1.48 mL, 17.3 mmol) was dissolved in methylene chloride (55 mL), the mixture was cooled to -78°C, followed by addition of a solution of dimethyl sulfoxide (2.44 mL, 34.4 mmol) in methylene chloride (23 mL), and the mixture was stirred at -78°C for 15 min. A solution of methyl-2,3,4-tri-O-benzyl-4-C-methyl-α-D-glucopyranoside(Bull. Chem. Soc. Jpn., EN, 67, 6, 1994; 1633-1640.) (5.49 g, 11.5 mmol) in methylene chloride (17 mL) was added to the resulting solution, and the mixture was stirred at -78°C for 30 min. Triethylamine (9.50 mL, 68.6 mmol) was further added, and the mixture was heated to 0°C over 1 h. Subsequently, water (40 mL) was added, the mixture was extracted with ethyl acetate (200 mL), and then the organic layer was washed with 1 N hydrochloric acid (50 mL) and saturated aqueous sodium hydrogencarbonate (50 mL). The organic layer was dried over anhydrous sodium sulfate, and then the solvent was removed under reduced pressure. The residue was filtered using a silica gel to obtain a crude product (5.49 g) as an amorphous aldehyde compound. The resulting aldehyde compound was dehydrated azeotropically with toluene and used in the subsequent reaction as it was.

The aldehyde compound (5.49 g, 11.5 mmol) was dissolved in tetrahydrofuran (100 mL), the mixture was cooled to -78°C, a solution of methylmagnesium bromide (34.6 mmol) in tetrahydrofuran (36 mL) was added dropwise over 10 min, and the mixture was heated to 0°C and stirred for 30 min. Subsequently, saturated aqueous ammonium chloride (150 mL) was added, the mixture was extracted with ethyl acetate (300 mL), and then the organic layer was washed with saturated brine (100 mL). The organic layer was dried over anhydrous sodium sulfate, and then the solvent was removed under reduced pressure. The residue was purified by silica gel flash chromatography (hexane:ethyl acetate 20:1 to 3:1, v/v) to obtain a high polarity side title compound (3.65 g, yield 64%) and a low polarity side C6 epimer (1.27 g, yield 64%). The high polarity side compound (Rf value: 0.19) was used in the subsequent reaction.

### Title compound

### Rf value: 0.19 (hexane:ethyl acetate 3:1)

¹H NMR (400 MHz, CDCl₃): δ 1.25 - 1.28 (3H, m), 1.46 (3H, s), 2.10 (1H, d, J = 7.4 Hz), 3.41 (3H, s), 3.50 - 3.54 (2H, m), 4.02 (1H, d, J = 9.8 Hz), 4.10 - 4.14 (1H, m), 4.59 - 4.79 (6H, m), 5.05 (1H, d, J = 11.0 Hz), 7.25 - 7.36 (15H, m);
MS (FAB)m/z: 493 (M+H)⁺.

### C6 epimer

### Rf value: 0.33 (hexane:ethyl acetate 3:1)

¹H NMR (400 MHz, CDCl₃): δ 1.22 (3H, d, J = 5.9 Hz), 1.53 (3H, s), 3.40 (3H, s), 3.47 - 3.50 (2H, m), 4.07 - 4.19 (3H, m), 4.53 (1H, d, J = 3.9 Hz), 4.59 (1H, d, J = 12.1 Hz), 4.69 - 4.82 (4H, m), 5.14 (1H, d, J = 11.4 Hz), 7.25 - 7.34 (15H, m);
MS (FAB)m/z: 493 (M+H)⁺.

### (39b) Methyl-4-O-acetyl-4-C-methyl-2,3,6-tri-O-benzoyl-7-deoxy-glycero-α-D-gluco-heptapyranoside

The compound obtained in (39a) (4.60 g, 17.3 mmol) was dissolved in methyl alcohol (55 mL), followed by addition of 2 N hydrochloric acid (0.93 mL) and 20% wet palladium hydroxide on carbon (4.2 g), and the mixture was stirred at room temperature under a hydrogen atmosphere for 3 h. The solvent was removed from the resulting solution by filtration under reduced pressure. The resulting crude product (approx. 2.0 g) was dehydrated azeotropically with toluene and used in the subsequent reaction as it was.

The resulting crude product (approx. 2.0 g) was dissolved in methylene chloride (70 mL), followed by addition of triethylamine (25 mL, 180 mmol), the mixture was cooled to 0°C, followed by addition of benzoyl chloride (10.5 mL, 90.4 mmol), and stirred at 40°C for 2 h and at room temperature for 10 h. The mixture was cooled to 0°C, followed by addition of methyl alcohol (5 mL), and stirred at room temperature for 30 min, followed by addition of ethyl acetate (200 mL). The mixture was washed with saturated brine (50 mL), saturated aqueous sodium hydrogencarbonate (50 mL), and saturated brine (50 mL) in this order. The organic layer was dried over anhydrous sodium sulfate, and then the solvent was removed under reduced pressure. The residue was filtered using a silica gel, and the resulting crude product (approx. 13.0 g) was used in the subsequent reaction as it was.

The resulting crude product (approx. 13.0 g) was dissolved in pyridine (36 mL), followed by addition of acetic anhydride (4.3 mL, 45.5 mmol) and 4-dimethylaminopyridine (220 mg, 1.80 mmol), and the mixture was stirred at 60°C for 6 h. The mixture was cooled to 0°C and extracted with ethyl acetate (200 mL) and 1 N hydrochloric acid (100 mL), and then the organic layer was washed with saturated brine (100 mL). The organic layer was dried over anhydrous sodium sulfate, and then the solvent was removed under reduced pressure. The residue was purified by silica gel flash chromatography (hexane:ethyl acetate 19:1 to 4:1, v/v) to obtain the title compound (4.62 g, yield 86%) as an amorphous compound.
¹H NMR (400 MHz, CD₃OD): δ 1.48 (3H, d, J = 6.7 Hz), 1.62 (3H, s), 1.99 (3H, s), 3.43 (3H, s), 5.16 - 5.20 (2H, m), 5.26 (1H, dd, J = 10.4 and 4.1 Hz), 5.52 - 5.55 (1H, m), 6.56 (1H, d, J = 10.4 Hz), 7.33 - 7.61 (9H, m), 7.94 - 8.00 (4H, m), 8.09 - 8.11 (2H, m);
MS (FAB)m/z: 577(M+H)⁺.

### (39c) 4-O-Acetyl-4-C-methyl-2,3,6-tri-O-benzoyl-7-deoxy-glycero-α,β-D-gluco-heptapyranoside

The compound obtained in (39b) (4.62 g, 8.01 mmol), acetic acid (14 mL), acetic anhydride (23 mL), and concentrated sulfuric acid (2.20 mL) were used to obtain acetyl-2,3,6-tri-O-benzoyl-4-O-acetyl-4-C-methyl-7-deoxy-glycero-α-D-glucopyranoside (4.56 g) as an amorphous compound by the same method as in (2a).

This amorphous residue (4.56 g), hydrazine acetate (1.04 g, 11.3 mmol), and N,N-dimethylformamide (50 mL) were used to obtain the title compound (3.98 g, yield 88%) as an amorphous compound by the same method as in (2a).
¹H NMR (500 MHz, CDCl₃): δ 1.46 (2H, d, J = 6.3 Hz), 1.48 (1H, d, J = 6.4 Hz), 2.00 (2H, s), 2.01 (1H, s), 3.16 (2/3H, d, J = 4.2 Hz), 3.24 (1/3 H, d, J = 3.9 Hz), 3.79 (2/3H, d, J = 8.2 Hz), 3.84 (1/3H, d, J = 8.0 Hz), 4.99 (2/3H, d, J = 4.4 Hz), 5.05 (2/3H, t, J = 8.2 Hz), 5.13 (1/3H, t, J = 8.0 Hz), 5.18 (1/3H, d, J = 4.8 Hz), 5.29 - 5.36 (4/3H, m), 5.41 (2/3H, d, J = 3.9 Hz), 5.47 - 5.55 (1H, m), 5.70 (2/3H, t, J = 4.2 Hz), 5.74 (1/3H, t, J = 3.9 Hz), 6.42 (2/3H, d, J = 10.2 Hz), 6.61 (2/3H, d, J = 10.2 Hz), 6.68 (1/3H, d, J = 11.1 Hz), 6.87 (1/3H, d, J = 11.1 Hz), 7.31 - 7.60 (8H, m), 7.86 - 7.99 (4H, m), 8.10 (2H, d, J = 6.8 Hz);
MS (FAB)m/z: 573(M+H)⁺.

### (39d) 5-Acetoxymethyl-2-(4-ethylbenzyl)phenyl 4-O-acetyl-4-C-methyl-2,3,6-tri-O-benzoyl-7-deoxy-glycero-β-D-gluco-heptapyranoside

The compound obtained in (39c) (200 mg, 0.36 mmol), trichloroacetonitrile (180 mL, 1.78 mmol), 1,8-diazabicyclo[5.4.0]-7-undecene (50 µL, 0.03 mmol), and methylene chloride (4 mL) were used to prepare an imidate by the same method as in (1b). Subsequently, 5-acetoxymethyl-2-(4-ethylbenzyl)phenol (96 mg, 0.34 mmol), a boron trifluoride-diethyl ether complex (0.044 mL, 0.35 mmol), and methylene chloride (4 mL) were used to obtain a crude product of the title compound (120 mg) by the same method as in (1b).

### (39e) 2-(4-Ethylbenzyl)-5-hydroxymethylphenyl 4-C-methyl-7-deoxy-glycero-β-D-gluco-heptapyranoside

The crude product obtained in (39d) (120 mg), potassium carbonate (200 mg, 1.45 mmol), methanol (6 mL), and methylene chloride (1.5 mL) were used to obtain the title compound (25 mg, yield 17%) as a colorless solid by the same method as in (1c). However, purification was performed by silica gel flash column chromatography (methylene chloride:methanol, 50:1 to 15:1, v/v).
¹H NMR (400 MHz, CD₃OD): δ 1.19 (3H, t, J = 7.6 Hz), 1.27 (3H, s), 1.34 (3H, d, J = 6.2 Hz), 2.57 (2H, q, J = 7.6 Hz), 3.12 (1H, d, J = 3.5 Hz), 3.40 (1H, d, J = 9.6 Hz), 3.50 (1H, dd, J = 9.6 and 7.4 Hz), 3.92 (1H, d, J = 14.9 Hz), 4.03 - 4.08 (2H, m), 4.55 (2H, s), 4.93 (1H, d, J = 7.4 Hz), 6.93 (1H, d, J = 7.8 Hz), 7.05 (1H, d, J = 7.8 Hz), 7.06 (2H, d, J = 7.9 Hz), 7.14 (2H, d, J = 7.9 Hz), 7.18 (1H, s);
MS (FAB)m/z: 471 (M+K)⁺.

### (Example 40) 2-(4-Methoxybenzyl)-5-hydroxymethylphenyl 4-C-methyl-7-deoxy-glycero-β-D-gluco-heptapyranoside (Example Compound No. 1-111)

### (40a) 5-Acetoxymethyl-2-(4-methoxybenzyl)phenyl 4-O-acetyl-4-C-methyl-2,3,6-tri-O-benzoyl-7-deoxy-glycero-β-D-gluco-heptapyranoside

The compound obtained in (39c) (200 mg, 0.36 mmol), trichloroacetonitrile (180 mL, 1.78 mmol), 1,8-diazabicyclo[5.4.0]-7-undecene (50 µL, 0.03 mmol), and methylene chloride (4 mL) were used to prepare an imidate by the same method as in (1b). Subsequently, 5-acetoxymethyl-2-(4-methoxybenzyl)phenol (96 mg, 0.34 mmol), a boron trifluoride-diethyl ether complex (0.044 mL, 0.35 mmol), and methylene chloride (4 mL) were used to obtain a crude product of the title compound (150 mg) by the same method as in (1b).

### (40b) 2-(4-Methoxybenzyl)-5-hydroxymethylphenyl 4-C-methyl-7-deoxy-glycero-β-D-gluco-heptapyranoside

The crude product obtained in (40a) (150 mg), potassium carbonate (250 mg, 1.80 mmol), methanol (6 mL), and methylene chloride (1.5 mL) were used to obtain the title compound (50 mg, yield 34%) as a colorless solid by the same method as in (1c). However, purification was performed by silica gel flash column chromatography (methylene chloride:methanol, 50:1 to 15:1, v/v).
¹H NMR (400 MHz, CD₃OD): δ 1.28 (3H, s), 1.34 (3H, d, J = 6.6 Hz), 3.12 (1H, d, J = 3.6 Hz), 3.40 (1H, d, J = 9.8 Hz), 3.50 (1H, dd, J = 9.8 and 7.6 Hz), 3.74 (3H, s), 3.90 (1H, d, J = 14.8 Hz), 4.02 (1H, d, J = 14.8 Hz), 4.07 (1H, dd, J = 6.6 and 3.6 Hz), 4.54 (2H, s), 4.93 (1H, d, J = 7.6 Hz), 6.79 (2H, d, J = 8.6 Hz), 6.93 (1H, d, J = 7.8 Hz), 7.04 (1H, d, J= 7.8 Hz), 7.15 (2H, d, J = 8.6 Hz), 7.18 (1H, s);
MS (FAB)m/z: 473 (M+K)⁺.

### (Example 41) 5-Amino-2-(4-ethylbenzyl)phenyl 4-C-methyl-7-deoxy-glycero-β-D-gluco-heptapyranoside (Example Compound No. 1-112)

The compound obtained in (39c) (1.00 g, 1.78 mmol), trichloroacetonitrile (0.89 mL, 8.81 mmol), 1,8-diazabicyclo[5.4.0]-7-undecene (0.027 mL, 0.18 mmol), and methylene chloride (20 mL) were used to prepare an imidate by the same method as in (1b). Subsequently, benzyl N-{4-(4-ethylbenzyl)-3-hydroxyphenyl}carbamate (600 mg, 1.66 mmol), a boron trifluoride-diethyl ether complex (0.22 mL, 1.74 mmol), and methylene chloride (20 mL) were used to obtain 5-benzyloxycarboamino-2-(4-ethylbenzyl)phenyl 2,3,6-tri-O-benzoyl-4-O-acetyl-4-C-methyl-7-deoxy-glycero-α-D-glucopyranoside (730 mg) as an amorphous crude product by the same method as in (16).

The resulting glycoside compound (730 mg), 20% wet palladium hydroxide on carbon (240 mg), methyl alcohol (12 mL), and tetrahydrofuran (3 mL) were used to obtain 5-amino-2-(4-ethylbenzyl)phenyl 2,3,6-tri-O-benzoyl-4-O-acetyl-4-C-methyl-7-deoxy-glycero-α-D-glucopyranoside (620 mg) as an amorphous crude product by the same method as in (16).

The resulting amino compound (620 mg, 0.80 mmol), potassium carbonate (1.11 g, 8.03 mmol), methanol (20 mL), and methylene chloride (5 mL) were used to obtain the title compound (220 mg, yield 32%) as a colorless amorphous compound by the same method as in (1c). However, purification was performed by silica gel flash column chromatography (methylene chloride:methanol, 20:1 to 10:1, v/v).
¹H NMR (400 MHz, CD₃OD): δ 1.18 (3H, t, J = 7.6 Hz), 1.24 (3H, s), 2.59 (3H, d, J = 7.5 Hz), 2.57 (2H, q, J = 7.6 Hz), 3.08 (1H, d, J = 4.3 Hz), 3.38 (1H, d, J = 9.8 Hz), 3.45 (1H, dd, J = 9.8 and 7.5 Hz), 3.80 (1H, d, J = 14.9 Hz), 3.92 (1H, d, J = 14.9 Hz), 4.02 (1H, dd, J = 7.5 and 4.3 Hz), 4.85 (1H, d, J = 7.5 Hz), 6.34 (1H, dd, J = 7.9 and 2.1 Hz), 6.57 (1H, d, J = 2.1 Hz), 6.80 (1H, d, J = 7.9 Hz), 7.04 (2H, d, J = 8.2 Hz), 7.11 (2H, d, J = 8.2 Hz);
MS (ESI)m/z: 440 (M+Na)⁺.

### (Example 42) 3-Chloro-2-(4-methoxybenzyl)phenyl 7-deoxy-D-glycero-β-D-gluco-heptopyranoside (Example Compound No. 1-113)

### (42a) 3-Hydroxy-2-(4-methoxybenzoyl)-cyclohex-2-enone

A solution of 4-methoxybenzoylchloride (6.10 g, 35.7 mmol) in acetonitrile (15 mL) was added to a solution of 1,3-cyclohexanedione (4.00 g, 35.7 mmol) and triethylamine (15.0 mL, 108 mmol) in acetonitrile (60 mL) in a water bath, and the mixture was stirred for 10 min. Subsequently, trimethylsilyl cyanide (0.29 mL, 2.17 mmol) was added, and the mixture was stirred at 60°C for 4 h. The mixture was cooled to room temperature, and then the solvent was removed under reduced pressure. Subsequently, water (50 mL) and 2 N hydrochloric acid (50 mL) were added, the mixture was extracted with ethyl acetate (100 mL), and then the organic layer was washed with saturated brine (50 mL). The organic layer was dried over anhydrous sodium sulfate, and then the solvent was removed under reduced pressure. The residue was passed through a short column to obtain a crude product of the title compound (4.30 g).

### (42b) 3-Chloro-2-(4-methoxybenzoyl)-cyclohex-2-enone

Oxalyl chloride (0.86 mL, 10.0 mmol) and 1 drop of dimethylformamide were added to a solution of the crude product obtained in (42a) (2.20 g, 9.16 mmol) in methylene chloride (20 mL) on an ice bath, and the mixture was stirred at room temperature for 1.5 h. The solvent was removed under reduced pressure. The residue was purified by silica gel flash chromatography (hexane:ethyl acetate 10:1 1 to 3:1, v/v) to obtain the title compound (1.93 g, yield 80%) as a pale yellow solid.
¹H NMR (400 MHz, CDCl₃): δ 2.19 - 2.26 (2H, m), 2.58 (2H, t, J=6.6 Hz), 2.88 (2H, t, J=6.3 Hz), 3.88 (3H, s), 6.95 (2H, d, J=9.0 Hz), 7.83 (2H, d, J=9.0 Hz);
MS (EI)m/z: 264,266 (M)⁺.

### (42c) (2-Chloro-6-hydroxyphenyl)-(4-methoxyphenyl)-methanone

The compound obtained in (42b) (460 mg, 1.74 mmol), sodium iodide (1.30 g, 8.67 mmol), triethylamine (1.20 mL, 8.61 mmol), and trimethylsilane chloride (1.00 mL, 8.11 mmol) were dissolved in acetonitrile (20 mL), and the mixture was stirred at room temperature for 10 h and at 50°C for 5 h. The mixture was cooled to 0°C, followed by addition of toluene (20 mL), and washed with a neutral phosphate-buffered pH standard solution (40 mL). The organic layer was dried over anhydrous sodium sulfate, and then the solvent was removed under reduced pressure.

The resulting oily crude product was dissolved in toluene (5 mL), followed by addition of N-iodosuccinimide (390 mg, 1.74 mmol), and the mixture was stirred at 80°C for 1 h. Subsequently, triethylamine (0.25 mL, 1.74 mmol) was added, and the mixture was stirred at 50°C for 10 min. Subsequently, 2 N hydrochloric acid (10 mL) was added, the mixture was extracted with ethyl acetate (20 mL), and then the organic layer was washed with saturated brine (20 mL). The organic layer was dried over anhydrous sodium sulfate, and then the solvent was removed under reduced pressure. The residue was purified by silica gel flash chromatography (hexane:ethyl acetate 10:1 1 to 1:1, v/v) to obtain the title compound (230 mg, yield 48%) as an amorphous compound.
¹H NMR (400 MHz, CDCl₃): δ 3.89 (3H, s), 6.94 - 7.00 (3H, m), 7.31 (1H, t, J= 8.2 Hz), 7.78 (2H, d, J= 9.0 Hz), 8.11 (1H, s);
MS (EI)m/z: 262, 264 (M)⁺.

### (42d) 3-Chloro-2-{hydroxy(4-methoxyphenyl)methyl}phenol

Sodium borohydride (0.55 g, 14.5 mmol) was added to a solution of the compound obtained in (42c) (1.90 g, 7.23 mmol) in methanol (20 mL) with ice cooling, and the mixture was stirred at room temperature for 30 min. An appropriate amount of saturated aqueous ammonium chloride was added, and the solvent was removed under reduced pressure. Ethyl acetate (50 mL) was added, and the mixture was washed with saturated aqueous ammonium chloride (50 mL) and saturated brine (50 mL) in this order. The organic layer was dried over anhydrous sodium sulfate, and then the solvent was removed under reduced pressure. The residue was purified by silica gel flash chromatography (hexane:ethyl acetate 10:1 to 1:1, v/v) to obtain the title compound (1.40 g, yield 74%) as a white solid.
¹H NMR (400 MHz, CDCl₃): δ 2.99 (1H, d, J = 2.8 Hz), 3.79 (3H, s), 6.45 (1H, d, J = 2.8 Hz), 6.85 - 6.90 (3H, m), 7.13 (1H, t, J = 8.2 Hz), 7.37 (2H, d, J = 8.6 Hz), 9.02 (1H, s);
MS (EI)m/z: 264, 266 (M)⁺.

### (42e) 3-Chloro-2-(4-methoxybenzyl)phenol

A solution of the compound obtained in (42d) (1.40 g, 5.29 mmol) in acetonitrile (30 mL) was cooled to -5°C, followed by addition of triethylsilane (2.50 mL, 15.7 mmol) and a boron fluoride-diethyl ether complex (0.99 mL, 7.88 mmol), and the mixture was stirred at -5°C for 1 h and then at room temperature for 1 h. Saturated aqueous sodium hydrogencarbonate (60 mL) was added with ice cooling, the mixture was extracted with ethyl acetate (100 mL), and then the organic layer was washed with saturated brine (50 mL). The organic layer was dried over anhydrous sodium sulfate, and then the solvent was removed under reduced pressure. The residue was purified by silica gel flash chromatography (hexane:ethyl acetate 10:1 1 to 3:1, v/v) to obtain the title compound (1.27 g, yield 96%) as a white solid.
¹H NMR (400 MHz, CDCl₃): δ 4.71(3H, s), 5.16 (2H, s), 6.26 (1H, s), 8.39(2H, d, J = 7.8 Hz), 8.52 (2H, d, J = 8.6 Hz), 8.75 - 8.83 (3H, m), 8.98 (2H, d, J = 8.6 Hz);
MS (EI)m/z: 248, 250 (M)⁺.

### (42f) 3-Chloro-2-(4-methoxybenzyl)phenyl 2,3,4,6-tetra-O-benzoyl-7-deoxy-D-glycero-β-D-gluco-heptopyranoside

The compound obtained in (7d) (200 mg, 0.33 mmol), trichloroacetonitrile (0.165 mL, 1.63 mmol), 1,8-diazabicyclo[5.4.0]-7-undecene (40 µL, 0.27 mmol), and methylene chloride (5 mL) were used to prepare an imidate by the same method as in (1b). Subsequently, the compound obtained in (42e) (74 mg, 0.30 mmol), a boron trifluoride-diethyl ether complex (0.041 mL, 0.33 mmol), and methylene chloride (5 mL) were used to obtain a crude product of the title compound (170 mg) by the same method as in (1b). The resulting crude product was used in the subsequent reaction as it was.

### (42g) 3-Chloro-2-(4-methoxybenzyl)phenyl 7-deoxy-D-glycero-β-D-gluco-heptopyranoside

The crude product obtained in (42f) (170 mg), potassium carbonate (270 mg, 1.95 mmol), methanol (8 mL), and methylene chloride (2 mL) were used to obtain the title compound (49 mg, yield 39%) as a colorless solid by the same method as in (1c). However, purification was performed by silica gel flash column chromatography (methylene chloride:methanol, 50:1 1 to 15:1, v/v). ¹H NMR (400 MHz, CD₃OD): δ 1.20 (3H, d, J = 6.2 Hz), 3.35 - 3.36 (2H, m), 3.44-3.46 (2H, m), 3.72 (3H, s), 4.04 - 4.07 (1H, m), 4.10 (1H, d, J = 14.5 Hz), 4.22 (1H, d, J = 14.5 Hz), 4.91 (1H, d, J = 7.4 Hz), 6.75 (2H, d, J = 8.6 Hz), 7.07 (1H, dd, J = 7.0 and 2.0 Hz), 7.13-7.18 (4H, m);
MS (FAB)m/z: 463 (M+K)⁺.

### (Example 43) 3-Chloro-5-methyl-2-(4-methoxybenzyl)phenyl 7-deoxy-D-glycero-β-D-gluco-heptopyranoside (Example Compound No. 1-114)

### (43a) 3-Hydroxy-5-methyl-2-(4-methoxybenzoyl)cyclohex-2-enone

5-Methyl-1,3-cyclohexanedione (1.06 g, 8.40 mmol), triethylamine (3.51 mL, 25.2 mmol), 4-methoxybenzoylchloride (1.43 g, 8.38 mmol), acetonitrile (20 mL), and trimethylsilyl cyanide (0.13 mL, 0.97 mmol) were used to obtain a crude product of the title compound (2.14 g) by the same method as in (42a).

### (43b) 3-Chloro-5-methyl-2-(4-methoxybenzoyl)cyclohex-2-enone

The crude product obtained in (43a) (2.14 g, 8.42 mmol), oxalyl chloride (0.86 mL, 10.0 mmol), methylene chloride (20 mL), and dimethylformamide were used to obtain the title compound (1.63 g, yield 70%) as a pale yellow oil by the same method as in (42b).
¹H NMR (400 MHz, CDCl₃): δ 1.19 (3H, d, J = 6.3 Hz), 2.29 (1H, dd, J = 16.0 and 12.1 Hz), 2.45-2.55 (1H, m), 2.60-2.67 (2H, m), 2.84-2.89 (1H, m), 3.88 (3H, s), 6.95 (2H, d, J = 9.0 Hz), 7.83 (2H, d, J = 9.0 Hz);
MS (EI)m/z: 278, 280 (M)⁺.

### (43c) (2-Chloro-6-hydroxy-4-methyl-phenyl)-(4-methoxyphenyl)-methanone

The compound obtained in (43b) (2.20 g, 7.89 mmol) and triethylamine (3.30 mL, 23.6 mmol) were dissolved in acetonitrile (30 mL), trimethylsilane iodide (2.80 mL, 19.7 mmol) was added in a water bath, and the mixture was stirred at 50°C for 30 min. The mixture was cooled to 0°C, followed by addition of toluene (30 mL), and the mixture was washed with a neutral phosphate-buffered pH standard solution (60 mL). The organic layer was dried over anhydrous sodium sulfate, and then the solvent was removed under reduced pressure.

The resulting oily crude product was dissolved in toluene (30 mL), followed by addition ofN-iodosuccinimide (1.78 g, 7.91 mmol), and the mixture was stirred at room temperature for 15 min. Subsequently, triethylamine (1.10 mL, 7.89 mmol) was added, and the mixture was stirred at room temperature for 15 min. Subsequently, tetrahydrofuran (45 mL) and 2 N aqueous sodium hydroxide (15 mL) were added, and the mixture was stirred at 40°C for 1.5 h. The mixture was extracted with ethyl acetate (100 mL), and then the organic layer was washed with saturated brine (50 mL). The organic layer was dried over anhydrous sodium sulfate, and then the solvent was removed under reduced pressure. The residue was purified by silica gel flash chromatography (hexane:ethyl acetate 19:1 to 3:1, v/v) to obtain the title compound (1.23 g, yield 56%) as an amorphous compound.
¹H NMR (400 MHz, CDCl₃): δ 2.35 (3H, s), 3.89 (3H, s), 6.80 (1H, d, J = 8.6 Hz), 6.94 (2H, d, J = 8.8 Hz), 7.75 (2H, d, J = 8.8 Hz), 8.73 (1H, s);
MS (FAB)m/z: 277, 279 (M+H)⁺.

### (43d) 3-Chloro-2-{hydroxy(4-methoxyphenyl)methyl}-5-methylphenol

The compound obtained in (43c) (1.23 g, 4.44 mmol), methanol (20 mL), and sodium borohydride (340 mg, 8.99 mmol) were used to obtain the title compound (790 mg, yield 64%) as a white solid by the same method as in (42d).
¹H NMR (400 MHz, CDCl₃): δ 2.27 (3H, s), 2.87 (1H, d, J = 2.7 Hz), 3.79 (3H, s), 6.41 (1H, d, J = 2.7 Hz), 6.68 (1H, s), 6.73 (1H, s), 6.88 (2H, d, J = 8.6 Hz), 7.36 (2H, d, J = 8.6 Hz), 8.89 (1H, s);
MS (EI)m/z: 278, 280 (M)⁺.

### (43e) 3-Chloro-5-methyl-2-(4-methoxybenzyl)phenol

The compound obtained in (43d) (790 mg, 2.83 mmol), acetonitrile (16 mL), triethylsilane (1.35 mL, 8.48 mmol), and a boron fluoride-diethyl ether complex (0.53 mL, 4.22 mmol) were used to obtain the title compound (598 mg, yield 76%) as an oil by the same method as in (42e).
¹H NMR (500 MHz, CDCl₃): δ 2.25 (3H, s), 3.77 (3H, s), 4.08 (2H, s), 4.77 (1H, s), 6.53 (1H, s), 6.81 (2H, d, J = 8.3 Hz), 6.84 (1H, s), 7.17 (2H, d, J = 8.3 Hz);
MS (EI)m/z: 262, 264 (M)⁺.

### (43f) 3-Chloro-5-methyl-2-(4-methoxybenzyl)phenyl 2,3,4,6-tetra-O-benzoyl-7-deoxy-D-glycero-β-D-gluco-heptopyranoside

The compound obtained in (7d) (200 mg, 0.33 mmol), trichloroacetonitrile (0.165 mL, 1.63 mmol), 1,8-diazabicyclo[5.4.0]-7-undecene (40 µL, 0.27 mmol), and methylene chloride (5 mL) were used to prepare an imidate by the same method as in (1b). Subsequently, the compound obtained in (43e) (78 mg, 0.30 mmol), a boron trifluoride-diethyl ether complex (0.041 mL, 0.33 mmol), and methylene chloride (5 mL) were used to obtain a crude product of the title compound (129 mg) by the same method as in (1b). The resulting crude product was used in the subsequent reaction as it was.

### (43g) 3-Chloro-5-methyl-2-(4-methoxybenzyl)phenyl 7-deoxy-D-glycero-β-D-gluco-heptopyranoside

The crude product obtained in (43f) (129 mg), potassium carbonate (200 mg, 1.45 mmol), methanol (6 mL), and methylene chloride (1.5 mL) were used to obtain the title compound (42 mg, yield 32%) as a colorless solid by the same method as in (1c).
¹H NMR (400 MHz, CD₃OD): δ 1.21 (3H, d, J = 6.6 Hz), 2.29 (3H, s), 3.35 - 3.38 (2H, m), 3.44 - 3.45 (1H, m), 3.73 (2H, s), 4.02 - 4.06 (2H, m), 4.17 (1H, d, J = 14.1 Hz), 6.75 (2H, d, J = 8.8 Hz), 6.92 (1H, s), 6.97 (1H, s), 7.16 (2H, d, J = 8.8 Hz); MS (FAB)m/z: 477 (M+K)⁺.

### (Example 44) 3-Chloro-5-hydroxymethyl-2-(4-methoxybenzyl)phenyl 7-deoxy-D-glycero-β-D-gluco-heptopyranoside (Example Compound No. 1-115)

### (44a) (6-Acetoxy-2-chloro-4-methyl-phenyl)-(4-methoxyphenyl)-methanone

The compound obtained in (43c) (1.86 g, 6.72 mmol) was dissolved in methylene chloride (20 mL), followed by addition of pyridine (1.09 mL, 13.5 mmol), acetic anhydride (0.95 mL, 10.0 mmol), and N,N-dimethylaminopyridine (250 mg, 2.05 mmol), and the mixture was stirred at room temperature for 12 h. The solvent was removed under reduced pressure, followed by addition of ethyl acetate (30 mL), and the mixture was washed with 1 N hydrochloric acid (10 mL), saturated aqueous sodium hydroxide (20 mL), and brine (20 mL) in this order. The organic layer was dried over anhydrous sodium sulfate, and then the solvent was removed under reduced pressure. The residue was purified by silica gel flash chromatography (hexane:ethyl acetate 10:1 1 to 3:1, v/v) to obtain the title compound (1.77 g, yield 83%) as an oil.
¹H NMR (400 MHz, CDCl₃): δ 1.98 (3H, s), 2.41 (3H, s), 3.88 (3H, s), 6.93 (2H, d, J = 9.0 Hz), 6.96 (1H, s), 7.18 (1H, s), 7.79 (2H, d, J = 9.0 Hz);
MS (FAB)m/z: 319, 321 (M+H)⁺.

### (44b) (2-Chloro-6-hydroxy-4-hydroxymethyl-phenyl)-(4-methoxyphenyl)-methanone

The compound obtained in (44a) (1.77 g, 5.55 mmol) was dissolved in carbon tetrachloride (25 mL), followed by addition of N-bromosuccinimide (1.04 g, 5.84 mmol) and 2,2-azobis(isobutyronitrile) (90 mg, 0.55 mmol), and the mixture was refluxed for 4 h. The reaction mixture was cooled to room temperature and filtered using Celite, and then the solvent was removed under reduced pressure. The residue was purified by silica gel flash chromatography (hexane:ethyl acetate 10:1 1 to 3:1, v/v) to obtain a mixture (1.20 g).

The resulting mixture (1.20 g) was dissolved in dioxane-water (24 mL/8 mL), followed by addition of carbonate calcium (1.80 g, 18.0 mmol), and the mixture was refluxed for 24 h. The reaction mixture was cooled to room temperature and filtered using Celite, and then the solvent was removed under reduced pressure. Methyl alcohol (20 mL) and 2 N aqueous sodium hydroxide (5 mL) were added to the residue, the mixture was stirred at room temperature for 30 min, and the solvent was removed under reduced pressure. Ethyl acetate (50 mL) was added, and the mixture was washed with saturated aqueous ammonium chloride (50 mL) and saturated brine (50 mL) in this order. The organic layer was dried over anhydrous sodium sulfate, and then the solvent was removed under reduced pressure. Ethyl acetate-hexane was added to the residue, and the resulting precipitates were filtered to obtain a crude product of the title compound (750 mg, yield 83%). The resulting crude product was used in the subsequent reaction as it was.

### (44c) (4-Acetoxymethyl-2-chloro-6-hydroxy-phenyl)-(4-methoxyphenyl)-methanone

The crude product obtained in (44b) (750 mg, 2.56 mmol) was dissolved in tetrahydrofuran (15 mL), followed by addition of vinyl acetate (7.5 mL) and bis(dibutylchlorotin)oxide (140 mg, 0.25 mmol), and the mixture was stirred at 30°C for 16 h. The solvent was removed under reduced pressure. The residue was purified by silica gel flash chromatography (hexane:ethyl acetate 5:1 1 to 1:1, v/v) to obtain the title compound (680 mg, yield 79%) as an amorphous compound.
¹H NMR (400 MHz, CDCl₃): δ 2.17 (3H, s), 3.89 (3H, s), 5.08 (2H, s), 6.94 (2H, d, J = 8.8 Hz), 6.96 (1H, s), 7.77 (2H, d, J = 8.8 Hz), 8.36 (1H, s);
MS (FAB)m/z: 335, 337 (M+H)⁺.

### (44d) 5-Acetoxymethyl-3-chloro-2-{hydroxy(4-methoxyphenyl)methyl}phenol

Sodium borohydride (150 mg, 3.97 mmol) was added to a solution of the compound obtained in (44c) (680 mg, 2.03 mmol) in methanol (15 mL) with ice cooling, and the mixture was stirred at room temperature for 30 min. An appropriate amount of saturated aqueous ammonium chloride was added, and the solvent was removed under reduced pressure. Ethyl acetate (30 mL) was added, and the mixture was washed with saturated aqueous ammonium chloride (30 mL) and saturated brine (30 mL) in this order. The organic layer was dried over anhydrous sodium sulfate, and then the solvent was removed under reduced pressure. The residue was purified by silica gel flash chromatography (hexane:ethyl acetate 5:1 to 1:1, v/v) to obtain the title compound (510 mg, yield 75%) as an oil.
¹H NMR (400 MHz, CDCl₃): δ 2.13 (3H, s), 3.80 (3H, s), 5.02 (2H, s), 6.42 (1H, d, J = 2.7 Hz), 6.85 (1H, s), 6.88 (2H, d, J = 8.6 Hz), 7.36 (2H, d, J = 8.6 Hz), 9.13 (1H, s);
MS (FAB)m/z: 375, 377 (M+K)⁺.

### (44e) 5-Acetoxymethyl-3-chloro-2-(4-methoxybenzyl)phenol

The compound obtained in (44d) (510 mg, 1.51 mmol), acetonitrile (10 mL), triethylsilane (0.72 mL, 4.52 mmol), and a boron fluoride-diethyl ether complex (0.29 mL, 2.31 mmol) were used to obtain the title compound (420 mg, yield 86%) as a white solid by the same method as in (42e).
¹H NMR (500 MHz, CDCl₃): δ 2.11 (3H, s), 3.77 (3H, s), 4.11 (2H, s), 4.99 (2H, s), 6.71 (1H, s), 6.81 (2H, d, J = 8.8 Hz), 7.01 (1H, s), 7.18 (2H, d, J = 8.8 Hz);
MS (EI)m/z: 320, 322 (M)⁺.

### (44f) 5-Acetoxymethyl-3-chloro-2-(4-methoxybenzyl)phenyl 7-deoxy-D-glycero-β-D-gluco-heptopyranoside

The compound obtained in (7d) (250 mg, 0.41 mmol), trichloroacetonitrile (0.210 mL, 2.08 mmol), 1,8-diazabicyclo[5.4.0]-7-undecene (60 µL, 0.04 mmol), and methylene chloride (5 mL) were used to prepare an imidate by the same method as in (1b). Subsequently, the compound obtained in (44e) (100 mg, 0.31 mmol), a boron trifluoride-diethyl ether complex (0.050 mL, 0.40 mmol), and methylene chloride (5 mL) were used to obtain a crude product of the title compound (256 mg) by the same method as in (1b). The resulting crude product was used in the subsequent reaction as it was.

### (44g) 3-Chloro-5-hydroxymethyl-2-(4-methoxybenzyl)phenyl 7-deoxy-D-glycero-β-D-gluco-heptopyranoside

The crude product obtained in (44f) (256 mg), potassium carbonate (386 mg, 2.79 mmol), methanol (8 mL), and methylene chloride (2 mL) were used to obtain the title compound (85 mg, yield 60%) as a colorless solid by the same method as in (1c).
¹H NMR (400 MHz, CD₃OD): δ 1.21 (3H, d, J = 6.2 Hz), 3.36 - 3.47 (4H, m), 3.73 (3H, s), 4.05 - 4.10 (2H, m), 4.20 (1H, d, J = 14.0 Hz), 4.55 (2H, s), 4.93 (1H, d, J = 7.4 Hz), 6.75 (2H, d, J = 8.8 Hz), 7.10 (1H, s), 7.12 (1H, s), 7.18 (2H, d, J = 8.8 Hz); MS (FAB)m/z: 493 (M+K)⁺.

### (Example 45) 3-Chloro-2-(4-ethoxybenzyl)-5-hydroxymethyl-phenyl 7-deoxy-D-glycero-β-D-gluco-heptopyranoside (Example Compound No. 1-116)

### (45a) 2-(4-Ethoxybenzoyl)-3-hydroxy-5-methylcyclohex-2-enone

5-Methyl-1,3-cyclohexanedione (9.69 g, 76.8 mmol), triethylamine (32.1 mL, 230 mmol), 4-ethoxybenzoylchloride (14.2 g, 76.9 mmol), acetonitrile (120 mL), and trimethyl cyanonitrile (1.00 mL, 7.50 mmol) were used to obtain a crude product of the title compound (20.6 g) by the same method as in (42a).

### (45b) 3-Chloro-5-methyl-2-(4-ethoxybenzoyl)cyclohex-2-enone

The crude product obtained in (45a) (8.00 g, 29.8 mmol), oxalyl chloride (2.68 mL, 31.2 mmol), methylene chloride (80 mL), and dimethylformamide were used to obtain the title compound (7.40 g, yield 85%) as a pale yellow solid by the same method as in (42b).
¹H NMR (400 MHz, CDCl₃): δ 1.19 (3H, d, J = 6.6 Hz), 1.44 (3H, t, J = 7.0 Hz), 2.29 (2H, dd, J = 16.1 and 12.2 Hz), 2.43 - 2.55 (1H, m), 2.60 - 2.67 (2H, m), 2.87 (1H, dd, J = 17.8 and 4.1 Hz), 4.10 (2H, q, J = 7.0 Hz), 6.93 (2H, d, J = 9.0 Hz), 7.81 (2H, d, J = 9.0 Hz);
MS (EI)m/z: 292, 294 (M)⁺.

### (45c) (2-Chloro-6-hydroxy-4-methyl-phenyl)-(4-ethoxyphenyl)-methanone

The compound obtained in (45b) (7.40 g, 25.3 mmol), triethylamine (11.7 mL, 82.8 mmol), acetonitrile (100 mL), and trimethylsilane iodide (9.80 mL, 69.1 mmol) were used to obtain an oily silyl enol ether compound by the same method as in (43c).

The resulting oily crude product was treated with toluene (70 mL), N-iodosuccinimide (6.20 g, 27.6 mmol), triethylamine (5.00 mL, 35.4 mmol), tetrahydrofuran (140 mL), and 2 N aqueous sodium hydroxide (10 mL) in this order by the same method as in (43c), and the reaction mixture was passed through a short column to obtain a crude product of the title compound (5.54 g).

### (45d) (6-Acetoxy-2-chloro-4-methyl-phenyl)-(4-ethoxyphenyl)-methanone

The crude product obtained in (45c) (5.54 g, 19.1 mmol), methylene chloride (60 mL), pyridine (3.00 mL, 37.1 mmol), acetic anhydride (2.70 mL, 28.6 mmol), and N,N-dimethylaminopyridine (700 mg, 5.23 mmol) were used to obtain the title compound (3.65 g, yield 58%) as an oil by the same method as in (44a).
¹H NMR (400 MHz, CDCl₃): δ 1.44 (3H, t, J = 7.0 Hz), 1.97 (3H, s), 2.40 (3H, s), 4.10 (2H, q, J = 7.0 Hz), 6.90 (2H, d, J = 9.3 Hz), 6.95 (1H, s), 7.16 (1H, s), 7.76 (2H, d, J = 8.3 Hz);
MS (FAB)m/z: 333, 335 (M+H)⁺.

### (45e) (2-Chloro-6-hydroxy-4-hydroxymethyl-phenyl)-(4-ethoxyphenyl)-methanone

The compound obtained in (45d) (3.65 g, 11.0 mmol), carbon tetrachloride (60 mL), N-bromosuccinimide (2.05 g, 11.5 mmol), and 2,2-azobis(isobutyronitrile) (360 mg, 2.19 mmol) were used to obtain a mixture (5.36 g) by the same method as in (44b). The resulting mixture was used in the subsequent reaction as it was.

The resulting mixture (5.36 g) was treated with dioxane-water (45 mL/15 mL), calcium carbonate (6.59 g, 65.8 mmol), methyl alcohol (20 mL), and 2 N aqueous sodium hydroxide (5 mL) in this order by the same method as in (44b), and the reaction mixture was purified by silica gel flash chromatography (methylene chloride:methyl alcohol 40:1 to 10:1, v/v) to obtain the title compound (1.23 g, yield 37%) as a white solid.
¹H NMR (400 MHz, CD₃OD): δ 1.41 (3H, t, J = 7.1 Hz), 4.13 (2H, q, J = 7.1 Hz), 4.58 (2H, s), 6.86 (1H, s), 6.96 (1H, s), 6.98 (2H, d, J = 9.0 Hz), 7.76 (2H, d, J = 9.0 Hz);
MS (EI)m/z: 306, 308 (M)⁺.

### (45f) (4-Acetoxymethyl-2-chloro-6-hydroxy-phenyl)-(4-ethoxyphenyl)-methanone

The compound obtained in (45e) (1.23 g, 4.00 mmol) was dissolved in tetrahydrofuran (24 mL), and vinyl acetate (12 mL) and bis(dibutylchlorotin)oxide (440 mg, 0.80 mmol) were used to obtain the title compound (1.21 g, yield 86%) as an amorphous compound by the same method as in (44c).
¹H NMR (500 MHz, CDCl₃): δ 1.45 (3H, t, J = 7.0 Hz), 2.17 (3H, s), 4.12 (2H, q, J = 7.0 Hz), 5.08 (2H, s), 6.92 (2H, d, J = 8.8 Hz), 6.96 (1H, s), 7.75 (2H, d, J = 8.8 Hz), 8.33 (1H, s);
MS (FAB)m/z: 349, 351 (M+H)⁺.

### (45g) 5-Acetoxymethyl-3-chloro-2-{hydroxy-(4-ethoxyphenyl)methyl}phenol

The compound obtained in (45f) (1.21 g, 3.47 mmol), methanol (15 mL), and sodium borohydride (260 mg, 6.87 mmol) were used to obtain the title compound (800 mg, yield 66%) as a white solid by the same method as in (44d).
¹H NMR (400 MHz, CDCl₃): δ 1.40 (3H, t, J = 7.1 Hz), 2.12 (3H, s), 2.97 (1H, brs), 4.01 (2H, q, J = 7.1 Hz), 5.01 (2H, s), 6.41 (1H, d, J = 2.7 Hz), 6.84 - 6.89 (4H, m), 7.34(2H, d, J = 8.6 Hz), 9.14 (1H, s);
MS (FAB)m/z: 389, 391 (M+K)⁺.

### (45h) 5-Acetoxymethyl-3-chloro-2-(4-ethoxybenzyl)phenol

The compound obtained in (45g) (800 mg, 2.28 mmol), acetonitrile (12 mL), triethylsilane (1.09 mL, 6.84 mmol), and a boron fluoride-diethyl ether complex (0.43 mL, 3.42 mmol) were used to obtain the title compound (710 mg, yield 93%) as a white solid by the same method as in (42e).
¹H NMR (400 MHz, CDCl₃): 81.39 (3H, t, J = 7.1 Hz), 2.12 (3H, s), 3.99 (2H, q, J = 7.1 Hz), 4.11 (2H, s), 5.00 (2H, s), 5.10 (1H, s), 6.72 (1H, d, J = 1.6 Hz), 6.81 (2H, d, J = 8.8 Hz), 7.02 (1H, d, J = 1.6 Hz), 7.17 (2H, d, J = 8.8 Hz);
MS (FAB)m/z: 334, 336 (M)⁺.

### (45i) 5-Acetoxymethyl-3-chloro-2-(4-ethoxybenzyl)phenyl 7-deoxy-D-glycero-β-D-gluco-heptopyranoside

The compound obtained in (7d) (250 mg, 0.41 mmol), trichloroacetonitrile (0.210 mL, 2.08 mmol), 1,8-diazabicyclo[5.4.0]-7-undecene (60 µL, 0.04 mmol), and methylene chloride (5 mL) were used to prepare an imidate by the same method as in (1b). Subsequently, the compound obtained in (45h) (100 mg, 0.30 mmol), a boron trifluoride-diethyl ether complex (0.050 mL, 0.40 mmol), and methylene chloride (5 mL) were used to obtain a crude product of the title compound (198 mg) by the same method as in (1b). The resulting crude product was used in the subsequent reaction as it was.

### (45j) 3-Chloro-2-(4-ethoxybenzyl)-5-hydroxymethyl-phenyl 7-deoxy-D-glycero-β-D-gluco-heptopyranoside

The crude product obtained in (45i) (198 mg), potassium carbonate (295 mg, 2.13 mmol), methanol (6 mL), and methylene chloride (1.5 mL) were used to obtain the title compound (65 mg, yield 65%) as a colorless solid by the same method as in (1c).
¹H NMR (400 MHz, CD₃OD): δ 1.21 (3H, d, J = 6.6 Hz), 1.34 (3H, t, J = 7.1 Hz), 3.35 - 3.38 (2H, m), 3.44 - 3.47 (2H, m), 3.96 (2H, q, J = 7.1 Hz), 4.05 - 4.09 (2H, m), 4.20 (1H, d, J = 14.5 Hz), 4.55 (2H, s), 4.93 (1H, d, J = 7.0 Hz), 6.74 (2H, d, J = 8.8 Hz), 7.10 (1H, s), 7.12 (1H, s), 7.16 (2H, d, J = 8.8 Hz);
MS (FAB)m/z: 507 (M+K)⁺.

### (Example 46) 2-(4-Ethoxybenzyl)-3-fluoro-5-hydroxymethyl-phenyl 7-deoxy-D-glycero-β-D-gluco-heptopyranoside (Example Compound No. 1-117)

### (46a) 2-(4-Ethoxybenzoyl)-3-fluoro-5-methyl-cyclohex-2-enone

The crude product obtained in (45a) (8.00 g, 29.8 mmol) was dissolved in methylene chloride (80 mL), followed by addition of diethylaminosulfur trifluoride (11.7 mL, 89.3 mmol) with ice cooling, and the mixture was stirred at room temperature for 3 h. The mixture was cooled to 0°C, followed by addition of methyl alcohol (8 mL), and the mixture was stirred for 20 min. The organic layer was washed with water (150 mL x 3), saturated aqueous sodium hydrogencarbonate (150 mL), and saturated brine (50 mL) in this order. The organic layer was dried over anhydrous sodium sulfate, and then the solvent was removed under reduced pressure. The residue was purified by silica gel flash chromatography (hexane:ethyl acetate 20:1 to 3:1, v/v) to obtain the title compound (7.00 g, yield 85%) as a pale yellow solid.
¹H NMR (400 MHz, CDCl₃): δ 1.21 (3H, d, J = 5.5 Hz), 1.44 (3H, t, J = 7.1 Hz), 2.27 (1H, dd, J = 16.0 and 12.1 Hz), 2.45 - 2.53 (2H, m), 2.58 - 2.63 (1H, m), 2.69-2.79 (1H, m), 4.10 (2H, q, J = 7.1 Hz), 6.92 (2H, d, J = 9.0 Hz), 7.81 (2H, d, J = 9.0 Hz);
MS (EI)m/z: 276 (M)⁺.

### (46b) (2-Fluoro-6-hydroxy-4-methyl-phenyl)-(4-ethoxyphenyl)-methanone

The compound obtained in (46a) (7.00 g, 25.3 mmol), triethylamine (10.7 mL, 75.7 mmol), acetonitrile (100 mL), and trimethylsilane iodide (9.00 mL, 63.4 mmol) were used to obtain an oily silyl enol ether compound by the same method as in (43c).

The resulting oily crude product was treated with toluene (60 mL), N-iodosuccinimide (5.70 g, 25.3 mmol), triethylamine (4.70 mL, 33.3 mmol), tetrahydrofuran (120 mL), and 2 N aqueous sodium hydroxide (10 mL) in this order by the same method as in (43c), and the reaction mixture was purified by silica gel flash chromatography (hexane:ethyl acetate 20:1 1 to 1:1, v/v) to obtain the title compound (4.36 g, yield 63%) as a pale yellow solid.
¹H NMR (400 MHz, CDCl₃): δ 1.45 (3H, t, J = 7.1 Hz), 2.36 (3H, s), 4.12 (2H, q, J = 7.1 Hz), 6.45 (1H, d, J = 11.3 Hz), 6.69 (1H, s), 6.93 (2H, d, J = 8.8 Hz), 7.72 (2H, dd, J = 8.8 and 3. 5Hz), 11.0 (1H, s);
MS (EI)m/z: 274 (M)⁺.

### (46c) (6-Acetoxy-2-fluoro-4-methyl-phenyl)-(4-ethoxyphenyl)-methanone

The compound obtained in (46b) (4.36 g, 15.9 mmol), methylene chloride (40 mL), pyridine (2.57 mL, 31.8 mmol), acetic anhydride (2.25 mL, 23.8 mmol), and N,N-dimethylaminopyridine (580 mg, 4.75 mmol) were used to obtain a crude product of the title compound (4.62 g) as a pale yellow solid by the same method as in (44a). However, the resulting crude product was only washed with hexane and used in the subsequent reaction as it was without purification.

### (46d) (2-Fluoro-6-hydroxy-4-hydroxymethyl-phenyl)-(4-ethoxyphenyl)-methanone

The crude product obtained in (46c) (4.62 g), carbon tetrachloride (65 mL), N-bromosuccinimide (2.73 g, 15.3 mmol), and 2,2'-azobis(isobutyronitrile) (480 mg, 2.92 mmol) were used to obtain a mixture (5.77 g) by the same method as in (44b). The resulting mixture was used in the subsequent reaction as it was.

The resulting mixture (5.77 g) was treated with dioxane-water (60 mL/20 mL), calcium carbonate (8.70 g, 86.9 mmol), methyl alcohol (40 mL), and 2 N aqueous sodium hydroxide (10 mL) in this order by the same method as in (44b). Finally, the reaction mixture was purified by silica gel flash chromatography (methylene chloride:methyl alcohol 40:1 to 10:1, v/v) to obtain the title compound (2.00 g, yield 47%) as a white solid.
¹H NMR (400 MHz, CDCl₃): δ 1.46 (3H, t, J=7.1 Hz), 4.12 (2H, q, J=7.1 Hz), 4.72 (2H, d, J=5.9 Hz), 6.68 (1H, d, J=11.7 Hz), 6.86 (1H, s), 6.94 (2H, d, J=9.0 Hz), 7.73 (2H, dd, J=9.0 and 3.2 Hz), 10.9 (1H, s);
MS (FAB)m/z: 601 (M+K)⁺.

### (46e) (4-Acetoxymethyl-2-fluoro-6-hydroxy-phenyl)-(4-ethoxyphenyl)-methanone

The compound obtained in (46d) (2.00 g, 6.89 mmol) was dissolved in tetrahydrofuran (20 mL), and vinyl acetate (20 mL) and bis(dibutylchlorotin)oxide (760 mg, 1.37 mmol) were added to obtain the title compound (1.76 g, yield 77%) as an oil by the same method as in (44c). ¹H NMR (400 MHz, CDCl₃): δ 1.46 (3H, t, J = 7.1 Hz), 2.17 (3H, s), 4.13 (2H, q, J = 7.1 Hz), 5.10 (2H, s), 6.62 (1H, d, J = 10.5 Hz), 6.85 (1H, s), 6.94 (2H, d, J = 8.8 Hz), 7.74 (2H, dd, J = 8.8 and 3.5 Hz), 10.8 (1H, s);
MS (FAB)m/z: 333 (M+H)⁺.

### (46f) 5-Acetoxymethyl-3-fluoro-2-{hydroxy-(4-ethoxyphenyl)methyl}phenol

The compound obtained in (46e) (1.76 g, 5.30 mmol), methanol (20 mL), and sodium borohydride (400 mg, 10.6 mmol) were used to obtain the title compound (1.21 g, yield 69%) as an oil by the same method as in (44d).
¹H NMR (400 MHz, CDCl₃): δ1.40 (3H, t, J = 7.1 Hz), 2.12 (3H, s), 2.77 (1H, d, J = 2.8 Hz), 4.02 (2H, q, J = 7.1 Hz), 5.01 (2H, s), 6.29 (1H, d, J = 2.8 Hz), 6.56 (1H, d, J = 10.2 Hz), 6.71 (1H, s), 6.87 (2H, d, J = 8.4 Hz), 7.35 (2H, d, J = 8.4 Hz), 8.88 (1H, s);
MS (FAB)m/z: 373 (M+H)⁺.

### (46g) 5-Acetoxymethyl-3-fluoro-2-(4-ethoxybenzyl)phenol

The compound obtained in (46f) (1.21 g, 3.62 mmol), acetonitrile (16 mL), triethylsilane (1.73 mL, 10.9 mmol), and a boron fluoride-diethyl ether complex (0.68 mL, 5.41 mmol) were used to obtain the title compound (710 mg, yield 93%) as a white solid by the same method as in (42e). However, the resulting crude product was only washed with hexane and used in the subsequent reaction as it was without purification by silica gel flash chromatography.
¹H NMR (400 MHz, CDCl₃): δ 1.39 (3H, t, J = 6.9 Hz), 2.11 (3H, s), 3.95 (2H, s), 3.99 (2H, q, J = 6.9 Hz), 5.00 (2H, s), 5.03 (1H, s), 6.60 (1H, s), 6.70 (1H, dd, J = 9.8 and 1.5 Hz), 6.81 (2H, d, J = 8.8 Hz), 7.18 (2H, d, J = 8.8 Hz);
MS (FAB)m/z: 318 (M)⁺.

### (46h) 5-Acetoxymethyl-2-(4-ethoxybenzyl)-3-fluorophenyl 7-deoxy-D-glycero-β-D-gluco-heptopyranoside

The compound obtained in (7d) (250 mg, 0.41 mmol), trichloroacetonitrile (0.210 mL, 2.08 mmol), 1,8-diazabicyclo[5.4.0]-7-undecene (60 µL, 0.04 mmol), and methylene chloride (5 mL) were used to prepare an imidate by the same method as in (1b). Subsequently, the compound obtained in (46g) (100 mg, 0.31 mmol), a boron trifluoride-diethyl ether complex (0.050 mL, 0.40 mmol), and methylene chloride (5 mL) were used to obtain a crude product of the title compound (298 mg) by the same method as in (1b). The resulting crude product was used as it was in the subsequent reaction.

### (46i) 2-(4-Ethoxybenzyl)-3-fluoro-5-hydroxymethylphenyl 7-deoxy-D-glycero-β-D-gluco-heptopyranoside

The crude product obtained in (46h) (298 mg), potassium carbonate (450 mg, 3.26 mmol), methanol (8 mL), and methylene chloride (2 mL) were used to obtain the title compound (87 mg, yield 59%) as a colorless solid by the same method as in (1c).
¹H NMR (400 MHz, CD₃OD): δ 1.21 (3H, d, J = 6.8Hz), 1.33 (3H, t, J = 7.1 Hz), 3.36 - 3.39 (2H, m), 3.43 - 3.50 (2H, m), 3.91 - 4.07 (5H, m), 4.54 (2H, s), 4.93 (1H, d, J = 6.8 Hz), 6.73 (2H, d, J = 8.3 Hz), 6.77 (1H, d, J = 9.8 Hz), 6.97 (1H, s), 7.17 (2H, d, J = 8.3 Hz);
MS (FAB)m/z: 491 (M+K)⁺.

### (Example 47) 2-(4-Ethylbenzyl)-3-fluoro-5-hydroxymethyl-phenyl 7-deoxy-D-glycero-β-D-gluco-heptopyranoside (Example Compound No. 1-118)

### (47a) Ethyl 4-(4-Ethylbenzoyl)-3-hydroxy-5-oxo-cyclohex-3-enecarboxylate

Ethyl-3-hydroxy-5-oxo-cyclohex-3-enecarboxylate (EP1571148A1) (1.83 g, 9.94 mmol), triethylamine (4.15 mL, 29.8 mmol), 4-ethylbenzoylchloride (1.76 g, 12.0 mmol), acetonitrile (10 mL), and trimethyl cyanonitrile (0.16 mL, 1.19 mmol) were used to obtain a crude product of the title compound (3.14 g) by the same method as in (42a).

### (47b) Ethyl 4-(4-ethylbenzoyl)-3-fluoro-5-oxo-cyclohex-3-enecarboxylate

The crude product obtained in (47a) (5.40 g, 17.1 mmol), and methylene chloride (60 mL), and diethylaminosulfur trifluoride (6.70 mL, 51.1 mmol) were used to obtain the title compound (4.28 g, yield 79%) as an oil by the same method as in (46a).
¹H NMR (400 MHz, CDCl₃): δ 1.25 (3H, t, J = 7.7 Hz), 1.32 (3H, t, J = 7.0 Hz), 2.71 (2H, q, J = 7.7 Hz), 2.79 - 2.82 (2H, m), 2.94 - 3.01 (1H, m), 3.06 - 3.13 (1H, m), 3.25 - 3.33 (1H, m), 4.26 (2H, q, J = 7.0 Hz), 7.29 (2H, d, J = 8.4 Hz), 7.77 (2H, d, J = 8.4 Hz);
MS (EI)m/z: 318 (M)⁺.

### (47c) Ethyl 4-(4-ethylbenzoyl)-3-fluoro-5-hydroxybenzoate

The compound obtained in (47b) (4.28 g, 13.4 mmol) and triethylamine (5.70 mL, 40.3 mmol) were dissolved in acetonitrile (30 mL), trimethylsilane iodide (4.77 mL, 33.6 mmol) was added in a water bath, and the mixture was stirred at room temperature for 30 min. The mixture was cooled to 0°C, followed by addition of toluene (60 mL), and washed with a neutral phosphate-buffered pH standard solution (120 mL). The organic layer was dried over anhydrous sodium sulfate, and then the solvent was removed under reduced pressure.

The resulting amorphous crude product was dissolved in toluene (30 mL), followed by addition of a silica gel (SK-85) (17.1 g), and the mixture was stirred at room temperature for 1 h. After cotton-string filtration, the solvent was removed under reduced pressure. The residue was dissolved in ethyl alcohol (20 mL), followed by addition of potassium carbonate (0.37 g, 2.68 mmol), and the mixture was stirred at 50°C for 1 h. The mixture was cooled to room temperature, and the solvent was removed under reduced pressure. Ethyl acetate (60 mL) was added to the residue, and the mixture was washed with saturated brine (30 mL). The organic layer was dried over anhydrous sodium sulfate, and then the solvent was removed under reduced pressure. The residue was purified by silica gel flash chromatography (hexane:ethyl acetate 10:1 1 to 1:1, v/v) to obtain the title compound (3.23 g, yield 76%) as a white solid. ¹H NMR (400 MHz, CDCl₃): δ 1.21 (3H, t, J = 7.7 Hz), 1.37 (3H, t, J = 7.1 Hz), 2.64 (2H, q, J = 7.7 Hz), 4.35 (2H, q, J = 7.1 Hz), 6.35 (1H, s), 7.20 (2H, d, J = 8.2 Hz), 7.36 (2H, d, J = 8.2 Hz), 7.40 (1H, s), 8.96 (1H, s);
MS (EI)m/z: 315 (M-H)⁺.

### (47d) 2-{(4-Ethylphenyl)hydroxymethyl}-3-fluoro-5-hydroxymethylphenol

A solution of the compound obtained in (47c) (693 mg, 2.19 mmol) in tetrahydrofuran (2 mL) was added to a suspension of lithium aluminium hydride (250 mg, 6.59 mmol) in tetrahydrofuran (12 mL) with ice cooling, and the mixture was stirred at room temperature for 1 h. The mixture was cooled to 0°C, water (0.25 mL), 5 N aqueous sodium hydroxide (0.25 mL), and water (0.75 mL) were successively added, and the mixture was stirred at room temperature for 30 min. Subsequently, 2 N hydrochloric acid (10 mL) was added, the mixture was extracted with ethyl acetate (30 mL), and the organic layer was washed with saturated brine (30 mL). The organic layer was dried over anhydrous sodium sulfate, and then the solvent was removed under reduced pressure. The resulting crude product (606 mg) was used in the subsequent reaction as it was.

### (47e) 5-Acetoxymethyl-2-{(4-ethylphenyl)hydroxymethyl}-3-fluorophenol

The crude product obtained in (47d) (606 mg) was dissolved in tetrahydrofuran (6.0 mL), and vinyl acetate (6.0 mL) and bis(dibutylchlorotin)oxide (242 mg, 0.44 mmol) were used to obtain the title compound (595 mg, yield 85%) as a white solid by the same method as in (44c).
¹H NMR (400 MHz, CDCl₃): δ 1.21 (3H, t, J = 7.7 Hz), 2.12 (3H, s), 2.63 (2H, q, J = 7.7 Hz), 2.82 (1H, d, J = 2.5 Hz), 5.01 (2H, s), 6.32 (1H, d, J = 2.5 Hz), 6.57 (1H, dd, J = 10.1 and 1.5 Hz), 6.70 (1H, s), 7.20 (2H, d, J = 8.4 Hz), 7.36 (2H, d, J = 8.4 Hz), 8.84 (1H, s);
MS (FAB)m/z: 319 (M+H)⁺.

### (47f) 5-Acetoxymethyl-3-fluoro-2-(4-ethylbenzyl)phenol

The compound obtained in (47e) (590 mg, 1.85 mmol), acetonitrile (8 mL), triethylsilane (0.89 mL, 5.59 mmol), and a boron fluoride-diethyl ether complex (0.35 mL, 2.79 mmol) were used to obtain the title compound (440 mg, yield 79%) as a white solid by the same method as in (42e).
¹H NMR (400 MHz, CDCl₃): δ 1.20 (3H, t, J = 7.4 Hz), 2.11 (3H, s), 2.60 (2H, q, J = 7.4 Hz), 3.98 (2H, s), 5.01 (2H, s), 5.02 (1H, s), 6.60 (1H, s), 6.70 (1H, d, J =9.8 Hz), 7.12 (2H, d, J = 7.9Hz), 7.19 (2H, d, J = 7.9 Hz);
MS (FAB)m/z: 341 (M+K)⁺.

### (47g) 5-Acetoxymethyl-2-(4-ethylbenzyl)-3-fluorophenyl 7-deoxy-D-glycero-β-D-gluco-heptopyranoside

The compound obtained in (7d) (1.80 g, 2.95 mmol), trichloroacetonitrile (1.49 mL, 15.8 mmol), 1,8-diazabicyclo[5.4.0]-7-undecene (0.044 mL, 0.27 mmol), and methylene chloride (35 mL) were used to prepare an imidate by the same method as in (1b). Subsequently, the compound obtained in (47f) (636 mg, 2.10 mmol), a boron trifluoride-diethyl ether complex (0.37 mL, 2.95 mmol), and methylene chloride (35 mL) were used to obtain a crude product of the title compound (1.88 g) by the same method as in (1b). The resulting crude product was used in the subsequent reaction as it was.

### (47h) 2-(4-Ethylbenzyl)-3-fluoro-5-hydroxymethylphenyl 7-deoxy-D-glycero-β-D-gluco-heptopyranoside

The crude product obtained in (47g) (1.88 g), potassium carbonate (2.90 g, 21.0 mmol), methanol (40 mL), and methylene chloride (10 mL) were used to obtain the title compound (687 mg, yield 75%) as a white solid by the same method as in (1c).
¹H NMR (400 MHz, CD₃OD): δ 1.17 (3H, t, J = 7.6 Hz), 1.21 (3H, d, J = 6.3 Hz), 2.56 (2H, q, J = 7.6 Hz), 3.36 - 3.38 (2H, m), 3.45 - 3.50 (2H, m), 3.95 (1H, d, J = 13.7 Hz), 4.03 - 4.07 (2H, m), 4.55 (2H, s), 4.93 (1H, d, J = 7.4 Hz), 6.79 (1H, d, J = 10.1 Hz), 6.98 (1H, s), 7.03 (2H, d, J = 8.3 Hz), 7.17 (2H, d, J = 8.3 Hz);
MS (FAB)m/z: 475 (M+K)⁺.

### (Example 48) 2-(4-Ethylbenzyl)-3-fluoro-5-hydroxymethylphenyl 4-C-methyl-7-deoxy-glycero-β-D-gluco-heptapyranoside (Example Compound No. 1-119)

### (48a) 5-Acetoxymethyl-2-(4-ethylbenzyl)-3-fluorophenyl 4-O-acetyl-4-C-methyl-2,3,6-tri-O-benzoyl-7-deoxy-glycero-β-D-gluco-heptapyranoside

The compound obtained in (39c) (200 mg, 0.36 mmol), trichloroacetonitrile (0.20 mL, 1.98 mmol), 1,8-diazabicyclo[5.4.0]-7-undecene (60 µL, 0.04 mmol), and methylene chloride (5 mL) were used to prepare an imidate by the same method as in (1b). Subsequently, the compound obtained in (47f) (80 mg, 0.26 mmol), a boron trifluoride-diethyl ether complex (0.045 mL, 0.36 mmol), and methylene chloride (4 mL) were used to obtain a crude product of the title compound (166 mg) by the same method as in (1b). The resulting crude product was used in the subsequent reaction as it was.

### (48b) 2-(4-Ethylbenzyl)-3-fluoro-5-hydroxymethylphenyl 4-C-methyl-7-deoxy-glycero-β-D-gluco-heptapyranoside

The crude product obtained in (48a) (166 mg), potassium carbonate (260 mg, 1.88 mmol), methanol (6 mL), and methylene chloride (1.5 mL) were used to obtain the title compound (45 mg, yield 52%) as a white solid by the same method as in (1c). ¹H NMR (400 MHz, CD₃OD): δ 1.17 (3H, t, J = 7.6 Hz), 1.27 (3H, s), 1.33 (3H, d, J = 6.2 Hz), 2.56 (2H, q, J = 7.6 Hz), 3.12 (1H, d, J = 3.9 Hz), 3.40 (1H, d, J = 9.8 Hz), 3.51 (1H, dd, J = 9.8 and 7.8 Hz), 3.96 (1H, d, J = 14.5 Hz), 4.03 - 4.07 (2H, m), 4.55 (2H, s), 4.95 (1H, d, J = 7.8 Hz), 6.80 (1H, d, J = 10.2 Hz), 7.02 (1H, s), 7.03 (2H, d, J =8.1 Hz), 7.18 (2H, d, J = 8.1 Hz);
MS (FAB)m/z: 451 (M+H)⁺.

### (Example 49) 2-(4-Methoxybenzyl)phenyl 4-deoxy-β-D-glucopyranoside (Example Compound No 2-17)

### (49a) 2-(4-Methoxybenzyl)phenyl 2,3,6-tri-O-benzoyl-4-deoxy-β-D-glucopyranoside

2,3,6-Tri-O-benzoyl-4-deoxy-D-glucopyranoside (Liebigs Ann. Chem., GE, 1992, 7, 747-758) (248 mg, 0.52 mmol), trichloroacetonitrile (0.26 mL, 2.60 mmol), 1,8-diazabicyclo[5.4.0]-7-undecene (8 µL, 0.05 mmol), and methylene chloride (6 mL) were used to prepare an imidate by the same method as in (1b). Subsequently, 2-(4-methoxybenzyl)phenol (100 mg, 0.47 mmol), a boron trifluoride-diethyl ether complex (66 µL, 0.52 mmol), and methylene chloride (6 mL) were used to obtain a crude product of the title compound (306 mg) by the same method as in (1b).

### (49b) 2-(4-Methoxybenzyl)phenyl 4-deoxy-β-D-glucopyranoside

The crude product obtained in (49a) (306 mg, 0.46 mmol), potassium carbonate (630 mg, 4.55 mmol), methanol (8 mL), and methylene chloride (4 mL) were used to obtain the title compound (121 mg, yield 72% in 2 steps) as a colorless solid by the same method as in (1c). However, purification was performed by silica gel flash column chromatography (methylene chloride:methanol, 100:0 to 85:15, v/v). ¹H NMR (400 MHz, CD₃OD): δ 1.48 (1H, dd, J=24.2 and 11.7 Hz), 1.98 (1H, d, J=12.9, 5.2 and 1.9 Hz), 3.39 (1H, dd, J=9.0 and 7.5 Hz), 3.59 (2H, d, J=4.7 Hz), 3.73-3.66 (2H, m), 3.75 (3H, s), 3.93 (1H, d, J=15.0 Hz), 4.03 (1H, d, J=15.0 Hz), 4.92-4.84 (1H, m), 6.80 (2H, d like, J=8.6 Hz), 6.94-6.88 (1H, m), 7.04 (1H, d, J=7.4 Hz), 7.19-7.13 (4H, m);
MS (FAB)m/z: 383 (M + Na)⁺.

### (Example 50) 2-(4-Methoxybenzyl)phenyl 4-C-methyl-β-D-glucopyranoside

### (Example Compound No. 1-120)

### (50a) 2-(4-Methoxybenzyl)phenyl 4-O-acetyl-2,3,6-tri-O-benzoyl-4-C-methyl-β-D-glucopyranoside

The compound obtained in (3c) (285 mg, 0.52 mmol), trichloroacetonitrile (0.26 mL, 2.60 mmol), 1,8-diazabicyclo[5.4.0]-7-undecene (8 µL, 0.05 mmol), and methylene chloride (6 mL) were used to prepare an imidate by the same method as in (1b). Subsequently, 2-(4-methoxybenzyl)phenol (100 mg, 0.47 mmol), a boron trifluoride-diethyl ether complex (66 µL, 0.52 mmol), and methylene chloride (6 mL) were used to obtain a crude product of the title compound (243 mg) by the same method as in (1b).

### (50b) 2-(4-Methoxybenzyl)phenyl 4-C-methyl-β-D-glucopyranoside

The crude product obtained in (50a) (243 mg, 0.33 mmol), potassium carbonate (451 mg, 3.26 mmol), methanol (8 mL), and methylene chloride (2 mL) were used to obtain the title compound (88 mg, yield 48% in 2 steps) as a colorless solid by the same method as in (1c). However, purification was performed by silica gel flash column chromatography (methylene chloride:methanol, 100:0 to 85:15, v/v). ¹H NMR (400 MHz, CD₃OD): δ 1.14 (3H, s), 3.50-3.42 (3H, m), 3.62 (1H, dd, J=11.8 and 8.2 Hz), 3.74 (3H, s), 3.96-3.88 (2H, m), 4.03 (1H, d, J=15.3 Hz), 4.93-4.86 (1H, m), 6.80 (2H, d like, J=8.6 Hz), 6.91 (1H, dt like, J=10.2 and 3.7 Hz), 7.04 (1H, dd, J=7.4 and 1.6 Hz), 7.16 (2H, d like, J=8.6 Hz), 7.23-7.12 (2H, m);
MS (FAB)m/z: 413 (M + Na)⁺.

### (Example 51) 2-(4-Methoxybenzyl)phenyl 5-C-methyl-β-D-glucopyranoside (Example Compound No. 1-121)

### (51a) 2-(4-Methoxybenzyl)phenyl 2,3,4,6-tetra-O-benzoyl-5-C-methyl-β-D-glucopyranoside

The compound obtained in (4c) (318 mg, 0.52 mmol), trichloroacetonitrile (0.26 mL, 2.60 mmol), 1,8-diazabicyclo[5.4.0]-7-undecene (8 µL, 0.05 mmol), and methylene chloride (6 mL) were used to prepare an imidate by the same method as in (1b). Subsequently, 2-(4-methoxybenzyl)phenol (100 mg, 0.47 mmol), a boron trifluoride-diethyl ether complex (66 µL, 0.52 mmol), and methylene chloride (6 mL) were used to obtain a crude product of the title compound (335 mg) by the same method as in (1b).

### (51b) 2-(4-Methoxybenzyl)phenyl 5-C-methyl-β-D-glucopyranoside

The compound obtained in (51a) (327 mg, 0.41 mmol), potassium carbonate (560 mg, 4.05 mmol), methanol (10 mL), and methylene chloride (1 mL) were used to obtain the title compound (126 mg, yield 69% in 2 steps) as a colorless solid by the same method as in (1c). However, purification was performed by silica gel flash column chromatography (methylene chloride:methanol, 100:0 to 85:15, v/v).
¹H NMR (400 MHz, CD₃OD): δ 1.26 (3H, s), 3.50-3.45 (1H, m), 3.52 (2H, d, J=6.6Hz), 3.67-3.55 (2H, m), 3.74 (3H, s), 3.91 (1H, d, J=15.2 Hz), 4.02 (1H, d, J=15.2 Hz), 5.15 (1H, d, J=7.8 Hz), 6.80 (2H, d like, J=8.6 Hz), 6.93-6.87 (1H, m), 7.03 (1H, d, J=7.4 Hz), 7.17-7.12 (4H, m);
MS (FAB)m/z: 413 (M + Na)⁺.

### (Example 52) 2-(4-Methoxybenzyl)phenyl 7-deoxy-L-glycero-β-D-gluco-heptopyranoside (Example Compound No. 1-122)

### (52a) 2-(4-Methoxybenzyl)phenyl 2,3,4,6-tetra-O-benzoyl-7-deoxy-L-glycero-β-D-gluco-heptopyranoside

The compound obtained in (6c) (318 mg, 0.52 mmol), trichloroacetonitrile (0.26 mL, 2.60 mmol), 1,8-diazabicyclo[5.4.0]-7-undecene (8 µL, 0.05 mmol), and methylene chloride (6 mL) were used to prepare an imidate by the same method as in (1b). Subsequently, 2-(4-methoxybenzyl)phenol (100 mg, 0.47 mmol), a boron trifluoride-diethyl ether complex (66 µL, 0.52 mmol), and methylene chloride (6 mL) were used to obtain a crude product of the title compound (337 mg) by the same method as in (1b).

### (52b) 2-(4-Methoxybenzyl)phenyl 7-deoxy-L-glycero-β-D-gluco-heptopyranoside

The compound obtained in (52a) (333 mg, 0.41 mmol), potassium carbonate (560 mg, 4.05 mmol), methanol (8 mL), and methylene chloride (2 mL) were used to obtain the title compound (82 mg, yield 45% in 2 steps) as a colorless solid by the same method as in (1c). However, purification was performed by silica gel flash column chromatography (methylene chloride:methanol, 95:5 to 88:12, v/v).
¹H NMR (400 MHz, CD₃OD): δ 1.29 (3H, d, J=6.6 Hz), 3.19 (1H, dd, J=9.5 and 1.7 Hz), 3.54-3.42 (2H, m), 3.62 (1H, t, J=9.2 Hz), 3.74 (3H, s), 3.94 (1H, d, J=15.0 Hz), 4.02 (1H, d, J=15.0 Hz), 4.14-4.07 (1H, m), 4.89 (1H, d, J=7.4 Hz), 6.80 (2H, d like, J=8.6 Hz), 6.91 (1H, dt like, J=10.2 and 3.7 Hz), 7.10-7.02 (2H, m), 7.15 (2H, d like, J=8.6 Hz), 7.17-7.11 (1H, m);
MS (FAB)m/z: 413 (M + Na)⁺.

### (Example 53) 2-[4-(2-Hydroxyethyl)benzyl]-5-hydroxymethylphenyl 7-deoxy-D-glycero-β-D-gluco-heptopyranoside (Example Compound No. 1-123)

### (53a) 2-[4-(2-Acetoxyethyl)benzyl]-5-acetoxymethylphenyl 2,3,4,6-tetra-O-benzoyl-7-deoxy-D-glycero-β-D-gluco-heptopyranoside)

The compound obtained in (7d) (165 mg, 0.27 mmol), trichloroacetonitrile (0.14 mL, 1.36 mmol), 1,8-diazabicyclo[5.4.0]-7-undecene (4 µL, 0.03 mmol), and methylene chloride (4 mL) were used to prepare an imidate by the same method as in (1b). Subsequently, 2-[4-(2-acetoxyethyl)benzyl]-5-acetoxymethylphenol [EP1270584A1 (2003/01/02)] (84 mg, 0.24 mmol), a boron trifluoride-diethyl ether complex (34 µL, 0.27 mmol), and methylene chloride (6 mL) were used to obtain a crude product of the title compound by the same method as in (1b).

### (53b) 2-[4-(2-Hydroxyethyl)benzyl]-5-hydroxymethylphenyl 7-deoxy-D-glycero-β-D-gluco-heptopyranoside

The crude product obtained in (53a) was dissolved in a mixed solution of methanol (2 mL) and tetrahydrofuran (2 mL), followed by addition of 2 M aqueous sodium hydroxide (1.6 mL, 3.25 mmol), and the mixture stirred overnight at room temperature. After the reaction was completed, saturated brine was added, the mixture was extracted with ethyl acetate, and the organic layer was washed with saturated aqueous sodium hydrogencarbonate, and saturated brine in this order. The organic layer was dried over sodium sulfate, and the residue was purified by filtration, concentration, and silica gel flash column chromatography (methylene chloride:methanol, 93:7 to 85:15, v/v) to obtain the title compound (17 mg, yield 16% in 2 steps) as a colorless solid.
¹H NMR (400 MHz, CD₃OD): δ 1.22 (3H, d, J=6.2 Hz), 2.76 (2H, t, J=7.0 Hz), 3.38-3.35 (2H, m), 3.49-3.42 (2H, m), 3.70 (2H, t, J=7.0 Hz), 3.95 (1H, d, J=14.8 Hz), 4.08-4.01 (2H, m), 4.54 (2H, brs), 4.92-4.81 (1H, m), 6.94-6.90 (1H, m), 7.03 (1H, d, J=7.4 Hz), 7.10 (2H, d like, J=7.9 Hz), 7.18-7.12 (3H, m);
MS (FAB)m/z: 457 (M + Na)⁺.

### (Example 54) 2-[4-(4-Hydroxypiperidin-1-yl)benzyl]phenyl 7-deoxy-D-glycero-β-D-gluco-heptopyranoside (Example Compound No. 1-124)

### (54a) 2-[4-(4-Acetoxypiperidin-1-yl)benzyl]phenyl 2,3,4,6-tetra-O-benzoyl-7-deoxy-D-glycero-β-D-gluco-heptopyranoside

2-[4-(4-Acetoxypiperidin-1-yl)benzyl]phenol (W02003/011880) (313 mg, 0.51 mmol), trichloroacetonitrile (0.26 mL, 2.56 mmol), 1,8-diazabicyclo[5.4.0]-7-undecene (8 µL, 0.51 mmol), and methylene chloride (6 mL) were used to prepare an imidate by the same method as in (1b), and 1-[4-(2-hydroxy benzyl)phenyl]piperidin-4-yl acetate (129 mg, 0.40 mmol), a boron trifluoride-diethyl ether complex (162µL, 1.28 mmol), and methylene chloride (7 mL) were used to obtain a crude product of the title compound by the same method as in (1b).

### (54b) 2-[4-(4-Hydroxypiperidin-1-yl)benzyl]phenyl 7-deoxy-D-glycero-β-D-gluco-heptopyranoside

The crude product obtained in (54a), 2 M aqueous sodium hydroxide (3.0 mL, 6.00 mmol), methanol (3 mL), and tetrahydrofuran (3 mL) were used to obtain the title compound (60 mg, yield 33% in 2 steps) as a colorless solid by the same method as in (53b). However, purification was performed by silica gel flash column chromatography (methylene chloride:methanol, 93:7 to 85:15, v/v).
¹H NMR (500 MHz, CD₃OD): δ 1.21 (3H, d, J=6.8 Hz), 1.68-1.59 (2H, m), 1.98-1.90 (2H, m), 2.81 (2H, ddd, J=12.7, 10.0 and 2.7 Hz), 3.40-3.30 (2H, m), 3.51-3.42 (4H, m), 3.75-3.67 (1H, m), 3.92 (1H, d, J=14.8 Hz), 4.00 (1H, d, J=14.8 Hz), 4.07-4.02 (1H, m), 4.91-4.84 (1H, m), 6.90 (2H, d like, J=8.8 Hz), 6.93-6.87 (1H, m), 7.03 (1H, d, J=6.9 Hz), 7.16-7.08 (4H, m);
MS (FAB)m/z: 498 (M + K)⁺.

### (Example 55) 3-Chloro-5-hydroxymethyl-2-(4-methoxybenzyl)phenyl 5-C-methyl-β-D-glucopyranoside (Example Compound No. 1-125)

### (55a) 5-Acetoxymethyl-3-chloro-2-(4-methoxybenzyl)phenyl 2,3,4,6-tetra-O-benzoyl-5-C-methyl-β-D-glucopyranoside

The compound obtained in (4c) (168 mg, 0.28 mmol), trichloroacetonitrile (0.14 mL, 1.38 mmol), 1,8-diazabicyclo[5.4.0]-7-undecene (4 µL, 0.03 mmol), and methylene chloride (4 mL) were used to prepare an imidate by the same method as in (1b). The compound obtained in (44e) (75 mg, 0.23 mmol), a boron trifluoride-diethyl ether complex (35 µL, 0.28 mmol), and methylene chloride (4 mL) were used to obtain a crude product of the title compound by the same method as in (1b).

### (55b) 3-Chloro-5-hydroxymethyl-2-(4-methoxybenzyl)phenyl 5-C-methyl-β-D-glucopyranoside

The crude product obtained in (55a), 2 M aqueous sodium hydroxide (2.1 mL, 4.13 mmol), methanol (3 mL), and tetrahydrofuran (2 mL) were used to obtain the title compound (54 mg, yield 51% in 2 steps) as a colorless solid by the same method as in (53b). However, purification was performed by silica gel flash column chromatography (methylene chloride:methanol, 95:5 to 87:13, v/v).
¹H NMR (400 MHz, CD₃OD): δ 1.28 (3H, s), 3.47 (1H, t, J=8.5 Hz), 3.52 (2H, d, J=2.3 Hz), 3.68-3.54 (2H, m), 3.73 (3H, s), 4.07 (1H, d, J=14.4 Hz), 4.20 (1H, d, J=14.4 Hz), 4.56 (2H, s), 5.18 (1H, d, J=7.8 Hz), 6.75 (2H, d like, J=8.6 Hz), 7.06 (1H, brs), 7.17 (2H, d like, J=8.6 Hz), 7.19 (1H, brs);
MS (FAB)m/z: 493, 495 (M + K)⁺.

### (Example 56) 3-Chloro-5-hydroxymethyl-2-(4-methoxybenzyl)phenyl 4-C-methyl-β-D-glucopyranoside (Example Compound No. 1-126)

### (56a) 5-Acetoxymethyl-3-chloro-2-(4-methoxybenzyl)phenyl 4-O-acetyl-2,3,6-tri-O-benzoyl-4-C-methyl-β-D-glucopyranoside

The compound obtained in (3c) (151 mg, 0.28 mmol), trichloroacetonitrile (0.14 mL, 1.38 mmol), 1,8-diazabicyclo[5.4.0]-7-undecene (4 µL, 0.03 mmol), and methylene chloride (4 mL) were used to prepare an imidate by the same method as in (1b). The compound obtained in (44e) (75 mg, 0.23 mmol), a boron trifluoride-diethyl ether complex (35 µL, 0.28 mmol), and methylene chloride (4 mL) were used to obtain a crude product of the title compound by the same method as in (1b).

### (56b) 3-Chloro-5-hydroxymethyl-2-(4-methoxybenzyl)phenyl 4-C-methyl-β-D-glucopyranoside

The crude product obtained in (56a), 2 M aqueous sodium hydroxide (2.1 mL, 4.13 mmol), methanol (3 mL), and tetrahydrofuran (2 mL) were used to obtain the title compound (44 mg, yield 41% in 2 steps) as a colorless solid by the same method as in (53b). However, purification was performed by silica gel flash column chromatography (methylene chloride:methanol, 95:5 to 87:13, v/v).
¹H NMR (400 MHz, CD₃OD): δ 1.11 (3H, s), 3.49-3.43 (3H, m), 3.61 (1H, dd, J=11.8 and 8.6 Hz), 3.72 (3H, s), 3.90 (1H, dd, J=11.8 and 2.4 Hz), 4.08 (1H, d, J=14.5 Hz), 4.20 (1H, d, J=14.5 Hz), 4.55 (2H, s), 4.94 (1H, dd, J=5.5 and 2.0 Hz), 6.75 (2H, d like, J=8.6 Hz), 7.10 (1H, brs), 7.16 (1H, brs), 7.18 (2H, d like, J=8.6 Hz);
MS (FAB)m/z: 493, 495 (M + K)⁺.

### (Example 57) 3-Chloro-5-hydroxymethyl-2-(4-methoxybenzyl)phenyl 7-deoxy-L-glycero-β-D-gluco-heptopyranoside (Example Compound No. 1-127)

### (57a) 5-Acetoxymethyl-3-chloro-2-(4-methoxybenzyl)phenyl 2,3,4,6-tetra-O-benzoyl-7-deoxy-L-glycero-β-D-gluco-heptopyranoside

The compound obtained in (6c) (168 mg, 0.28 mmol), trichloroacetonitrile (0.14 mL, 1.38 mmol), 1,8-diazabicyclo[5.4.0]-7-undecene (4 µL, 0.03 mmol), and methylene chloride (4 mL) were used to prepare an imidate by the same method as in (1b). The compound obtained in (44e) (75 mg, 0.23 mmol), a boron trifluoride-diethyl ether complex (35 µL, 0.28 mmol), and methylene chloride (4 mL) were used to obtain a crude product of the title compound by the same method as in (1b).

### (57b) 3-Chloro-5-hydroxymethyl-2-(4-methoxybenzyl)phenyl 7-deoxy-L-glycero-β-D-gluco-heptopyranoside

The crude product obtained in (57a), 2 M aqueous sodium hydroxide (2.1 mL, 4.13 mmol), methanol (3 mL), and tetrahydrofuran (2 mL) were used to obtain the title compound (60 mg, yield 56% in 2 steps) as a colorless solid by the same method as in (53b). However, purification was performed by silica gel flash column chromatography (methylene chloride:methanol, 95:5 to 87:13, v/v).
¹H NMR (400 MHz, CD₃OD): δ 1.31 (3H, d, J=6.6 Hz), 3.23 (1H, dd, J=9.6 and 1.8 Hz), 3.54-3.41 (2H, m), 3.62 (1H, t, J=9.6 Hz), 3.73 (3H, s), 4.09 (1H, d, J=14.5 Hz), 4.14-4.09 (1H, m), 4.20 (1H, d, J=14.5 Hz), 4.55 (2H, s), 4.92 (1H, d, J=7.4 Hz), 6.75 (2H, d like, J=8.8 Hz), 7.09 (1H, brs), 7.12 (1H, brs), 7.18 (2H, d like, J=8.8 Hz);
MS (FAB)m/z: 493,495 (M + K)⁺.

### (Example 58) 2-(4-Difluoromethoxybenzyl)-5-hydroxymethylphenyl 7-deoxy-D-glycero-β-D-gluco-heptopyranoside (Example Compound No. 1-128)

### (58a) Methyl 4-(diethoxyphosphoryloxymethyl)-3-methoxymethoxybenzoate

Potassium carbonate (9.9 g, 71.6 mmol) and chloromethyl methyl ether (4.3 g, 53.4 mmol) were successively added to a solution of methyl 4-formyl-3-hydroxybenzoate (5.2 g, 28.9 mmol) in N,N-dimethylformamide (60 mL) in an ice water bath, and the mixture was stirred at room temperature for 1 h. The reaction mixture was filtered using Celite, and the filtrate was diluted with ethyl acetate and washed with saturated brine. The organic layer was dried over anhydrous sodium sulfate, and then the solvent was removed under reduced pressure. The residue was purified by silica gel column chromatography to obtain a crude product (5.99 g, yield 92%).

The resulting crude product (2.05 g, 9.14 mmol) was dissolved in a mixed solution of methanol (20 mL) and tetrahydrofuran (10 mL), sodium borohydride (346 mg, 9.14 mmol) was added in an ice water bath, and the mixture was stirred at room temperature for 2 h. After the reaction was completed, water was added, the mixture was extracted with ethyl acetate, and the organic layer was washed with water and saturated brine in this order. The organic layer was dried over anhydrous sodium sulfate, and then the solvent was removed under reduced pressure to obtain a crude product (2.03 g, yield 98%).

The resulting crude product (2.03 g, 8.97 mmol) was dissolved in tetrahydrofuran (25 mL), followed by addition of triethylamine (5.6 mL, 40.4 mmol) and 4-(dimethylamino)pyridine (329 mg, 2.69 mmol), and chlorophosphate diethyl (4.3 mL, 29.6 mmol) was slowly added in an ice water bath. The mixture was stirred at room temperature for 4 h, followed by addition of tetrahydrofuran (5.0 mL), triethylamine (3.8 mL, 26.9 mmol), and chlorophosphate diethyl (2.8 mL, 19.7 mmol), and the mixture was stirred at room temperature for 19 h. Saturated aqueous ammonium chloride was added to the reaction mixture, the mixture was extracted with ethyl acetate, and then the organic layer was washed with saturated brine. The organic layer was dried over anhydrous sodium sulfate, and then the solvent was removed under reduced pressure. The residue was purified by silica gel flash column chromatography (hexane:ethyl acetate, 3:1 to 1:4, v/v) to obtain the title compound (2.69 g, yield 83%) as a colorless oil.
¹H NMR (500 MHz, CD₃OD): δ 1.33 (6H, t, J=7.1 Hz), 3.49 (3H, s), 3.92 (3H, s), 4.19-4.09 (4H, m), 5.19 (2H, d, J=6.8 Hz), 5.27 (2H, s), 7.51 (1H, d, J=7.8 Hz), 7.72 (1H, d, J=7.8 Hz), 7.74 (1H, s).

### (58b) Methyl 4-(4-difluoromethoxybenzyl)-3-methoxymethoxybenzoate

The compound obtained in (58a) (890 mg, 2.46 mmol) was dissolved in toluene (15 mL), then 2-(4-difluoromethoxyphenyl)-4,4,5,5-tetramethyl-[1,3,2]dioxaborolane (862 mg, 3.19 mmol), potassium phosphate (730 mg, 3.44 mmol), and tetrakis triphenylphosphine palladium (284 mg, 0.25 mmol) were successively added, and the mixture was stirred at 90°C for 3 h under a nitrogen atmosphere. Subsequently, 2-(4-difluoromethoxyphenyl)-4,4,5,5-tetramethyl-[1,3,2]dioxaborolane (300 mg, 1.11 mmol), potassium phosphate (208 mg, 0.98 mmol), and tetrakis triphenylphosphine palladium (284 mg, 0.25 mmol) were successively added, and the mixture was stirred under a nitrogen atmosphere at 90°C for 18 h. The reaction mixture was cooled to room temperature, diluted with ethyl acetate, and washed with water and saturated brine. The organic layer was dried over anhydrous sodium sulfate, and then the solvent was removed under reduced pressure. The residue was purified by silica gel flash column chromatography (hexane:ethyl acetate, 95:5 to 75:25, v/v) to obtain the title compound (215 mg, yield 25%) as a yellow oil.
¹H NMR (400 MHz, CDCl₃): δ 3.36 (3H, s), 3.90(3H, s), 4.01 (2H, s), 5.23 (2H, s), 6.47 (1H, t, J=74.1 Hz), 7.03 (2H, d, J=8.6 Hz), 7.18 (1H, d, J=7.9 Hz), 7.19 (2H, d, J=8.6 Hz), 7.65 (1H, brd, J=7.9 Hz), 7.72 (1H, brs);
MS (FAB)m/z: 353 (M + H)⁺.

### (58c) 5-Acetoxymethyl-2-(4-difluoromethoxybenzyl)phenol

The compound obtained in (58b) (215 mg, 0.61 mmol) was dissolved in methanol (3.0 mL) and tetrahydrofuran (1.0 mL), followed by addition of hydrochloric acid-methanol (ca. 2.2 M; 6.2 mL), and the mixture was stirred at room temperature for 15 h. The solvent was removed under reduced pressure, and then the residue was purified by silica gel flash column chromatography (hexane:ethyl acetate, 95:5 to 75:25, v/v) to obtain a crude product (160 mg, yield 85%).

The resulting crude product (157 mg, 0.51 mmol) was dissolved in tetrahydrofuran (3.0 mL), and the mixture was added dropwise to a suspension of lithium aluminium hydride (157 mg) in tetrahydrofuran (3.0 mL)with ice cooling and stirred at room temperature for 1 h. As a posttreatment, water (0.06 mL), 15% aqueous sodium hydroxide (0.06 mL), and water (0.18 mL) were successively added with ice cooling, the mixture was stirred and filtered using Celite, and then the solvent was removed from the filtrate under reduced pressure. The residue was purified by silica gel flash column chromatography (hexane:ethyl acetate, 3:1 to 1:3, v/v) to obtain a crude product (103 mg, yield 72%).

The resulting crude product (100 mg, 0.36 mmol), tetrahydrofuran (1.5 mL), vinyl acetate (1.5 mL), and bis(dibutylchlorotin)oxide (40 mg, 0.07 mmol) were used to obtain the title compound (104 mg, yield 90%) by the same method as in (8b). However, purification was performed by silica gel flash column chromatography (hexane:ethyl acetate, 88:12 to 55:45, v/v).
¹H NMR (400 MHz, CDCl₃):δ 2.12 (3H, s), 3.98 (2H, s), 4.84 (1H, s), 5.06 (2H, s), 6.49 (1H, t, J=74.1 Hz), 6.81 (1H, s), 6.90 (1H, d, J=7.7 Hz), 7.06 (2H, d, J=8.2 Hz), 7.11 (1H, d, J=7.7 Hz), 7.23 (2H, d, J=8.2 Hz);
MS (FAB)m/z: 322 (M + H)⁺.

### (58d) 5-Acetoxymethyl-2-(4-difluoro-methoxybenzyl)phenyl 2,3,4,6-tetra-O-benzoyl-7-deoxy-D-glycero-β-D-gluco-heptopyranoside

The compound obtained in (7d) (223 mg, 0.37 mmol), trichloroacetonitrile (0.18 mL, 1.83 mmol), 1,8-diazabicyclo[5.4.0]-7-undecene (5.5 µL, 0.04 mmol), and methylene chloride (4 mL) were used to prepare an imidate by the same method as in (1b). The compound obtained in (58c) (100 mg, 0.31 mmol), a boron trifluoride-diethyl ether complex (46 µL, 0.37 mmol), and methylene chloride (4 mL) were used to obtain a crude product of the title compound by the same method as in (1b).

### (58e) 2-(4-Difluoromethoxybenzyl)-5-hydroxymethylphenyl 7-deoxy-D-glycero-β-D-gluco-heptopyranoside

The crude product obtained in (58d), 2 M aqueous sodium hydroxide (2.7 mL, 5.5 mmol), methanol (3 mL), and tetrahydrofuran (3 mL) were used to obtain the title compound (84 mg, yield 41 % in 2 steps) as a colorless solid by the same method as in (53b). However, purification was performed by silica gel flash column chromatography (methylene chloride:methanol, 95:5 to 83:17, v/v).
¹H NMR (400 MHz, CD₃OD): δ 1.22 (3H, d, J=6.3 Hz), 3.40-3.35 (2H, m), 3.50-3.43 (2H, m), 3.98 (1H, d, J=14.7 Hz), 4.09-4.02 (1H, m), 4.08 (1H, d, J=14.7 Hz), 4.54 (2H, s), 4.95-4.90 (1H, m), 6.72 (1H, t, J=74.5 Hz), 6.94 (1H, dd, J=7.8 and 1.5 Hz), 7.00 (2H, d like, J=8.6 Hz), 7.06 (1H, d, J=7.8 Hz), 7.16 (1H, d, J=1.5 Hz), 7.28 (2H, d like, J=8.6 Hz);
MS (FAB)m/z: 495 (M + K)⁺.

### (Example 59) 5-Hydroxymethyl-2-(4-methoxymethylbenzyl)phenyl 7-deoxy-D-glycero-β-D-gluco-heptopyranoside (Example Compound No. 1-129)

### (59a) 5-Acetoxymethyl-2-(4-methoxymethylbenzyl)phenol

1-Bromo-4-methoxymethylbenzene (2.20 g, 10.9 mmol), metal magnesium (300 mg, 12.3 mmol), a catalytic amount of iodine, and tetrahydrofuran (6.0 mL) were used to prepare Grignard reagent by a usual method. The resulting Grignard reagent was added to a solution of ethyl 4-formyl-3-hydroxybenzoate (532 mg, 2.74 mmol) in tetrahydrofuran (6.0 mL) at -50°C, and the mixture was stirred for 16 h while heated to room temperature. Saturated aqueous ammonium chloride was added to the reaction mixture, the mixture was extracted with ethyl acetate, and then the organic layer was washed with saturated brine. The organic layer was dried over anhydrous sodium sulfate, and then the solvent was removed under reduced pressure. The residue was purified by silica gel flash column chromatography (hexane:ethyl acetate, 90:10 to 20:80, v/v) to obtain a crude product (234 mg, yield 27%).

The resulting crude product (231 mg, 0.73 mmol) was dissolved in acetonitrile (3.0 mL) and methylene chloride (1.5 mL), followed by addition of triethylsilane (0.35 mL, 2.19 mmol), then a boron trifluoride-diethyl ether complex (0.14 mL, 1.10 mmol) was added with ice cooling, and the mixture was stirred at room temperature for 2 h. Saturated aqueous sodium hydrogencarbonate was added to the reaction mixture with ice cooling, the mixture was extracted with ethyl acetate, and then the organic layer was washed with saturated brine. The organic layer was dried over anhydrous sodium sulfate, and then the solvent was removed under reduced pressure. The residue was purified by silica gel flash column chromatography (hexane:ethyl acetate, 89:11 to 60:40, v/v) to obtain a crude product (117 mg, yield 53%).

The resulting crude product (113 mg, 0.38 mmol) was dissolved in tetrahydrofuran (2.0 mL), the mixture was added dropwise to a suspension of lithium aluminium hydride (43 mg) in tetrahydrofuran (2.0 mL) with ice cooling and stirred at room temperature for 1.5 h. As a posttreatment, water (0.05 mL), 15% aqueous sodium hydroxide (0.05 mL), and water (0.15 mL) were successively added with ice cooling, and the mixture was stirred. Then, 1 N hydrochloric acid was added, the mixture was extracted with ethyl acetate, and then the organic layer was washed with saturated aqueous sodium hydrogencarbonate and saturated brine. The organic layer was dried over anhydrous sodium sulfate, and then the solvent was removed under reduced pressure. The residue was purified by silica gel flash column chromatography (hexane:ethyl acetate, 70:30 to 25:75, v/v) to obtain a crude product (95 mg, yield 98%).

The resulting crude product (93 mg, 0.36 mmol), tetrahydrofuran (1.5 mL), vinyl acetate (1.5 mL), and bis(dibutylchlorotin)oxide (40 mg, 0.07 mmol) were used to obtain the title compound (105 mg, yield 97%) by the same method as in (8b). However, purification was performed by silica gel flash column chromatography (hexane:ethyl acetate, 88:12 to 55:45, v/v).
¹H NMR (400 MHz, CDCl₃): δ 2.10 (3H, s), 3.38 (3H, s), 3.98 (2H, s), 4.42 (2H, s), 4.88 (1H, s), 5.03 (2H, s), 6.79 (1H, s), 6.87 (1H, d, J=7.8 Hz), 7.09 (1H, d, J=7.8 Hz), 7.21 (2H, d, J=8.2 Hz), 7.26 (2H, d, J=8.2 Hz);
MS (FAB)m/z: 339 (M + K)⁺.

### (59b) 5-Acetoxymethyl-2-(4-methoxymethylbenzyl)phenyl 2,3,4,6-tetra-O-benzoyl-7-deoxy-D-glycero-β-D-gluco-heptopyranoside

The compound obtained in (7d) (244 mg, 0.40 mmol), trichloroacetonitrile (0.20 mL, 2.00 mmol), 1,8-diazabicyclo[5.4.0]-7-undecene (6.0 µL, 0.04 mmol), and methylene chloride (4 mL) were used to prepare an imidate by the same method as in (1b). The compound obtained in (59a) (102 mg, 0.34 mmol), a boron trifluoride-diethyl ether complex (50 µL, 0.40 mmol), and methylene chloride (4 mL) were used to obtain a crude product of the title compound by the same method as in (1b).

### (59c) 5-Hydroxymethyl-2-(4-methoxymethylbenzyl)phenyl 7-deoxy-D-glycero-β-D-gluco-heptopyranoside

The crude product obtained in (59b), 2 M aqueous sodium hydroxide (3.0 mL, 6.00 mmol), methanol (3 mL), and tetrahydrofuran (3 mL) were used to obtain the title compound (100 mg, yield 68% in 2 steps) as a colorless solid by the same method as in (53b). However, purification was performed by silica gel flash column chromatography (methylene chloride:methanol, 95:5 to 83:17, v/v).
¹H NMR (400 MHz, CD₃OD): δ 1.22 (3H, d, J=6.3 Hz), 3.34 (3H, s), 3.40-3.36 (2H, m), 3.51-3.44 (2H, m), 3.99 (1H, d, J=15.1 Hz), 4.10-4.03 (1H, m), 4.09 (1H, d, J=15.1 Hz), 4.40 (2H, s), 4.55 (2H, s), 4.94-4.90 (1H, m), 6.94 (1H, dd, J=7.7 Hz, 1.4Hz), 7.05 (1H, d, J=7.7 Hz), 7.16 (1H, d, J=1.4 Hz), 7.21 (2H, d like, J=8.4 Hz), 7.24 (2H, d like, J=8.4 Hz);
MS (FAB)m/z: 435 (M + H)⁺.

### (Example 60) 2-(4-Cyclopropyloxybenzyl)-5-hydroxymethylphenyl 7-deoxy-D-glycero-β-D-gluco-heptopyranoside (Example Compound No. 1-130)

### (60a) Methyl 4-(4-Hydroxy benzyl)-3-methoxymethoxybenzoate

The compound obtained in (58a) (650 mg, 1.79 mmol) was dissolved in toluene (12 mL), 4-benzyloxyphenyl boronic acid (450 mg, 1.97 mmol), potassium phosphate (418 mg, 1.97 mmol), and tetrakis triphenylphosphine palladium (207 mg, 0.18 mmol) were successively added, and the mixture was stirred under a nitrogen atmosphere at 90°C for 15 h. The reaction mixture was cooled back to room temperature, followed by addition of saturated aqueous ammonium chloride, and extracted with ethyl acetate, and then the organic layer was washed with water and saturated brine in this order. The organic layer was dried over anhydrous sodium sulfate, and then the solvent was removed under reduced pressure. The residue was purified by silica gel flash column chromatography (hexane:ethyl acetate, 90:10 to 75:25, v/v) to obtain a crude product (519 mg, yield 74%).

The resulting crude product (519 mg, 1.32 mol) was dissolved in methanol (2.5 mL) and tetrahydrofuran (2.5 mL), followed by addition of 20% palladium hydroxide on carbon (150 mg), and the mixture was stirred under a hydrogen atmosphere at room temperature for 2 h. Methylene chloride was added to the reaction mixture, the mixture was stirred for 10 min, and then 20% palladium hydroxide on carbon was removed by filtration. The solvent was removed from the filtrate under reduced pressure. The residue was purified by silica gel flash column chromatography (hexane:ethyl acetate, 90:10 to 60:40, v/v) to obtain the title compound (370 mg, yield 93%) as a colorless solid.
¹H NMR (400 MHz, CDCl₃): δ 3.39 (3H, s), 3.89 (3H, s), 3.95 (2H, s), 4.71 (1H, s), 5.23 (2H, s), 6.74 (2H, d, J=8.6 Hz), 7.06 (2H, d, J=8.6 Hz), 7.15 (1H, d, J=7.8 Hz), 7.62 (1H, dd, J=7.8 and 1.8 Hz), 7.70 (1H, d, J=1.8 Hz).

### (60b) Methyl 3-methoxymethoxy-4-(4-vinyloxybenzyl)benzoate

The compound obtained in (60a) (244 mg, 0.74 mmol) was dissolved in acetonitrile (3.5 mL), followed by addition of copper(II) acetate (175 mg, 0.966 mmol) and tetravinyl tin (0.18 mL, 0.966 mmol), and the mixture was stirred under an oxygen atmosphere at 40°C for 19 h. 25% aqueous ammonium acetate was added to the reaction mixture, the mixture was stirred for 10 min and extracted with ethyl acetate, and then the organic layer was washed with saturated brine. The organic layer was dried over anhydrous sodium sulfate, and then the solvent was removed under reduced pressure. The residue was purified by silica gel flash column chromatography (hexane:ethyl acetate, 90:10 to 70:30, v/v) to obtain the title compound (229 mg, yield 86%) as a colorless oil.
¹H NMR (400 MHz, CDCl₃): δ 3.38(3H, s), 3.90 (3H, s), 3.99 (2H, s), 4.39 (1H, dd, J=6.0 and 1.6 Hz), 4.72 (1H, dd, J=13.8 and 1.6 Hz), 5.23 (2H, s), 6.61 (1H, dd, J=13.8 and 6.0 Hz), 6.92 (2H, d, J=8.6 Hz), 7.19-7.13 (3H, m), 7.64 (1H, dd, J=7.8 and 1.5 Hz), 7.72 (1H, d, J=1.5 Hz).

### (60c) Methyl 4-(4-cyclopropyloxybenzyl)-3-methoxymethoxybenzoate

The compound obtained in (60b) (225 mg, 0.69 mol) was dissolved in 1,2-dichloroethane (5.0 mL), followed by addition of chloroiodomethane (0.20 mL, 2.74 mmol), and then diethylzinc (1.0 M hexane solution; 1.37 mL, 1.37 mmol) was added dropwise over 45 min with ice cooling. The mixture was stirred at room temperature for 3 h, followed by addition of saturated aqueous ammonium chloride, and extracted with ethyl acetate, and then the organic layer was washed with saturated brine. The organic layer was dried over anhydrous sodium sulfate, and then the solvent was removed under reduced pressure. The residue was purified by silica gel flash column chromatography (hexane:ethyl acetate, 90:10 to 75:25, v/v) to obtain the title compound (162 mg, yield 69%) as a colorless oil.
¹H NMR (400 MHz, CDCl₃): δ 0.78-0.72 (4H, m), 3.40 (3H, s), 3.73-3.66 (1H, m), 3.89 (3H, s), 3.97 (2H, s), 5.24 (2H, s), 6.96 (2H, d, J=8.4 Hz), 7.11 (2H, d, J=8.4 Hz), 7.16 (1H, d, J=7.8 Hz), 7.63 (1H, d, J=7.8 Hz), 7.71 (1H, s).

### (60d) 5-Acetoxymethyl-2-(4-cyclopropyloxybenzyl)phenol

The compound obtained in (60c) (160 mg, 0.47 mmol) was dissolved in tetrahydrofuran (1.0 mL), followed by addition of hydrochloric acid-methanol (ca. 2.2 M; 3.0 mL), and the mixture was stirred at 40°C for 4 h. The solvent was removed under reduced pressure, and then the residue was purified by silica gel flash column chromatography (hexane:ethyl acetate, 90:10 to 70:30, v/v) to obtain a crude product (128 mg, yield 92%).

The resulting crude product (118 mg, 0.40 mmol) was dissolved in tetrahydrofuran (2.0 mL), and the mixture was added dropwise to a suspension of lithium aluminium hydride (118 mg) in tetrahydrofuran (2.0 mL) with ice cooling and stirred at room temperature for 1 h. As a posttreatment, water (0.05 mL), 15% aqueous sodium hydroxide (0.05 mL), and water (0.15 mL) were successively added with ice cooling, and the mixture was stirred. Then, 1 N hydrochloric acid was added, the mixture was extracted with ethyl acetate, and then the organic layer was washed with saturated aqueous sodium hydrogencarbonate and saturated brine. The organic layer was dried over anhydrous sodium sulfate, and then the solvent was removed under reduced pressure. The residue was purified by silica gel flash column chromatography (hexane:ethyl acetate, 70:30 to 25:75, v/v) to obtain a crude product (106 mg, yield 99%).

The resulting crude product (104 mg, 0.39 mmol), tetrahydrofuran (1.0 mL), vinyl acetate (2.0 mL), and bis(dibutylchlorotin)oxide (43 mg, 0.08 mmol) were used to obtain the title compound (113 mg, yield 94%) by the same method as in (8b). However, purification was performed by silica gel flash column chromatography (hexane:ethyl acetate, 88:12 to 55:45, v/v).
¹H NMR (500 MHz, CDCl₃): δ 0.77-0.72 (4H, m), 2.10 (3H, s), 3.72-3.67 (1H, m), 3.93 (2H, s), 4.78(1H, s), 5.03 (2H, s), 6.80 (1H, brs), 6.87 (1H, brd, J=7.3 Hz), 6.97 (2H, d, J=8.8 Hz), 7.10 (1H, d, J=7.3 Hz), 7.13 (2H, d, J=8.8 Hz);
MS (FAB)m/z: 351 (M + K)⁺.

### (60e) 5-Acetoxymethyl-2-(4-cyclopropyloxybenzyl)phenyl 2,3,4,6-tetra-O-benzoyl-7-deoxy-D-glycero-β-D-gluco-heptopyranoside

The compound obtained in (7d) (260 mg, 0.43 mmol), trichloroacetonitrile (0.21 mL, 2.13 mmol), 1,8-diazabicyclo[5.4.0]-7-undecene (6.5 µL, 0.04 mmol), and methylene chloride (4 mL) were used to prepare an imidate by the same method as in (1b). The compound obtained in (60d) (113 mg, 0.36 mmol), a boron trifluoride-diethyl ether complex (54 µL, 0.43 mmol), and methylene chloride (4 mL) were used to obtain a crude product of the title compound by the same method as in (1b).

### (60f) 2-(4-Cyclopropyloxybenzyl)-5-hydroxymethylphenyl 7-deoxy-D-glycero-β-D-gluco-heptopyranoside

The crude product obtained in (60e), 2 M aqueous sodium hydroxide (3.2 mL, 6.4 mmol), methanol (3 mL), and tetrahydrofuran (3 mL) were used to obtain the title compound (100 mg, yield 62% in 2 steps) as a colorless solid by the same method as in (53b). However, purification was performed by silica gel flash column chromatography (methylene chloride:methanol, 95:5 to 83:17, v/v).
¹H NMR (400 MHz, CD₃OD): δ 0.68-0.62 (2H, m), 0.77-0.68 (2H, m), 1.22 (3H, d, J=6.7 Hz), 3.39-3.36 (2H, m), 3.50-3.43 (2H, m), 3.75-3.69 (1H, m), 3.93 (1H, d, J=14.9 Hz), 4.01 (1H, d, J=14.9 Hz), 4.09-4.02 (1H, m), 4.54 (2H, s), 4.91 (1H, d like, J=7.8 Hz), 6.91 (2H, d like, J=8.6 Hz), 6.95-6.91 (1H, m), 7.03 (1H, d, J=7.4 Hz), 7.14 (2H, d like, J=8.6 Hz), 7.16-7.13 (1H, m)
MS (FAB)m/z: 485 (M + K)⁺.

### (Example 61) 2-(4-Acetylbenzyl)-5-hydroxymethylphenyl 7-deoxy-D-glycero-β-D-gluco-heptopyranoside (Example Compound No. 1-131)

### (61a) Methyl 4-(4-acetylbenzyl)-3-hydroxybenzoate

The compound obtained in (58a) (300 mg, 0.83 mmol) was dissolved in toluene (6.0 mL), 4-acetylphenylboronic acid (208 mg, 0.91 mmol), potassium phosphate (193 mg, 0.911 mmol), tetrakis triphenylphosphine palladium (96 mg, 0.083 mmol) were successively added, and the mixture was stirred under a nitrogen atmosphere at 90°C for 4 h. The reaction mixture was cooled back to room temperature, followed by addition of saturated aqueous ammonium chloride, and extracted with ethyl acetate, and then the organic layer was washed with water and saturated brine in this order. The organic layer was dried over anhydrous sodium sulfate, and then the solvent was removed under reduced pressure. The residue was purified by silica gel flash column chromatography (hexane:ethyl acetate, 90:10 to 70:30, v/v) to obtain a crude product (208 mg, yield 76%).

The resulting crude product (205 mg, 0.62 mmol) was dissolved in methanol (2.0 mL), tetrahydrofuran (1.0 mL), and water (1.0 mL), followed by addition of hydrochloric acid-1,4-dioxane (4 N; 2.0 mL), and the mixture was stirred for 30 min, followed by addition of 5 N hydrochloric acid (1.0 mL), and stirred at 40°C at 1.5 h. Saturated brine was added to the reaction mixture, the mixture was extracted with ethyl acetate, and then the organic layer was washed with saturated aqueous sodium hydrogencarbonate and saturated brine in this order. The organic layer was dried over anhydrous sodium sulfate, and then the solvent was removed under reduced pressure to obtain the title compound (176 mg, quantitative) as a colorless solid.
¹H NMR (400 MHz, DMSO-d6): δ 2.51 (3H, s), 3.79 (3H, s), 3.98 (2H, s), 7.19 (1H, d, J=7.8 Hz), 7.30-7.37 (3H, m), 7.42 (1H, d, J=1.2 Hz), 7.85 (2H, d, J=7.8 Hz), 9.96 (1H, brs).

### (61b) 5-Acetoxymethyl-2-(4-acetylbenzyl)phenol

The compound obtained in (61a) (173 mg, 0.61 mmol) was dissolved in toluene (5.0 mL), followed by addition of ethan-1,2-diol(1.2 mL), paratoluenesulfonic acid (10 mg, 0.06 mmol), and a molecular sieve 4A (ca. 500 mg), and the mixture was stirred for 2 days while heated to reflux. Saturated aqueous sodium hydrogencarbonate was added to the reaction mixture, the mixture was extracted with ethyl acetate, and then the organic layer was washed with saturated brine. The organic layer was dried over anhydrous sodium sulfate, and then the solvent was removed under reduced pressure. The residue was purified by silica gel flash column chromatography (hexane:ethyl acetate, 90:10 to 60:40, v/v) to obtain a crude product (70 mg, yield 35%).

The resulting crude product (90 mg, 0.27 mmol) was dissolved in tetrahydrofuran (2.0 mL), the mixture was added dropwise to a suspension of lithium aluminium hydride (31 mg) in tetrahydrofuran (2.0 mL) with ice cooling and stirred at room temperature for 1 h. As a posttreatment, water (0.04 mL), 15% aqueous sodium hydroxide (0.04 mL), and water (0.11 mL) were successively added and stirred with ice cooling. Subsequently, 1 N hydrochloric acid was added, the mixture was stirred and extracted with ethyl acetate, and then the organic layer was washed with saturated aqueous sodium hydrogencarbonate and saturated brine. The organic layer was dried over anhydrous sodium sulfate, and then the solvent was removed under reduced pressure. The residue was purified by silica gel flash column chromatography (hexane:ethyl acetate, 70:30 to 25:75, v/v) to obtain a crude product (78 mg, quantitative).

The resulting crude product (78 mg, 0.27 mmol), tetrahydrofuran (1.0 mL), vinyl acetate (2.0 mL), and bis(dibutylchlorotin)oxide (53 mg, 0.10 mmol) were used to obtain the title compound (71 mg, yield 87%) by the same method as in (8b). However, purification was performed by silica gel flash column chromatography (hexane:ethyl acetate, 90:10 to 55:45, v/v).
¹H NMR (400 MHz, CDCl₃): δ 2.10 (3H, s), 2.58 (3H, s), 4.04 (2H, s), 4.95 (1H, s), 5.04 (2H, s), 6.80 (1H, s), 6.89 (1H, d, J=7.4 Hz), 7.10 (1H, d, J=7.4 Hz), 7.32 (2H, d, J=7.8 Hz), 7.88 (2H, d, J=7.8 Hz)
MS (EI)m/z: 298 (M)⁺.

### (61c) 5-Acetoxymethyl-2-(4-acetylbenzyl)phenyl 2,3,4,6-tetra-O-benzoyl-7-deoxy-D-glycero-β-D-gluco-heptopyranoside

The compound obtained in (7d) (176 mg, 0.29 mmol), trichloroacetonitrile (0.15 mL, 1.45 mmol), 1,8-diazabicyclo[5.4.0]-7-undecene (4.5 µL, 0.03 mmol), and methylene chloride (4 mL) were used to prepare an imidate by the same method as in (1b). Subsequently, the compound obtained in (61b) (69 mg, 0.23 mmol), a boron trifluoride-diethyl ether complex (37 µL, 0.29 mmol), and methylene chloride (4 mL) were used to obtain a crude product of the title compound by the same method as in (1b).

### (61d) 2-(4-Acetylbenzyl)-5-hydroxymethylphenyl 7-deoxy-D-glycero-β-D-gluco-heptopyranoside

The crude product obtained in (61c), potassium carbonate (600 mg, 4.34 mmol), methanol (2 mL), and methylene chloride (2 mL) were used to obtain the title compound (75 mg, yield 75% in 2 steps) as a colorless solid by the same method as in (1c). However, purification was performed by silica gel flash column chromatography (methylene chloride:methanol, 95:5 to 83:17, v/v).
¹H NMR (400 MHz, CD₃OD): δ 1.21 (3H, d, J=6.6 Hz), 2.56 (3H, s), 3.40-3.34 (2H, m), 3.49-3.42 (2H, m), 4.05 (1H, d, J=14.9 Hz), 4.09-4.01 (1H, m), 4.17 (1H, d, J=14.9 Hz), 4.55 (2H, s), 4.94-4.90 (1H, m), 6.95 (1H, brd, J=7.8 Hz), 7.09 (1H, d, J=7.8 Hz), 7.17 (1H, brs), 7.39 (2H, d like, J=8.6 Hz), 7.87 (2H, d like, J=8.6 Hz);
MS (FAB)m/z: 433 (M + H)⁺.

### (Example 62) 2-(4-Cyclopropylbenzyl)-5-hydroxymethylphenyl 7-deoxy-D-glycero-β-D-gluco-heptopyranoside (Example Compound No. 1-131)

### (62a) Methyl 4-(4-cyclopropylbenzyl)-3-methoxymethoxybenzoate

The compound obtained in (58a) (481 mg, 1.33 mmol) was dissolved in toluene (9.0 mL), 4-vinylphenyl boronic acid (236 mg, 1.59 mmol), potassium phosphate (338 mg, 1.59 mmol), and tetrakis triphenylphosphine palladium (154 mg, 0.13 mmol) were successively added, and the mixture was stirred under a nitrogen atmosphere at 90°C for 4 h. The reaction mixture was cooled back to room temperature, followed by addition of saturated aqueous ammonium chloride, the mixture was extracted with ethyl acetate, and the organic layer was washed with water and saturated brine in this order. The organic layer was dried over anhydrous sodium sulfate, and then the solvent was removed under reduced pressure. The residue was purified by silica gel flash column chromatography (hexane:ethyl acetate, 95:5 to 82:18, v/v) to obtain a crude product (246 mg, yield 59%).

The resulting crude product (244 mg, 0.78 mmol) was dissolved in 1,2-dichloroethane (6.0 mL), followed by addition of chloroiodomethane (0.57 mL, 7.81 mmol), and then diethylzinc (1.0 M hexane solution; 3.9 mL, 3.91 mmol) was added dropwise at room temperature over 1 h. The mixture was stirred at room temperature for 24 h, followed by addition of saturated aqueous ammonium chloride, and extracted with ethyl acetate, and then the organic layer was washed with saturated brine. The organic layer was dried over anhydrous sodium sulfate, and then the solvent was removed under reduced pressure. The residue was purified by silica gel flash column chromatography (hexane:ethyl acetate, 95:5 to 80:20, v/v) to obtain the title compound (204 mg, yield 80%) as a colorless oil.
¹H NMR (400 MHz, CDCl₃): δ 0.67-0.61 (2H, m), 0.95-0.89 (2H, m), 1.90-1.81 (1H, m), 3.40 (3H, s), 3.89 (3H, s), 3.98 (2H, s), 5.23 (2H, s), 6.98 (2H, d, J=8.2 Hz), 7.08 (2H, d, J=8.2 Hz), 7.15 (1H, d, J=7.8 Hz), 7.62 (1H, dd, J=7.8 and 1.5Hz), 7.71 (1H, d, J=1.5 Hz);
MS (FAB)m/z: 327 (M + H)⁺.

### (62b) 5-Acetoxymethyl-2-(4-cyclopropylbenzyl)phenol

The compound obtained in (62a) (200 mg, 0.61 mmol) was dissolved in tetrahydrofuran (1.0 mL), followed by addition of hydrochloric acid-methanol (ca. 2.2 M; 3.0 mL), and the mixture was stirred at 40°C for 3 h. The solvent was removed under reduced pressure, and then the residue was purified by silica gel flash column chromatography (hexane:ethyl acetate, 90:10 to 67:33, v/v) to obtain a crude product (155 mg, yield 90%).

The resulting crude product (155 mg, 0.55 mmol) was dissolved in tetrahydrofuran (3.0 mL), and the mixture was added dropwise to a suspension of lithium aluminium hydride (63 mg) in tetrahydrofuran (3.0 mL) with ice cooling and stirred at room temperature for 1 h. As a posttreatment, water (0.07 mL), 15% aqueous sodium hydroxide (0.07 mL), and water (0.20 mL) were successively added, and the mixture was stirred with ice cooling. Subsequently, 1 N hydrochloric acid was added, the mixture was extracted with ethyl acetate, and then the organic layer was washed with saturated aqueous sodium hydrogencarbonate and saturated brine in this order. The organic layer was dried over anhydrous sodium sulfate, and then the solvent was removed under reduced pressure. The residue was purified by silica gel flash column chromatography (hexane:ethyl acetate, 85:15 to 30:70, v/v) to obtain a crude product (137 mg, yield 98%).

The resulting crude product (134 mg, 0.53 mmol), tetrahydrofuran (1.0 mL), vinyl acetate (2.5 mL), and bis(dibutylchlorotin)oxide (58 mg, 0.11 mmol) were used to obtain the title compound (148 mg, yield 95%) by the same method as in (8b). However, purification was performed by silica gel flash column chromatography (hexane:ethyl acetate, 90:10 to 55:45, v/v).
¹H NMR (400 MHz, CDCl₃): δ 0.67-0.63 (2H, m), 0.95-0.90 (2H, m), 1.90-1.81 (1H, m), 2.10 (3H, s), 3.94 (2H, s), 4.74 (2H, s), 5.04 (1H, s), 6.80 (1H, d, J=1.6 Hz), 6.87 (1H, dd, J=7.6 and 1.6 Hz), 7.00 (2H, d, J=8.2 Hz), 7.14-7.08 (3H, m);
MS (FAB)m/z: 335 (M + K)⁺.

### (62c) 5-Acetoxymethyl-2-(4-cyclopropylbenzyl)phenyl 2,3,4,6-tetra-O-benzoyl-7-deoxy-D-glycero-β-D-gluco-heptopyranoside

The compound obtained in (7d) (185 mg, 0.30 mmol), trichloroacetonitrile (0.15 mL, 1.52 mmol), 1,8-diazabicyclo[5.4.0]-7-undecene (4.5 µL, 0.03 mmol), and methylene chloride (4 mL) were used to prepare an imidate by the same method as in (1b). Subsequently, the compound obtained in (62b) (72 mg, 0.24 mmol), a boron trifluoride-diethyl ether complex (38 µL, 0.3 mmol), and methylene chloride (4 mL) were used to obtain a crude product of the title compound by the same method as in (1b).

### (62d) 2-(4-Cyclopropylbenzyl)-5-hydroxymethylphenyl 7-deoxy-D-glycero-β-D-gluco-heptopyranoside

The crude product obtained in (62c), 2 M aqueous sodium hydroxide (2.3 mL, 4.6 mmol), methanol (3 mL), and tetrahydrofuran (3 mL) were used to obtain the title compound (92 mg, yield 88% in 2 steps) as a colorless solid by the same method as in (53b). However, purification was performed by silica gel flash column chromatography (methylene chloride:methanol, 95:5 to 83:17, v/v).
¹H NMR (400 MHz, CD₃OD): δ 0.63-0.58 (2H, m), 0.92-0.86 (2H, m), 1.22 (3H, d, J=6.6 Hz), 3.39-3.34 (2H, m), 3.50-3.42 (2H, m), 3.93-3.89 (1H, m), 3.94 (1H, d, J=15.0 Hz), 4.02 (1H, d, J=15.0 Hz), 4.09-4.02 (1H, m), 4.54 (2H, s), 4.92-4.88 (1H, m), 6.94-6.90 (1H, m), 6.94 (2H, d like, J=8.2 Hz), 7.02 (1H, d, J=7.8 Hz), 7.10 (2H, d like, J=8.2 Hz), 7.14 (1H, d, J=1.1 Hz);
MS (FAB)m/z: 469 (M + K)⁺,

### (Example 63) 2-(4-Cyclopropoxybenzyl)-3-fluoro-5-hydroxymethyl-phenyl 7-deoxy-D-glycero-β-D-gluco-heptopyranoside (Example Compound No. 1-133)

### (63a) Ethyl 4-(4-cyclopropoxybenzoyl)-3-hydroxy-5-oxo-cyclohex-3-enecarboxylate

Oxalyl chloride (0.17 mL, 1.95 mmol) and a catalytic amount of N,N-dimethylformamide were added to a solution of 4-cyclopropoxybenzoate (US4009208) (350 mg, 1.96 mmol) in tetrahydrofuran (4 mL), and the mixture was stirred at room temperature for 1 h. The solvent was removed under reduced pressure, and a crude product of the resulting 4-cyclopropoxybenzoyl chloride (386 mg) was used in the subsequent reaction as it was.

Ethyl-3-hydroxy-5-oxo-cyclohex-3-ene carboxylate (EP1571148A1) (310 mg, 1.68 mmol), triethylamine (0.82 mL, 5.88 mmol), 4-cyclopropoxybenzoyl chloride (386 mg, 1.96 mmol), acetonitrile (5 mL), and trimethyl cyanonitrile (0.031 mL, 0.23 mmol) were used to obtain a crude product of the title compound (580 mg) by the same method as in (42a).

### (63b) Ethyl 4-(4-cyclopropoxybenzoyl)-3-fluoro-5-oxo-cyclohex-3-enecarboxylate

The crude product obtained in (63a) (580 mg), methylene chloride (6 mL), and diethylaminosulfur trifluoride (0.66 mL, 5.04 mmol) were used to obtain the title compound (289 mg, yield 50%) as an oil by the same method as in (46a).
¹H NMR (500 MHz, CDCl₃): δ 0.79 - 0.84 (4H, m), 1.31 (3H, t, J = 7.4 Hz), 2.79 - 2.81 (2H, m), 2.97 (1H, dt, J = 18.6 and 5.2 Hz), 3.08 (1H, dd, J = 18.6 and 8.6 Hz), 3.25 - 3.30 (1H, m), 3.78 - 3.82 (1H, m), 4.25 (2H, q, J = 7.4 Hz), 7.08 (2H, d, J = 8.8 Hz), 7.80 (2H, d, J = 8.8 Hz);
MS (EI)m/z: 346 (M)⁺.

### (63c) Ethyl 4-(4-cyclopropoxybenzoyl)-3-fluoro-5-hydroxybenzoate

The compound obtained in (63b) (289 mg, 0.83 mmol), triethylamine (0.35 mL, 2.48 mmol), acetonitrile (4 mL), and trimethylsilane iodide (0.30 mL, 2.09 mmol) were used to obtain an amorphous crude product by the same method as in (47c). Subsequently, the amorphous crude product, toluene (5 mL), and silica gel (SK-85) (1.16 g) were used to obtain the title compound (130 mg, yield 45%) by the same method as in (47c).
¹H NMR (500 MHz, CDCl₃): δ 0.73 - 0.77 (4H, m), 1.37 (3H, t, J = 7.0 Hz), 3.69 - 3.72 (1H, m), 4.35 (2H, q, J = 7.0 Hz), 6.32 (1H, s), 7.02 (2H, d, J = 8.8 Hz), 7.21 (1H, d, J = 10.3 Hz), 7.35 (2H, d, J = 8.8 Hz), 8.99 (1H, s);
MS (FAB)m/z: 347 (M+H)⁺.

### (63d) Ethyl 4-(4-cyclopropoxybenzyl)-3-fluoro-5-hydroxylbenzoate

The compound obtained in (63c) (130 mg, 0.38 mmol), methanol (2 mL), and sodium borohydride (36 mg, 0.95 mmol) were used to obtain a crude product (131 mg) as an amorphous diol compound by the same method as in (42d). However, the product was used in the subsequent reaction without purification. The crude product as a diol compound (131 mg), acetonitrile (2 mL), triethylsilane (0.18 mL, 1.13 mmol), and a boron fluoride-diethyl ether complex (0.072 mL, 0.57 mmol) were used to obtain a crude product of the title compound (111 mg, yield 89%) as a solid by the same method as in (42e).

### (63e) 5-Acetoxymethyl-2-(4-cyclopropoxybenzyl)3-fluorophenol

The compound obtained in (63d) (111 mg, 0.34 mmol), lithium aluminium hydride (38 mg, 1.00 mmol), and tetrahydrofuran (3 mL) were used to obtain a crude product (97 mg) as a diol compound by the same method as in (47d). The product was used in the subsequent reaction as it was.

The resulting crude product (97 mg, 0.34 mmol) was dissolved in tetrahydrofuran (1.5 mL), and vinyl acetate (1.5 mL) and bis(dibutylchlorotin)oxide (74 mg, 0.13 mmol) were used to obtain the title compound (72 mg, yield 65%) as a white solid by the same method as in (44c).
¹H NMR (400 MHz, CDCl₃): δ 0.74 - 0.75 (4H, m), 2.12 (3H, s), 3.67 - 3.71 (1H, m), 3.96 (2H, s), 4.91 (1H, s), 5.01 (2H, s), 6.60 (1H, s), 6.70 (1H, d, J = 9.3 Hz), 6.96 (2H, d, J = 8.3 Hz), 7.19 (2H, d, J = 8.3 Hz);
MS (FAB)m/z: 369 (M+K)⁺.

### (63f) 5-Acetoxymethyl-2-(4-cyclopropoxybenzyl)-3-fluorophenyl 7-deoxy-D-glycero-β-D-gluco-heptopyranoside

The compound obtained in (7d) (200 mg, 0.33 mmol), trichloroacetonitrile (0.165 mL, 1.63 mmol), 1,8-diazabicyclo[5.4.0]-7-undecene (50 µL, 0.033 mmol), and methylene chloride (4 mL) were used to prepare an imidate by the same method as in (1b). Subsequently, the compound obtained in (63e) (72 mg, 0.22 mmol), a boron trifluoride-diethyl ether complex (0.041 mL, 0.33 mmol), and methylene chloride (4 mL) were used to obtain a crude product of the title compound (330 mg) by the same method as in (1b). The product was used in the subsequent reaction as it was.

### (63g) 2-(4-Cyclopropoxybenzyl)-3-fluoro-5-hydroxymethyl-phenyl 7-deoxy-D-glycero-β-D-gluco-heptopyranoside

The compound obtained in (63f) (330 mg), potassium carbonate (450 mg, 3.26 mmol), methanol (8 mL), and methylene chloride (2 mL) were used to obtain the title compound (72 mg, yield 71 %) as a white solid by the same method as in (1c). ¹H NMR (500 MHz, CD₃OD): δ 0.62 - 0.65 (2H, m), 0.72 - 0.75 (2H, m), 1.22 (3H, d, J = 6.8 Hz), 3.37 - 3.38 (2H, m), 3.44 - 3.51 (2H, m), 3.68 - 3.73 (1H, m), 3.93 (1H, d, J = 18.1 Hz), 4.03 (1H, d, J = 18.1 Hz), 4.04 - 4.07 (1H, m), 4.55 (2H, s), 4.94 (1H, d, J = 7.3 Hz), 6.79 (1H, d, J = 10.2 Hz), 6.88 (2H, d, J = 8.8 Hz), 6.98 (1H, s), 7.19 (2H, d, J = 8.8 Hz);
MS (FAB)m/z: 503 (M+K)⁺.

### (Example 64) 5-Amino-2-(4-methoxybenzyl)phenyl 4-C-methyl-β-D-glucopyranoside (Example Compound No. 1-134)

4-O-Acetyl-4-C-methyl-2,3,6-tri-O-benzoyl-α,β-D-glucopyranoside obtained in (3c) (308 mg, 0.56 mmol), methylene chloride (6 mL), trichloroacetonitrile (0.28 mL, 2.77 mmol), and 1,8-diazabicyclo[5.4.0]-7-undecene (17 µL, 0.11 mmol) were used to obtain an imidate (0.48 g) by the same method as in (16). The resulting imidate (0.48 g), benzyl N-{3-hydroxy-4-(4-methoxybenzyl)phenyl}carbamate obtained in (2 1 g) (0.17 g, 0.47 mmol), methylene chloride (6 mL), and a boron trifluoride-diethyl ether complex (59 µL, 0.47 mmol) were used to obtain 5-benzyloxycarbonylamino-2-(4-methoxybenzyl)phenyl 4-O-acetyl-4-C-methyl-2,3,6-tri-O-benzoyl-β-D-glucopyranoside (0.42 g) as a pale brown oil by the same method as in (16).

The resulting glycoside compound (0.41 g), methanol (4 mL), tetrahydrofuran (4 mL), and 10% palladium on carbon (0.20 g) were used to obtain 5-amino-2-(4-methoxybenzyl)phenyl 4-O-acetyl-4-C-methyl-2,3,6-tri-O-benzoyl-β-D-glucopyranoside (0.35 g) as a crude product by the same method as in (16). The resulting amino compound (0.35 g), methylene chloride (3 mL), methanol (15 mL), and potassium carbonate (0.65 g, 4.70 mmol) were used to obtain 5-amino-2-(4-methoxybenzyl)phenyl 4-C-methyl-β-D-glucopyranoside (40 mg, yield 21%) as a pale yellow powder by the same method as in (16).
¹H NMR (400 MHz, CD₃OD): δ 1.12 (3H, s), 3.42-3.44 (3H, m), 3.63 (1H, dd, J=11.7 and 8.2 Hz), 3.74 (3H, s), 3.78 (1H, d, J=15.3 Hz), 3.87-3.93 (2H, m), 4.83 (1H, d, J=7.8 Hz), 6.33 (1H, dd, J=8.0 and 2.2 Hz), 6.64 (1H, d, J=2.2 Hz), 6.76-6.78 (3H, m), 7.11 (2H, d, J=8.6 Hz);
MS (FAB)m/z: 405 (M)⁺.

### (Example 65) 5-Amino-2-(4-methoxybenzyl)phenyl 4-deoxy-β-D-glucopyranoside

### (Example Compound No. 2-18)

2,3,6-Tri-O-benzoyl-4-deoxy-α,β-D-glucopyranoside (Liebigs Ann. Chem. GE, 1992, 7, 747-758) (0.24 g, 0.50 mmol), methylene chloride (5 mL), trichloroacetonitrile (0.25 mL, 2.48 mmol), and 1,8-diazabicyclo[5.4.0]-7-undecene (15 µL, 0.10 mmol) were used to obtain an imidate (0.40 g) by the same method as in (16). The resulting imidate (0.40 g), benzyl N-{3-hydroxy-4-(4-methoxybenzyl)phenyl} carbamate obtained in (21 g) (0.15 g, 0.41 mmol), methylene chloride (5 mL), and a boron trifluoride-diethyl ether complex (52 µL, 0.41 mmol) were used to obtain 5-benzyloxycarbonylamino-2-(4-methoxybenzyl)phenyl 2,3,6-tri-O-benzoyl-4-deoxy-β-D-glucopyranoside (0.48 g) as a pale brown oil by the same method as in (16).

The resulting glycoside compound (0.47 g), methanol (5 mL), tetrahydrofuran (5 mL), and 10% palladium on carbon (0.20 g) were used to obtain 5-amino-2-(4-methoxybenzyl)phenyl 2,3,6-tri-O-benzoyl-4-deoxy-β-D-glucopyranoside (0.39 g) as a crude product by the same method as in (16). The resulting amino compound (0.39 g), methylene chloride (4 mL), methanol (20 mL), and potassium carbonate (0.57 g, 4.12 mmol) were used to obtain 5-amino-2-(4-methoxybenzyl)phenyl 4-deoxy-β-D-glucopyranoside (91 mg, yield 59%) as a pale yellow powder by the same method as in (16).
¹H NMR (400 MHz, CD₃OD): δ 1.45 (1H, dd, J=24.2 and 11.7 Hz), 1.95-2.00 (1H, m), 3.32-3.36 (1H, m), 3.60 (2H, d, J=5.1 Hz), 3.64-3.71 (2H, m), 3.74 (3H, s), 3.79 (1H, d, J=15.2 Hz), 3.89 (1H, d, J=15.2 Hz), 4.79 (1H, d, J=7.8 Hz), 6.33 (1H, dd, J= 8.2 and 2.2 Hz), 6.59 (1H, d, J=2.2 Hz), 6.76-6.79 (3H, m), 7.11 (2H, d, J=8.6 Hz); MS (FAB)m/z: 375 (M)⁺.

### (Example 66) 5-Hydroxymethyl-2-(4-methylsulfanylbenzyl)phenyl 7-deoxy-D-glycero-β-D-gluco-heptopyranoside (Example Compound No. 1-135)

### (66a) Methyl 3-hydroxy-4-[hydroxy-(4-methylsulfanylphenyl)methyl]benzoate

4-Bromothioanisole (9.02 g, 44.4 mmol) was dissolved in tetrahydrofuran (50 mL), followed by addition of magnesium (1.08 g, 44.4 mmol) and a catalytic amount of iodine, and the mixture was stirred at room temperature for 30 min and further heated to reflux for 1 h. The reaction mixture was added dropwise to a solution of 2-hydroxy-4-methoxycarbonylbenzaldehyde (2.00 g, 11.1 mmol) in tetrahydrofuran (40 mL) at -50°C, and the mixture was stirred from -50 to 2°C for 1 h. Aqueous ammonium chloride was added to the reaction mixture, the mixture was extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 3:1 to 2:1, v/v) to obtain the title compound (3.38 g, yield 100%) as a yellow oil.
¹H NMR (400 MHz, CDCl₃): δ 2.48 (3H, s), 2.89 (1H, d, J=3.1 Hz), 3.89 (3H, s), 6.04 (1H, d, J=3.1 Hz), 6.96 (2H, d, J=7.8 Hz), 7.25 (2H, d, J=8.6 Hz), 7.31 (2H, d, J=8.6 Hz), 7.49 (1H, dd, J=7.8 and 1.6 Hz), 7.56 (1H, d, J=1.6 Hz), 8.00 (1H, s);
MS (FAB)m/z: 304 (M)⁺.

### (66b) 5-Hydroxymethyl-2-(4-methylsulfanylbenzyl)phenol

The compound obtained in (66a) (1.20 g, 3.94 mmol) was dissolved in methanol (20 mL), followed by addition of concentrated hydrochloric acid (0.33 mL, 3.99 mmol) and 10% palladium on carbon (1.20 g), and the mixture was stirred under a hydrogen atmosphere at room temperature for 3 h. Insoluble matters were removed from the reaction mixture by filtration, then the solvent was removed under reduced pressure to obtain methyl 3-hydroxy-4-(4-methylsulfanylbenzyl)benzoate (1.22 g) as a pale brown solid crude product. A solution of the resulting compound (1.22 g) in tetrahydrofuran (5 mL) was added dropwise to a suspension of lithium aluminium hydride (0.45 g, 11.9 mmol) in tetrahydrofuran (20 mL) with ice cooling, and the mixture was stirred at room temperature for 1 h. Water (0.5 mL), 15% aqueous sodium hydroxide (0.5 mL), and water (1.5 mL) were successively added dropwise with ice cooling, and the mixture was stirred at room temperature for 1 h and allowed to stand overnight at room temperature. Insoluble matters were removed by filtration using Celite, and then the solvent was removed under reduced pressure. The residue was purified by silica gel column chromatography (hexane:ethyl acetate, 1:1 to 1:2, v/v) to obtain the title compound (0.83 g) as a colorless oil.

### (66c) 5-Acetoxymethyl-2-(4-methylsulfanylbenzyl)phenol

The compound obtained in (66b) (0.82 g) was dissolved in tetrahydrofuran (8 mL), followed by addition of vinyl acetate (8 mL) and bis(dibutylchlorotin)oxide (87 mg, 0.16 mmol), and the mixture was stirred at room temperature for 4 h, allowed to stand overnight at room temperature, and further stirred at room temperature for 10 h. The reaction mixture was allowed to stand overnight at room temperature, and then the solvent was removed under reduced pressure. The residue was purified by silica gel column chromatography (hexane:ethyl acetate, 5:1 to 3:1, v/v) to obtain the title compound (0.65 g, yield 55%) as a colorless oil.
¹H NMR (400 MHz, CDCl₃): δ 2.11 (3H, s), 2.47 (3H, s), 3.94 (2H, s), 4.77 (1H, s), 5.05 (2H, s), 6.90-6.79 (2H, m), 7.27-7.09 (5H, m).

### (66d) 5-Hydroxymethyl-2-(4-methylsulfanylbenzyl)phenyl 7-deoxy-D-glycero-β-D-gluco-heptopyranoside

2,3,4,6-Tetra-O-benzoyl-7-deoxy-D-glycero-α,β-D-gluco-heptopyranoside (0.36 g, 0.59 mmol) obtained in (7d), methylene chloride (6 mL), trichloroacetonitrile (0.30 mL, 2.97 mmol), and 1,8-diazabicyclo[5.4.0]-7-undecene (18 µL, 0.12 mmol) were used to obtain an imidate (0.50 g) by the same method as in (1b). The resulting imidate (0.50 g), the compound obtained in (66c) (0.15 g, 0.50 mmol), methylene chloride (6 mL), and a boron trifluoride-diethyl ether complex (126 µL, 0.99 mmol) were used to obtain 5-acetoxymethyl-2-(4-methylsulfanylbenzyl)phenyl 2,3,4,6-tetra-O-benzoyl-7-deoxy-D-glycero-β-D-gluco-heptopyranoside (0.35 g) as a colorless oil by the same method as in (1b).

The resulting glycoside compound (0.35 g), methylene chloride (3 mL), methanol (15 mL), and potassium carbonate (0.69 g, 4.99 mmol) were used to obtain 5-hydroxymethyl-2-(4-methylsulfanylbenzyl)phenyl 7-deoxy-D-glycero-β-D-gluco-heptopyranoside (65 mg, yield 38%) as a white powder by the same method as in (1c).
¹H NMR (500 MHz, CD₃OD): δ 1.22 (3H, d, J=6.3 Hz), 2.42 (3H, s), 3.36-3.37 (2H, m), 3.45-3.47 (2H, m), 3.94 (1H, d, J=15.0 Hz), 4.04 (1H, d, J=15.0 Hz), 4.02-4.06 (1H, m), 4.54 (2H, s), 4.91 (1H, d, J=7.3 Hz), 6.93 (1H, d, J=7.6 Hz), 7.03 (1H, d, J=7.6 Hz), 7.14-7.20 (5H, m);
MS (FAB)m/z: 436 (M)⁺.

### (Example 67) 2-(4-Ethylbenzyl)-5-(2-hydroxyethyl)phenyl 7-deoxy-D-glycero-β-D-gluco-heptopyranoside (Example Compound No. 1-136)

### (67a) 3-Benzyloxy-4-(4-ethylbenzyl)benzyl acetate

5-Acetoxymethyl-2-(4-ethylbenzyl)phenol (WO2002/064606) (13.8 g, 48.5 mmol) was dissolved in dimethylformamide (200 mL), followed by addition of benzyl bromide (6.34 mL, 53.4 mmol) and potassium carbonate (10.1 g, 73.1 mmol), and the mixture was stirred at 50°C for 10 h. The reaction mixture was allowed to stand overnight, followed by addition of potassium carbonate (6.70 g, 48.5 mmol) and benzyl bromide (3.17 mL, 26.7 mmol), and the mixture was stirred at 50°C for 10 h. The reaction mixture was allowed to stand for 2 days, poured into water, and extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 10:1 to 8:1, v/v) to obtain the title compound (18.2 g, yield 100%) as a colorless oil.
¹H NMR (400 MHz, CDCl₃): δ 1.19 (3H, t, J=7.6 Hz), 1.22 (3H, d, J=6.7 Hz), 2.58 (2H, q, J=7.4 Hz), 2.77 (2H, t, J=6.9 Hz), 3.38-3.35 (2H, m), 3.48-3.45 (2H, m), 3.74 (2H, t, J=7.2 Hz), 3.93 (1H, d, J=14.9 Hz), 4.08-3.99 (2H, m), 4.92 (1H, d, J=10.5 Hz), 6.81 (1H, d, J=7.4 Hz), 6.97 (1H, d, J=8.2 Hz), 7.02 (1H, s), 7.06 (2H, d, J=8.3 Hz), 7.14 (2H, d, J=8.2 Hz);
MS (FAB)m/z: 433 (M+H)⁺.

### (67b) 3-Benzyloxy-4-(4-ethylbenzyl)benzyl alcohol

The compound obtained in (67a) (18.2 g, 48.6 mmol) was dissolved in methanol (150 mL) and tetrahydrofuran (50 mL), followed by addition of 2 N aqueous potassium hydroxide (100 mL), and the mixture was stirred at room temperature for 2 h. The reaction mixture was poured into water and extracted with ethyl acetate, and then the organic layer was washed with saturated brine. The organic layer was dried over anhydrous sodium sulfate, and then the solvent was removed under reduced pressure to obtain 3-benzyloxy-4-(4-ethylbenzyl)benzyl alcohol (18.4 g) as a colorless oil.
¹H NMR (400 MHz, CDCl₃): δ 1.22 (3H, t, J=7.6 Hz), 2.61 (2H, q, J=7.6 Hz), 3.98 (2H, s), 4.65 (2H, s), 5.08 (2H, s), 6.88 (1H, d, J=8.3 Hz), 6.97 (1H, s), 7.13-7.07 (5H, m), 7.38-7.31 (5H, m).

### (67c) 3-Benzyloxy-4-(4-ethylbenzyl)benzaldehyde

The compound obtained in (67b) (6.00 g) was dissolved in methylene chloride (100 mL), followed by addition of manganese dioxide (15.7 g, 181 mmol), and the mixture was stirred at room temperature for 4 h and further heated to reflux for 10 h. The reaction mixture was allowed to stand overnight, then insoluble matters were removed by filtration, and the solvent was removed under reduced pressure. The residue was purified by silica gel column chromatography (hexane:ethyl acetate, 5:1, v/v) to obtain the title compound (4.65 g, yield 88% in 2 steps) as a colorless oil.
¹H NMR (400 MHz, CDCl₃): δ 1.23 (3H, t, J=7.8 Hz), 2.62 (2H, q, J=7.8 Hz), 4.05 (2H, s), 5.14 (2H, s), 7.11 (4H, s), 7.41-7.26 (7H, m), 7.44 (1H, d, J=1.1 Hz), 9.93 (1H, s).

### (67d) 2-[3-Benzyloxy-4-(4-ethylbenzyl)phenyl]ethanol

Methoxymethylphosphonium chloride (14.9 g, 43.5 mmol) was dissolved in tetrahydrofuran (100 mL), a solution of 1.0 mol/L lithium hexamethyldisilazane in tetrahydrofuran (43.5 mL, 43.5 mmol) was added dropwise with ice cooling, and the mixture was stirred at room temperature for 1 h. A solution of the compound obtained in (67c) (4.63 g, 14.0 mmol) in tetrahydrofuran (50 mL) was added dropwise to the reaction mixture, and the mixture was stirred at room temperature for 1 h. The reaction mixture was allowed to stand overnight at room temperature, then poured into water, and extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 20:1 to 15:1, v/v) to obtain a mixture of 2-benzyloxy-1-(4-ethylbenzyl)-4-((E)-2-methoxyvinyl)benzene and 2-benzyloxy-1-(4-ethylbenzyl)-4-((Z)-2-methoxyvinyl)benzene (7.70 g) as a colorless oil.

The resulting compound (7.70 g) was dissolved in dioxane (50 mL), followed by addition of 4 N dioxane hydrochloride (50 mL), and the mixture was stirred at room temperature for 1 h. The solvent was removed from the reaction mixture under reduced pressure, and the residue was purified by silica gel column chromatography (hexane:ethyl acetate, 20:1 to 10:1 to 5:1 to 3:1 to 1:1 to ethyl acetate, v/v) to obtain [3-benzyloxy-4-(4-ethylbenzyl)phenyl]acetaldehyde (7.70 g) as a pale yellow oil.

The resulting compound (1.80 g mmol) was dissolved in methanol (20 mL) and tetrahydrofuran (10 mL), followed by addition of sodium borohydride (0.20 g, 5.29 mmol) with ice cooling, and the mixture was stirred at room temperature for 2 h. The reaction mixture was poured into water and extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 5:1 to 3:1 to 2:1, v/v) to obtain the title compound (0.90 g, yield 19%) as a colorless oil.
¹H NMR (400 MHz, CDCl₃): δ 1.22 (3H, t, J=7.5 Hz), 2.61 (2H, q, J=7.5 Hz), 2.82 (2H, t, J=6.4 Hz), 3.83 (2H, q, J=6.4 Hz), 3.96 (2H, s), 5.06 (2H, s), 6.78-6.75 (2H, m), 7.13-7.05 (5H, m), 7.37-7.31 (5H, m).

### (67e) 2-(4-Ethylbenzyl)-5-(2-hydroxyethyl)phenol

The compound obtained in (67d) (0.90 g, 2.60 mmol) was dissolved in methanol (20 mL), followed by addition of 10% palladium on carbon (0.50 g), and the mixture was stirred under a hydrogen atmosphere at room temperature for 3 h. The insoluble matters were removed from the reaction mixture by filtration, and then the solvent was removed under reduced pressure. The residue was purified by silica gel column chromatography (ethyl acetate) to obtain the title compound (0.62 g, yield 93%) as a colorless oil.

### (67f) 5-(2-Acetoxyethyl)-2-(4-ethylbenzyl)phenol

The compound obtained in (67e) (0.60 g, 2.34 mmol), tetrahydrofuran (5 mL), vinyl acetate (5 mL), and bis(dibutylchlorotin)oxide (0.15 g, 0.27 mmol) were used to obtain the title compound (0.70 g, yield 100%) as a colorless oil by the same method as in (66c).
¹H NMR (400 MHz, CDCl₃): δ 1.21 (3H, t, J=7.7 Hz), 2.04 (3H, s), 2.61 (2H, q, J=7.7 Hz), 2.87 (2H, t, J=7.0 Hz), 3.93 (2H, s), 4.26 (2H, t, J=7.0 Hz), 4.69 (1H, s), 6.66 (1H, d, J=1.6 Hz), 6.75 (1H, dd, J=7.8 and 1.6 Hz), 7.05 (1H, d, J=7.8 Hz), 7.13 (4H, s).

### (67g) 2-(4-Ethylbenzyl)-5-(2-hydroxyethyl)phenyl 7-deoxy-D-glycero-β-D-gluco-heptopyranoside

2,3,4,6-Tetra-O-benzoyl-7-deoxy-D-glycero-α,β-D-gluco-heptopyranoside obtained in (7d) (0.37 g, 0.61 mmol), methylene chloride (6 mL), trichloroacetonitrile (0.31 mL, 3.07 mmol), and 1,8-diazabicyclo[5.4.0]-7-undecene (18 µL, 0.12 mmol) were used to obtain an imidate (0.48 g) by the same method as in (1b). The resulting imidate (0.48 g), the compound obtained in (67f) (0.15 g, 0.50 mmol), methylene chloride (6 mL), and a boron trifluoride-diethyl ether complex (64 µL, 0.51 mmol) were used to obtain 5-(2-acetoxyethyl)-2-(4-ethylbenzyl)phenyl 2,3,4,6-tetra-O-benzoyl-7-deoxy-D-glycero-β-D-gluco-heptopyranoside (0.54 g) as a pale yellow oil by the same method as in (1b).

The resulting glycoside compound (0.54 g), methylene chloride (5 mL), methanol (25 mL), and potassium carbonate (0.69 g, 4.99 mmol) were used to obtain 2-(4-ethylbenzyl)-5-(2-hydroxyethyl)phenyl 7-deoxy-D-glycero-β-D-gluco-heptopyranoside (150 mg, yield 69%) as a white powder by the same method as in (1c). ¹H NMR (400 MHz, CD₃OD): δ 1.19 (3H, t, J=7.6 Hz), 1.22 (3H, d, J=6.7 Hz), 2.58 (2H, q, J=7.4 Hz), 2.77 (2H, t, J=6.9 Hz), 3.35-3.38 (2H, m), 3.45-3.48 (2H, m), 3.74 (2H, t, J=7.2 Hz), 3.93 (1H, d, J=14.9 Hz), 3.99-4.08 (2H, m), 4.92 (1H, d, J=10.5 Hz), 6.81 (1H, d, J=7.4 Hz), 6.97 (1H, d, J=8.2 Hz), 7.02 (1H, s), 7.06 (2H, d, J=8.3 Hz), 7.14 (2H, d, J=8.2 Hz);
MS (FAB)m/z: 433 (M+H)⁺.

### (Example 68) 5-Hydroxymethyl-2-[4-(3-hydroxypropyl)benzyl]phenyl 7-deoxy-D-glycero-β-D-gluco-heptopyranoside (Example Compound No. 1-137)

### (68a) Methyl 4-{[4-(3-benzyloxypropyl)phenyl]hydroxymethyl}-3-hydroxybenzoate

1-Bromo-4-(3-benzyloxypropyl)benzene (2.71 g, 8.88 mmol) was dissolved in tetrahydrofuran (50 mL), the mixture was cooled to -78°C, a solution of 2.6 mol/L n-butyllithium hexane (3.4 mL, 8.84 mmol) was added dropwise, and the mixture was stirred at -78°C for 10 min. A solution of 2-hydroxy-4-methoxycarbonyl benzaldehyde (0.40 g, 2.22 mmol) in tetrahydrofuran (10 mL) was added dropwise to the reaction mixture, and the mixture was stirred from -70 to 0°C for 1 h. Aqueous ammonium chloride was added to the reaction mixture, the mixture was extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 5:1 to 3:1 to 2:1, v/v) to obtain the title compound (0.45 g, yield 50%) as a pale yellow oil.

### (68b) 5-Hydroxymethyl-2-[4-(3-hydroxypropyl)benzyl]phenol

The compound obtained in (68a) (0.44 g, 1.08 mmol), methanol (10 mL), concentrated hydrochloric acid (0.09 mL, 1.09 mmol), and 10% palladium on carbon (0.50 g) were used to obtain methyl 3-hydroxy-4-[4-(3-hydroxypropyl)benzyl]benzoate (0.35 g) as a crude product by the same method as in (66b). The resulting compound (0.35 g, 1.17 mmol), lithium aluminium hydride (88 mg, 2.32 mmol), and tetrahydrofuran (8 mL) were used to obtain the title compound (0.13 g, yield 44%) as a colorless oil by the same method as in (66b).
¹H NMR (400 MHz, CDCl₃): δ 1.84-1.91 (2H, m), 2.68 (2H, t, J=7.6 Hz), 3.67 (2H, t, J=6.5 Hz), 3.96 (2H, s), 4.63 (2H, d, J=3.1 Hz), 4.82 (1H, s), 6.83 (1H, d, J=1.6 Hz), 6.88 (1H, dd, J=7.8 and 1.6 Hz), 7.11-7.16 (5H, m);
MS (FAB)m/z: 272 (M)⁺.

### (68c) 5-Acetoxymethyl-2-[4-(3-acetoxypropyl)benzyl]phenol

The compound obtained in (68b) (0.13 g, 0.48 mmol), tetrahydrofuran (2 mL), vinyl acetate (2 mL), and bis(dibutylchlorotin)oxide (26 mg, 0.047 mmol) were used to obtain the title compound (0.17 g, yield 100%) as a colorless oil by the same method as in (66c).
¹H NMR (400 MHz, CDCl₃): δ 1.90-1.97 (2H, m), 2.05 (3H, s), 2.10 (3H, s), 2.65 (2H, t, J=7.6 Hz), 3.95 (2H, s), 4.07 (2H, t, J=6.6 Hz), 4.78 (1H, s), 5.04 (2H, s), 6.80 (1H, d, J=1.1 Hz), 6.88 (1H, dd, J=7.7 and 1.1 Hz), 7.10-7.15 (5H, m);
MS (FAB)m/z: 356 (M)⁺.

### (68d) 5-Hydroxymethyl-2-[4-(3-hydroxypropyl)benzyl]phenyl 7-deoxy-D-glycero-β-D-gluco-heptopyranoside

2,3,4,6-Tetra-O-benzoyl-7-deoxy-D-glycero-α,β-D-gluco-heptopyranoside obtained in (7d) (103 mg, 0.17 mmol), methylene chloride (2 mL), trichloroacetonitrile (85 µL, 0.84 mmol), and 1,8-diazabicyclo[5.4.0]-7-undecene (5 µL, 0.035 mmol) were used to obtain an imidate (140 mg) by the same method as in (1b). The resulting imidate (140 mg), the compound obtained in (68c) (60 mg, 0.17 mmol), methylene chloride (2 mL), and a boron trifluoride-diethyl ether complex (21 µL, 0.17 mmol) were used to obtain 5-(2-acetoxymethyl)-2-[4-(3-acetoxypropyl)benzyl]phenyl 2,3,4,6-tetra-O-benzoyl-7-deoxy-D-glycero-β-D-gluco-heptopyranoside (83 mg) as a colorless oil by the same method as in (1b).

The resulting glycoside compound (80 mg), methylene chloride (1 mL), methanol (5 mL), and potassium carbonate (121 mg, 0.88 mmol) were used to obtain 5-hydroxymethyl-2-[4-(3-hydroxypropyl)benzyl]phenyl 7-deoxy-D-glycero-β-D-gluco-heptopyranoside (20 mg, yield 53%) as a white powder by the same method as in (1c).
¹H NMR (400 MHz, CD₃OD): δ 1.22 (3H, d, J=6.6 Hz), 1.74-1.83 (2H, m), 2.62 (2H, t, J=7.6 Hz), 3.36-3.38 (2H, m), 3.45-3.47 (2H, m), 3.54 (2H, t, J=6.5 Hz), 3.95 (1H, d, J=14.9 Hz), 4.02-4.06 (2H, m), 4.54 (2H, s), 4.90 (1H, d, J=9.0 Hz), 6.92 (1H, d, J=7.5 Hz), 7.02 (1H, d, J=7.5 Hz), 7.14 (2H, d, J=8.3 Hz), 7.13-7.15 (3H, m);
MS (FAB)m/z: 449 (M+H)⁺.

### (Example 69) 2-(3-Fluoro-4-methoxybenzyl)-3,5-dimethylphenyl 7-deoxy-D-glycero-β-D-gluco-heptopyranoside (Example Compound No. 1-138)

### (69a) 2-(3-Fluoro-4-methoxyphenyl)hydroxymethyl-3,5-dimethylphenol

4-Bromo-2-fluoroanisole (4.10 g, 20.0 mmol), a solution of 2.6 mol/L n-butyllithium in hexane (7.69 mL, 20.0 mmol), 2-hydroxy-4,6-dimethylbenzaldehyde (1.00 g, 6.66 mmol), and tetrahydrofuran (100 mL) were used to obtain the title compound (1.44 g, yield 78%) as a yellow powder by the same method as in (68a).
¹H NMR (400 MHz, CDCl₃): δ 2.14 (3H, s), 2.27 (3H, s), 2.82 (1H, d, J=3.2 Hz), 3.87 (3H, s), 6.11 (1H, d, J=3.2 Hz), 6.54 (1H, s), 6.63 (1H, s), 6.90 (1H, t, J=8.6 Hz), 7.04 (1H, dd, J=8.4 and 0.9 Hz), 7.12 (1H, dd, J=12.2 and 1.9 Hz), 8.23 (1H, s);
MS (FAB)m/z: 276 (M)⁺.

### (69b) 2-(3-Fluoro-4-methoxybenzyl)-3,5-dimethylphenol

The compound obtained in (69a) (1.44 g, 5.21 mmol), methanol (20 mL), tetrahydrofuran (5 mL), and 20% palladium hydroxide on carbon (2.00 g) were used to obtain the title compound (1.15 g, yield 85%) by the same method as in (66b).
¹H NMR (500 MHz, CDCl₃): δ 2.21 (3H, s), 2.26 (3H, s), 3.84 (3H, s), 3.93 (2H, s), 4.56 (1H, s), 6.49 (1H, s), 6.62 (1H, s), 6.81-6.89 (3H, m);
MS (FAB)m/z: 260 (M)⁺.

### (69c) 2-(3-Fluoro-4-methoxybenzyl)-3,5-dimethylphenyl 7-deoxy-D-glycero-β-D-gluco-heptopyranoside

2,3,4,6-Tetra-O-benzoyl-7-deoxy-D-glycero-α,β-D-gluco-heptopyranoside (0.28 g, 0.46 mmol) obtained in (7d), methylene chloride (5 mL), trichloroacetonitrile (0.23 mL, 2.28 mmol), and 1,8-diazabicyclo[5.4.0]-7-undecene (14 µL, 0.094 mmol) were used to obtain an imidate (0.36 g) by the same method as in (1b). The resulting imidate (0.36 g), the compound obtained in (69b) (0.10 g, 0.38 mmol), methylene chloride (5 mL), and a boron trifluoride-diethyl ether complex (49 µL, 0.39 mmol) were used to obtain 2-(3-fluoro-4-methoxybenzyl)-3,5-dimethylphenyl 2,3,4,6-tetra-O-benzoyl-7-deoxy-D-glycero-β-D-gluco-heptopyranoside (0.34 g) as a pale brown oil by the same method as in (1b).

The resulting glycoside compound (0.34 g), methylene chloride (10 mL), methanol (15 mL), and potassium carbonate (0.53 g, 3.83 mmol) were used to obtain 2-(3-fluoro-4-methoxybenzyl)-3,5-dimethylphenyl 7-deoxy-D-glycero-β-D-gluco-heptopyranoside (98 mg, yield 58%) as a white powder by the same method as in (1c).
¹H NMR (400 MHz, CD₃OD): δ 1.23 (3H, d, J=6.2 Hz), 2.15 (3H, s), 2.28 (3H, s), 3.30-3.49 (3H, m), 3.81 (3H, s), 3.94 (1H, d, J=15.2 Hz), 4.03-4.07 (1H, m), 4.11 (1H, d, J=15.3 Hz), 4.85-4.89 (1H, m), 6.70 (1H, s), 6.83-6.93 (4H, m);
MS (FAB)m/z: 436 (M)⁺.

### (Example 70) 2-(3-Fluoro-4-methoxybenzyl)-3,5-dimethylphenyl 7-deoxy-D-glycero-β-D-gluco-heptopyranoside (Example Compound No. 1-139)

### (70a) 2-[4-(2-Hydroxyethyl)benzyl]-3,5-dimethylphenol

1-Bromo-4-(2-benzyloxyethyl)benzene (5.82 g, 20.0 mmol), a solution of 2.6 mol/L n-butyllithium in hexane (7.69 mL, 20.0 mmol), 2-hydroxy-4,6-dimethylbenzaldehyde (1.00 g, 6.66 mmol), and tetrahydrofuran (120 mL) were used to obtain 2-[4-(2-benzyloxyethyl)phenyl]hydroxymethyl-3,5-dimethylphenol (6.00 g) as a pale yellow oil by the same method as in (68a). The resulting compound (6.00 g), methanol (100 mL), concentrated hydrochloric acid (0.55 mL, 6.66 mmol), and 10% palladium on carbon (2.00 g) were used to obtain the title compound (2.22 g) as a colorless oil by the same method as in (66b).

### (70b) 2-[4-(2-Acetoxyethyl)benzyl]-3,5-dimethylphenol

The compound obtained in (70a) (2.22 g), tetrahydrofuran (20 mL), vinyl acetate (20 mL), and bis(dibutylchlorotin)oxide (0.45 g, 0.81 mmol) were used to obtain the title compound (1.85 g, yield 93%) as a pale yellow oil by the same method as in (66c).
¹H NMR (400 MHz, CDCl₃): δ 2.03 (3H, s), 2.23 (3H, s), 2.26 (3H, s), 2.88 (2H, t, J=7.2 Hz), 3.99 (2H, s), 4.24 (2H, t, J=7.2 Hz), 4.62 (1H, s), 6.52 (1H, s), 6.64 (1H, s), 7.10 (4H, s);
MS (FAB)m/z: 298 (M)⁺.

### (70c) 2-[4-(2-Hydroxyethyl)benzyl]-3,5-dimethylphenyl 7-deoxy-D-glycero-β-D-gluco-heptopyranoside

2,3,4,6-Tetra-O-benzoyl-7-deoxy-D-glycero-α,β-D-gluco-heptopyranoside obtained in (7d) (0.25 g, 0.41 mmol), methylene chloride (5 mL), trichloroacetonitrile (0.21 mL, 2.08 mmol), and 1,8-diazabicyclo[5.4.0]-7-undecene (12 µL, 0.080 mmol) were used to obtain an imidate (0.32 g) by the same method as in (1b). The resulting imidate (0.32 g), the compound obtained in (70b) (0.11 g, 0.37 mmol), methylene chloride (5 mL), and a boron trifluoride-diethyl ether complex (46 µL, 0.36 mmol) were used to obtain 2-[4-(2-acetoxyethyl)benzyl]-3,5-dimethylphenyl 2,3,4,6-tetra-O-benzoyl-7-deoxy-D-glycero-β-D-gluco-heptopyranoside (0.30 g) as a colorless oil by the same method as in (1b).

The resulting glycoside compound (0.30 g), methylene chloride (3 mL), methanol (15 mL), and potassium carbonate (0.46 g, 3.33 mmol) were used to obtain 2-[4-(2-hydroxyethyl)benzyl]-3,5-dimethylphenyl 7-deoxy-D-glycero-β-D-gluco-heptopyranoside (68 mg, yield 43%) as a white powder by the same method as in (1c). ¹H NMR (400 MHz, CD₃OD): δ 1.22 (3H, d, J=6.6 Hz), 2.14 (3H, s), 2.27 (3H, s), 2.74 (2H, t, J=7.2 Hz), 3.31-3.42 (4H, m), 3.69 (2H, t, J=7.1 Hz), 3.97 (1H, d, J=15.6 Hz), 4.02-4.09 (1H, m), 4.14 (1H, d, J=15.3 Hz), 4.84-4.86 (1H, m), 6.68 (1H, s), 6.88 (1H, s), 7.05 (4H, s);
MS (FAB)m/z: 433 (M+H)⁺.

### (Example 71) 5-(2-Hydroxyethyl)-2-(4-methoxybenzyl)phenyl 7-deoxy-D-glycero-β-D-gluco-heptopyranoside (Example Compound No. 1-140)

### (71a) 3-Benzyloxy-4-(4-methoxybenzyl)benzyl acetate

5-Acetoxymethyl-2-(4-methoxybenzyl)phenol obtained in (8b) (15.0 g, 52.4 mmol), dimethylformamide (200 mL), benzyl bromide (9.34 mL, 78.6 mmol), and potassium carbonate (15.5 g, 112 mmol) were used to obtain the title compound (19.8 g, yield 100%) as a colorless oil by the same method as in (67a).
¹H NMR (400 MHz, CDCl₃): δ 2.08 (3H, s), 3.78 (3H, s), 3.95 (2H, s), 5.07 (2H, s), 5.07 (2H, s), 6.80 (2H, d, J=9.0 Hz), 6.92-6.88 (2H, m), 7.12-7.07 (3H, m), 7.39-7.30 (5H, m);
MS (FAB)m/z: 376 (M)⁺.

### (71b) 3-Benzyloxy-4-(4-methoxybenzyl)benzyl alcohol

The compound obtained in (71a) (19.8 g, 52.6 mmol) (150 mL), tetrahydrofuran (100 mL), and 2 N aqueous potassium hydroxide (100 mL) were used to obtain the title compound (19.0 g) as a colorless oil by the same method as in (67b).
¹H NMR (400 MHz, CDCl₃): δ 1.59 (1H, t, J=6.1 Hz), 3.78 (3H, s), 3.96 (2H, s), 4.65 (2H, d, J=5.9 Hz), 5.08 (2H, s), 6.80 (2H, d, J=8.6 Hz), 6.88 (1H, dd, J=7.4 and 1.6 Hz), 6.98 (1H, s), 7.13-7.08 (3H, m), 7.39-7.31 (5H, m).

### (71 c) 3-Benzyloxy-4-(4-methoxybenzyl)benzaldehyde

The compound obtained in (71b) (10.0 g) was dissolved in chloroform (200 mL), followed by addition of manganese dioxide (15.6 g, 179 mmol), and the mixture was stirred at 60°C for 1 h. The reaction mixture was allowed to stand overnight, then insoluble matters were removed by filtration using Celite, and the solvent was removed under reduced pressure to obtain the title compound (9.70 g) as a colorless oil.
¹H NMR (400 MHz, CDCl₃): δ 3.79 (3H, s), 4.02 (2H, s), 5.14 (2H, s), 6.82 (2H, d, J=8.6 Hz), 7.11 (2H, d, J=8.6 Hz), 7.27-7.25 (1H, m), 7.41-7.34 (6H, m), 7.45 (1H, d, J=1.6 Hz), 9.93 (1H, s);
MS (FAB)m/z: 332 (M)⁺.

### (71d) 2-Benzyloxy-1-(4-methoxybenzyl)-4-((E)-2-methoxyvinyl)benzene and 2-benzyloxy-1-(4-methoxybenzyl)-4-((Z)-2-methoxyvinyl)benzene

Methoxymethylphosphonium chloride (20.0 g, 58.3 mmol), a solution of 1.0 mol/L lithium hexamethyldisilazane in tetrahydrofuran (58.3 mL, 58.3 mmol), the compound obtained in (71c) (9.70 g), and tetrahydrofuran (200 mL) were used to obtain the title compound (9.39 g, yield 94%) as a colorless oil by the same method as in (67d).
¹H NMR (400 MHz, CDCl₃): δ 3.78-3.67 (6H, m), 3.93-3.92 (2H, m), 5.05 (2H, s), 5.18 (0.5H, d, J=7.1 Hz), 5.77 (0.5H, d, J=12.9 Hz), 6.09 (0.5H, d, J=7.1 Hz), 6.81-6.76 (3H, m), 7.05-6.98 (2H, m), 7.12-7.09 (2H, m), 7.37-7.29 (5H, m);
MS (FAB)m/z: 360 (M)⁺.

### (71e) 2-[3-Benzyloxy-4-(4-methoxybenzyl)phenyl]ethanol

The compound obtained in (71d) (9.38 g, 26.0 mmol), dioxane (50 mL), and 4 N dioxane hydrochloride (50 mL) were used to obtain [3-benzyloxy-4-(4-methoxybenzyl)phenyl]acetaldehyde (9.30 g) as a pale yellow oil by the same method as in (67d).

The resulting compound (9.30 g), methanol (80 mL), tetrahydrofuran (40 mL), and sodium borohydride (0.91 g, 24.1 mmol) were used to obtain the title compound (9.53 g) as a colorless oil by the same method as in (67d).
¹H NMR (400 MHz, CDCl₃): δ 1.67 (1H, t, J=6.1 Hz), 3.52 (2H, t, J=6.4 Hz), 4.72 (3H, s), 4.78 (2H, q, J=6.1 Hz), 4.91 (2H, s), 6.32 (2H, s), 8.44 (1H, d, J=7.6 Hz), 8.51-8.48 (3H, m), 8.79 (1H, d, J=7.6 Hz), 8.89 (2H, d, J=8.3 Hz), 9.22-9.12 (5H, m);
MS (FAB)m/z: 348 (M)⁺.

### (71f) 5-(2-Hydroxyethyl)-2-(4-methoxybenzyl)phenol

The compound obtained in (71e) (5.76 g), methanol (50 mL), and 10% palladium on carbon (2.00 g) were used to obtain the title compound (3.03 g, yield 75%) as a white solid by the same method as in (67e).
¹H NMR (400 MHz, CDCl₃): δ 1.40 (1H, t, J=6.2 Hz), 2.80 (2H, t, J=6.2 Hz), 3.79 (3H, s), 3.85 (2H, q, J=6.2 Hz), 3.91 (2H, s), 4.75 (1H, s), 6.68 (1H, d, J=1.7 Hz), 6.76 (1H, dd, J=7.5 and 1.7 Hz), 6.85 (2H, d, J=8.6 Hz), 7.06 (1H, d, J=7.5 Hz), 7.15 (2H, d, J=8.6 Hz);
MS (FAB)m/z: 258 (M)⁺.

### (71 g) 5-(2-Acetoxyethyl)-2-(4-methoxybenzyl)phenol

The compound obtained in (71f) (3.03 g, 11.7 mmol), tetrahydrofuran (30 mL), vinyl acetate (30 mL), and bis(dibutylchlorotin)oxide (0.65 g, 1.18 mmol) were used to obtain the title compound (3.40 g, yield 97%) as a colorless oil by the same method as in (66c).
¹H NMR (400 MHz, CDCl₃): δ 2.04 (3H, s), 2.87 (2H, t, J=7.0 Hz), 3.78 (3H, s), 3.91 (2H, s), 4.26 (2H, t, J=7.0 Hz), 4.67 (1H, s), 6.67 (1H, s), 6.75 (1H, d, J=7.6 Hz), 6.84 (2H, d, J=8.6 Hz), 7.04 (1H, d, J=7.6 Hz), 7.14 (2H, d, J=8.6 Hz);
MS (FAB)m/z: 300 (M)⁺.

### (71h) 5-(2-Hydroxyethyl)-2-(4-methoxybenzyl)phenyl 7-deoxy-D-glycero-β-D-gluco-heptopyranoside

2,3,4,6-Tetra-O-benzoyl-7-deoxy-D-glycero-α,β-D-gluco-heptopyranoside obtained in (7d) (0.24 g, 0.39 mmol), methylene chloride (4 mL), trichloroacetonitrile (0.20 mL, 1.98 mmol), and 1,8-diazabicyclo[5.4.0]-7-undecene (12 µL, 0.080 mmol) were used to obtain an imidate (0.33 g) by the same method as in (1b). The resulting imidate (0.33 g), the compound obtained in (71g) (0.10 g, 0.33 mmol), methylene chloride (4 mL), and a boron trifluoride-diethyl ether complex (42 µL, 0.33 mmol) were used to obtain 5-(2-acetoxyethyl)-2-(4-methoxybenzyl)phenyl 2,3,4,6-tetra-O-benzoyl-7-deoxy-D-glycero-β-D-gluco-heptopyranoside (0.39 g) as a colorless oil by the same method as in (1b).

The resulting glycoside compound (0.39 g), methylene chloride (3 mL), methanol (15 mL), and potassium carbonate (0.46 g, 3.33 mmol) were used to obtain 5-(2-hydroxyethyl)-2-(4-methoxybenzyl)phenyl 7-deoxy-D-glycero-β-D-gluco-heptopyranoside (84 mg, yield 58%) as a white powder by the same method as in (1c).
¹H NMR (400 MHz, CD₃OD): δ 1.23 (3H, d, J=6.7 Hz), 2.77 (2H, t, J=6.8 Hz), 3.35-3.38 (2H, m), 3.45-3.48 (2H, m), 3.74 (3H, s), 3.72-3.75 (2H, m), 3.90 (1H, d, J=14.9 Hz), 3.98 (1H, d, J=14.9 Hz), 4.04-4.06 (1H, m), 4.92 (1H, d, J=10.5 Hz), 6.78-6.82 (3H, m), 6.96 (1H, d, J=7.8 Hz), 7.02 (1H, d, J=1.5 Hz), 7.15 (2H, d, J=8.6 Hz);
MS (FAB)m/z: 434 (M)⁺.

### (Example 72) 5-Hydroxy iminomethyl-2-(4-methoxybenzyl)phenyl 7-deoxy-D-glycero-β-D-gluco-heptopyranoside (Example Compound No. 1-141)

### (72a) 5-Hydroxymethyl-2-(4-methoxybenzyl)phenyl 2,3,4,6-tetra-O-benzoyl-7-deoxy-D-glycero-β-D-gluco-heptopyranoside

The compound obtained in (9a) (1.95 g, crude product) was dissolved in methanol (10 mL) and dioxane (10 mL), followed by addition of 2 N aqueous sodium hydroxide (1.0 mL, 2.00 mmol) with ice cooling, and the mixture was stirred at 2°C for 1.5 h. The reaction mixture was neutralized with 2 N hydrochloric acid and extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 2:1 to 3:2 to 1:1, v/v) to obtain the title compound (0.66 g, yield 40%) as a colorless amorphous compound. ¹H NMR (400 MHz, CDCl₃): δ 1.30 (1H, t, J=6.1 Hz), 1.57 (3H, d, J=6.6 Hz), 3.70 (3H, s), 3.79 (1H, d, J=15.6 Hz), 3.79 (1H, d, J=15.6 Hz), 4.32-4.34 (2H, m), 4.36-4.39 (1H, m), 5.37-5.42 (1H, m), 5.45 (1H, d, J=7.8 Hz), 5.66-5.71 (1H, m), 5.84-5.88 (1H, m), 5.96-6.00 (1H, m), 6.64 (2H, d, J=9.0 Hz), 6.90-6.92 (4H, m), 7.04 (1H, s), 7.26-7.59 (12H, m), 7.84 (2H, dd, J=8.5 and 1.3 Hz), 7.89 (2H, dd, J=8.4 and 1.3 Hz), 7.95 (2H, dd, J=8.4 and 1.4 Hz), 8.03 (2H, dd, J=8.4 and 1.4 Hz);
MS (FAB)m/z: 836 (M)⁺.

### (72b) 5-Formyl-2-(4-methoxybenzyl)phenyl 2,3,4,6-tetra-O-benzoyl-7-deoxy-D-glycero-β-D-gluco-heptopyranoside

The compound obtained in (72a) (0.65 g, 0.78 mmol) was dissolved in chloroform (10 mL), followed by addition of manganese dioxide (0.41 g, 4.72 mmol), and the mixture was stirred at 60°C for 2 h. The reaction mixture was allowed to stand overnight at room temperature, followed by addition of manganese dioxide (0.41 g, 4.72 mmol), and the mixture was stirred at 60°C for 2 h. Insoluble matters were removed by filtration using Celite, and then solvent was removed under reduced pressure to obtain the title compound (0.61 g, yield 94%) as a white amorphous compound.
¹H NMR (400 MHz, CDCl₃): δ 1.56 (3H, d, J=6.7 Hz), 3.71 (3H, s), 3.80 (1H, d, J=15.7 Hz), 3.89 (1H, d, J=15.6 Hz), 4.40-4.43 (1H, m), 5.40-5.42 (1H, m), 5.57 (1H, d, J=7.4 Hz), 5.71-5.73 (1H, m), 5.91-5.93 (1H, m), 6.03-6.05 (1H, m), 6.66 (2H, d, J=8.6 Hz), 6.94 (2H, d, J=8.6 Hz), 7.14 (1H, d, J=7.8 Hz), 7.28-7.58 (14H, m), 7.85 (2H, dd, J=8.4 and 1.3 Hz), 7.91 (2H, dd, J=8.6 and 1.2 Hz), 7.95 (2H, dd, J=8.4 and 1.3 Hz), 8.00 (2H, dd, J=8.2 and 1.2 Hz), 9.61 (1H, s).

### (72c) 5-Hydroxyiminomethyl-2-(4-methoxybenzyl)phenyl 2,3,4,6-tetra-O-benzoyl-7-deoxy-D-glycero-β-D-gluco-heptopyranoside

The compound obtained in (72b) (100 mg, 0.12 mmol) was dissolved in methanol (2 mL) and tetrahydrofuran (1 mL), followed by addition of hydroxyammonium chloride (13 mg, 0.19 mmol) and pyridine (19 µL, 0.23 mmol), and the mixture was stirred at room temperature for 1 h. The reaction mixture was poured into water and extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 1:1, v/v) to obtain the title compound (82 mg, yield 81%) as a colorless amorphous compound. ¹H NMR (400 MHz, CDCl₃): δ 1.58 (3H, d, J=6.6 Hz), 3.71 (3H, s), 3.70 (1H, d, J=15.6 Hz), 3.82 (1H, d, J=15.7 Hz), 4.41-4.44 (1H, m), 5.38-5.40 (1H, m), 5.50 (1H, d, J=7.8 Hz), 5.65-5.70 (1H, m), 5.86-5.91 (1H, m), 5.99-6.03 (1H, m), 6.54 (1H, s), 6.65 (2H, d, J=9.0 Hz), 6.91-6.96 (3H, m), 7.05-7.08 (1H, m), 7.28-7.59 (12H, m), 7.83-7.86 (3H, m), 7.89-7.91 (2H, m), 7.95-7.98 (2H, m), 8.02-8.05 (2H, m).

### (72d) 5-Hydroxyiminomethyl-2-(4-methoxybenzyl)phenyl 7-deoxy-D-glycero-β-D-gluco-heptopyranoside

The compound obtained in (72c) (80 mg, 0.094 mmol), methylene chloride (1 mL), methanol (5 mL), and potassium carbonate (133 mg, 0.96 mmol) were used to obtain 5-hydroxyiminomethyl-2-(4-methoxybenzyl)phenyl 2,3,4,6-tetra-O-benzoyl-7-deoxy-D-glycero-β-D-gluco-heptopyranoside (20 mg, yield 49%) as a white powder by the same method as in (1c).
¹H NMR (400 MHz, CD₃OD): δ 1.23 (3H, d, J=6.6 Hz), 3.35-3.40 (2H, m), 3.47-3.49 (2H, m), 3.75 (3H, s), 3.94 (1H, d, J=15.6 Hz), 4.00-4.07 (2H, m), 4.93 (1H, d, J=7.8 Hz), 6.81 (2H, d, J=8.6 Hz), 7.05 (1H, d, J=8.2 Hz), 7.14-7.18 (3H, m), 7.39 (1H, d, J=1.2 Hz), 8.03 (1H, s);
MS (FAB)m/z: 434 (M+H)⁺.

### (Example 73) 5-(1-Hydroxyethyl)-2-(4-methoxybenzyl)phenyl 7-deoxy-D-glycero-β-D-gluco-heptopyranoside (Example Compound No. 1-142)

### (73a) 5-(1-Hydroxyethyl)-2-(4-methoxybenzyl)phenyl 2,3,4,6-tetra-O-benzoyl-7-deoxy-D-glycero-β-D-gluco-heptopyranoside

The compound obtained in (72b) (100 mg, 0.12 mmol) was dissolved in tetrahydrofuran (2 mL), followed by addition of a solution of 0.96 mol/L methylmagnesium bromide in tetrahydrofuran (0.13 ml, 0.12 mmol) at -70°C, and the mixture was stirred at -70°C for 2 h. Aqueous ammonium chloride was added to the reaction mixture, the mixture was extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 1:1, v/v) to obtain the title compound (72 mg, yield 71%) as a colorless solid.
¹H NMR (400 MHz, CDCl₃): δ 1.23-1.32 (3H, m), 1.58 (3H, d, J=5.5 Hz), 3.66-3.70 (1H, m), 3.71 (3H, s), 3.77-3.82 (1H, m), 4.35-4.41 (1H, m), 4.47-4.58 (1H, m), 5.39-5.47 (2H, m), 5.67-5.73 (1H, m), 5.84-5.88 (1H, m), 5.97-6.02 (1H, m), 6.65 (2H, d, J=8.2 Hz), 6.92-6.94 (4H, m), 7.03-7.09 (1H, m), 7.26-7.57 (12H, m), 7.84 (2H, d, J=8.6 Hz), 7.90-7.95 (4H, m), 7.98-8.02 (2H, m).

### (73b) 5-(1-Hydroxyethyl)-2-(4-methoxybenzyl)phenyl 7-deoxy-D-glycero-β-D-gluco-heptopyranoside

The compound obtained in (73a) (70 mg, 0.082 mmol), methylene chloride (1 mL), methanol (5 mL), and potassium carbonate (117 mg, 0.85 mmol) were used to obtain 5-(1-hydroxyethyl)-2-(4-methoxybenzyl)phenyl 7-deoxy-D-glycero-β-D-gluco-heptopyranoside (28 mg, yield 78%) as a white amorphous compound by the same method as in (1c).
¹H NMR (400 MHz, CD₃OD): δ 1.22-1.24 (3H, m), 1.42 (3H, d, J=6.6 Hz), 3.36-3.39 (2H, m), 3.46-3.48 (2H, m), 3.74 (3H, s), 3.92 (1H, d, J=14.9 Hz), 4.00 (1H, d, J=14.9 Hz), 4.04-4.08 (1H, m), 4.75-4.78 (1H, m), 4.91 (1H, d, J=7.4 Hz), 6.79 (2H, d, J=8.6 Hz), 6.92-6.95 (1H, m), 7.01 (1H, d, J=7.9 Hz), 7.14-7.16 (3H, m);
MS (FAB)m/z: 434 (M)⁺.

### (Example 74) 3-Fluoro-5-hydroxymethyl-2-(4-propoxybenzyl)phenyl 7-deoxy-D-glycero-β-D-gluco-heptopyranoside (Example Compound No. 1-143)

### (74a) 3-Fluoro-5-methyl-2-(4-propoxybenzoyl)-2-cyclohexenone

5-Methyl-cyclohexan-1,3-dione (4.95 g, 39.2 mmol) was dissolved in acetonitrile (50 mL), followed by addition of triethylamine (16.6 mL, 119 mmol), propoxybenzoyl chloride (7.80 g, 39.3 mmol), and trimethylsilyl nitrile (0.64 mL, 4.80 mmol) at room temperature, and the mixture was stirred at 60°C for 4 h. The mixture was allowed to stand for 1 day, then neutralized with 2 N hydrochloric acid, and extracted with ethyl acetate, and then the organic layer was washed with saturated brine. The organic layer was dried over anhydrous sodium sulfate, and then the solvent was removed under reduced pressure to obtain 5-methyl-2-(4-propoxybenzoyl)cyclohexan-1,3-dione (12.3 g) as a brown oil.

The resulting compound (12.2 g) was dissolved in methylene chloride (200 mL), followed by addition of (diethylamino)sulfur trifluoride (15.4 mL, 118 mmol) with ice cooling, and the mixture was stirred at room temperature for 3 h, followed by addition of methanol and water with ice cooling, and extracted with methylene chloride. The organic layer was washed with water and aqueous sodium hydrogencarbonate and dried over anhydrous sodium sulfate, and then the solvent was removed under reduced pressure. The residue was purified by silica gel column chromatography (hexane:ethyl acetate, 3:1 to 2:1, v/v) to obtain the title compound (9.35 g, yield 82%) as a yellow oil.
¹H NMR (400 MHz, CDCl₃): δ 1.04 (3H, t, J=7.5 Hz), 1.21 (3H, d, J=5.5 Hz), 1.79-1.87 (2H, m), 2.27 (1H, dd, J=16.0 and 12.1 Hz), 2.46-2.53 (2H, m), 2.61 (1H, dd, J=16.0 and 3.5 Hz), 2.69-2.79 (1H, m), 3.99 (2H, t, J=6.6 Hz), 6.92 (2H, d, J=8.8 Hz), 7.81 (2H, d, J=8.8 Hz);
MS (FAB)m/z: 291 (M+H)⁺.

### (74b) 3-Fluoro-5-methyl-2-(4-propoxybenzoyl)phenyl acetate

The compound obtained in (74a) (9.30 g, 32.0 mmol) was dissolved in acetonitrile (100 mL), followed by addition of triethylamine (13.4 mL, 96.1 mmol), then trimethylsilyl iodide (11.4 mL, 80.1 mmol) was added with ice cooling, and the mixture was stirred at 50°C for 30 min. Toluene and a neutral phosphate pH standard solution (type 2) were added to the reaction mixture, and the mixture was extracted with toluene. The organic layer was dried over anhydrous sodium sulfate, and then the solvent was removed under reduced pressure. The residue was dissolved in toluene (100 mL), followed by addition of N-iodosuccinimide (7.21 g, 32.0 mmol) with ice cooling, and the mixture was stirred at room temperature for 30 min. Triethylamine (5.80 mL, 41.6 mmol) was added to the reaction mixture, the mixture was stirred at room temperature for 15 min, tetrahydrofuran (100 mL) and 2 N aqueous sodium hydroxide (50 mL) were further added, and the mixture was stirred at 40°C for 30 min. Water was added to the reaction mixture, the mixture was extracted with ethyl acetate, and then the organic layer was washed with saturated brine. The organic layer was dried over anhydrous sodium sulfate, and then the solvent was removed under reduced pressure to obtain 3-fluoro-5-methyl-2-(4-propoxybenzoyl)phenol (10.2 g) as a brown oil.

The resulting compound (10.2 g) was dissolved in methylene chloride (100 mL), followed by addition of pyridine (5.18 mL, 64.0 mmol), acetic anhydride (4.54 mL, 48.0 mmol), and dimethylaminopyridine (0.78 g, 6.38 mmol), and the mixture was stirred at room temperature for 2 h. The solvent was removed under reduced pressure, ethyl acetate was added to the residue, the mixture was washed with 1 N hydrochloric acid, aqueous sodium hydrogencarbonate, and saturated brine and dried over anhydrous sodium sulfate, and then the solvent was removed under reduced pressure. The residue was purified by silica gel column chromatography (hexane:ethyl acetate, 10:1 to 8:1, v/v) to obtain the title compound (6.70 g, yield 63%) as a yellow oil.
¹H NMR (400 MHz, CDCl₃): δ 1.05 (3H, t, J=7.4 Hz), 1.79-1.88 (2H, m), 2.03 (3H, s), 2.42 (3H, s), 3.99 (2H, t, J=6.6 Hz), 6.85-6.89 (2H, m), 6.92 (2H, d, J=9.0 Hz), 7.80 (2H, d, J=9.0 Hz);
MS (FAB)m/z: 331 (M+H)⁺.

### (74c) 3-Fluoro-5-hydroxymethyl-2-(4-propoxybenzoyl)phenol

The compound obtained in (74b) (6.70 g, 20.3 mmol) was dissolved in carbon tetrachloride (120 mL), followed by addition of N-bromosuccinimide (3.61 g, 20.3 mmol) and azobisisobutyronitrile (0.33 g, 2.01 mmol), and the mixture was heated to reflux for 3 h. The reaction mixture was cooled to room temperature, insoluble matters were removed by filtration, and the solvent was removed under reduced pressure to obtain 5-bromomethyl-3-fluoro-2-(4-propoxybenzoyl)phenol (9.00 g) as a brown oil.

The resulting compound (9.00 g) was dissolved in dioxane (120 mL) and water (40 mL), followed by addition of carbonate calcium (10.1 g, 101 mmol), and the mixture was heated to reflux for 1 day. The reaction mixture was cooled to room temperature, insoluble matters were removed by filtration, followed by addition of 2 N aqueous sodium hydroxide (20 mL), and the mixture was stirred at room temperature for 1 h. The solvent was removed under reduced pressure, water was added to the residue, the mixture was neutralized with 2 N hydrochloric acid and extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 2:1 to 1:1 to 1:2, v/v) to obtain the title compound (1.35 g, yield 22%) as a pale brown solid.
¹H NMR (400 MHz, CDCl₃): δ 1.06 (3H, t, J=7.4 Hz), 1.82-1.88 (2H, m), 4.01 (2H, t, J=6.6 Hz), 4.73 (2H, d, J=5.8 Hz), 6.67-6.70 (1H, m), 6.87 (1H, s), 6.95 (2H, d, J=9.0 Hz), 7.72-7.75 (2H, m), 10.9 (1H, s);
MS (FAB)m/z: 305 (M+H)⁺.

### (74d) 5-Acetoxymethyl-3-fluoro-2-(4-propoxybenzoyl)phenol

The compound obtained in (74c) (1.34 g, 4.40 mmol), tetrahydrofuran (15 mL), vinyl acetate (15 mL), and bis(dibutylchlorotin)oxide (0.73 g, 1.32 mmol) were used to obtain the title compound (1.50 g, yield 98%) as a pale yellow solid by the same method as in (66c).
¹H NMR (400 MHz, CDCl₃): δ 1.06 (3H, t, J=7.5 Hz), 1.80-1.89 (2H, m), 2.17 (3H, s), 4.01 (2H, t, J=6.7 Hz), 5.10 (2H, s), 6.60-6.63 (1H, m), 6.85 (1H, s), 6.95 (2H, d, J=9.0 Hz), 7.72-7.75 (2H, m), 10.8 (1H, s);
MS (FAB)m/z: 347 (M+H)⁺.

### (74e) 5-Acetoxymethyl-3-fluoro-2-[hydroxy-(4-propoxyphenyl)methyl]phenol

The compound obtained in (74d) (1.47 g, 4.24 mmol) was dissolved in methanol (30 mL), followed by addition of sodium borohydride (0.32 g, 8.46 mmol) with ice cooling, and the mixture was stirred at room temperature for 1 h. Aqueous ammonium chloride was added to the reaction mixture, and the mixture was extracted with ethyl acetate. The organic layer was washed with aqueous ammonium chloride and saturated brine and dried over anhydrous sodium sulfate, and then the solvent was removed under reduced pressure to obtain the title compound (1.36 g, yield 92%) as a pale yellow oil.
¹H NMR (400 MHz, CDCl₃): δ 1.02 (3H, t, J=7.4 Hz), 1.75-1.83 (2H, m), 2.11 (3H, s), 2.80 (1H, d, J=2.7 Hz), 3.90 (2H, t, J=6.6 Hz), 5.01 (2H, s), 6.29 (1H, d, J=1.6 Hz), 6.54-6.57 (1H, m), 6.70 (1H, s), 6.87 (2H, d, J=9.0 Hz), 7.35 (2H, d, J=9.0 Hz), 8.90 (1H, s).

### (74f) 5-Acetoxymethyl-3-fluoro-2-(4-propoxybenzyl)phenol

The compound obtained in (74e) (1.35 g, 3.88 mmol) was dissolved in acetonitrile (30 mL), followed by addition of triethylsilane (1.95 mL, 12.2 mmol) and a boron trifluoride-diethyl ether complex (0.77 mL, 6.08 mmol) with ice cooling, and the mixture was stirred at room temperature for 2 h. Aqueous sodium hydrogencarbonate was added to the reaction mixture with ice cooling, the mixture was extracted with ethyl acetate, and then the organic layer was washed with saturated brine. The organic layer was dried over anhydrous sodium sulfate, and then the solvent was removed under reduced pressure. The residue was purified by silica gel column chromatography (hexane:ethyl acetate, 5:1 to 3:1, v/v) to obtain the title compound (1.12 g, yield 87%) as a white powder.
¹H NMR (400 MHz, CDCl₃): δ 1.01 (3H, t, J=7.4 Hz), 1.73-1.82 (2H, m), 2.11 (3H, s), 3.88 (2H, t, J=6.6 Hz), 3.94 (2H, s), 5.00 (3H, s), 6.60 (1H, s), 6.80-6.83 (1H, m), 6.82 (2H, d, J=8.6 Hz), 7.18 (2H, d, J=8.6 Hz);
MS (FAB)m/z: 332 (M)⁺.

### (74g) 3-Fluoro-5-hydroxymethyl-2-(4-propoxybenzyl)phenyl 7-deoxy-D-glycero-β-D-gluco-heptopyranoside

2,3,4,6-Tetra-O-benzoyl-7-deoxy-D-glycero-α,β-D-gluco-heptopyranoside obtained in (7d) (0.22 g, 0.36 mmol), methylene chloride (4 mL), trichloroacetonitrile (0.18 mL, 1.78 mmol), and 1,8-diazabicyclo[5.4.0]-7-undecene (16 µL, 0.11 mmol) were used to obtain an imidate (0.34 g) by the same method as in (1b). The resulting imidate (0.34 g), the compound obtained in (74g) (0.10 g, 0.30 mmol), methylene chloride (4 mL), and a boron trifluoride-diethyl ether complex (38 µL, 0.30 mmol) were used to obtain 5-acetoxymethyl-3-fluoro-2-(4-propoxybenzyl)phenyl 2,3,4,6-tetra-O-benzoyl-7-deoxy-D-glycero-β-D-gluco-heptopyranoside (0.37 g) as a colorless oil by the same method as in (1b).

The resulting glycoside compound (0.37 g), methylene chloride (3 mL), methanol (15 mL), and potassium carbonate (0.42 g, 3.04 mmol) were used to obtain 3-fluoro-5-hydroxymethyl-2-(4-propoxybenzyl)phenyl 7-deoxy-D-glycero-β-D-gluco-heptopyranoside (100 mg, yield 71%) as a white powder by the same method as in (1c).
¹H NMR (400 MHz, CD₃OD): δ 1.01 (3H, t, J=7.5 Hz), 1.21 (3H, d, J= 6.6Hz), 1.70-1.79 (2H, m), 3.36-3.38 (2H, m), 3.43-3.51 (2H, m), 3.86 (2H, t, J=6.5 Hz), 3.92 (1H, d, J=14.8 Hz), 3.94-4.00 (2H, m), 4.55 (2H, s), 4.93 (1H, d, J=7.4 Hz), 6.75 (2H, d, J=8.6 Hz), 6.77-6.79 (1H, m), 6.98 (1H, s), 7.18 (2H, d, J=8.6 Hz);
MS (FAB)m/z: 466 (M)⁺.

### (Example 75) 3-Fluoro-5-hydroxymethyl-2-(4-isopropoxybenzyl)phenyl 7-deoxy-D-glycero-β-D-gluco-heptopyranoside (Example Compound No. 1-144)

### (75a) 3-Fluoro-5-methyl-2-(4-isopropoxy benzoyl)-2-cyclohexenone

5-Methyl-cyclohexane-1,3-dione (4.95 g, 39.2 mmol), acetonitrile (50 mL), triethylamine (16.6 mL, 119 mmol), isopropoxybenzoyl chloride (7.90 g, 39.8 mmol), and trimethylsilyl nitrile (0.53 mL, 3.97 mmol) were used to obtain 5-methyl-2-(4-isopropoxybenzoyl)cyclohexan-1,3-dione (13.0 g) as a brown oil by the same method as in (74a).

The resulting compound (13.0 g), methylene chloride (200 mL), and (diethylamino)sulfur trifluoride (15.4 mL, 118 mmol) were used to obtain the title compound (9.74 g, yield 86%) as a yellow oil by the same method as in (74a).
¹H NMR (400 MHz, CDCl₃): δ 1.21 (3H, d, J=5.5 Hz), 1.36 (6H, d, J=5.8 Hz), 2.27 (1H, dd, J=16.2 and 12.3 Hz), 2.45-2.53 (2H, m), 2.61 (1H, dd, J=16.0 and 3.5 Hz), 2.71-2.77 (1H, m), 4.62-4.68 (1H, m), 6.90 (2H, d, J=9.0 Hz), 7.80 (2H, d, J=9.0 Hz);
MS (FAB)m/z: 290 (M)⁺.

### (75b) 3-Fluoro-5-methyl-2-(4-isopropoxybenzoyl)phenyl acetate

The compound obtained in (75a) (9.70 g, 33.4 mmol), acetonitrile (100 mL), triethylamine (14.1 mL, 101 mmol), trimethylsilyl iodide (12.0 mL, 84.3 mmol), toluene (100 mL), N-iodosuccinimide (7.59 g, 33.7 mmol), triethylamine (6.12 mL, 43.9 mmol), tetrahydrofuran (100 mL), and 2 N aqueous sodium hydroxide (50 mL) were used to obtain 3-fluoro-5-methyl-2-(4-isopropoxybenzoyl)phenol (10.2 g) as a brown oil by the same method as in (74b).

The resulting compound (10.3 g), methylene chloride (100 mL), pyridine (5.40 mL, 66.8 mmol), acetic anhydride (4.74 mL, 50.1 mmol), and dimethylaminopyridine (0.82 g, 6.71 mmol) were used to obtain the title compound (6.54 g, yield 59%) as a yellow oil by the same method as in (74b). ¹H NMR (400 MHz, CDCl₃): δ 1.36 (6H, d, J=6.3 Hz), 2.03 (3H, s), 2.42 (3H, s), 4.62-4.68 (1H, m), 6.85-6.91 (4H, m), 7.79 (2H, d, J=8.6 Hz);
MS (FAB)m/z: 330 (M)⁺.

### (75c) 3-Fluoro-5-hydroxymethyl-2-(4-isopropoxy benzoyl)phenol

The compound obtained in (75b) (6.52 g, 19.7 mmol) was dissolved in carbon tetrachloride (120 mL), N-bromosuccinimide (3.86 g, 21.7 mmol), and azobisisobutyronitrile (0.32 g, 1.95 mmol) were used to obtain 5-bromomethyl-3-fluoro-2-(4-isopropoxybenzoyl)phenol (9.20 g) as a yellow oil by the same method as in (74c).

The resulting compound (9.20 g), dioxane (120 mL), water (40 mL), and carbonate calcium (9.88 g, 98.7 mmol) were used to obtain the title compound (2.10 g, yield 35%) as a yellow oil by the same method as in (74c).
¹H NMR (400 MHz, CDCl₃): δ 1.38 (6H, d, J=5.8 Hz), 1.82 (1H, t, J=5.5 Hz), 4.64-4.70 (1H, m), 4.72 (2H, d, J=5.5 Hz), 6.67-6.70 (1H, m), 6.86 (1H, s), 6.92 (2H, d, J=9.0 Hz), 7.73 (2H, dd, J=9.0 and 3.5 Hz), 10.9 (1H, s);
MS (FAB)m/z: 305 (M+H)⁺.

### (75d) 5-Acetoxymethyl-3-fluoro-2-(4-isopropoxy benzoyl)phenol

The compound obtained in (75c) (2.08 g, 6.83 mmol), tetrahydrofuran (25 mL), vinyl acetate (25 mL), and bis(dibutylchlorotin)oxide (1.20 g, 2.17 mmol) were used to obtain the title compound (2.34 g, yield 99%) as a yellow oil by the same method as in (66c).
¹H NMR (400 MHz, CDCl₃): δ 1.39 (6H, d, J=6.2 Hz), 2.17 (3H, s), 4.64-4.70 (1H, m), 5.10 (2H, s), 6.61-6.64 (1H, m), 6.85 (1H, s), 6.92 (2H, d, J=8.9 Hz), 7.73 (2H, dd, J=8.9 and 3.3 Hz), 10.8 (1H, s);
MS (FAB)m/z: 347 (M+H)⁺.

### (75e) 5-Acetoxymethyl-3-fluoro-2-[hydroxy-(4-isopropoxy phenyl)methyl]phenol

The compound obtained in (75d) (2.30 g, 6.64 mmol), methanol (50 mL), and sodium borohydride (0.50 g, 13.2 mmol) were used to obtain the title compound (1.99 g, yield 86%) as a yellow oil by the same method as in (74e).
¹H NMR (400 MHz, CDCl₃): δ 1.32 (6H, d, J=6.2 Hz), 2.11 (3H, s), 2.85 (1H, d, J=2.7 Hz), 4.48-4.57 (1H, m), 5.01 (2H, s), 6.28 (1H, s), 6.56 (1H, d, J=10.6 Hz), 6.70 (1H, s), 6.85 (2H, d, J=8.6 Hz), 7.34 (2H, d, J=8.6 Hz), 8.91 (1H, s);
MS (FAB)m/z: 348 (M)⁺.

### (75f) 5-Acetoxymethyl-3-fluoro-2-(4-isopropoxybenzyl)phenol

The compound obtained in (75e) (1.95 g, 5.60 mmol), acetonitrile (40 mL), triethylsilane (2.75 mL, 17.2 mmol), and a boron trifluoride-diethyl ether complex (1.09 mL, 8.60 mmol) were used to obtain the title compound (1.71 g, yield 92%) as a white powder by the same method as in (74f).
¹H NMR (400 MHz, CDCl₃): δ 1.31 (6H, d, J=6.2 Hz), 2.11 (3H, s), 3.94 (2H, s), 4.46-4.52 (1H, m), 5.00 (2H, s), 5.06 (1H, s), 6.60 (1H, s), 6.70 (1H, dd, J=9.8 and 1.5 Hz), 6.80 (2H, d, J=8.4 Hz), 7.17 (2H, d, J=8.4 Hz);
MS (FAB)m/z: 332 (M)⁺.

### (75g) 3-Fluoro-5-hydroxymethyl-2-(4-isopropoxybenzyl)phenyl 7-deoxy-D-glycero-β-D-gluco-heptopyranoside

2,3,4,6-Tetra-O-benzoyl-7-deoxy-D-glycero-α,β-D-gluco-heptopyranoside obtained in (7d) (0.22 g, 0.36 mmol), methylene chloride (4 mL), trichloroacetonitrile (0.18 mL, 1.78 mmol), and 1,8-diazabicyclo[5.4.0]-7-undecene (16 µL, 0.11 mmol) were used to obtain an imidate (0.34 g) by the same method as in (1b). The resulting imidate (0.34 g), the compound obtained in (75g) (0.10 g, 0.30 mmol), methylene chloride (4 mL), and a boron trifluoride-diethyl ether complex (38 µL, 0.30 mmol) were used to obtain 5-acetoxymethyl-3-fluoro-2-(4-isopropoxybenzyl)phenyl 2,3,4,6-tetra-O-benzoyl-7-deoxy-D-glycero-β-D-gluco-heptopyranoside (0.40 g) as a colorless oil by the same method as in (1b).

The resulting glycoside compound (0.40 g), methylene chloride (3 mL), methanol (15 mL), and potassium carbonate (0.42 g, 3.04 mmol) were used to obtain 3-fluoro-5-hydroxymethyl-2-(4-isopropoxybenzyl)phenyl 7-deoxy-D-glycero-β-D-gluco-heptopyranoside (122 mg, yield 87%) as a white powder by the same method as in (1c).
¹H NMR (500 MHz, CD₃OD): δ 1.21 (3H, d, J=6.3 Hz), 1.25 (6H, d, J=6.3 Hz), 3.36-3.37 (2H, m), 3.43-3.50 (2H, m), 3.92 (1H, d, J=14.1 Hz), 4.00 (1H, d, J=14.2 Hz), 4.04-4.06 (1H, m), 4.47-4.52 (1H, m), 4.54 (2H, s), 4.93 (1H, d, J=7.4 Hz), 6.73 (2H, d, J=8.6 Hz), 6.77 (1H, d, J=9.8 Hz), 6.97 (1H, s), 7.16 (2H, d, J=8.6 Hz);
MS (FAB)m/z: 466 (M)⁺.

### (Example 76) 2-(4-Propoxybenzyl)-5-hydroxymethylphenyl 7-deoxy-D-glycero-β-D-gluco-heptopyranoside (Example Compound No. 1-145)

### (76a) 5-Acetoxymethyl-2-(4-propoxybenzyl)phenyl 2,3,4,6-tetra-O-benzoyl-7-deoxy-D-glycero-β-D-gluco-heptopyranoside

The compound obtained in (7d) (256 mg, 0.42 mmol), trichloroacetonitrile (126 µL, 1.26 mmol), 1,8-diazabicyclo[5.4.0]-7-undecene (6 µL, 0.04 mmol), and methylene chloride (5 mL) were used to prepare an imidate by the same method as in (1b). Subsequently, 5-acetoxymethyl-2-(4-propoxybenzyl)phenol (EP2001/912380) (110 mg, 0.35 mmol), a boron trifluoride-diethyl ether complex (7 µL, 0.06 mmol), and methylene chloride (10 mL) were used to obtain a crude product of the title compound (317 mg) by the same method as in (1b).

### (76b) 2-(4-Propoxybenzyl)-5-hydroxymethylphenyl 7-deoxy-D-glycero-β-D-gluco-heptopyranoside

The compound obtained in (76a) (317 mg, 0.35 mmol) was dissolved in tetrahydrofuran (1 mL) and methanol (4 mL), followed by addition of potassium carbonate (484 mg, 3.50 mmol), and the mixture was stirred at room temperature for 14 h. The solvent was removed under reduced pressure, then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (40 mg, 25.5%) as a colorless solid.
¹H NMR (400 MHz, CD₃OD): δ 1.02 (3H, t, J=7.4 Hz), 1.22 (3H, d, J=6.7 Hz), 1.71-1.80 (2H, m), 3.35-3.38 (2H, m), 3.45-3.48 (2H, m), 3.88 (2H, t, J=7.8 Hz), 3.92 (1H, d, J=15.2 Hz), 4.00 (1H, d, J=14.9 Hz), 4.05-4.07 (1H, m), 4.54 (2H, s), 4.91 (1H, d, J=5.1 Hz), 6.78 (2H, d, J=8.6 Hz), 6.93 (1H, d, J=7.8 Hz), 7.02 (1H, d, J=7.8 Hz), 7.12 (2H, d, J=8.6 Hz), 7.13 (1H, s);
MS (FAB)m/z: 471 (M+Na)⁺.

### (Example 77) 3,5-Dimethyl-2-(4-methoxybenzyl)phenyl 7-deoxy-D-glycero-β-D-gluco-heptopyranoside (Example Compound No. 1-146)

### (77a) 3,5-Dimethyl-2-(4-methoxybenzyl)phenyl 2,3,4,6-tetra-O-benzoyl-7-deoxy-D-glycero-β-D-gluco-heptopyranoside

The compound obtained in (7d) (302 mg, 0.49 mmol), trichloroacetonitrile (150 µL, 1.50 mmol), 1,8-diazabicyclo[5.4.0]-7-undecene (7 µL, 0.05 mmol), and methylene chloride (5 mL) were used to prepare an imidate by the same method as in (1b). Subsequently, 3,5-dimethyl-2-(4-methoxybenzyl)phenol (WO2002/44192) (100 mg, 0.41 mmol), a boron trifluoride-diethyl ether complex (8 µL, 0.06 mmol), and methylene chloride (10 mL) were used to obtain a crude product of the title compound (340 mg) by the same method as in (1b).

### (77b) 3,5-Dimethyl-2-(4-methoxybenzyl)phenyl 7-deoxy-D-glycero-β-D-gluco-heptopyranoside

The compound obtained in (77a) (340 mg, 0.41 mmol) was dissolved in tetrahydrofuran (1 mL) and methanol (4 mL), followed by addition of 2 M aqueous sodium hydroxide (2 mL, 4.00 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (74 mg, 43.2%) as a colorless solid.
¹H NMR (400 MHz, CD₃OD): δ 1.23 (3H, d, J=6.7 Hz), 2.14 (3H, s), 2.17 (3H, s), 3.33-3.36(2H, m), 3.41-3.43 (2H, m), 3.31 (3H, s), 3.94 (1H, d, J=15.3 Hz), 4.04 (1H, dd, J=6.6, 3.9 Hz), 4.10 (1H, d, J=15.3 Hz), 4.86 (1H, d, J=9.8 Hz), 6.68 (1H, s), 6.75 (1H, d, J=9.0 Hz), 6.89 (1H, s), 7.05 (2H, d, J=8.2 Hz);
MS (FAB)m/z: 417 (M-H)⁺, 441 (M+Na)⁺.

### (Example 78) 2-(3-Fluoro-4-methoxybenzyl)-5-hydroxymethylphenyl 7-deoxy-D-glycero-β-D-gluco-heptopyranoside (Example Compound No. 1-147)

### (78a) 5-Hydroxymethyl-2-(3-fluoro-4-methoxybenzyl)phenol

1-Bromo-3-fluoro-4-methoxybenzene (1.59 mL, 12.3 mmol), metal magnesium (330 mg, 13.6 mmol), a catalytic amount of iodine, and tetrahydrofuran (6 mL) were used to prepare Grignard reagent according to a usual method. The resulting Grignard reagent was added to a solution of ethyl 4-formyl-3-hydroxybenzoate (600 mg, 3.09 mmol) in tetrahydrofuran (6 mL), and the mixture was stirred at -50°C for 20 min. Saturated aqueous ammonium chloride (30 mL) was added to the reaction mixture, the mixture was extracted with ethyl acetate (30 mL) and then washed with saturated brine (30 mL). The organic layer was dried over anhydrous sodium sulfate, and then the solvent was removed under reduced pressure. The resulting crude product was used in the subsequent reaction as it was.

The crude product was dissolved in methanol-tetrahydrofuran (8 mL/2 mL), followed by addition of 20% palladium hydroxide on carbon (320 mg), and the mixture was stirred under a hydrogen atmosphere at room temperature for 6 h. Methylene chloride (2 mL) was added to the reaction mixture, the mixture was stirred for 10 min, then 20% palladium hydroxide on carbon was removed by filtration, and the solvent was removed under reduced pressure. The resulting crude product was used in the subsequent reaction as it was.

The crude product was dissolved in tetrahydrofuran (6 mL), followed by addition of lithium aluminium hydride (270 mg, 7.11 mmol) with ice cooling, and the mixture was stirred at room temperature for 30 min. Subsequently, 2 mol/L hydrochloric acid (5 mL) was added with ice cooling, and the mixture was extracted with ethyl acetate (40 mL) and then washed with saturated aqueous sodium hydrogencarbonate (50 mL) and saturated brine (50 mL). The organic layer was dried over anhydrous sodium sulfate, and then the solvent was removed under reduced pressure. The residue was purified by using a short column to obtain a crude product of the title compound (670 mg, yield 83%) as a colorless solid.

### (78b) 5-Acetoxymethyl-2-(3-fluoro-4-methoxybenzyl)phenol

The crude product obtained in (78a) (670 mg, 2.55 mmol), tetrahydrofuran (7 m), vinyl acetate (7 mL), bis(dibutylchlorotin)oxide (70 mg, 0.13 mmol) were used to obtain the title compound (570 mg, yield 78%) as a colorless solid by the same method as in (8b).
¹H NMR (400MHz, CDCl₃): δ 2.10 (3H, s), 3.86 (3H, s), 3.91 (2H, s), 4.80 (1H, s), 5.04 (2H, s), 6.80 (1H, d, J=1.5 Hz), 6.89 - 6.96 (4H, m), 7.09 (1H, d, J=7.8 Hz);
MS (FAB)m/z: 304(M)⁺.

### (78c) 5-Acetoxymethyl-2-(3-fluoro-4-methoxybenzyl)phenyl 2,3,4,6-tetra-O-benzoyl-7-deoxy-D-glycero-β-D-gluco-heptopyranoside

The compound obtained in (7d) (260 mg, 0.43 mmol), trichloroacetonitrile (130 µL, 1.30 mmol), 1,8-diazabicyclo[5.4.0]-7-undecene (6 µL, 0.04 mmol), and methylene chloride (5 mL) were used to prepare an imidate by the same method as in (1b). Subsequently, the compound obtained in (78b) (104 mg, 0.34 mmol), a boron trifluoride-diethyl ether complex (43 µL, 0.34 mmol), and methylene chloride (10 mL) were used to obtain a crude product of the title compound (243 mg) by the same method as in (1b).

### (78d) 2-(3-Fluoro-4-methoxybenzyl)-5-hydroxymethylphenyl 7-deoxy-D-glycero-β-D-gluco-heptopyranoside

The compound obtained in (78c) (243 mg, 0.27 mmol) was dissolved in tetrahydrofuran (1 mL) and methanol (4 mL), followed by addition of 2 M aqueous sodium hydroxide (1.4 mL, 2.8 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (40 mg, 33.9%) as a colorless solid.
¹H NMR (400 MHz, CD₃OD): δ 1.22 (3H, d, J=6.2 Hz), 3.35-3.38 (2H, m), 3.46-3.48 (2H, m), 3.82 (3H, s), 3.91 (1H, d, J=14.8 Hz), 4.02 (1H, d, J=14.9 Hz), 4.06-4.07 (1H, m), 4.55 (2H, s), 4.92 (1H, d, J=7.8 Hz), 6.92-7.00 (4H, m), 7.06 (1H, d, J=7.8 Hz), 7.16 (1H, s);
MS (FAB)m/z: 437 (M-H)⁺, 438 (M)⁺, 461 (M+Na)⁺.

### (Example 79) 2-(4-Fluorobenzyl)-5-hydroxymethylphenyl 7-deoxy-D-glycero-β-D-gluco-heptopyranoside (Example Compound No. 1-148)

### (79a) 5-Hydroxymethyl-2-(4-fluorobenzyl)phenol

1-Bromo-4-fluorobenzene (4.14 mL, 37.6 mmol), metal magnesium (1.37 g, 56.4 mmol), a catalytic amount of iodine, and tetrahydrofuran (20 mL) were used to prepare Grignard reagent according to a usual method. A solution of the resulting Grignard reagent in tetrahydrofuran (13 mL) was added to a solution of ethyl 4-formyl-3-hydroxybenzoate (1.0 g, 5.15 mmol) in tetrahydrofuran (12 mL), and the mixture was stirred at -50°C for 20 min. Saturated aqueous ammonium chloride (50 mL) was added to the reaction mixture, and the mixture was extracted with ethyl acetate (40 mL) and then washed with saturated brine (50 mL). The organic layer was dried over anhydrous sodium sulfate, and then the solvent was removed under reduced pressure. The resulting crude product was used in the subsequent reaction as it was.

The crude product was dissolved in methanol (15 mL), followed by addition of 20% palladium hydroxide on carbon (430 mg), and the mixture was stirred under a hydrogen atmosphere at room temperature for 6 h. Methylene chloride (2 mL) was added to the reaction mixture, the mixture was stirred for 10 min, then 20% palladium hydroxide on carbon was removed by filtration, and the solvent was removed under reduced pressure. The resulting crude product was used in the subsequent reaction as it was.

The crude product was dissolved in tetrahydrofuran (20 mL), followed by addition of lithium aluminium hydride (500 mg, 13.2 mmol) with ice cooling, and the mixture was stirred at room temperature for 30 min. Subsequently, 2 mol/L hydrochloric acid (30 mL) was added with ice cooling, and the mixture was extracted with ethyl acetate (40 mL) and then washed with saturated aqueous sodium hydrogencarbonate (50 mL) and saturated brine (50 mL). The organic layer was dried over anhydrous sodium sulfate, and then the solvent was removed under reduced pressure. The residue was purified by silica gel flash column chromatography (methylene chloride:methanol, 30:1, v/v) to obtain the title compound (900 mg, yield 83%) as an oil.
¹H NMR (400MHz,DMSO-d6): δ 4.77 (2H, s), 5.45 (2H, d, J=5.8 Hz), 6.30 (1H, t, J=5.8 Hz), 8.31 (1H, d, J=7.8 Hz), 8.49 (1H, s), 8.71 (1H, d, J=7.8 Hz), 8.82 (2H, t, J=8.9 Hz), 9.01 (2H, dd, J=8.9, 5.9 Hz), 11.7 (1H, s);
MS (FAB)m/z: 232 (M)⁺.

### (79b) 5-Acetoxymethyl-2-(4-fluorobenzyl)phenol

The compound obtained in (79a) (900 mg, 3.88 mmol), tetrahydrofuran (9 mL), vinyl acetate (9 mL), and bis(dibutylchlorotin)oxide (110 mg, 0.20 mmol) were used to obtain the title compound (1.02 g, yield 96%) as a colorless solid by the same method as in (8b).
¹H NMR (400MHz, CDCl₃): δ 2.10 (3H, s), 3.95 (2H, s), 4.76 (1H, s), 5.04 (2H, s), 6.80 (1H, d, J=1.6 Hz), 6.88 (1H, dd, J=7.8, 1.6 Hz), 6.97 (2H, t, J=8.9 Hz), 7.08 (1H, d, J=7.8 Hz), 7.18 (2H, dd, J=8.9, 5.4 Hz);
MS (EI⁺)m/z: 274 (M)⁺.

### (79c) 5-Acetoxymethyl-2-(4-fluorobenzyl)phenyl 2,3,4,6-tetra-O-benzoyl-7-deoxy-D-glycero-β-D-gluco-heptopyranoside

The compound obtained in (7d) (288 mg, 0.47 mmol), trichloroacetonitrile (140 µL, 1.40 mmol), 1,8-diazabicyclo[5.4.0]-7-undecene (7 µL, 0.05 mmol), and methylene chloride (5 mL) were used to prepare an imidate by the same method as in (1b). Subsequently, the compound obtained in (79b) (107 mg, 0.32 mmol), a boron trifluoride-diethyl ether complex (49 µL, 0.39 mmol), and methylene chloride (10 mL) were used to obtain a crude product of the title compound (309 mg) by the same method as in (1b).

### (79d) 2-(4-Fluorobenzyl)-5-hydroxymethylphenyl 7-deoxy-D-glycero-β-D-gluco-heptopyranoside

The compound obtained in (79c) (309 mg, 0.36 mmol) was dissolved in tetrahydrofuran (1 mL) and methanol (4 mL), followed by addition of potassium carbonate (493 mg, 3.75 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (68 mg, 46.3%) as a colorless solid.
¹H NMR (400 MHz, CD₃OD): δ 1.22 (3H, d, J=6.6 Hz), 3.30-3.31 (1H, m), 3.36-3.38 (1H, m), 3.46-3.48 (2H, m), 3.96 (1H, d, J=14.8 Hz), 4.07 (1H, d, J=14.9 Hz), 4.05-4.08 (1H, m), 4.54 (2H, s), 4.93 (1H, d, J=7.4 Hz), 6.91-6.95 (3H, m), 7.05 (1H, d, J=7.8 Hz), 7.15 (1H, s), 7.22-7.26 (2H, m);
MS (FAB)m/z: 408 (M)⁺, 431 (M+Na)⁺.

### (Example 80) 2-(4-Hydroxymethylbenzyl)-5-hydroxymethylphenyl 7-deoxy-D-glycero-β-D-gluco-heptopyranoside (Example Compound No. 1-149)

### (80a) Methyl 4-(4-ethoxycarbonylbenzoyl)-3-hydroxybenzoate

4-Ethoxycarbonylphenylboronic acid (510 mg, 2.63 mmol), tetrakis triphenylphosphine palladium (256 mg, 0.22 mmol), and tripotassium phosphate (560 mg, 2.64 mmol) were added to a solution of the compound obtained in (58a) (800 mg, 2.21 mmol) in toluene (10 mL), and the mixture was stirred at 90°C for 4 h. The mixture was cooled to room temperature, followed by addition of water (50 mL), extracted with ethyl acetate (50 mL), and washed with saturated brine (50 mL), and then the organic layer was dried over anhydrous sodium sulfate, and then the solvent was removed under reduced pressure. The residue was purified by using a short column, and a crude product was used in the subsequent reaction as it was.

The crude product was dissolved in methanol-tetrahydrofuran (8 mL/2 mL), followed by addition of hydrochloric acid-methanol (R10) (2.5 mL), and the mixture was stirred at room temperature for 2 days. The solvent was removed under reduced pressure, and the residue was purified by silica gel flash column chromatography (hexane:ethyl acetate, 3:1, v/v) to obtain the title compound (480 mg, yield 70%) as a white solid.
¹H NMR (400MHz, CDCl₃): δ 1.38 (3H, t, J=7.2 Hz), 3.90 (2H, s), 4.08 (3H, s), 4.37 (2H, q, J=7.2 Hz), 4.99 (1H, s), 7.17 (1H, d, J=7.6 Hz), 7.29 (2H, d, J=8.3 Hz), 7.47 (1H, s), 7.58 (1H, d, J=7.6 Hz), 7.98 (2H, d, J=8.3 Hz);
MS (EI⁺)m/z: 314 (M)⁺.

### (80b) 5-Hydroxymethyl-2-(4-hydroxymethylbenzyl)phenol

The compound obtained in (80a) (660 mg, 2.10 mmol) was dissolved in tetrahydrofuran (12 mL), followed by addition of lithium aluminium hydride (320 mg, 8.43 mmol) with ice cooling, and the mixture was stirred at room temperature for 30 min. Subsequently, water (0.32 mL), 15% aqueous sodium hydroxide (0.32 mL), and water (0.96 mL) were added with ice cooling, and the mixture was stirred at room temperature for 10 h. After Celite filtration, the solvent was removed under reduced pressure. The residue was purified by silica gel flash column chromatography (methylene chloride:methanol , 30:1 to 10:1, v/v) to obtain the title compound (264 mg, yield 51%) as a white solid.
¹H NMR (400MHz, CD₃OD): δ 4.87 (2H, s), 5.60 (2H, s), 5.67 (2H, s), 8.39 (1H, d, J=7.8 Hz), 8.49 (1H, s), 8.68 (1H, d, J=7.8 Hz), 8.97 (2H, d, J=7.8 Hz), 9.02 (2H, d, J=7.8 Hz);
MS (EI⁺)m/z: 244 (M)⁺.

### (80c) 5-Acetoxymethyl-2-(4-acetoxymethylbenzyl)phenol

The compound obtained in (80b) (260 mg, 1.06 mmol), tetrahydrofuran (3 m), vinyl acetate (3 mL), and bis(dibutylchlorotin)oxide (59 mg, 0.11 mmol) were used to obtain the title compound (294 mg, yield 84%) as a colorless solid by the same method as in (8b).
¹H NMR (400MHz, CDCl₃): δ 2.09 (3H, s), 2.10 (3H, s), 3.98 (2H, s), 5.03 (2H, s), 5.07 (2H, s), 6.80 (1H, d, J=1.5 Hz), 6.87 (1H, dd, J=7.7, 1.5Hz), 7.10 (1H, d, J=7.7 Hz), 7.22 (2H, d, J=8.3 Hz), 7.28 (2H, d, J=8.3 Hz);
MS (FAB)m/z: 329 (M+H)⁺.

### (80d) 5-Acetoxymethyl-2-(4-acetoxymethylbenzyl)phenyl 2,3,4,6-tetra-O-benzoyl-7-deoxy-L-glycero-β-D-gluco-heptopyranoside

The compound obtained in (7d) (235 mg, 0.38 mmol), trichloroacetonitrile (116 µL, 1.16 mmol), 1,8-diazabicyclo[5.4.0]-7-undecene (5 µL, 0.03 mmol), and methylene chloride (5 mL) were used to prepare an imidate by the same method as in (1b). Subsequently, the compound obtained in (80c) (104 mg, 0.32 mmol), a boron trifluoride-diethyl ether complex (40 µL, 0.32 mmol), and methylene chloride (10 mL) were used to obtain a crude product of the title compound (226 mg) by the same method as in (1b).

### (80e) 2-(4-Hydroxymethylbenzyl)-5-hydroxymethylphenyl 7-deoxy-D-glycero-β-D-gluco-heptopyranoside

The compound obtained in (80d) (226 mg, 0.25 mmol) was dissolved in tetrahydrofuran (1 mL) and methanol (4 mL), followed by addition of potassium carbonate (339 mg, 2.45 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (32 mg, 30.5%) as a colorless solid.
¹H NMR (400 MHz, CD₃OD): δ 1.22 (3H, d, J=6.7 Hz), 3.35-3.38 (2H, m), 3.45-3.47 (2H, m), 3.98 (1H, d, J=14.9 Hz), 4.05-4.07 (1H, m), 4.08 (1H, d, J=14.9 Hz), 4.54 (4H, s), 4.93 (1H, d, J=4.7 Hz), 6.93 (1H, d, J=7.4 Hz), 7.03 (1H, d, J=7.5 Hz), 7.15 (1H, s), 7.23 (4H, s);
MS (FAB)m/z: 421 (M+H)⁺, 443 (M+Na)⁺.

### (Example 81) 2-(4-Isopropoxybenzyl)-5-hydroxymethylphenyl 7-deoxy-D-glycero-β-D-gluco-heptopyranoside (Example Compound No. 1-150)

### (81a) Methyl 4-(4-propoxyphenyl) hydroxymethyl-3-hydroxybenzoate

4-Bromophenyl-isopropyl ether (J.Chem.Soc., 1926, 2363.) (4.30 g, 20.00 mmol), metal magnesium (486 mg, 20.00 mmol), a catalytic amount of iodine, and tetrahydrofuran (20 mL) were used to prepare Grignard reagent according to a usual method. The resulting Grignard reagent was added to a solution of methyl 4-formyl-3-hydroxybenzoate (900 mg, 5.00 mmol) in tetrahydrofuran (20 mL), and the mixture was stirred at -50°C for 20 min. Saturated aqueous ammonium chloride (50 mL) was added to the reaction mixture, and the mixture was extracted with ethyl acetate (50 mL) and then washed with saturated brine (20 mL). The organic layer was dried over anhydrous sodium sulfate, and then the solvent was removed under reduced pressure. The residue was purified by silica gel flash column chromatography (hexane:ethyl acetate, 6:1 to 4:1, v/v) to obtain the title compound (1.10 g, 69.6%) as a pale yellow amorphous compound.
¹H NMR (400 MHz, CDCl₃): δ 1.33 (6H, d, J=5.9 Hz), 3.89 (3H, s), 4.50-4.59 (1H, m), 6.02 (1H, d, J=2.7 Hz), 6.88 (2H, d, J=8.6 Hz), 6.94 (1H, d, J=7.8 Hz), 7.28 (2H, d, J=9.0 Hz), 7.47 (1H, d, J=8.0 Hz), 7.56(1H, s), 8.29 (1H, s).

### (81b) 5-Acetoxymethyl-2-(4-isopropoxybenzyl)phenol

The compound obtained in (81a) (1.10 g, 3.48 mmol) was dissolved in methanol (10 mL), followed by addition of concentrated hydrochloric acid (0.32 mL) and 10% palladium on carbon (220 mg), and the mixture was stirred under a hydrogen atmosphere at room temperature for 30 h. The mixture was neutralized with triethylamine (700 µL, 5.02 mmol) and then filtered using Celite, and the solvent was removed under reduced pressure. The residue was diluted with ethyl acetate (20 mL) and washed with saturated brine (10 mL). The organic layer was dried over anhydrous sodium sulfate, and then the solvent was removed under reduced pressure.

The resulting crude product was dissolved in tetrahydrofuran (10 mL), followed by addition of lithium aluminium hydride (290 mg, 7.64 mmol) with ice cooling, and the mixture was stirred at room temperature for 2 h. 2 mol/L hydrochloric acid (30 mL) was added with ice cooling, and the mixture was extracted with ethyl acetate (40 mL) and then washed with saturated aqueous sodium hydrogencarbonate (50 mL) and saturated brine (20 mL). The organic layer was dried over anhydrous sodium sulfate, and then the solvent was removed under reduced pressure.

The resulting crude product was dissolved in tetrahydrofuran (10 mL), followed by addition of vinyl acetate (10 mL) and bis(dibutylchlorotin)oxide (70 mg, 0.13 mmol), and the mixture was stirred at 30°C for 16 h. The solvent was removed under reduced pressure, and then the residue was purified by silica gel flash column chromatography (hexane:ethyl acetate, 8:1 to 6:1, v/v) to obtain the title compound (724 mg, 91.4%) as a colorless solid.
¹H NMR (400MHz, CDCl₃): δ 1.31 (6H, d, J=5.9 Hz), 2.09 (3H, s), 3.91 (2H, s), 4.45-4.54 (1H, m), 5.03 (2H, s), 6.82 (1H, d, J=8.6 Hz), 6.86 (2H, d, J=7.8 Hz), 7.09 (1H, d, J=7.8 Hz), 7.12 (2H, d, J=8.2 Hz).

### (81c) 5-Acetoxymethyl-2-(4-isopropoxybenzyl)phenyl 2,3,4,6-tetra-O-benzoyl-7-deoxy-D-glycero-β-D-gluco-heptopyranoside

The compound obtained in (7d) (240 mg, 0.39 mmol), trichloroacetonitrile (120 µL, 1.20 mmol), 1,8-diazabicyclo[5.4.0]-7-undecene (6 µL, 0.04 mmol), and methylene chloride (5 mL) were used to prepare an imidate by the same method as in (1b). Subsequently, the compound obtained in (81b) (100 mg, 0.32 mmol), a boron trifluoride-diethyl ether complex (40 µL, 0.32 mmol), and methylene chloride (10 mL) were used to obtain a crude product of the title compound (229 mg) by the same method as in (1b).

### (81d) 2-(4-Isopropoxybenzyl)-5-hydroxymethylphenyl 7-deoxy-D-glycero-β-D-gluco-heptopyranoside

The compound obtained in (81c) (229 mg, 0.25 mmol) was dissolved in tetrahydrofuran (1 mL) and methanol (4 mL), followed by addition of potassium carbonate (350 mg, 2.53 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (64 mg, 57.1%) as a colorless solid.
¹H NMR (400 MHz, CD₃OD): δ 1.22 (3H, d, J=6.7 Hz), 1.27 (6H, d, J=5.9 Hz), 3.37-3.38 (2H, m), 3.43-3.51 (2H, m), 3.92 (1H, d, J=14.9 Hz), 4.00 (1H, d, J=14.9 Hz), 4.03-4.07 (1H, m), 4.48-4.55 (1H, m), 4.54 (2H, s), 4.91 (1H, d, J=7.4 Hz), 6.77 (2H, d, J=8.6 Hz), 6.93 (1H, d, J=7.8 Hz), 7.03 (1H, d, J=7.9 Hz), 7.11 (1H, s), 7.13 (2H, s);
MS (FAB)m/z: 448 (M)⁺, 471 (M+Na)⁺.

### (Example 82) 2-(2-Fluoro-4-methoxybenzyl)-5-hydroxymethylphenyl 7-deoxy-D-glycero-β-D-gluco-heptopyranoside (Example Compound No. 1-151)

### (82a) Methyl 4-(2-fluoro-4-methoxyphenyl)hydroxymethyl-3-hydroxybenzoate

1-Bromo-2-fluoro-4-methoxybenzene (1.27 g, 6.19 mmol) was dissolved in tetrahydrofuran (10 mL), then n-butyllithium in 2.6 M n-hexane solution (2.4 mL, 6.24 mmol) were added dropwise with cooling at -78°C, and the mixture was stirred at the same temperature for 10 min. A solution of methyl 4-formyl-3-hydroxybenzoate (298 mg, 1.65 mmol) in tetrahydrofuran (10 mL) was added to the mixture, and the mixture was stirred at -78°C for 2 h. Saturated aqueous ammonium chloride (20 mL) was added to the reaction mixture, and the mixture was extracted with ethyl acetate (20 mL) and then washed with saturated brine (10 mL). The organic layer was dried over anhydrous sodium sulfate, and then the solvent was removed under reduced pressure. The residue was purified by silica gel flash column chromatography (hexane:ethyl acetate, 6:1 1 to 4:1, v/v) to obtain the title compound (276 mg, 54.7%) as a pale yellow amorphous compound.
¹H NMR (400MHz, CDCl₃): δ 3.80 (3H, s), 3.90 (3H, s), 6.33 (1H, d, J=3.4 Hz), 6.65-6.68 (2H, m), 6.94 (1H, d, J=7.8 Hz), 7.16-7.20 (1H, m), 7.47 (1H, d, J=8.1 Hz), 7.58 (1H, s), 8.23 (1H, s);
MS (FAB⁺)m/z: 307(M+H)⁺.

### (82b) 5-Acetoxymethyl-2-(2-fluoro-4-methoxybenzyl)phenol

The compound obtained in (82a) (276 mg, 0.90 mmol) was dissolved in methanol (10 mL), followed by addition of concentrated hydrochloric acid (0.15 mL) and 10% palladium on carbon (100 mg), and the mixture was stirred under a hydrogen atmosphere at room temperature for 30 h. After Celite filtration, the solvent was removed under reduced pressure.

The resulting crude product (120 mg, 0.41 mmol) was dissolved in tetrahydrofuran (3 mL), followed by addition of lithium aluminium hydride (47 mg, 1.24 mmol) with ice cooling, and the mixture was stirred at room temperature for 2 h. 2 mol/L hydrochloric acid (5 mL) was added with ice cooling, and the mixture was extracted with ethyl acetate (10 mL) and then washed with saturated aqueous sodium hydrogencarbonate (10 mL) and saturated brine (5 mL). The organic layer was dried over anhydrous sodium sulfate, and then the solvent was removed under reduced pressure.

The resulting crude product was dissolved in tetrahydrofuran (2.5 mL), followed by addition of vinyl acetate (2.5 mL) and bis(dibutylchlorotin)oxide (12 mg, 0.02 mmol), and the mixture was stirred at 30°C for 16 h. The solvent was removed under reduced pressure, and then the residue was purified by silica gel flash column chromatography (hexane:ethyl acetate, 6:1 to 4:1, v/v) to obtain the title compound (108 mg, 86.4%) as a colorless solid.
¹H NMR (400MHz, CDCl₃): δ 2.09 (3H, s), 3.77 (3H, s), 3.91 (2H, s), 5.02 (2H, s), 6.60-6.65 (2H, m), 6.80 (1H, s), 6.86 (1H, d, J=7.4 Hz), 7.03-7.09(2H, m);
MS (FAB⁺)m/z: 304 (M)⁺.

### (82c) 5-Acetoxymethyl-2-(2-fluoro-4-methoxybenzyl)phenyl 2,3,4,6-tetra-O-benzoyl-7-deoxy-L-glycero-β-D-gluco-heptopyranoside

The compound obtained in (7d) (260 mg, 0.43 mmol), trichloroacetonitrile (130 µL, 1.30 mmol), 1,8-diazabicyclo[5.4.0]-7-undecene (6 µL, 0.04 mmol), and methylene chloride (5 mL) were used to prepare an imidate by the same method as in (1b). Subsequently, the compound obtained in (82b) (108 mg, 0.35 mmol), a boron trifluoride-diethyl ether complex (45 µL, 0.36 mmol), and methylene chloride (10 mL) were used to obtain a crude product of the title compound (282 mg) by the same method as in (1b).

### (82d) 2-(2-Fluoro-4-methoxybenzyl)-5-hydroxymethylphenyl 7-deoxy-D-glycero-β-D-gluco-heptopyranoside

The compound obtained in (82c) (282 mg, 0.31 mmol) was dissolved in tetrahydrofuran (1 mL) and methanol (4 mL), followed by addition of potassium carbonate (435 mg, 3.15 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (55 mg, 40.4%) as a colorless solid.
¹H NMR (400 MHz, CD₃OD): δ 1.22 (3H, d, J=6.6 Hz), 3.37-3.38 (2H, m), 2.44-3.52 (2H, m), 3.76 (3H, s), 3.95 (1H, d, J=15.2 Hz), 4.01 (1H, d, J=15.7 Hz), 4.06 (1H, dd, J=6.3, 2.8 Hz), 4.54 (2H, s), 4.93 (1H, d, J=7.4 Hz), 6.64 (1H, d, J=10.5 Hz), 6.65 (1H, d, J=4.7 Hz), 6.92 (1H, d, J=8.6 Hz), 6.97 (1H, d, J=7.8 Hz), 7.12 (1H, d, J=9.0 Hz), 7.15 (1H, s);
MS (FAB)m/z: 461 (M+Na)⁺.

### (Example 83) 5-Hydroxymethyl-3-methoxy-2-(4-methoxybenzyl)phenyl 7-deoxy-D-glycero-β-D-gluco-heptopyranoside (Example Compound No. 1-152)

### (83a) 3-Methoxy-2-(4-methoxybenzoyl)-5-methyl-cyclohex-2-enone

Methyl iodide (0.54 mL, 8.67 mmol) and potassium carbonate (1.20 g, 8.68 mmol) were added to a solution of the compound obtained in Example (43c) (1.10 g, 4.33 mmol) in acetone (20 mL), and the mixture was stirred at room temperature for 24 h. After Celite filtration, the solvent was removed under reduced pressure. The residue was purified by silica gel flash column chromatography (hexane:ethyl acetate, 3:1 to 1:3, v/v) to obtain the title compound (650 mg, yield 56%).
¹H NMR (400 MHz, CDCl₃): δ 1.19 (3H, d, J=6.4 Hz), 2.19 (1H, dd, J=16.1, 11.7 Hz), 2.32 (1H, dd, J=17.1, 10.2 Hz), 2.36-2.42 (1H, m), 2.53 (1H, dd, J=16.1, 4.0 Hz), 2.75 (1H, dd, J=17.1, 3.9 Hz), 3.74 (3H, s), 3.86 (3H, s), 6.91 (2H, d, J=8.8 Hz), 7.84 (2H, d, J=8.8 Hz);
MS (EI)m/z: 274 (M)⁺.

### (83b) (2-Hydroxy-6-methoxy-4-methylphenyl)-(4-methoxyphenyl)-methanone

The compound obtained in (83a) (750 mg, 2.71 mmol), triethylamine (1.14 mL, 8.17 mmol), acetonitrile (10 mL), and trimethylsilane iodide (0.96 mL, 6.76 mmol) were used to obtain a silyl enol ether compound as an oily crude product by the same method as in (43c). The resulting oily crude product was treated with toluene (8 mL), N-iodosuccinimide (610 mg, 2.71 mmol), triethylamine (0.49 mL, 3.51 mmol), tetrahydrofuran (32 mL), and 2 N aqueous sodium hydroxide (4 mL) in this order by the same method as in (43c), and the reaction mixture was passed through a short column to obtain a crude product of the title compound (510 mg).
¹H NMR (400 MHz, CDCl₃): δ 2.35 (3H, s), 3.56 (3H, s), 3.88 (3H, s), 6.25 (1H, s), 6.50 (1H, s), 6.90 (2H, d, J=8.8 Hz), 7.65 (2H, d, J=8.8 Hz), 10.7 (1H, s);
MS (EI)m/z: 272(M)⁺.

### (83c) 3-Methoxy-2-(4-methoxybenzoyl)-5-methylphenyl acetate

The compound obtained in (83b) (0.51 g, 1.87 mmol) was dissolved in methylene chloride (10 mL), followed by addition of acetic anhydride (270 µL, 2.87 mmol), pyridine (300 µL, 3.73 mmol), and 4-dimethylaminopyridine (69 mg, 0.65 mmol), and the mixture was stirred at room temperature for 20 h. The solvent was removed under reduced pressure, and then the residue was diluted with ethyl acetate (10 mL) and washed with 2 M aqueous hydrochloric acid (20 mL), saturated aqueous sodium hydrogencarbonate (20 mL), and saturated brine (5 mL). The organic layer was dried over anhydrous sodium sulfate, and then the solvent was removed under reduced pressure. The residue was purified by silica gel flash column chromatography (hexane:ethyl acetate, 6:1 to 4:1, v/v) to obtain the title compound (548 mg, 93.4%) as a colorless solid.
¹H NMR (400 MHz, CDCl₃): δ 1.97 (3H, s), 2.41 (3H, s), 3.72 (3H, s), 3.86 (3H, s), 6.66 (2H, d, J=12.5 Hz), 6.90 (2H, d, J=9.0 Hz), 7.80 (2H, d, J=9.0 Hz);
MS (FAB)m/z: 315 (M+H)⁺.

### (83d) 5-Hydroxymethyl-3-methoxy-2-(4-methoxybenzoyl)phenol

The compound obtained in (83c) (548 mg, 1.74 mmol) was dissolved in carbon tetrachloride (10 mL), followed by addition of N-bromosuccinimide (326 mg, 1.83 mmol) and 2,2-azobis(isobutyronitrile) (57 mg, 0.35 mmol), and the mixture was stirred for 6 h with heating to reflux. The mixture was cooled to room temperature and then filtered using Celite, and the solvent was removed under reduced pressure.

The resulting crude product was dissolved in 1,4-dioxane (15 mL) and distilled water (5 mL), followed by addition of carbonate calcium (886 mg, 8.85 mmol), and the mixture was stirred for 30 h with heating to reflux. The mixture was cooled to room temperature, and the filtered residue was washed with methanol and 2 M aqueous sodium hydroxide. The solvent was removed under reduced pressure, and then the residue was diluted with ethyl acetate (10 mL) and washed with saturated aqueous ammonium chloride (20 mL) and saturated brine (5 mL). The organic layer was dried over anhydrous sodium sulfate, and then the solvent was removed under reduced pressure. The residue was purified by silica gel flash column chromatography (hexane:ethyl acetate, 2:1 to 1:1, v/v) to obtain the title compound (209 mg, 41.4%) as a colorless solid.
¹H NMR (400 MHz, CDCl₃): δ 3.60 (3H, s), 3.88 (3H, s), 4.70 (2H, d, J=5.0 Hz), 6.50 (1H, s), 6.64 (1H, s), 6.90 (2H, d, J=8.6 Hz), 7.66 (2H, d, J=9.0 Hz);
MS (EI)m/z: 287 (M-H)⁺.

### (83e) 5-Acetoxymethyl-3-methoxy-2-(4-methoxybenzoyl)phenol

The compound obtained in (83d) (209 mg, 0.72 mmol) was dissolved in tetrahydrofuran (5 mL), followed by addition of vinyl acetate (5 mL) and bis(dibutylchlorotin)oxide (40 mg, 0.07 mmol), and the mixture was stirred at room temperature for 30 h. The solvent was removed under reduced pressure, and then the residue was purified by silica gel flash column chromatography (hexane:ethyl acetate, 4:1 to 2:1, v/v) to obtain the title compound (260 mg, quantitative) as a pale yellow syrup.
¹H NMR (400 MHz, CDCl₃): δ 2.17 (3H, s), 3.59 (3H, s), 3.88 (3H, s), 5.08 (2H, s), 6.40 (1H, s), 6.66 (1H, s), 6.90 (2H, d, J=8.3 Hz), 7.67 (2H, d, J=9.0 Hz), 10.36 (1H, s);
MS (FAB)m/z: 331(M+H)⁺.

### (83f) 5-Acetoxymethyl-3-methoxy-2-(4-methoxyphenyl)hydroxymethylphenol

The compound obtained in (83e) (260 mg, 0.72 mmol) was dissolved in methanol (8 mL), followed by addition of sodium borohydride (64 mg, 1.69 mmol) with ice cooling, and the mixture was stirred at room temperature for 30 min. Saturated aqueous ammonium chloride was added with ice cooling, and then the solvent was removed under reduced pressure. The residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (hexane:ethyl acetate, 4:1 to 2:1, v/v) to obtain the title compound (224 mg, 93.7%) as a colorless solid.
¹H NMR (400 MHz, CDCl₃): δ 2.11 (3H, s), 2.85 (1H, brs), 3.75 (3H, s), 3.78 (3H, s), 5.02 (2H, s), 6.37 (1H, d, J=2.8 Hz), 6.39 (1H, s), 6.57 (1H, s), 6.86 (2H, d, J=7.9 Hz), 7.36 (2H, d, J=8.6 Hz), 8.85 (1H, s);
MS (FAB)m/z: 331 (M-H)⁺, 332 (M)⁺.

### (83g) 5-Acetoxymethyl-3-methoxy-2-(4-methoxybenzyl)phenol

The compound obtained in (83f) (224 mg, 0.67 mmol) was dissolved in acetonitrile (5 mL), followed by addition of triethylsilane (320 µL, 2.01 mmol) and a boron trifluoride-diethyl ether complex (130 µL, 1.04 mmol), and the mixture was stirred at room temperature for 30 min. The mixture was neutralized with saturated aqueous sodium hydrogencarbonate with ice cooling, followed by addition of ethyl acetate (10 mL), and washed with saturated brine (5 mL). The residue was dried over anhydrous sodium sulfate, and then the solvent was removed under reduced pressure. The residue was purified by silica gel flash column chromatography (hexane:ethyl acetate, 5:1 to 3:1, v/v) to obtain the title compound (157 mg, 74.8%) as a colorless syrup.
¹H NMR (400 MHz, CDCl₃): δ 2.11 (3H, s), 3.76 (3H, s), 3.83 (3H, s), 3.96 (2H, s), 5.02 (2H, s), 6.49 (2H, d, J=11.4 Hz), 6.80 (1H, d, J=8.6 Hz), 7.18 (2H, d, J=8.6 Hz); MS (FAB)m/z: 316 (M)⁺.

### (83h) 5-Acetoxymethyl-3-methoxy-2-(4-methoxybenzyl)phenyl 2,3,4,6-tetra-O-benzoyl-7-deoxy-D-glycero-β-D-gluco-heptopyranoside

The compound obtained in (7d) (233 mg, 0.38 mmol), trichloroacetonitrile (110 µL, 1.10 mmol), 1,8-diazabicyclo[5.4.0]-7-undecene (6 µL, 0.04 mmol), and methylene chloride (5 mL) were used to prepare an imidate by the same method as in (1b). Subsequently, the compound obtained in (83g) (100 mg, 0.32 mmol), a boron trifluoride-diethyl ether complex (40 µL, 0.32 mmol), and methylene chloride (10 mL) were used to obtain a crude product of the title compound (183 mg) by the same method as in (1b).

### (83i) 5-Hydroxymethyl-3-methoxy-2-(4-methoxybenzyl)phenyl 7-deoxy-D-glycero-β-D-gluco-heptopyranoside

The compound obtained in (83h) (168 mg, 0.20 mmol) was dissolved in tetrahydrofuran (1 mL) and methanol (4 mL), followed by addition of potassium carbonate (280 mg, 2.03 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (60 mg, 66.4%) as a colorless solid.
¹H NMR (400 MHz, CD₃OD): δ 1.21 (3H, d, J=6.3 Hz), 3.36-3.36 (2H, m), 3.41-3.49 (2H, m), 3.71 (3H, s), 3.80 (3H, s), 3.92 (1H, d, J=14.8 Hz), 4.01 (1H, d, J=15.2 Hz), 4.02-4.05 (1H, m), 4.55 (2H, s), 4.90 (1H, d, J=6.6 Hz), 6.70 (2H, s), 6.72 (1H, s), 6.80 (1H, s), 7.17 (2H, d, J=7.0 Hz);
MS (FAB)m/z: 450 (M)⁺, 473 (M+Na)⁺.

### (Example 84) 3-Fluoro-5-hydroxymethyl-2-(4-methoxybenzyl)phenyl 7-deoxy-D-glycero-β-D-gluco-heptopyranoside (Example Compound No. 1-153)

### (84a) 3-Fluoro-2-(4-methoxybenzoyl)-5-methyl-cyclohex-2-enone

The compound obtained in Example (43c) (14.27 g, 54.82 mmol) was dissolved in dichloromethane (150 mL), followed by addition of diethylaminosulfur trifluoride (22.0 mL, 166.51 mmol) with ice cooling, and the mixtuer was stirred at room temperature for 3 h. Distilled water (10 mL) was added dropwise with ice cooling, and the mixture was diluted with dichloromethane and washed with distilled water (400 mL) and saturated brine (50 mL). The organic layer was dried over anhydrous sodium sulfate, and then the solvent was removed under reduced pressure. The residue was purified by silica gel flash column chromatography (hexane:ethyl acetate, 4:1 to 1:1, v/v) to obtain the title compound (13.24 g, yield 84.7%).
¹H NMR (400 MHz,CDCl₃): δ 1.22 (3H, d, J= 5.5 Hz), 2.27 (1H, dd, J = 16.0, 12.1 Hz), 2.46-2.53 (2H, m), 2.61 (1H, dd, J = 16.0, 3.5 Hz), 2.75 (1H, dd, J = 13.7, 8.2 Hz), 3.88 (3H, s), 6.94 (2H, d, J=9.0 Hz), 7.82 (2H, d, J=8.6 Hz);
MS (EI)m/z: 262 (M)⁺.

### (84b) (2-Hydroxy-6-fluoro-4-methylphenyl)-(4-methoxyphenyl) methanone

The compound obtained in (84a) (13.24 g, 50.48 mmol), triethylamine (21.0 mL, 150.07 mmol), acetonitrile (200 mL), and trimethylsilane iodide (18.0 mL, 126.48 mmol) were used to obtain a silyl enol ether compound as an oily crude product by the same method as in (43c). The resulting oily crude product was treated with toluene (200 mL), N-iodosuccinimide(11.36 g, 50.49 mmol), triethylamine (9.2 mL, 66.01 mmol), tetrahydrofuran (200 mL), and 2 N aqueous sodium hydroxide (50.5 mL) in this order by the same method as in (43c) and passed through a short column to obtain a crude product of the title compound (7.6 g).
¹H NMR (400 MHz, CDCl₃): δ 2.37 (3H, s), 3.90 (3H, s), 6.46 (1H, d, J=11.4 Hz), 6.69 (1H, s), 6.95 (2H, d, J= 7.1 Hz), 7.73 (2H, d, J=9.0 Hz), 11.03 (1H, s);
MS (EI)m/z: 260 (M)⁺.

### (84c) 2-(4-Methoxybenzoyl)-3-fluoro-5-methylphenyl acetate

The compound obtained in (84b) (7.6 g, 29.20 mmol) was dissolved in methylene chloride (150 mL), followed by addition of acetic anhydride (4.2 mL, 44.43 mmol), pyridine (4.7 mL, 58.11 mmol), and 4-dimethylaminopyridine (1.1 g, 9.00 mmol), and the mixture was stirred at room temperature for 20 h. The solvent was removed under reduced pressure, and the residue was diluted with ethyl acetate (100 mL) and washed with 2 M aqueous hydrochloric acid (150 mL), saturated aqueous sodium hydrogencarbonate (150 mL), and saturated brine (50 mL). The organic layer was dried over anhydrous sodium sulfate, and then the solvent was removed under reduced pressure. The residue was purified by silica gel flash column chromatography (hexane:ethyl acetate, 6:1 to 4:1, v/v) to obtain the title compound (9.53 g, quantitative) as a yellow syrup.
¹H NMR (400 MHz, CDCl₃): δ 2.04 (3H, s), 2.42 (3H, s), 3.88 (3H, s), 6.85 (1H, s), 6.88 (1H, d, J=9.8 Hz), 6.94 (2H, d, J=7.8 Hz), 7.82 (2H, d, J=8.6 Hz);
MS (EI)m/z: 302 (M)⁺.

### (84d) 3-Fluoro-5-hydroxymethyl-2-(4-methoxybenzoyl)phenol

The compound obtained in (84c) (9.53 g, 29.20 mmol) was dissolved in carbon tetrachloride (190 mL), followed by addition of N-bromosuccinimide (5.45 g, 30.62 mmol) and 2,2-azobis(isobutyronitrile) (960 mg, 5.85 mmol), and the mixture was stirred for 6 h with heating to reflux. The mixture was cooled to room temperature and filtered using Celite, and then the solvent was removed under reduced pressure.

The resulting crude product was dissolved in 1,4-dioxane (90 mL) and distilled water (30 mL), followed by addition of calcium carbonate (17.5 g, 174.84 mmol), and the mixture was stirred for 30 h with heating to reflux. The mixture was cooled to room temperature, and then the filtered residue was washed with methanol and 2 M aqueous sodium hydroxide. The solvent was removed under reduced pressure, and then the residue was diluted with ethyl acetate (100 mL) and washed with saturated aqueous ammonium chloride (50 mL) and saturated brine (20 mL). The organic layer was dried over anhydrous sodium sulfate, and then the solvent was removed under reduced pressure. The residue was purified by silica gel flash column chromatography (hexane:ethyl acetate, 2:1 to 1:1, v/v) to obtain the title compound (4.31 g, 53.4%) as a colorless solid.
¹H NMR (400 MHz, CDCl₃): δ 3.90 (3H, s), 4.73 (2H, s), 6.69 (1H, d, J=11.0 Hz), 6.87 (1H, s), 6.96 (2H, d, J=7.4 Hz), 7.74 (2H, d, J=9.0 Hz), 10.93 (1H, s);
MS (EI)m/z: 276 (M)⁺.

### (84e) 5-Acetoxymethyl-3-fluoro-2-(4-methoxybenzoyl)phenol

The compound obtained in (84d) (483 mg, 1.75 mmol) was dissolved in tetrahydrofuran (10 mL), followed by addition of vinyl acetate (10 mL) and bis(dibutylchlorotin)oxide (97 mg, 0.18 mmol), and the mixture was stirred at room temperature for 30 h. The solvent was removed under reduced pressure, and then the residue was purified by silica gel flash column chromatography (hexane:ethyl acetate, 4:1 to 2:1, v/v) to obtain the title compound (524 mg, 94.1%) as a pale yellow syrup.
¹H NMR (400 MHz, CDCl₃): δ 2.18 (3H, s), 3.91 (3H, s), 5.11 (2H, s), 6.62 (1H, d, J=11.0 Hz), 6.85 (1H, s), 6.97 (2H, d, J=9.0 Hz), 7.75 (2H, d, J=9.0 Hz), 10.86 (1H, s);
MS (FAB)m/z: 319 (M+H)⁺.

### (84f) 5-Acetoxymethyl-3-fluoro-2-(4-methoxyphenyl)hydroxymethyl phenol

The compound obtained in (84e) (524 mg, 1.65 mmol) was dissolved in methanol (15 mL), followed by addition of sodium borohydride (125 mg, 3.30 mmol) with ice cooling, and the mixture was stirred at room temperature for 30 min. Saturated aqueous ammonium chloride was added with ice cooling, and then the solvent was removed under reduced pressure. The residue was diluted with ethyl acetate (20 mL) and washed with saturated aqueous ammonium chloride (20 mL) and saturated brine (10 mL). The organic layer was dried over anhydrous sodium sulfate, and then the solvent was removed under reduced pressure. The residue was purified by silica gel flash column chromatography (hexane:ethyl acetate, 4:1 to 3:1, v/v) to obtain the title compound (382 mg, 72.3%) as a colorless syrup.
¹H NMR (400 MHz, CDCl₃): δ 2.10 (3H, s), 3.79 (3H, s), 4.99 (2H, s), 6.28 (1H, s), 6.55 (1H, d, J=10.2 Hz), 6.69 (1H, s), 6.87 (2H, d, J=7.0 Hz), 7.35 (2H, d, J=7.0 Hz), 8.94 (1H, s);
MS (FAB)m/z: 343 (M+Na)⁺.

### (84g) 5-Acetoxymethyl-3-fluoro-2-(4-methoxybenzyl)phenol

The compound obtained in (84f) (382 mg, 1.19 mmol) was dissolved in acetonitrile (7 mL), followed by addition of triethylsilane (570 µL, 3.58 mmol) and a boron trifluoride-diethyl ether complex (230 µL, 1.83 mmol), and the mixture was stirred at room temperature for 30 min. The mixture was neutralized with saturated aqueous sodium hydrogencarbonate with ice cooling, followed by addition of ethyl acetate (20 mL), and washed with saturated brine (10 mL). The residue was dried over anhydrous sodium sulfate, and then the solvent was removed under reduced pressure. The residue was purified by silica gel flash column chromatography (hexane:ethyl acetate, 4:1 to 3:1, v/v) to obtain the title compound (312 mg, 86.2%) as a colorless syrup.
¹H NMR (400 MHz, CDCl₃): δ 2.11 (3H, s), 3.77 (3H, s), 3.95 (2H, s), 5.00 (2H, s), 6.60 (1H, s), 6.69 (1H, d, J=10.9 Hz), 6.82 (2H, d, J=8.6 Hz), 7.20 (2H, d, J=8.2 Hz); MS (FAB)m/z: 304 (M)⁺.

### (84h) 5-Acetoxymethyl-3-fluoro-2-(4-methoxybenzyl)phenyl 2,3,4,6-tetra-O-benzoyl-7-deoxy-D-glycero-β-D-gluco-heptopyranoside

The compound obtained in (7d) (270 mg, 0.44 mmol), trichloroacetonitrile (140 µL, 1.40 mmol), 1,8-diazabicyclo[5.4.0]-7-undecene (7 µL, 0.05 mmol), and methylene chloride (5 mL) were used to prepare an imidate by the same method as in (1b). Subsequently, the compound obtained in (84g) (112 mg, 0.37 mmol), a boron trifluoride-diethyl ether complex (46 µL, 0.37 mmol), and methylene chloride (10 mL) were used to obtain a crude product of the title compound (272 mg) by the same method as in (1b).

### (84i) 3-Fluoro-5-hydroxymethyl-2-(4-methoxybenzyl)phenyl 7-deoxy-D-glycero-β-D-gluco-heptopyranoside

The compound obtained in (84h) (272 mg, 0.30 mmol) was dissolved in tetrahydrofuran (1 mL) and methanol (4 mL), followed by addition of potassium carbonate (420 mg, 3.04 mmol), and the mixture was stirred at room temperature for 14 h. The solvent was removed under reduced pressure, and the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (61 mg, 46.6%) as a colorless solid.
¹H NMR (400 MHz, CD₃OD): δ 1.21 (3H, d, J=6.2 Hz), 3.37-3.38 (2H, m), 3.43-3.48 (2H, m), 3.72 (3H, s), 3.93 (1H, d, J=14.0 Hz), 4.02 (1H, d, J=14.4 Hz), 4.06 (1H, dd, J=6.6, 3.5 Hz), 4.55 (2H, s), 4.93 (1H, d, J=7.4 Hz), 6.76 (2H, d, J=7.4 Hz), 6.78 (1H, d, J=10.5 Hz), 6.98 (1H, s), 7.19 (2H, d, J=8.6 Hz);
MS (FAB)m/z: 438 (M)⁺, 461 (M+Na)⁺.

### (Example 85) 3-Fluoro-5-hydroxymethyl-2-(4-methoxybenzyl)phenyl 4-deoxy-β-D-glucopyranoside (Example Compound No. 2-19)

### (85a) 5-Acetoxymethyl-3-fluoro-2-(4-methoxybenzyl)phenyl 2,3,4,6-tetra-O-benzoyl-4-deoxy-β-D-glucopyranoside

2,3,6-Tri-O-benzoyl-4-deoxy-α,β-D-glucopyranoside (Liebigs Ann. Chem. GE, 1992, 7, 747-758) (600 mg, 1.26 mmol), trichloroacetonitrile (380 µL, 3.79 mmol), 1,8-diazabicyclo[5.4.0]-7-undecene (20 µL, 0.13 mmol), and methylene chloride (12 mL) were used to prepare an imidate by the same method as in (1b). Subsequently, the compound obtained in (84d) (320 mg, 1.05 mmol), boron trifluoride-diethyl ether complex (30 µL, 1.04 mmol), and methylene chloride (12 mL) were used to obtain a crude product of the title compound (858 mg) by the same method as in (1b).

### (85b) 3-Fluoro-5-hydroxymethyl-(4-methoxybenzyl)phenyl 4-deoxy-β-D-glucopyranoside

The compound obtained in (85a) (858 mg, 1.05 mmol) was dissolved in tetrahydrofuran (2 mL) and methanol (8 mL), followed by addition of potassium carbonate (1.45 g, 10.50 mmol), and the mixture was stirred at room temperature for 14 h. The solvent was removed under reduced pressure, and then the residue was diluted with ethyl acetate (20 mL) and washed with saturated aqueous ammonium chloride (20 mL) and saturated brine (10 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (264 mg, 61.5%) as a colorless solid.
¹H NMR (400 MHz, CD₃OD): δ 1.48 (1H, dd, J=24.1, 12.0 Hz), 1.98 (1H, dd, J=12.2, 6.5 Hz), 3.31 (1H, s), 3.40 (1H, t, J=8.4 Hz), 3.59 (2H, d, J=5.1 Hz), 3.67-3.70 (1H, m), 3.73 (3H, s), 3.92 (1H, d, J=14.5 Hz), 4.03 (1H, d, J=14.5 Hz), 4.55 (2H, s), 4.90 (1H, d, J=7.8 Hz), 6.76 (2H, d, J=5.9 Hz), 6.78 (1H, d, J=6.3 Hz), 6.99 (1H, s), 7.19 (2H, d, J=8.6 Hz);
MS (FAB)m/z: 408 (M)⁺, 409 (M+H)⁺, 431 (M+Na)⁺.

### (Example 86) 3-Chloro-5-hydroxymethyl-2-(4-methoxybenzyl)phenyl 4-deoxy-β-D-glucopyranoside (Example Compound No. 2-20)

### (86a) 5-Acetoxymethyl-3-chloro-2-(4-methoxybenzyl)phenyl 2,3,4,6-tetra-O-benzoyl-4-deoxy-β-D-glucopyranoside

The compound obtained in (7d) (500 mg, 1.05 mmol), trichloroacetonitrile (320 µL, 3.19 mmol), 1,8-diazabicyclo[5.4.0]-7-undecene (16 µL, 0.11 mmol), and methylene chloride (10 mL) were used to prepare an imidate by the same method as in (1b). Subsequently, the compound obtained in (44e) (280 mg, 0.87 mmol), a boron trifluoride-diethyl ether complex (110 µL, 0.88 mmol), and methylene chloride (10 mL) were used to obtain a crude product of the title compound (714 mg) by the same method as in (1b).

### (86b) 3-Chloro-5-hydroxymethyl-2-(4-methoxybenzyl)phenyl 4-deoxy-β-D-glucopyranoside

The compound obtained in (86a) (714 mg, 0.87 mmol) was dissolved in tetrahydrofuran (2 mL) and methanol (8 mL), followed by addition of potassium carbonate (1.20 g, 8.68 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (227 mg, 61.4%) as a colorless solid.
¹H NMR (400 MHz, CD₃OD): δ 1.46 (1H, dd, J=24.2, 11.7 Hz), 1.98 (1H, ddd, J=12.9, 5.1, 2.0 Hz), 3.35 (1H, s), 3.38 (1H, t, J=8.4 Hz), 3.59 (2H, d, J=4.7 Hz), 3.66-3.71 (1H, m), 3.73 (3H, s), 4.08 (1H, d, J=14.1 Hz), 4.22 (1H, d, J=14.5 Hz), 4.55 (2H, s), 4.89 (1H, d, J=7.9 Hz), 6.76 (2H, d, J=8.6 Hz), 7.09 (1H, s), 7.13 (1H, s), 7.18 (2H, d, J=8.6 Hz);
MS (FAB)m/z: 424 (M)⁺, 447 (M+Na)⁺.

### (Example 87) 2-(4-Isopropoxybenzyl)-5-(hydroxyacetoxymethyl)phenyl 7-deoxy-D-glycero-β-D-gluco-heptopyranoside (Example Compound No. 1-154)

### (87a) 5-(Allyloxycarbonyloxy)acetoxymethyl-2-(4-propoxybenzyl)phenyl 7-deoxy-D-glycero-β-D-gluco-heptopyranoside

The compound obtained in Example (81d) (249 mg, 0.56 mmol), 2-(allyloxycarbonyloxy)acetyl chloride (EP1362856A1) (250 mg, 1.39 mmol), 2,4,6-trimethylpyridine (2.5 mL), and dichloromethane (2.5 mL) were used to obtain the title compound (127 mg, 38.6%) as a colorless syrup by the same method as in (34a). ¹H NMR (400 MHz, CD₃OD): δ 1.23 (3H, d, J=6.3 Hz), 1.26 (6H, d, J=6.3 Hz), 3.39-3.41 (2H, m), 3.48-3.50 (2H, m), 3.92 (1H, d, J=14.8 Hz), 4.00 (1H, d, J=15.2 Hz), 4.03-4.06 (1H, m), 4.47-4.53 (1H, m), 4.62 (2H, d, J=5.9 Hz), 4.69 (2H, s), 4.93 (1H, d, J=7.4 Hz), 5.12 (1H, d, J=12.1 Hz), 5.18 (1H, d, J=12.1 Hz), 5.21 (1H, d, J=9.1 Hz), 5.33 (1H, dd, J=17.1, 1.5 Hz), 5.86-5.96 (1H, m), 6.77 (2H, d, J=8.6 Hz), 6.93 (1H, d, J=7.8 Hz), 7.03 (1H, d, J=7.4 Hz), 7.11 (1H, s), 7.14 (2H, d, J=6.3 Hz); MS (FAB)m/z: 591 (M+H)⁺, 613 (M+Na)⁺.

### (87b) 2-(4-Isopropoxybenzyl)-5-(hydroxyacetoxymethyl)phenyl 7-deoxy-D-glycero-β-D-gluco-heptopyranoside

The compound obtained in (87a) (127 mg, 0.22 mmol) was dissolved in tetrahydrofuran (1 mL), followed by addition of tetrakis(triphenylphosphine)palladium(0) (24.8 mg, 0.0021 mmol), triphenylphosphine (24.8 mg, 0.094 mmol), and dimedone (18.1 mg, 0.13 mmol), and the mixture was stirred at room temperature for 1 h. The mixture was diluted with dichloromethane (1 mL) and purified by silica gel flash column chromatography (ethanol:methylene chloride, 1:20 to 1:10 to 1:5, v/v) to obtain the title compound (68 mg, 67.3%) as a colorless solid.
¹H NMR (400 MHz, CD₃OD): δ 1.22 (3H, d, J=6.6 Hz), 1.27 (6H, d, J=5.9 Hz), 3.37-3.38 (2H, m), 3.45-3.48 (2H, m), 3.92 (1H, d, J=14.8 Hz), 4.00 (1H, d, J=15.3 Hz), 4.03-4.09 (1H, d), 4.14 (2H, s), 4.48-4.54 (1H, m), 4.90 (1H, d, J=7.8 Hz), 5.13 (2H, s), 6.77 (2H, d, J=8.6 Hz), 6.95 (1H, d, J=9.4 Hz), 7.04 (1H, d, J=7.4 Hz), 7.12 (2H, d, J=8.6 Hz), 7.16 (1H, s);
MS (FAB)m/z: 506 (M)⁺, 529 (M+Na)⁺.

### (Example 88) 2-(4-Cyclopropylbenzyl)-3-fluoro-5-hydroxymethylphenyl 7-deoxy-D-glycero-β-D-gluco-heptopyranoside (Example Compound No. 1-155)

### (88a) 2-(4-Cyclopropylbenzoyl)-5-ethoxycarbonyl-3-hydroxy-cyclohex-2-enone

Ethyl-3-hydroxy-5-oxo-cyclohex-3-enecarboxylate (EP1571148A1) (1.18 g, 6.41 mmol) dissolved in acetonitrile (24 mL), 4-cyclopropylbenzoic acid chloride (Org. Magn. Reson., 13, 1980, 372-375) (1.16 g, 6.41 mmol), triethylamine (2.68 mL, 19.23 mmol), and trimethylsilyl nitrile (102 µL, 0.76 mmol) were used to obtain the title compound (2.16 g, quantitative) as a brown syrup by the same method as in (42a).
¹H NMR (400 MHz, CDCl₃): δ 0.76-0.80 (2H, m), 1.01-1.06 (2H, m), 1.29 (3H, t, J=7.1 Hz), 1.90-1.97 (1H, m), 2.75 (2H, d, J=6.6 Hz), 2.95 (1H, dd, J=18.4, 5.5 Hz), 3.05 (1H, dd, J=18.4, 8.2 Hz), 3.01-3.03 (1H, m), 4.22 (2H, q, J=7.1 Hz), 7.08 (2H, d, J=8.2 Hz), 7.47 (2H, d, J=8.6 Hz);
MS (FAB)m/z: 329 (M+H)⁺, 351 (M+Na)⁺, 373 (M+2Na-H)⁺.

### (88b) 2-(4-Cyclopropylbenzoyl)-5-ethoxycarbonyl-3-fluoro-cyclohex-2-enone

The compound obtained in (88a) (2.16 g, 6.41 mmol) was dissolved in dichloromethane (25 mL), followed by addition of diethylaminosulfur trifluoride (2.54 mL, 19.22 mmol), and the mixture was stirred at room temperature for 3 h. Distilled water (10 mL) was added dropwise with ice cooling, and then the mixture was diluted with dichloromethane (20 mL) and washed with distilled water (150 mL) and saturated brine (20 mL). The organic layer was dried over anhydrous sodium sulfate, and then the solvent was removed under reduced pressure. The residue was purified by silica gel flash column chromatography (hexane:ethyl acetate, 6:1 to 3:1, v/v) to obtain the title compound (1.74 g, 82.5%) as a brown syrup.
¹H NMR (400MHz, CDCl₃): δ 0.77-0.81 (2H, m), 1.05-1.10 (2H, m), 1.31 (3H, t, J=7.1 Hz), 1.91-1.98 (1H, m), 2.79 (1H, d, J=3.5 Hz), 2.81 (1H, s), 2.96 (1H, dt, J=18.4, 5.3 Hz), 3.25-3.32 (1H, m), 4.26 (2H, q, J=7.3 Hz), 7.12 (2H, d, J=8.2 Hz), 7.72 (2H, d, J=8.6 Hz);
MS (FAB)m/z: 331 (M+H)⁺.

### (88c) 2-(4-Cyclopropylbenzoyl)-5-ethoxycarbonyl-3-fluorophenol

The compound obtained in (88b) (1.74 g, 5.27 mmol), triethylamine (2.20 mL, 15.78 mmol), acetonitrile (20 mL), and trimethylsilane iodide (1.87 mL, 13.14 mmol) were used to obtain a silyl enol ether compound as an oily crude product by the same method as in (43c). The resulting oily crude product was treated with toluene (20 mL), a silica gel SK-85 (6.86 g), potassium carbonate (728 mg, 5.27 mmol), and ethanol (20 mL) in this order by the same method as in (47c), and the product was purified by silica gel flash column chromatography (hexane:ethyl acetate, 4:1, v/v) to obtain the title compound (1.06 g, 61.3%) as a pale yellow solid. ¹H NMR (400 MHz,CDCl₃): δ 0.65-0.69 (2H, m), 0.93-0.98 (2H, m), 1.36 (3H, t, J=7.1 Hz), 1.83-1.90 (1H, m), 4.34 (2H, q, J=7.0 Hz), 7.04 82H, d, J=8.2 Hz), 7.20 (1H, d, J=10.6 Hz), 7.31 (2H, d, J=8.2 Hz), 7.38 (1H, s), 9.01 (1H, s);
MS (FAB)m/z: 329 (M+H)⁺.

### (88d) 5-Acetoxymethyl-2-(4-cyclopropylbenzyl)hydroxymethyl-3fluorophenol

The compound obtained in (88c) (1.06 g, 3.23 mmol) was dissolved in tetrahydrofuran (10 mL), followed by addition of lithium aluminium hydride (368 mg, 9.70 mmol) with ice cooling, and the mixture was stirred at room temperature for 2 h. Distilled water (2 mL) was added with ice cooling, the mixture was diluted with ethyl acetate (10 mL) and washed with 1 M hydrochloric acid (20 mL), saturated aqueous sodium hydrogencarbonate (20 mL), and saturated brine (10 mL). The mixture was dried over sodium sulfate, and then the solvent was removed under reduced pressure to obtain a crude product (838 mg).

The resulting crude product (838 mg) was dissolved in tetrahydrofuran (8 mL), followed by addition of vinyl acetate (8 mL) and bis(dibutylchlorotin)oxide (161 mg, 0.29 mmol), and the mixture was stirred at room temperature for 30 h. The solvent was removed under reduced pressure, and then the residue was purified by silica gel flash column chromatography (hexane:ethyl acetate, 6:1 to 4:1, v/v) to obtain the title compound (980 mg, quantitative) as a pale yellow oil.
¹H NMR (400 MHz, CDCl₃): δ 0.65-0.68 (2H, m), 0.93-0.98 (2H, m), 1.84-1.90 (1H, m), 2.11 (3H, s), 3.01 (1H, brs), 4.99 (2H, s), 6.29 (1H, s), 6.55 (1H, d, J=10.2 Hz), 6.68 (1H, s), 7.04 (2H, d, J=8.2 Hz), 7.31 (2H, d, J=8.3 Hz), 8.87 (1H, s);
MS (FAB)m/z: 353 (M+Na)⁺.

### (88e) 5-Acetoxymethyl-2-(4-cyclopropylbenzyl)-3-fluorophenol

The compound obtained in (88d) (980 mg, 2.91 mmol) was dissolved in acetonitrile (10 mL), followed by addition of triethylsilane (1.40 mL, 8.79 mmol) and a boron trifluoride-diethyl ether complex (550 µL, 4.38 mmol), and the mixture was stirred at room temperature for 30 min. The mixture was neutralized with saturated aqueous sodium hydrogencarbonate with ice cooling, followed by addition of ethyl acetate (20 mL), and washed with saturated brine (10 mL). The residue was dried over anhydrous sodium sulfate, and then the solvent was removed under reduced pressure. The residue was purified by silica gel flash column chromatography (hexane:ethyl acetate, 6:1 to 4:1, v/v) to obtain the title compound (805 mg, 88.1%) as a colorless solid. ¹H NMR (400 MHz, CDCl₃): δ 0.62-0.66 (2H, m), 0.89-0.94 (2H, m), 1.81-1.87 (1H, m), 2.11 (3H, s), 3.96 (2H, s), 5.00 (2H, s), 6.59 (1H, s), 6.68 (1H, d, J=9.4 Hz), 6.98 (2H, d, J=8.2 Hz), 7.15 (2H, d, J=8.2 Hz);
MS (FAB)m/z: 314 (M)⁺, 337 (M+Na)⁺.

### (88f) 5-Acetoxymethyl-2-(4-cyclopropylbenzyl)-3-fluorophenyl 2,3,4,6-tetra-O-benzoyl-7-deoxy-D-glycero-β-D-gluco-heptopyranoside

The compound obtained in (7d) (233 mg, 0.38 mmol), trichloroacetonitrile (120 µL, 1.20 mmol), 1,8-diazabicyclo[5.4.0]-7-undecene (11 µL, 0.07 mmol), and methylene chloride (5 mL) were used to prepare an imidate by the same method as in (1b). Subsequently, the compound obtained in (88e) (100 mg, 0.32 mmol), a boron trifluoride-diethyl ether complex (40 µL, 0.32 mmol), and methylene chloride (10 mL) were used to obtain a crude product of the title compound (325 mg) by the same method as in (1b).

### (88g) 2-(4-Cyclopropylbenzyl)-3-fluoro-5-hydroxymethylphenyl 7-deoxy-D-glycero-β-D-gluco-heptopyranoside

The compound obtained in (88f) (325 mg) was dissolved in tetrahydrofuran (1 mL) and methanol (4 mL), followed by addition of potassium carbonate (44 mg, 0.32 mmol), and the mixture was stirred at room temperature for 14 h. The solvent was removed under reduced pressure, and then the residue was diluted with ethyl acetate (10 mL) and washed with saturated aqueous ammonium chloride (10 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (68 mg, 47.6%) as a colorless solid.
¹H NMR (400 MHz, CD₃OD): δ 0.58-0.62 (2H, m), 0.86-0.91 (2H, m), 1.22 (3H, d, J=5.3 Hz), 1.78-1.85 (1H, m), 3.36-3.38 (2H, m), 3.44-3.50 (2H, m), 3.94 (1H, d, J=13.2 Hz), 4.05 (2H, d, J=14.2 Hz), 4.56 (2H, s), 4.94 (1H, d, J=6.4 Hz), 6.79 (1H, d, J=9.8 Hz), 6.92 (2H, d,J=6.4 Hz), 6.98 (1H, s), 7.14 (2H, d,J=5.8 Hz);
MS (FAB)m/z: 449 (M+H)⁺, 471 (M+Na)⁺.

### (Example 89) 2-(4-Ethylbenzyl)-5-hydroxymethyl-3-methylphenyl 7-deoxy-D-glycero-β-D-gluco-heptopyranoside (Example Compound No. 1-156)

### (89a) 3-Bromo-2-(4-ethylbenzoyl)-5-ethoxycarbonylcyclohex-2-enone

The compound obtained in (47a) (500 mg, 1.58 mmol) was dissolved in dichloromethane (5 mL), followed by addition of 2-methyl-2-butene (670 µL, 6.31 mmol), oxalyl dibromide (230 µL, 1.62 mmol), and N,N-dimethylformamide (2 drops) with ice cooling, and the mixture was stirred at room temperature for 1 h. Distilled water (500 µL) was added with ice cooling, and the mixture was diluted with dichloromethane (5 mL) and then washed with saturated brine (5 mL). The organic layer was dried over sodium sulfate, and then the solvent was removed under reduced pressure. The residue was purified by silica gel flash column chromatography (hexane:ethyl acetate, 6:1 to 4:1, v/v) to obtain the title compound (415 mg, 69.3%) as a brown syrup.
¹H NMR (400 MHz, CDCl₃): δ 1.26 (3H, t, J=7.7 Hz), 1.32 (3H, t, J=7.2 Hz), 2.71 (2H, q, J=7.4 Hz), 2.83 (2H, d, J=6.3 Hz), 3.24-3.33 (3H, m), 4.26 (2H, q, J=7.0 Hz), 7.30 (2H, d, J=8.2 Hz), 7.78 (2H, d, J=8.2 Hz);
MS (FAB)m/z: 379 (M+H)⁺, 401 (M+Na)⁺.

### (89b) 2-(4-Ethylbenzoyl)-5-ethoxycarbonyl-3-methyl-cyclohex-2-enone

Tetrahydrofuran (10 mL) was added to copper(I) thiophenolate (468 mg, 2.71 mmol), then methyl lithium (1.13 M diethyl ether solution) (2.4 mL, 2.71 mmol) was added dropwise under a nitrogen atmosphere at -20°C, and the mixture was stirred at the same temperature for 15 min. The mixture was cooled to -78°C, a solution of the compound obtained in (89a) (500 mg, 1.32 mmol) in tetrahydrofuran (5 mL) was added dropwise, and the mixture was stirred for 2 h. Saturated aqueous ammonium chloride (2 mL) was added, and then the mixture was diluted with diethyl ether (10 mL) and washed with saturated brine (10 mL). The organic layer was dried over anhydrous sodium sulfate, and then the solvent was removed under reduced pressure. The residue was purified by silica gel flash column chromatography (hexane:ethyl acetate, 5:1 to 4:1, v/v) to obtain the title compound (299 mg, 72.2%) as a brown syrup.
¹H NMR (400MHz, CDCl₃): δ 1.25 (3H, t, J=7.7 Hz), 1.30 (3H, t, J=7.1 Hz), 1.90 (3H, s), 2.70 (2H, q, J=7.5 Hz), 2.74-2.84 (4H, m), 3.18-3.25 (1H, m), 4.23 (2H, q, J=7.0 Hz), 7.27 (2H, d, J=8.2Hz), 7.75 (2H, d, J=8.2 Hz);
MS (FAB)m/z: 315(M+H)⁺.

### (89c) 5-Ethoxycarbonyl-2-(4-ethylbenzoyl)-3-methyl phenol

The compound obtained in (89b) (299 mg, 0.95 mmol), triethylamine (400 µL, 2.87 mmol), acetonitrile (3 mL), and trimethylsilane iodide (340 µL, 2.39 mmol) were used to obtain a silyl enol ether compound as an oily crude product by the same method as in (43c). The resulting oily crude product was treated with toluene (3 mL), N-iodosuccinimide (214 mg, 0.95 mmol), triethylamine (170 µL, 1.22 mmol), tetrahydrofuran (3 mL), and 2 N aqueous sodium hydroxide (1.0 mL) in this order by the same method as in (43c), and the product was purified by silica gel flash column chromatography (hexane:ethyl acetate, 4:1, v/v) to obtain the title compound (124 mg, 41.9%) as a pale yellow syrup.
¹H NMR (400 MHz,CDCl₃): δ 1.27 (3H, d, J=7.6 Hz), 1.41 (3H, d, J=7.1 Hz), 2.18 (3H, s), 2.73 (2H, q, J=7.5 Hz), 4.40 (2H, q, J=7.1 Hz), 7.30 (2H, d, J=8.2 Hz), 7.50 (1H, d, J=10.6 Hz), 7.52 (1H, s), 7.69 (2H, d, J=8.2 Hz);
MS (FAB)m/z: 313(M+H)⁺.

### (89d) 5-Acetoxymethyl-2-(4-ethylbenzyl)hydroxymethyl-3-methylphenol

The compound obtained in (89c) (124 mg, 0.40 mmol) was dissolved in tetrahydrofuran (5 mL), followed by addition of lithium aluminium hydride (45 mg, 1.21 mmol) with ice cooling, and the mixture was stirred at room temperature for 2 h. Distilled water (500 µL) was added with ice cooling, and the mixture was diluted with ethyl acetate (5 mL) and washed with 1 M hydrochloric acid (5 mL), saturated aqueous sodium hydrogencarbonate (5 mL), and saturated brine (5 mL). The mixture was dried over sodium sulfate, and then the solvent was removed under reduced pressure to obtain a crude product (100 mg).

The resulting crude product (100 mg) was dissolved in tetrahydrofuran (1 mL), followed by addition of vinyl acetate (1 mL) and bis(dibutylchlorotin)oxide (22 mg, 0.04 mmol), and the mixture was stirred at room temperature for 30 h. The solvent was removed under reduced pressure, and then the residue was purified by silica gel flash column chromatography (hexane:ethyl acetate, 6:1 to 5:1, v/v) to obtain the title compound (73 mg, 58.4%) as a colorless solid.
¹H NMR (400 MHz, CDCl₃): δ 1.21 (3H, t, J=7.7 Hz), 2.16 (3H, s), 2.17 (3H, s), 2.63 (2H, q, J=7.8 Hz), 2.93 (1H, brs), 5.02 (2H, s), 6.17 (1H, d, J=2.7 Hz), 6.67 (1H, s), 6.81 (1H, s), 7.18 (2H, d, J=8.2 Hz), 7.29 (2H, d, J=8.2 Hz), 8.84 (1H, s);
MS (FAB)m/z: 314 (M)⁺.

### (89e) 5-Acetoxymethyl-2-(4-ethylbenzyl)-3-methylphenol

The compound obtained in (89d) (73 mg, 0.23 mmol) was dissolved in acetonitrile (2 mL), followed by addition of triethylsilane (110 µL, 0.69 mmol) and a boron trifluoride-diethyl ether complex (45 µL, 0.36 mmol), and the mixture was stirred at room temperature for 30 min. The mixture was neutralized with saturated aqueous sodium hydrogencarbonate with ice cooling, followed by addition of ethyl acetate (5 mL), and washed with saturated brine (5 mL). The residue was dried over anhydrous sodium sulfate, and then the solvent was removed under reduced pressure. The residue was purified by silica gel flash column chromatography (hexane:ethyl acetate, 6:1, v/v) to obtain the title compound (37 mg, 54.4%) as a colorless solid.
¹H NMR (400 MHz, CDCl₃): δ 1.20 (3H, t, J=7.7 Hz), 2.18 (3H, s), 2.29 (3H, s), 2.60 (2H, q, J=7.4 Hz), 4.01 (2H, s), 5.02 (2H, s), 6.70 (1H, s), 6.80 (1H, s), 7.08 (4H, d, J=3.2 Hz);
MS (EI)m/z: 298 (M)⁺.

### (89f) 5-Acetoxymethyl-2-(4-ethylbenzyl)-3-methylphenyl 2,3,4,6-tetra-O-benzoyl-7-deoxy-D-glycero-β-D-gluco-heptopyranoside

The compound obtained in (7d) (100 mg, 0.16 mmol), trichloroacetonitrile (50 µL, 0.50 mmol), 1,8-diazabicyclo[5.4.0]-7-undecene (5 µL, 0.03 mmol), and methylene chloride (1 mL) were used to prepare an imidate by the same method as in (1b). Subsequently, the compound obtained in (89e) (37 mg, 0.12 mmol), a boron trifluoride-diethyl ether complex (16 µL, 0.13 mmol), and methylene chloride (2 mL) were used to obtain a crude product of the title compound (143 mg) by the same method as in (1b).

### (89g) 2-(4-Ethylbenzyl)-5-hydroxymethyl-3-methylphenyl 7-deoxy-D-glycero-β-D-gluco-heptopyranoside

The compound obtained in (89f) (143 mg) was dissolved in tetrahydrofuran (0.5 mL) and methanol (2 mL), followed by addition of potassium carbonate (17 mg, 0.13 mmol), and the mixture was stirred at room temperature for 14 h. The solvent was removed under reduced pressure, and the residue was diluted with ethyl acetate (5 mL) and washed with saturated aqueous ammonium chloride (5 mL) and saturated brine (5 mL). The organic layer was dried over anhydrous sodium sulfate, and then the solvent was removed under reduced pressure. The residue was purified by silica gel flash column chromatography (methanol:methylene chloride, 1:20 to 1:15 to 1:10, v/v) to obtain the title compound (21 mg, 39.6%) as a colorless solid.
¹H NMR (400 MHz, CD₃OD): δ 1.17 (3H, t, J=7.7 Hz), 1.23 (3H, d, J=6.3 Hz), 2.19 (3H, s), 2.55 (2H, q, J=7.8 Hz), 3.35-3.36 (2H, m), 3.42-3.43 (2H, m), 3.99 (1H, d, J=15.2 Hz), 4.02-4.08 (1H, m), 4.18 (1H, d, J=15.2 Hz), 4.53 (2H, s), 6.86 (1H, s), 7.00-7.06 (5H, m);
MS (FAB)m/z: 455 (M+Na)⁺.

### (Example 90) 3-Chloro-5-hydroxymethyl-2-(4-methoxybenzyl)phenyl 4-C-methyl-7-deoxy-glycero-β-D-gluco-heptapyranoside (Example Compound No. 1-157)

### (90a) 5-Acetoxymethyl-3-chloro-2-(4-methoxybenzyl)phenyl 4-O-acetyl-4-C-methyl-2,3,6-tri-O-benzoyl-7-deoxy-glycero-β-D-gluco-heptapyranoside

The compound obtained in (39c) (200 mg, 0.356 mmol), trichloroacetonitrile (0.18 mL, 1.80 mmol), 1,8-diazabicyclo[5.4.0]-7-undecene (10 mg, 0.066 mmol), and methylene chloride (4 mL) were used to prepare an imidate by the same method as in (1b). Subsequently, the compound obtained in (44e) (95.0 mg, 0.296 mmol), a boron trifluoride-diethyl ether complex (45 µL, 0.355 mmol), and methylene chloride (4 mL) were used to obtain a crude product of the title compound (214 mg) by the same method as in (1b), and the resulting crude product was used in the subsequent reaction as it was.

### (90b) 3-Chloro-5-hydroxymethyl-2-(4-methoxybenzyl)phenyl 4-C-methyl-7-deoxy-glycero-β-D-gluco-heptapyranoside

The compound obtained in (90a) (214 mg), aqueous sodium hydroxide (2 mol/L, 1.48 mL, 2.96 mmol), tetrahydrofuran (2 mL), and methanol (5 mL) were used to obtain the title compound (39.6 mg, yield 29%) as a colorless solid by the same method as in (6e). However, purification was performed by silica gel flash column chromatography (methylene chloride:methanol, 9:1 to 7:1, v/v).
¹H NMR (400 MHz, CD₃OD): δ 1.26 (3H, s), 1.33 (3H, d, J=6.7 Hz), 3.12 (1H, d, J=3.9 Hz), 3.39 (1H, d, J=9.7 Hz), 3.49 (1H, dd, J=9.7 Hz and 7.4 Hz), 3.72 (3H, s), 4.03-4.09 (1H, m), 4.09 (1H, d, J=14.1 Hz), 4.20 (1H, d, J=14.1 Hz), 4.55 (2H, s), 4.94 (1H, d, J=7.4 Hz), 6.73-6.77 (2H, m), 7.09 (1H, brs), 7.17-7.20 (3H, m);
MS (FAB)m/z: 491 (M+Na)⁺.

### (Example 91) 3-Fluoro-5-hydroxymethyl-2-(4-methoxybenzyl)phenyl 4-C-methyl-7-deoxy-glycero-β-D-gluco-heptapyranoside (Example Compound No. 1-158)

### (91 a) 5-Acetoxymethyl-3-fluoro-2-(4-methoxybenzyl)phenyl 4-O-acetyl-4-C-methyl-2,3,6-tri-O-benzoyl-7-deoxy-glycero-β-D-gluco-heptapyranoside

The compound obtained in (39c) (1.20 g, 2.13 mmol), trichloroacetonitrile (0.64 mL, 6.38 mmol), 1,8-diazabicyclo[5.4.0]-7-undecene (35 µL, 0.234 mmol), and methylene chloride (24 mL) were used to prepare an imidate by the same method as in (1b). Subsequently, the compound obtained in (84g) (500 mg, 1.64 mmol), a boron trifluoride-diethyl ether complex (0.25 mL, 1.97 mmol), and methylene chloride (12 mL) were used to obtain a crude product of the title compound (1.80 g) by the same method as in (1b), and the resulting crude product was used in the subsequent reaction as it was.

### (91b) 3-Fluoro-5-hydroxymethyl-2-(4-methoxybenzyl)phenyl 4-C-methyl-7-deoxy-glycero-β-D-gluco-heptapyranoside

The compound obtained in (91a) (1.80 g), sodium methoxide (0.64 g, 3.32 mmol), tetrahydrofuran (2.4 mL), methanol (13 mL), and acetic acid (0.23 mL, 4.0 mmol) were used to obtain the title compound (346 mg, yield 47%) as a colorless solid by the same method as in (7f). However, purification was performed by silica gel flash column chromatography (methylene chloride:methanol, 6:1 to 5:1, v/v).
¹H NMR (500 MHz, CD₃OD): δ 1.28 (3H, s), 1.34 (3H, d, J=6.8 Hz), 3.13 (1H, d, J=3.4 Hz), 3.41 (1H, d, J=9.8 Hz), 3.52 (1H, dd, J=9.8 Hz and 7.8 Hz), 3.72 (3H, s), 3.94 (1H, d, J=14.6 Hz), 4.01 (1H, d, J=14.6 Hz), 4.04-4.09 (1H, m), 4.55 (2H, s), 4.95 (1H, d, J=7.8 Hz), 6.74-6.79 (3H, m), 7.02 (1H, s), 7.19-7.20 (2H, m);
MS (FAB)m/z: 475 (M+Na)⁺.

### (Example 92) 3-Fluoro-5-(hydroxyacetoxy)methyl-2-(4-methoxybenzyl)phenyl 7-deoxy-glycero-β-D-gluco-heptopyranoside (Example Compound No. 1-159)

### (92a) 5-(Allyloxycarbonyloxy)acetoxymethyl-3-fluoro-2-(4-methoxybenzyl)phenyl 7-deoxy-glycero-β-D-gluco-heptopyranoside

The compound obtained in (91b) (197 mg, 0.435 mmol), 2,4,6-trimethylpyridine (3 mL), methylene chloride (3.5 mL), and 2-(allyloxycarbonyloxy)acetyl chloride (194 mg, 1.09 mmol) were used to obtain the title compound (119 mg, yield 46%) as a colorless solid by the same method as in (34a). However, purification was performed by silica gel flash column chromatography (methylene chloride:ethanol, 12:1 to 9:1, v/v). ¹H NMR (500 MHz, CD₃OD): δ 1.27 (3H, s), 1.33 (3H, d, J=6.3 Hz), 3.16 (1H, d, J=3.9 Hz), 3.42 (1H, d, J=9.6 Hz), 3.52 (1H, dd, J=9.6 Hz and 7.5 Hz), 3.72 (3H, s), 3.94 (1H, d, J=13.7 Hz), 4.01-4.08 (2H, m), 4.63-4.64 (2H, m), 4.68-4.75 (2H, m), 4.98 (1H, d, J=7.5 Hz), 5.13 (1H, d, J=12.7 Hz), 5.18 (1H, d, J=12.7 Hz), 5.21-5.23 (1H, m), 5.32-5.36 (1H, m), 5.88-5.97 (1H, m), 6.75-6.77 (2H, m), 6.81 (1H, d, J=9.3 Hz), 7.04 (1H, s), 7.19-7.21 (2H, m);
MS (FAB)m/z: 595 (M+H)⁺.

### (92b) 3-Fluoro-5-(hydroxyacetoxy)methyl-2-(4-methoxybenzyl)phenyl 7-deoxy-glycero-β-D-gluco-heptopyranoside

The compound obtained in (92a) (115 mg, 0.193 mmol) was dissolved in tetrahydrofuran (1.7 mL), triphenylphosphine (22 mg, 0.084 mmol), tetrakis(triphenylphosphine)palladium (22 mg, 0.019 mmol), and dimedone (18 mg, 0.128 mmol) were successively added, and the mixture was stirred at room temperature for 30 min. The mixture was diluted with methylene chloride (10 mL) and purified by silica gel flash column chromatography (methylene chloride: ethanol, 9:1 to 7:1, v/v) as it was to obtain the title compound (71.3 mg, yield 72%) as a colorless solid.
¹H NMR (500 MHz, CD₃OD): δ 1.26 (3H, s), 1.33 (3H, d, J=6.3 Hz), 3.13 (1H, d, J=4.4 Hz), 3.41 (1H, d, J=9.8 Hz), 3.51 (1H, dd, J=9.8 Hz and 7.8 Hz), 3.72 (3H, s), 3.94 (1H, d, J=14.1 Hz), 4.02-4.00 (2H, m), 4.15 (2H, s), 4.94 (1H, d, J=7.8 Hz), 5.12 (2H, s), 6.74-6.76 (2H, m), 6.81 (1H, d, J=9.8 Hz), 7.05 (1H, s), 7.18-7.20 (2H, m);
MS (FAB)m/z: 533 (M+Na)⁺.

### (Example 93) 2-(4-Ethoxybenzyl)-5-hydroxymethylphenyl 7-deoxy-D-glycero-β-D-gluco-heptapyranoside (Example Compound No. 1-160)

### (93a) Ethyl 4-[(4-ethoxyphenyl)hydroxymethyl]-3-hydroxybenzoate

1-Bromo-4-ethoxybenzene (10.4 g, 51.5 mmol), metal magnesium (1.25 g, 51.5 mmol), and tetrahydrofuran (52 mL) were used to prepare Grignard reagent according to a usual method. A solution of ethyl 4-formyl-3-hydroxybenzoate (2.50 g, 12.9 mmol) in tetrahydrofuran (30 mL) was added dropwise to the resulting Grignard reagent at -78°C over 15 min, and the mixture was heated to -65°C and stirred for 2 h. Dilute hydrochloric acid (2 mol/L, 28 mL) was added to the reaction mixture, and the mixture was extracted with ethyl acetate (40 mL) and then washed successively with water (50 mL), saturated aqueous sodium hydrogencarbonate (50 mL), and saturated aqueous ammonium chloride (50 mL). The organic layer was dried over anhydrous magnesium sulfate, and then the solvent was removed under reduced pressure. The residue was purified by silica gel flash column chromatography (hexane:ethyl acetate, 7:3 to 6:4, v/v) to obtain the title compound (3.91 g, yield 96%) as a colorless oil.
¹H NMR (400 MHz, CDCl₃): δ 1.37 (3H, t, J=7.0 Hz), 1.41 (3H, t, J=7.0 Hz), 2.80 (1H, d, J=3.2 Hz), 4.03 (2H, q, J=7.0 Hz), 4.35 (2H, q, J=7.0 Hz), 6.03 (1H, d, J=3.2 Hz), 6.87-6.91 (2H, m), 6.93 (1H, d, J=7.8 Hz), 7.28-7.31 (2H, m), 7.48 (1H, dd, J=7.8 Hz and 1.6 Hz), 7.58 (1H, d, J=1.6 Hz), 8.22 (1H, s).

### (93b) 2-(4-Ethoxybenzyl)-5-hydroxymethylphenol

The compound obtained in (93a) (3.91 g, 12.4 mmol) was dissolved in methanol (78 mL), followed by addition of concentrated hydrochloric acid (1.0 mL) and 10% palladium on carbon (0.78 g), and the mixture was stirred under a hydrogen atmosphere at room temperature for 2 h. The 10% palladium on carbon was removed by filtration, followed by addition of toluene (10 mL) and methanol (10 mL), the solvent was removed under reduced pressure, and the resulting crude product was used in the subsequent reaction as it was.

The crude product was dissolved in tetrahydrofuran (70 mL), the mixture was added to a suspension of lithium aluminium hydride (1.18 g) in tetrahydrofuran (8 mL) with ice cooling and stirred at room temperature for 2 h. 2 mol/L hydrochloric acid (30 mL) was added dropwise with ice cooling, and the mixture was extracted with ethyl acetate (100 mL x 2) and then washed with saturated brine (50 mL). The organic layer was dried over anhydrous magnesium sulfate, and the solvent was removed under reduced pressure. The residue was purified by silica gel flash column chromatography (hexane:ethyl acetate, 7:3 to 1:1, v/v) to obtain the title compound (2.69 g, yield 84%) as a colorless solid.
¹H NMR (400 MHz, CDCl₃): δ 1.39 (3H, t, J=7.1 Hz), 1.66 (1H, brs), 3.92 (3H, s), 4.00 (2H, q, J=7.1 Hz), 4.62 (2H, brs), 4.89 (1H, s), 6.80-6.84 (3H, m), 6.85-6.87 (1H, m), 7.09 (1H, d, J=7.4Hz), 7.11-7.13 (2H, m).

### (93c) 5-Acetoxymethyl-2-(4-ethoxybenzyl)phenol

The compound obtained in (93b) (1.35 g, 5.23 mmol), tetrahydrofuran (6.8 mL), vinyl acetate (13.6 mL), and bis(dibutylchlorotin)oxide (0.87 g, 1.57 mmol) were used to obtain the title compound (1.57 g, yield 100%) as a colorless solid by the same method as in (8b). However, purification was performed by silica gel flash column chromatography (hexane:ethyl acetate, 3:2 to 1:1, v/v).
¹H NMR (400 MHz, CDCl₃): δ 1.39 (3H, t, J=6.8 Hz), 2.10 (3H, s), 3.92 (2H, s), 4.00 (2H, q, J=6.8 Hz), 4.89(1H, brs), 5.03 (2H, s), 6.80 (1H, d, J=1.8 Hz), 6.81-6.84 (2H, m), 6.87 (1H, dd, J=7.8 Hz and 1.8 Hz), 7.09 (1H, d, J=7.8 Hz), 7.11-7.14 (2H, m).

### (93d) 5-Acetoxymethyl-2-(4-ethoxybenzyl)phenyl 2,3,4,6-tetra-O-benzoyl-7-deoxy-D-glycero-β-D-gluco-heptopyranoside

The compound obtained in (7d) (2.33 g, 3.82 mmol), trichloroacetonitrile (0.77 mL, 7.68 mmol), 1,8-diazabicyclo[5.4.0]-7-undecene (60 µL, 0.40 mmol), and methylene chloride (46 mL) were used to prepare an imidate by the same method as in (1b). Subsequently, the compound obtained in (93c) (820 mg, 0.273 mmol), a boron trifluoride-diethyl ether complex (0.42 mL, 3.3 mmol), and methylene chloride (23 mL) were used to obtain a crude product of the title compound (6.0 g) by the same method as in (1b), and the resulting crude product was used in the subsequent reaction as it was.

### (93e) 2-(4-Ethoxybenzyl)-5-hydroxymethylphenyl 7-deoxy-D-glycero-β-D-gluco-heptapyranoside

The crude product obtained in (93d) (6.0 g), sodium methoxide (1.0 g, 5.2 mmol), tetrahydrofuran (5 mL), methanol (25 mL), and acetic acid (0.39 mL, 6.8 mmol) were used to obtain the title compound (1.10 g, yield 92%) as a colorless solid by the same method as in (7f). However, purification was performed by silica gel flash column chromatography (methylene chloride:methanol, 9:1 to 6:1, v/v).
¹H NMR (400 MHz, CD₃OD): δ 1.22 (3H, d, J=6.7 Hz), 1.35 (3H, t, J=7.0 Hz), 3.37-3.38 (2H, m), 3.43-3.50 (2H, m), 3.92 (1H, d, J=14.9 Hz), 3.98 (2H, q, J=7.0Hz), 4.00 (1H, d, J=14.9 Hz), 4.03-4.08 (1H, m), 4.54 (2H, s), 4.91 (1H, d, J=7.4 Hz), 6.76-6.80 (2H, m), 6.76-6.91 (1H, m), 7.01-7.03 (1H, m), 7.12-7.16 (3H, m);
MS (FAB)m/z: 457 (M+Na)⁺.

### (Example 94) 5-Hydroxymethyl-2-(4-methoxybenzyl)-3-methylphenyl 7-deoxy-D-glycero-β-D-gluco-heptopyranoside (Example Compound No. 1-161)

### (94a) 3-Bromo-5-methyl-2-(4-methoxybenzoyl)-cyclohex-2-enone

The compound obtained in (43a) (5.60 g, 22.0 mmol), oxalyl dibromide (3.10 mL, 21.8 mmol), methylene chloride (80 mL), 2-methyl-2-butene (9.4 mL, 88.5 mmol), and dimethylformamide were used to obtain the title compound (4.89 g, yield 69%) as a pale yellow amorphous compound by the same method as in (42b).
¹H NMR (400 MHz, CDCl₃): δ 1.19 (3H, d, J=6.6 Hz), 2.30 (1H, dd, J=16.1 and 12.1 Hz), 2.46 - 2.55 (1H, m), 2.64 (1H, dd, J=16.0 and 4.0 Hz), 2.75 (1H, dd, J=18.4 and 9.7 Hz), 3.05 (1H, dd, J=18.4 and 4.6 Hz), 3.88 (3H, s), 6.95 (2H, d, J=8.6 Hz), 7.84 (2H, d, J=8.6 Hz);
MS (EI)m/z: 322, 324 (M)⁺.

### (94b) (2-Bromo-6-hydroxy-4-methylphenyl)-(4-methoxyphenyl)-methanone

The compound obtained in (94a) (2.27 g, 7.02 mmol), triethylamine (2.94 mL, 21.1 mmol), acetonitrile (30 mL), and trimethylsilane iodide (2.50 mL, 17.6 mmol) were used to obtain a silyl enol ether compound as an oily crude product by the same method as in (43c).

The resulting oily crude product was treated with toluene (30 mL), N-iodosuccinimide (1.58 g, 7.02 mmol), triethylamine (1.27 mL, 9.11 mmol), tetrahydrofuran (60 mL), and 2 N aqueous sodium hydroxide (6 mL) in this order by the same method as in (43c), and the product was passed through a short column to obtain a crude product of the title compound (1.87 g).

### (94c) (6-Acetoxy-2-bromo-4-methylphenyl)-(4-methoxyphenyl)methanone

The crude product obtained in (94b) (1.87 g), methylene chloride (15 mL), pyridine (0.94 mL, 11.6 mmol), acetic anhydride (0.83 mL, 8.78 mmol), and N,N-dimethylaminopyridine (210 mg, 1.72 mmol) were used to obtain the title compound (1.37 g, yield 54%) as an oil by the same method as in (44a).
¹H NMR (400 MHz, CDCl₃): δ 1.96 (3H, s), 2.41 (3H, s), 3.88 (3H, s), 6.93 (2H, d, J=8.8 Hz), 7.00 (1H, s), 7.35 (1H, s), 7.79 (2H, d, J=8.8 Hz)
MS (FAB)m/z: 401,403 (M+K)⁺.

### (94d) [2-Bromo-6-hydroxy-4-(hydroxymethyl)phenyl](4-methoxyphenyl)-methanone

The compound obtained in (94c) (1.37 g, 3.78 mmol), carbon tetrachloride (15 mL), N-bromosuccinimide (710 mg, 3.99 mmol), and 2,2-azobis(isobutyronitrile) (120 mg, 0.73 mmol) were used to obtain a mixture (1.59 g) by the same method as in (44b), and the resulting mixture was used in the subsequent reaction as it was.

Subsequently, the resulting mixture (1.59 g) was treated with dioxane-water (24 mL/8 mL), carbonate calcium (2.16 g, 21.6 mmol), methyl alcohol (20 mL), and 2 N aqueous sodium hydroxide (5 mL) in this order by the same method as in (44b), and the product was finally purified by silica gel flash chromatography (methylene chloride:methyl alcohol 40:1 to 10:1, v/v) to obtain the title compound (710 mg, yield 58%) as a white solid. ¹H NMR (400 MHz, CD₃OD): δ 3.88 (3H, s), 4.58 (2H, s), 6.89 (1H, s), 7.00 (2H, d, J=9.0 Hz), 7.13 (1H, s), 7.77 (2H, d, J=9.0 Hz);
MS (FAB)m/z: 375, 377 (M+K)⁺.

### (94e) (4-Acetoxymethyl-2-bromo-6-hydroxyphenyl)-(4-methoxyphenyl)-methanone

The compound obtained in (94d) (710 mg, 2.11 mmol) was dissolved in tetrahydrofuran (14 mL), and vinyl acetate (7 mL) and bis(dibutylchlorotin)oxide (110 mg, 0.20 mmol) were added to obtain the title compound (420 mg, yield 53%) as an amorphous compound by the same method as in (44c).
¹H NMR (400 MHz, CDCl₃): δ 2.17 (3H, s), 3.90 (3H, s), 5.08 (2H, s), 6.95 (2H, d, J=9.0 Hz), 6.97 (1H, s), 7.17 (1H, s), 7.79 (2H, d, J=9.0 Hz);
MS (FAB)m/z: 379, 381 (M+H)⁺.

### (94f) 3-Bromo-4-(4-methoxybenzyl)-5-(tetrahydropyran-2-yloxy)benzyl acetate

The compound obtained in (94e) (2.37 g, 6.25 mmol) was dissolved in ethanol (24 mL), followed by addition of sodium borohydride (0.24 g, 6.3 mmol) with ice cooling, and the mixture was stirred at room temperature for 1 h. Then, dilute hydrochloric acid (1 mol/L, 15 mL) was added, and the mixture was extracted with ethyl acetate (50 mLx2) and then washed with saturated brine (50 mL). The organic layer was dried over anhydrous magnesium sulfate, and then the solvent was removed under reduced pressure. The resulting mixture (3.22 g) was used in the subsequent reaction as it was.

Subsequently, the resulting mixture (3.22 g) was dissolved in acetonitrile (24 mL), and triethylsilane (2.99 mL, 18.8 mmol) and a boron trifluoride-diethyl ether complex (1.18 mL, 9.39 mmol) were successively added with ice cooling. The mixture was stirred at room temperature for 1 h, followed by addition of saturated aqueous sodium hydrogencarbonate (15 mL), and extracted with ethyl acetate (50 mL x 2). The organic layer was washed with saturated brine (50 mL x 2) and dried over anhydrous magnesium sulfate, and then the solvent was removed under reduced pressure. The resulting mixture (2.29 g) was used in the subsequent reaction as it was.

The resulting mixture (2.01 g) was dissolved in methylene chloride (20 mL), then 3,4-dihydro-2H-pyran (0.75 mL, 8.29 mmol) and pyridinium p-toluenesulfonate (25 mg, 0.10 mmol) were successively added, and the mixture was allowed to stand overnight. Saturated aqueous sodium hydrogencarbonate (15 mL) was added to the reaction mixture, the mixture was extracted with ethyl acetate (100 mL), and the organic layer was washed with saturated brine (50 mL). The organic layer was dried over anhydrous magnesium sulfate, then the solvent was removed under reduced pressure, and the residue was purified by silica gel flash chromatography (hexane:ethyl acetate, 9:1 to 7:3, v/v) to obtain the crude product (2.11 g) as a colorless oil.

### (94g) 4-(4-Methoxybenzyl)-3-methyl-5-(tetrahydropyran-2-yloxy)benzyl acetate

The mixture obtained in (94f) (2.10 g) was dissolved in N,N-dimethylformamide (21 mL), followed by addition of tetrakis(triphenylphosphine)palladium (0.54 g, 0.47 mmol), potassium carbonate (3.23 g), and trimethylboroxin (2.35 g, 18.7 mmol), and the mixture was stirred at 90°C for 3 h. Tetrakis(triphenylphosphine)palladium (0.54 g, 0.47 mmol) was added, and the mixture was stirred at 90°C for 3 h, tetrakis(triphenylphosphine)palladium (1.08 g, 0.935 mmol) and trimethylboroxin (1.36 g, 10.8 mmol) were further added, and the mixture was stirred at 90°C for 3 h. The reaction mixture was cooled to room temperature, followed by addition of ethyl acetate (30 mL) and 10% brine (30 mL), and insoluble matters were removed by Celite filtration. The mixture was extracted with ethyl acetate (50 mL), and the organic layer was washed successively with 10% brine (25 mL) and 5% brine (25 mL) and dried over anhydrous magnesium sulfate. The solvent was removed under reduced pressure, and then the residue was purified by silica gel flash chromatography (hexane:ethyl acetate, 9:1 to 3:1, v/v) to obtain the title compound (1.53 g, yield 85%) as a colorless solid.
¹H NMR (400 MHz, CDCl₃): δ 1.48-1.92 (6H, m), 2.10 (3H, s), 2.26 (3H, s), 3.54-3.58 (1H, m), 3.71-3.74 (1H, m), 3.76 (3H, s), 3.98 (1H, d, J=14.9 Hz), 4.04 (1H, d, J=14.9 Hz), 5.02 (1H, d, J=12.1 Hz), 5.06 (1H, d, J=12.1 Hz), 5.42 (1H, t, J=2.9 Hz), 6.76-6.79 (2H, m), 6.84 (1H, brs), 7.02-7.07 (3H, m);
MS (FAB)m/z: 423 (M+K)⁺.

### (94h) 5-Hydroxy-4-(4-methoxybenzyl)-3-methylbenzyl acetate

The compound obtained in (94g) (1.50 g, 3.90 mmol) was dissolved in methanol (20 mL), methylene chloride (10 mL), and ethyl acetate (10 mL), followed by addition of pyridinium p-toluenesulfonate (100 mg, 0.40 mmol). The mixture was stirred at 30°C for 2 h and allowed to stand overnight at room temperature. Water (60 mL) was added, and the mixture was extracted with ethyl acetate (50 mL x 2) and then washed with saturated brine (30 mL). The organic layer was dried over anhydrous magnesium sulfate, then the solvent was removed under reduced pressure, and the residue was purified by silica gel flash chromatography (hexane:ethyl acetate, 4:1 to 7:3, v/v) to obtain the title compound (850 mg, yield 73%) as a colorless oil.
¹H NMR (400 MHz, CDCl₃): δ 2.11 (3H, s), 2.28 (3H, s), 3.76 (3H, s), 3.98 (2H, s), 4.98 (1H, s), 5.02 (2H, s), 6.70 (1H, brs), 6.79-6.82 (3H, m), 7.07-7.09 (2H, m);
MS (FAB)m/z: 300 (M)⁺.

### (94i) 5-Acetoxymethyl-2-(4-methoxybenzyl)-3-methylphenyl 2,3,4,6-tetra-O-benzoyl-7-deoxy-D-glycero-β-D-gluco-heptopyranoside

The compound obtained in (7d) (2.22 g, 3.64 mmol), trichloroacetonitrile (1.5 mL, mmol), 1,8-diazabicyclo[5.4.0]-7-undecene (55 µL, 0.36 mmol), and methylene chloride (44 mL) were used to prepare an imidate by the same method as in (1b). Subsequently, the compound obtained in (94h) (840 mg, 0.280 mmol), a boron trifluoride-diethyl ether complex (0.43 mL, 3.4 mmol), and methylene chloride (22 mL) were used to obtain a crude product of the title compound (3.6 g) by the same method as in (1b), and the resulting crude product was used in the subsequent reaction as it was.

### (94j) 5-Hydroxymethyl-2-(4-methoxybenzyl)-3-methylphenyl 7-deoxy-D-glycero-β-D-gluco-heptopyranoside

The crude product obtained in (94i) (3.6 g), sodium methoxide (1.08 g, 5.60 mmol), tetrahydrofuran (4.4 mL), and methanol (22 mL) were used to obtain the title compound (970 mg, yield 80%) as a colorless solid by the same method as in (7f). However, purification was performed by silica gel flash column chromatography (methylene chloride:methanol, 7:1 to 4:1, v/v).
¹H NMR (400 MHz, CD₃OD): δ 1.23 (3H, d, J=6.2 Hz), 2.19 (3H, s), 3.35-3.37 (2H, m), 3.42-3.44 (2H, m), 3.72 (3H, s), 3.97 (1H, d, J=14.8 Hz), 4.03-4.08 (1H, m), 4.14 (1H, d, J=14.8 Hz), 4.53 (2H, s), 4.90 (1H, d, J=7.8 Hz), 6.73-6.76 (2H, m), 6.86 (1H, brs), 7.04-7.07 (3H, m);
MS (FAB)m/z: 473 (M+K)⁺.

### (Example 95) 3-Chloro-2-(4-cyclopropoxybenzyl)-5-hydroxymethyl-phenyl 7-deoxy-D-glycero-β-D-gluco-heptopyranoside (Example Compound No. 1-162)

### (95a) 2-(4-Cyclopropoxybenzoyl)-3-hydroxy-5-methyl-cyclohex-2-enone

Oxalyl chloride (0.67 mL, 7.71 mmol) and a catalytic amount of N,N-dimethylformamide were added to a solution of 4-cyclopropoxybenzoate (US4009208) (1.37 g, 7.69 mmol) in tetrahydrofuran (12 mL), and the mixture was stirred at room temperature for 1 h. The solvent was removed under reduced pressure, and a crude product of the resulting 4-cyclopropoxybenzoylchloride (1.51 g) was used in the subsequent reaction as it was.

5-Methyl-1,3-cyclohexanedione (1.00 g, 7.93 mmol), triethylamine (3.31 mL, 23.7 mmol), 4-cyclopropoxybenzoylchloride (1.51 g, 7.68 mmol), acetonitrile (12 mL), and trimethyl cyanonitrile (0.106 mL, 1.20 mmol) were used to obtain a crude product of the title compound (2.31 g) by the same method as in (42a).

### (95b) 3-Chloro-5-methyl-2-(4-cyclopropoxybenzoyl)-cyclohex-2-enone

The crude product obtained in (95a) (2.31 g), methylene chloride (15 mL), oxalyl chloride (0.71 mL, 8.28 mmol), and a catalytic amount ofN,N-dimethylformamide were used to obtain the title compound (1.98 g, yield 82%) as an oil by the same method as in (46a).
¹H NMR (400 MHz, CDCl₃): δ 0.79 - 0.84 (4H, m), 1.19 (3H, d, J = 6.3 Hz), 2.29 (1H, dd, J = 16.0 and 12.1 Hz), 2.45 - 2.53 (1H, m), 2.60 - 2.67 (2H, m), 2.87 (1H, dd, J = 18.4 and 4.7 Hz), 3.78 - 3.83 (1H, m), 7.10 (2H, d, J = 9.0 Hz), 7.82 (2H, d, J = 9.0Hz);
MS (EI)m/z: 304, 306 (M)⁺.

### (95c) (2-Chloro-6-hydroxy-4-methyl-phenyl)-(4-cyclopropoxyphenyl)-methanone

The compound obtained in (95b) (1.98 g, 6.50 mmol), triethylamine (2.75 mL, 19.5 mmol), acetonitrile (30 mL), and trimethylsilane iodide (2.30 mL, 16.2 mmol) were used to obtain a silyl enol ether compound as an oily crude product by the same method as in (43c).

The resulting oily crude product was treated with toluene (20 mL), N-iodosuccinimide(1.46 g, 6.49 mmol), triethylamine (1.19 mL, 8.42 mmol), tetrahydrofuran (30 mL), and 2 N aqueous sodium hydroxide (2 mL) in this order by the same method as in (43c), and the product was finally purified by silica gel flash chromatography (hexane:ethyl acetate 10:1 1 to 3:1, v/v) to obtain the title compound (870 mg, yield 44%) as an amorphous compound.
¹H NMR (400 MHz, CDCl₃): δ 0.82- 0.85 (4H, m), 2.35 (3H, s), 3.79 - 3.84 (1H, m), 6.79 (1H, s), 6.80 (1H, s), 7.08 (2H, d, J = 8.9 Hz), 7.74 (2H, d, J = 8.9 Hz), 8.73 (1H, s);
MS (EI)m/z: 302, 304 (M)⁺.

### (95d) (6-Acetoxy-2-chloro-4-methyl-phenyl)-(4-cyclopropoxyphenyl)-methanone

The compound obtained in (95c) (870 mg, 2.87 mmol), methylene chloride (10 mL), pyridine (0.46 mL, 5.69 mmol), acetic anhydride (0.40 mL, 4.23 mmol), and N,N-dimethylaminopyridine (110 mg, 0.90 mmol) were used to obtain the title compound (807 mg, yield 81 %) as an oil by the same method as in (44a).
¹H NMR (400 MHz, CDCl₃): δ 0.80 - 0.84 (4H, m), 1.98 (3H, s), 2.41 (3H, s), 3.78 - 3.82 (1H, m), 6.96 (1H, s), 7.08 (2H, d, J = 9.0 Hz), 7.17 (1H, s), 7.78 (2H, d, J = 9.0 Hz);
MS (EI)m/z: 344, 346 (M)⁺.

### (95e) (2-Chloro-6-hydroxy-4-hydroxymethyl-phenyl)-(4-cyclopropoxyphenyl)-methanone

The compound obtained in (95d) (807 mg, 2.34 mmol), carbon tetrachloride (10 mL), N-bromosuccinimide (437 mg, 2.46 mmol), and 2,2-azobis(isobutyronitrile) (77 mg, 0.47 mmol) were used to obtain a mixture (990 mg) by the same method as in (44b), and the resulting mixture was used in the subsequent reaction as it was.

Subsequently, the resulting mixture (990 mg) was treated with dioxane-water (12 mL/4 mL), carbonate calcium (1.40 g, 14.0 mmol), methyl alcohol (10 mL), and 2 N aqueous sodium hydroxide (2 mL) in this order by the same method as in (44b), and the product was finally purified by silica gel flash chromatography (methylene chloride:methyl alcohol 40:1 to 10:1, v/v) to obtain the title compound (130 mg, yield 17%) as an amorphous compound.
¹H NMR (400 MHz, CDCl₃): δ 0.81- 0.85 (4H, m), 3.79 - 3.84 (1H, m), 4.71 (2H, d, J = 4.3Hz), 6.96 (1H, s), 7.01 (1H, s), 7.09 (2H, d, J = 8.8 Hz), 7.76 (2H, d, J = 8.8 Hz);
MS (FAB)m/z: 319, 321 (M+H)⁺.

### (95f) (4-Acetoxymethyl-2-chloro-6-hydroxy-phenyl)-(4-cyclopropoxyphenyl)-methanone

The compound obtained in (95e) (130 mg, 0.41 mmol) was dissolved in tetrahydrofuran (2 mL), and vinyl acetate (2 mL) and bis(dibutylchlorotin)oxide (135 mg, 0.24 mmol) were used to obtain a crude product of the title compound by the same method as in (44c). The resulting crude product was passed through a short column and used in the subsequent reaction as it was.

### (95g) 5-Acetoxymethyl-3-chloro-2{hydroxy-(4-cyclopropoxyphenyl)methyl}phenol

The crude product obtained in (95f) (110 mg), methanol (2 mL), and sodium borohydride (35 mg, 0.93 mmol) were used to obtain a crude product of the title compound (110 mg) by the same method as in (44d). The resulting crude product was used in the subsequent reaction as it was without purification.

### (95h) 5-Acetoxymethyl-3-chloro-2-(4-cyclopropoxybenzyl)-phenol

The crude product obtained in (95g) (110 mg), acetonitrile (2 mL), triethylsilane (0.145 mL, 0.91 mmol), and a boron fluoride-diethyl ether complex (0.057 mL, 0.45 mmol) were used to obtain the title compound (82 mg, yield %) as a white solid by the same method as in (42e).
¹H NMR (400 MHz, CDCl₃): δ 0.74 - 0.75 (4H, m), 2.12 (3H, s), 3.67 - 3.71 (1H, m), 4.12 (2H, s), 4.99 (1H, s), 5.00 (2H, s), 6.73 (1H, s), 6.97 (2H, d, J = 8.8 Hz), 7.03 (1H, s), 7.19 (2H, d, J = 8.8 Hz);
MS (FAB)m/z: 346, 348(M)⁺.

### (95i) 5-Acetoxymethyl-3-chloro-2-(4-cyclopropoxybenzyl)phenyl 7-deoxy-D-glycero-β-D-gluco-heptopyranoside

The compound obtained in (7d) (217 mg, 0.36 mmol), trichloroacetonitrile (0.179 mL, 1.77 mmol), 1,8-diazabicyclo[5.4.0]-7-undecene (53 µL, 0.036 mmol), and methylene chloride (5 mL) were used to prepare an imidate by the same method as in (1b). Subsequently, the compound obtained in (95h) (82 mg, 0.24 mmol), a boron trifluoride-diethyl ether complex (0.045 mL, 0.36 mmol), and methylene chloride (5 mL) were used to obtain a crude product of the title compound (300 mg) by the same method as in (1b), and the resulting crude product was used in the subsequent reaction as it was.

### (95j) 3-Chloro-2-(4-cyclopropoxybenzyl)-5-hydroxymethyl-phenyl 7-deoxy-D-glycero-β-D-gluco-heptopyranoside

The crude product obtained in (95i) (300 mg), potassium carbonate (490 mg, 3.55 mmol), methanol (8 mL), and methylene chloride (2 mL) were used to obtain the title compound (65 mg, yield 57%) as a colorless solid by the same method as in (1c).
¹H NMR (400 MHz, CD₃OD): δ 0.63 - 0.64 (2H, m), 0.71 - 0.74 (2H, m), 1.21 (3H, d, J = 6.6 Hz), 3.36 - 3.38 (2H, m), 3.44 - 3.47 (2H, m), 3.68 - 3.72 (1H, m), 4.05 - 4.10 (2H, m), 4.21 (1H, d, J = 14.1 Hz), 4.55 (2H, s), 4.94 (1H, d, J = 7.4 Hz), 6.87 (2H, d, J = 8.6 Hz), 7.10 (1H, s), 7.12 (1H, s), 7.17 (2H, d, J = 8.6 Hz);
MS (FAB)m/z: 503, 505(M+Na)⁺.

### (Example 96) 2-(4-Cyclopropoxybenzyl)-5-hydroxymethyl-3-methyl-phenyl 7-deoxy-D-glycero-β-D-gluco-heptopyranoside (Example Compound No. 1-163)

### (96a) Ethyl 3-bromo-4-(4-cyclopropoxybenzoyl)-5-oxo-cyclohex-3-enecarboxylate

The crude product obtained in (63a) (2.95 g, 8.57 mmol), methylene chloride (30 mL), oxalyl dibromide (1.22 mL, 8.59 mmol), 2-methyl-2-butene (3.64 mL, 34.3 mmol), and a catalytic amount of dimethylformamide were used to obtain the title compound (2.40 g, yield 69%) as a yellow amorphous compound by the same method as in (42b).
¹H NMR (400 MHz, CDCl₃): δ 0.80 - 0.85 (4H, m), 1.31 (3H, t, J = 7.1 Hz), 2.83 - 2.84 (2H, m), 3.27 - 3.30 (3H, m), 3.78 - 3.83 (1H, m), 4.25 (2H, q, J = 7.1Hz), 7.10 (2H, d, J = 9.0 Hz), 7.82 (2H, d, J = 9.0 Hz);
MS (FAB)m/z: 445, 447(M+K)⁺.

### (96b) Ethyl 4-(4-cyclopropoxybenzoyl)-5-methyl-3-oxo-cyclohex-3-enecarboxylate

The compound obtained in (96a) (2.40 g, 5.89 mmol), copper(I) thiophenolate (2.04 g, 11.8 mmol), methyllithium (1.13 M diethyl ether solution) (10.4 mL, 11.8 mmol), and tetrahydrofuran (60 mL) were used to obtain the title compound (1.83 g, yield 91%) as an oil by the same method as in (89b). ¹H NMR (500 MHz, CDCl₃): δ 0.79 - 0.84 (4H, m), 1.30 (3H, t, J = 7.2 Hz), 1.90 (3H, s), 2.69 - 2.83 (4H, m), 3.17 - 3.25 (1H, m), 3.77 - 3.82 (1H, m), 4.23 (2H, q, J = 7.3Hz), 7.08 (2H, d, J = 9.0Hz), 7.80 (2H, d, J = 9.0 Hz);
MS (FAB)m/z: 343(M)⁺.

### (96c) Ethyl 4-(4-cyclopropoxybenzoyl)-3-hydroxy-5-methyl-benzoate

The compound obtained in (96b) (1.83 g, 5.34 mmol), triethylamine (2.27 mL, 16.1 mmol), acetonitrile (25 mL), and trimethylsilane iodide (1.90 mL, 13.4 mmol) were used to obtain a silyl enol ether compound as an oily crude product by the same method as in (43c).

The resulting oily crude product was treated with toluene (15 mL), N-iodosuccinimide (1.20 g, 5.33 mmol), and triethylamine (0.98 mL, 6.93 mmol) in this order by the same method as in (43c). Saturated aqueous sodium sulfite (40 mL) was added, and the mixture was extracted with ethyl acetate (50 mL) and then washed with saturated brine (50 mL). The organic layer was dried over anhydrous sodium sulfate, and then the solvent was removed under reduced pressure. The residue was dissolved in ethanol (15 mL), followed by addition of potassium carbonate (740 mg, 5.35 mmol), and the mixture was stirred at 50°C for 1 h. The solvent was removed under reduced pressure, followed by addition of ethyl acetate (50 mL) and saturated aqueous ammonium chloride (50 mL), and then the mixture was extracted to washed with saturated brine (50 mL). The organic layer was dried over anhydrous sodium sulfate, and then the solvent was removed under reduced pressure. The residue was passed through a short column to obtain a crude product of the title compound (551 mg).

### (96d) Ethyl 4-[(4-cyclopropoxyphenyl)-hydroxy-methyl]-3-hydroxy-5-methylbenzoate

The compound obtained in (96c) (551 mg, 1.62 mmol), methanol (15 mL), and sodium borohydride (122 mg, 3.22 mmol) were used to obtain the title compound (286 mg, yield 52%) as an amorphous compound by the same method as in (42d). However, purification was performed by silica gel flash chromatography (hexane:ethyl acetate, 10:1 to 1:1, v/v).
¹H NMR (500 MHz, CDCl₃): δ 0.75 - 0.78 (4H, m), 1.39 (3H, t, J = 7.2 Hz), 2.19 (3H, s), 2.81 (1H, d, J = 2.5 Hz), 3.69 - 3.73 (1H, m), 4.36 (2H, q, J = 7.2 Hz), 6.19 (1H, d, J = 2.5 Hz), 7.02 (2H, d, J = 8.8 Hz), 7.29 (2H, d, J = 8.8 Hz), 7.37 (1H, s), 7.48 (1H, s), 8.97 (1H, s);
MS (FAB)m/z: 343(M)⁺.

### (96e) 5-Acetoxymethyl-2-(4-cyclopropoxybenzyl)-3-methylphenol

The compound obtained in (96d) (286 mg, 0.84 mmol), acetonitrile (5 mL), triethylsilane (0.40 mL, 2.51 mmol), and a boron fluoride-diethyl ether complex (0.157 mL, 1.25 mmol) were used to obtain a crude product of a phenol compound (254 mg) as a solid by the same method as in (42e). However, the crude product was used in the subsequent reaction as it was without purification.

The resulting crude product (254 mg, 0.78 mmol), lithium aluminium hydride (89 mg, 2.35 mmol), and tetrahydrofuran (4.5 mL) were used to obtain a crude product of a diol compound (221 mg) as a solid by the same method as in (47d). However, the crude product was used in the subsequent reaction as it was without purification.

The resulting crude product (221 mg, 0.79 mmol) was dissolved in tetrahydrofuran (3 mL), and vinyl acetate (3 mL) and bis(dibutylchlorotin)oxide (215 mg, 0.39 mmol) were added to obtain the title compound (230 mg, yield 84%) as a white solid by the same method as in (44c).
¹H NMR (400 MHz, CDCl₃): δ 0.73 - 0.75 (4H, m), 2.11 (3H, s), 2.29 (3H, s), 3.66 - 3.71 (1H, m), 3.98 (2H, s), 4.74 (1H, s), 5.02 (2H, s), 6.71 (1H, s), 6.80 (1H, s), 6.95 (2H, d, J = 8.4 Hz), 7.08 (2H, d, J = 8.4Hz);
MS (FAB)m/z: 326(M)⁺.

### (96f) 5-Acetoxymethyl-2-(4-cyclopropoxybenzyl)-3-methyl-phenyl 7-deoxy-D-glycero-β-D-gluco-heptopyranoside

The compound obtained in (7d) (645 mg, 1.06 mmol), trichloroacetonitrile (0.530 mL, 5.24 mmol), 1,8-diazabicyclo[5.4.0]-7-undecene (0.016 mL, 0.11 mmol), and methylene chloride (15 mL) were used to prepare an imidate by the same method as in (1b). Subsequently, the compound obtained in (96e) (230 mg, 0.70 mmol), a boron trifluoride-diethyl ether complex (0.133 mL, 1.06 mmol), and methylene chloride (15 mL) were used to obtain a crude product of the title compound (1.00 g) by the same method as in (1b), and the resulting crude product was used in the subsequent reaction as it was.

### (96g) 2-(4-Cyclopropoxybenzyl)-5-hydroxymethyl-3-methyl-phenyl 7-deoxy-D-glycero-β-D-gluco-heptopyranoside

The crude product obtained in (96f) (1.00 g), potassium carbonate (1.40 g, 10.1 mmol), methanol (24 mL), and methylene chloride (6 mL) were used to obtain the title compound (204 mg, yield 63%) as a white solid by the same method as in (1c).
¹H NMR (400 MHz, CD₃OD): δ 0.61 - 0.64 (2H, m),0.70 - 0.75 (2H, m), 1.23 (3H, d, J = 6.2 Hz), 2.20 (3H, s), 3.35 - 3.37 (2H, m), 3.42 - 3.44 (2H, m), 3.67 - 3.72 (1H, m), 3.97 (1H, d, J = 14.8 Hz), 4.05 - 4.07 (1H, m), 4.15 (1H, d, J = 14.8 Hz), 4.53 (2H, s), 4.91 (1H, d, J = 7.5 Hz), 6.85 (1H, s), 6.87 (2H, d, J = 8.8 Hz), 7.05 (2H, d, J = 8.8 Hz), 7.07 (1H, s);
MS (FAB)m/z: 483 (M+Na)⁺.

### (Example 97) 3-Fluoro-5-hydroxymethyl-2-(4-methylbenzyl)phenyl 7-deoxy-D-glycero-β-D-gluco-heptopyranoside (Example Compound No. 1-164)

### (97a) Ethyl 4-(4-methylbenzoyl)-3-hydroxy-5-oxo-cyclohex-3-enecarboxylate

Ethyl-3-hydroxy-5-oxo-cyclohex-3-enecarboxylate (EP1571148A1) (1.50 g, 8.14 mmol), triethylamine (3.40 mL, 24.4 mmol), 4-methylbenzoylchloride (1.32 g, 8.54 mmol), acetonitrile (14 mL), and trimethyl cyanonitrile (0.13 mL, 0.97 mmol) were used to obtain a crude product of the title compound (2.71 g) by the same method as in (42a).

### (97b) Ethyl 4-(4-methylbenzoyl)-3-fluoro-5-oxo-cyclohex-3-enecarboxylate

The crude product obtained in (97a) (2.71 g), methylene chloride (25 mL), and diethylaminosulfur trifluoride (3.20 mL, 24.4 mmol) were used to obtain an oily title compound (1.61 g, yield 65%) by the same method as in (46a), and the resulting oily title compound was used in the subsequent step as it was.

### (97c) Ethyl 4-(4-methylbenzoyl)-3-fluoro-5-hydroxybenzoate

The compound obtained in (97b) (1.60 g, 5.26 mmol), triethylamine (2.30 mL, 16.5 mmol), acetonitrile (20 mL), trimethylsilane iodide (2.76 g, 13.8 mmol), toluene (16 mL), silica gel (SK-85) (6.4 g), ethylalcohol (10 mL), and potassium carbonate (0.15 g, 1.09 mmol) were used to obtain a crude product of the title compound (0.94 g, yield 59%) by the same method as in (47c).

### (97d) 3-Fluoro-5-hydroxymethyl-2-{(4-methylphenyl)hydroxymethyl}phenol

The compound obtained in (97c) (0.92 g, 3.04 mmol), lithium aluminium hydride (300 mg, 7.91 mmol), and tetrahydrofuran (16 mL) were used to obtain a crude product (0.80 g) by the same method as in (47d), and the resulting crude product was used in the subsequent reaction as it was.

### (97e) 5-Acetoxymethyl-3-fluoro-2-{(4-methylphenyl)hydroxymethyl}phenol

The crude product obtained in (97d) (0.80 g, 3.05 mmol), tetrahydrofuran (8.0 mL), vinyl acetate (8.0 mL), and bis(dibutylchlorotin)oxide (0.34 g, 0.62 mmol) were used to obtain the title compound (835 mg, yield 90%) as a colorless solid by the same method as in (44c). However, purification was performed by silica gel flash chromatography (hexane:ethyl acetate, 4:1 1 to 3:1, v/v).
¹H NMR (400 MHz, CDCl₃): δ 2.11 (3H, s), 2.11 (3H, s), 2.89 (1H, d, J=2.7 Hz), 5.00 (2H, s), 6.31 (1H, d, J=2.7 Hz), 6.56 (1H, dd, J=10.6 Hz, 1.6 Hz), 6.69 (1H, s), 7.17 (2H, d, J= 7.8Hz), 7.33 (2H, d, J= 7.8Hz), 8.83 (1H, s);
MS (FAB)m/z: 327 (M+Na)⁺.

### (97f) 5-Acetoxymethyl-3-fluoro-2-(4-methylbenzyl)phenol

The compound obtained in (97e) (396 mg, 1.30 mmol), acetonitrile (6 mL), triethylsilane (0.62 mL, 3.89 mmol), and a boron fluoride-diethyl ether complex (0.25 mL, 1.97 mmol) were used to obtain the title compound (239 mg, yield 64%) as a colorless solid by the same method as in (42e).
¹H NMR (500 MHz, CDCl₃): δ 2.10 (3H, s), 2.30 (3H, s), 3.97 (2H, s), 5.00 (2H, s), 5.12 (1H, s), 6.59 (1H, s), 6.67-6.70 (1H, m), 7.08 (2H, d, J=7.8 Hz), 7.15 (2H, d, J=7.8 Hz);
MS (FAB)m/z: 288 (M)⁺.

### (97g) 3-Fluoro-5-hydroxymethyl-2-(4-methylbenzyl)phenyl 7-deoxy-D-glycero-β-D-gluco-heptopyranoside

The compound obtained in (7d) (0.51 g, 0.835 mmol), trichloroacetonitrile (0.33 mL, 3.29 mmol), 1,8-diazabicyclo[5.4.0]-7-undecene (0.015 mL, 0.10 mmol), and methylene chloride (5 mL) were used to prepare an imidate by the same method as in (1b). Subsequently, the compound obtained in (97f) (200 mg, 0.694 mmol), a boron trifluoride-diethyl ether complex (0.045 mL, 0.36 mmol), and methylene chloride (5 mL) were used to obtain a crude product of the title compound by the same method as in (1b), and the resulting crude product was used in the subsequent reaction as it was.

The above-mentioned crude product, sodium methoxide (0.27 g, 1.40 mmol), methanol (6 mL), and tetrahydrofuran (1.5 mL) were used to obtain the title compound (227 mg, yield 78%) as a colorless solid by the same method as in (7f). However, purification was performed by silica gel flash chromatography (methylene chloride:methanol 85:15 to 80:20, v/v).
¹H NMR (400 MHz, CD₃OD): δ 1.21 (3H, d, J=6.7 Hz), 2.24 (3H, s), 3.36-3.40 (2H, m), 3.42-3.50 (2H, m), 3.94 (1H, d, J=14.5 Hz), 4.04 (1H, d, J=14.5 Hz), 4.04-4.08 (1H, m), 4.55 (2H, s), 4.92 (1H, d, J=7.4 Hz), 6.78 (1H, d, J=10.2 Hz), 6.98 (1H, s), 7.00 (2H, d, J=7.8Hz), 7.14 (2H, d, J=7.8 Hz);
MS (FAB)m/z: 423 (M+H)⁺.

### (Example 98) 5-Hydroxymethyl-2-(4-methylbenzyl)phenyl 7-deoxy-D-glycero-β-D-gluco-heptapyranoside (Example Compound No. 1-165)

### (98a) Ethyl 3-hydroxy-4-(4-methylbenzyl)benzoate

Ethyl 4-formyl-3-hydroxybenzoate (1.00 g, 5.15 mmol), 4-bromotoluene (3.52 g, 20.6 mmol), metal magnesium (0.50 g, 20.6 mmol), and tetrahydrofuran (22 mL) were used to obtain a crude product (1.52 g) by the same method as in (93a), and the resulting crude product was used in the subsequent reaction as it was.

The above-mentioned crude product (1.52 g), 10% palladium on carbon (0.50 g), 2 M hydrochloric acid (0.26 mL), and methanol (30 mL) were used to obtain the title compound (1.28 g, yield 92%) by the same method as in (93b). However, purification was performed by silica gel flash chromatography (hexane:ethyl acetate, 85:15, v/v).
¹H NMR (400 MHz, CD₃OD): δ 1.36 (3H, t, J= 7.2 Hz), 2.28 (3H, s), 3.92 (2H, s), 4.31 (2H, q, J=7.2 Hz), 7.04-7.11 (5H, m), 7.39 (1H, dd, J=7.8 Hz, 2.0 Hz), 7.43 (1H, d, J=2.0 Hz);
MS (FAB)m/z: 271 (M+H)⁺.

### (98b) 5-Acetoxymethyl-2-(4-methylbenzyl)phenol

The compound obtained in (98a) (1.27 g, 4.70 mmol), lithium aluminium hydride (0.44 g, 11.6 mmol), and tetrahydrofuran (24 mL) were used to obtain a crude product (1.11 g) by the same method as in (93b), and the resulting crude product (1.11 g) was used in the subsequent reaction as it was.

The above-mentioned crude product (1.11 g), tetrahydrofuran (5.5 mL), vinyl acetate (5.5 mL), and bis(dibutylchlorotin)oxide (0.78 g, 1.41 mmol) were used to obtain the title compound (1.26 g, yield 99%) as a colorless solid by the same method as in (8b). However, purification was performed by silica gel flash column chromatography (hexane:ethyl acetate, 85:15, v/v).
¹H NMR (400 MHz, CDCl₃): δ 2.10 (3H, s), 2.31 (3H, s), 3.95 (2H, s), 4.92 (1H, s), 5.04 (2H, s), 6.80-6.81 (1H, m), 6.86-6.89 (1H, m), 7.09-7.12 (5H, m);
MS (FAB)m/z: 270 (M)⁺.

### (98c) 5-Hydroxymethyl-2-(4-methylbenzyl)phenyl 7-deoxy-D-glycero-β-D-gluco-heptapyranoside

The compound obtained in (7d) (0.39 g, 0.643 mmol), trichloroacetonitrile (0.26 mL, 2.59 mmol), 1,8-diazabicyclo[5.4.0]-7-undecene (10 µL, 0.067 mmol), and methylene chloride (4 mL) were used to prepare an imidate by the same method as in (1b). Subsequently, the compound obtained in (98b) (145 mg, 0.536 mmol), a boron trifluoride-diethyl ether complex (0.035 mL, 0.28 mmol), and methylene chloride (6 mL) were used to obtain a crude product of the title compound by the same method as in (1b), and the resulting crude product was used in the subsequent reaction as it was.

The above-mentioned crude product, sodium methoxide (0.21 g, 1.09 mmol), tetrahydrofuran (6 mL), and methanol (1.5 mL) were used to obtain the title compound (170 mg, yield 79%) as a colorless solid by the same method as in (7f). However, purification was performed by silica gel flash column chromatography (methylene chloride:methanol, 85:15, v/v).
¹H NMR (500 MHz, CD₃OD): δ 1.22 (3H, d, J=6.3 Hz), 2.26 (3H, s), 3.34-3.39 (2H, m), 3.34-3.39 (2H, m), 3.94 (1H, d, J=14.6 Hz), 4.02 (1H, d, J=14.6 Hz), 4.04-4.07 (1H, m), 4.53 (2H, s), 4.90 (1H, d, J=6.8 Hz), 6.91 (1H, d, J=7.8 Hz), 7.01 (1H, d, J=7.8 Hz), 7.03 (2H, d, J=7.8 Hz), 7.09 (2H, d, J=7.8 Hz), 7.13 (1H, s);
MS (FAB)m/z: 427 (M+Na)⁺.

### (Example 99) 5-Hydroxyacetyloxymethyl-2-(4-ethoxybenzyl)phenyl 7-deoxy-D-glycero-β-D-gluco-heptopyranoside (Example Compound No. 1-166)

### (99a) 5-Allyloxycarbonyloxyacetyloxymethyl-2-(4-ethoxybenzyl)phenyl 7-deoxy-D-glycero-β-D-gluco-heptopyranoside

5-Hydroxymethyl-2-(4-ethoxybenzyl)phenyl 7-deoxy-D-glycero-β-D-gluco-heptopyranoside obtained in (93e) (0.69 g, 1.59 mmol), 2,4,6-trimethylpyridine (10 mL), allyloxycarbonyloxyacetyl chloride (0.64 g, 3.28 mmol), and methylene chloride (14 mL) were used to obtain the title compound (0.55 g, yield 60%) as a white powder by the same method as in (34a).
¹H NMR (400 MHz, CD₃OD): δ 1.23 (3H, d, J=6.3 Hz), 1.35 (3H, t, J=7.0 Hz), 3.39-3.41 (2H, m), 3.47-3.49 (2H, m), 3.91-4.06 (5H, m), 4.63-4.65 (2H, m), 4.71 (2H, d, J=0.8 Hz), 4.93 (1H, d, J=7.4 Hz), 5.12-5.25 (3H, m), 5.32-5.37 (1H, m), 5.88-5.98 (1H, m), 6.79 (2H, d, J=9.0 Hz), 6.94 (1H, dd, J=7.7 and 1.5 Hz), 7.05 (1H, d, J=7.7 Hz), 7.13-7.16 (3H, m);
MS (FAB)m/z: 577 (M+H)⁺.

### (99b) 5-Hydroxyacetyloxymethyl-2-(4-ethoxybenzyl)phenyl 7-deoxy-D-glycero-β-D-gluco-heptopyranoside

The compound obtained in (99a) (0.55 g, 0.96 mmol) was dissolved in tetrahydrofuran (10 mL), followed by addition of triphenylphosphine (0.11 g, 0.42 mmol), dimedone (0.08 g, 0.57 mmol), and tetrakis triphenylphosphine palladium (0.11 g, 0.095 mmol), and the mixture was stirred at room temperature for 1 h. Methylene chloride was added to the reaction mixture, and the mixture was purified by silica gel column chromatography (methylene chloride to methylene chloride:ethanol, 20:1 to 10:1 to 6:1 to 5:1, v/v) to obtain a pale brown solid. Methylene chloride and hexane were added to the resulting compound, the mixture was sonicated, and insoluble matters were removed by filtration to obtain the title compound (0.38 g, yield 81%) as a white powder.
¹H NMR (400 MHz, CD₃OD): δ 1.22 (3H, d, J=6.7 Hz), 1.35 (3H, t, J=7.1 Hz), 3.37-3.39 (2H, m), 3.46-3.49 (2H, m), 4.08-4.14 (5H, m), 4.14 (2H, s), 4.90 (1H, d, J=7.4 Hz), 5.14 (2H, s), 6.79 (2H, d, J=8.8 Hz), 6.95 (1H, dd, J=7.7 and 1.4 Hz), 7.04 (1H, d, J=7.7 Hz), 7.13 (2H, d, J=8.8 Hz), 7.17 (1H, d, J=1.4 Hz);
MS (FAB)m/z: 492 (M)⁺.

### (Example 100) 5-Hydroxyacetyloxymethyl-2-(2-fluoro-4-methoxybenzyl)phenyl 7-deoxy-D-glycero-β-D-gluco-heptopyranoside (Example Compound No. 1-167)

### (100a) 5-Allyloxycarbonyloxyacetyloxymethyl-2-(2-fluoro-4-methoxybenzyl)phenyl 7-deoxy-D-glycero-β-D-gluco-heptopyranoside

5-Hydroxymethyl-2-(2-fluoro-4-methoxybenzyl)phenyl 7-deoxy-D-glycero-β-D-gluco-heptopyranoside obtained in (82d) (0.20 g, 0.46 mmol), 2,4,6-trimethylpyridine (2 mL), methylene chloride (3 mL), and allyloxycarbonyloxyacetyl chloride (0.19 g, 1.37 mmol) were used to obtain the title compound (0.12 g, yield 45%) as a white powder by the same method as in (34a).
¹H NMR (400 MHz, CD₃OD): δ 1.22 (3H, d, J=6.7 Hz), 3.38-3.41 (2H, m), 3.44-3.50 (2H, m), 3.76 (3H, s), 3.94-4.08 (3H, m), 4.63-4.65 (2H, m), 4.71 (2H, s), 4.95 (1H, d, J=7.0 Hz), 5.13-5.24 (3H, m), 5.32-5.37 (1H, m), 5.88-5.99 (1H, m), 6.63-6.66 (2H, m), 6.92-7.00 (2H, m), 7.12-7.16 (2H, m).

### (100b) 5-Hydroxyacetyloxymethyl-2-(2-fluoro-4-methoxybenzyl)phenyl 7-deoxy-D-glycero-β-D-gluco-heptopyranoside

The compound obtained in (100a) (0.11 g, 0.19 mmol), tetrahydrofuran (2 mL), triphenylphosphine (27 mg, 0.10 mmol), dimedone (18 mg, 0.13 mmol), and tetrakis triphenylphosphine palladium (24 mg, 0.0 mmol) were used to obtain the title compound (69 mg, yield 73%) as a white powder by the same method as in (99b).
¹H NMR (400 MHz, CD₃OD): δ 1.23 (3H, d, J=6.3 Hz), 3.37-3.39 (2H, m), 3.48-3.53 (2H, m), 3.77 (3H, s), 3.95-4.10 (3H, m), 4.15 (2H, s), 4.93 (1H, d, J=6.3 Hz), 5.15 (2H, s), 6.63-6.66 (2H, m), 6.94-7.00 (2H, m), 7.13-7.18 (2H, m);
MS (FAB)m/z: 496 (M)⁺.

### (Example 101) 5-Hydroxymethyl-2-(4-methoxybenzyl)-3-vinyl-phenyl 7-deoxy-D-glycero-β-D-gluco-heptopyranoside (Example Compound No. 1-168)

### (101a) 5-Acetoxymethyl-2-(4-methoxybenzyl)-3-vinylphenol

Tetrakis triphenylphosphine palladium (0.147 g, 0.13 mmol) and tributylvinyltin (0.41 mL, 1.40 mmol) were added to a solution of the compound obtained in (94f) (570 mg, 1.27 mmol) in toluene (4 mL), and the mixture was heated to reflux for 6 h. The mixture was cooled to room temperature, followed by addition of toluene (20 mL), and washed with saturated aqueous sodium hydrogencarbonate (20 mL) and saturated brine (20 mL). The organic layer was dried over anhydrous sodium sulfate, and then the solvent was removed under reduced pressure. The residue was passed through a short column, and the resulting crude product (302 mg) was used in the subsequent reaction as it was.

The resulting crude product (302 mg) was dissolved in methyl alcohol (3 mL), followed by addition of pyridine p-toluenesulfonate (19 mg, 0.076 mmol), and the mixture was stirred for 48 h. An appropriate amount of triethylamine was added, and the solvent was removed under reduced pressure. Ethyl acetate (20 mL) was added, and the mixture was washed with saturated aqueous ammonium chloride (20 mL) and saturated brine (20 mL). The organic layer was dried over anhydrous sodium sulfate, and then the solvent was removed under reduced pressure. The residue was purified by silica gel flash chromatography (hexane:ethyl acetate, 10:1 1 to 3:1, v/v) to obtain the title compound (220 mg, yield 55%) as a colorless solid.
¹H NMR (400 MHz, CDCl₃): δ 2.12 (3H, s), 3.76 (3H, s), 4.04 (2H, s), 4.88 (1H, s), 5.05 (2H, s), 5.31 (1H, dd, J = 11.0 Hz, 1.2 Hz), 5.65 (1H, dd, J = 17.4 Hz, 1.2 Hz), 6.77 (1H, d, J = 1.2 Hz), 6.80 (2H, d, J = 8.7 Hz), 6.95 (1H, dd, J = 17.4 Hz, 11.0 Hz), 7.08 (2H, d, J = 8.6 Hz), 7.12 (1H, d, J = 1.2 Hz);
MS (FAB)m/z: 312 (M)⁺.

### (101b) 5-Acetoxymethyl-2-(4-methoxybenzyl)-3-vinyl-phenyl 2,3,4,6-tetra-O-benzoyl-7-deoxy-D-glycero-β-D-gluco-heptopyranoside

The compound obtained in (7d) (400 mg, 0.66 mmol), trichloroacetonitrile (0.33 mL, 3.27 mmol), 1,8-diazabicyclo[5.4.0]-7-undecene (90 µL, 0.060 mmol), and methylene chloride (5 mL) were used to prepare an imidate by the same method as in (1b). Subsequently, the compound obtained in (99a) (136 mg, 0.44 mmol), a boron trifluoride-diethyl ether complex (0.082 mL, 0.65 mmol), and methylene chloride (6 mL) were used to obtain a crude product of the title compound (590 mg) by the same method as in (1b), and the resulting crude product was used in the subsequent reaction as it was.

### (101c) 5-Acetoxymethyl-2-(4-methoxybenzyl)-3-vinyl-phenyl 7-deoxy-D-glycero-β-D-gluco-heptopyranoside

The crude product obtained in (101b) (590 mg), potassium carbonate (905 mg, 6.55 mmol), methanol (8 mL), and methylene chloride (2 mL) were used to obtain the title compound (160 mg, yield 82%) as a colorless solid by the same method as in (1c).
¹H NMR (400 MHz, CD₃OD): δ 1.22 (3H, d, J = 6.6 Hz), 3.35-3.37 (2H, m), 3.42 - 3.44 (2H, m), 3.72 (3H, s), 4.01 - 4.06 (2H, m), 4.20 (1H, d, J = 14.9 Hz), 4.58 (2H, s), 4.91 (1H, d, J = 7.9 Hz), 5.22 (1H, dd, J = 11.1 Hz, 1.5 Hz), 5.63 (1H, dd, J = 17.5 Hz, 1.5 Hz), 6.74 (2H, d, J = 9.0 Hz), 6.97 (1H, dd, J = 17.5 Hz, 11.1 Hz), 7.06 (2H, d, J = 9.0 Hz), 7.15 (1H, s), 7.24 (1H, s);
MS (FAB)m/z: 469 (M+Na)⁺.

### (Test Example 1) Determination of SGLT1 inhibiting activity using human SGLT1 expressing cell

### 1) Preparation of vector expressing human SGLT1 cDNA in animal cell

Amplification was performed by PCR using human SGLT1 cDNA clone (Origene: Clone Number, TC119918; GenBank accession number, NM_000343) as a template. The sense oligonucleotide primer for PCR was
5'-ttaagcttaccatggacagtagcacctggagccc-3' (Primer 1: SEQ ID NO: 1 in the sequence listing),
and the antisense oligonucleotide primer was
5'-ttctcgagtcaggcaaaatatgcatggcaa-3' (Primer 2: SEQ ID NO: 2 in the sequence listing).
The PCR product was subjected to agarose electrophoresis, then a target DNA fragment was recovered from a single band corresponding to 2013 bases, digested with restriction enzymes *Hind*III and *Xho*I, and introduced into the *Hind*III/*Xho*I site of vector pCMV-Script (Stratagene) to obtain SGLT1 expressing plasmid pCMV-SGLT1. A *Hind*III/*Xho*I fragment was excised from pCMV-SGLT1 and introduced into the *Bam*HI/*Xho*I site of pENTR1A (Gateway, Invitrogen) to prepare pENTR-SGLT1. Retrovirus vector pLPCX (Clontech) into which Gateway Vector Conversion System Cassette A (Invitrogen) was introduced was used as the destination plasmid to prepare SGLT1 expressing retrovirus vector pLPCX-SGLT1.

### 2) Establishment of human SGLT1 expressing cell

The retrovirus pLPCX-SGLT1 obtained in 1) was transfected into integrin αsβ3 expressing HEK-293 cells and, the cells were treated with antibiotic G418 (brand name, Geneticin: Invitrogen) and puromycin (Clontech) to obtain HEK-SGLT1 cells stably expressing the target vector having resistance. The stably expressing cells were cultured and maintained in DMEM medium containing 250 mg/mL G418, 1 mg/mL puromycin, 3 mM KGT-1075, and 10% FBS.

### 3) Determination of SGLT1 inhibiting activity

HEK-SGLT1 cells were suspended at a density of 10⁶ cells/mL in DMEM medium containing 250 mg/mL G418, 1 mg/ml puromycin, and 10% FBS, and 100 µL was seeded in each well of a type I collagen-coated 96-well culture plate (Corning). On the following day, the medium was replaced with a sugar uptake buffer (10 mM HEPES [pH 7.5], 5 mM Tris-HCl [pH 7.5], 140 mM NaCl, 2 mM KCl, 1 mM CaCl₂, 1 mM MgCl₂), and the cells were cultured with 1 mM [¹⁴C]-α-methyl-D-glucopyranoside (0.1 mCi) and the test compound at 37°C for 30 min and then washed 3 times with a washing buffer (10 mM HEPES [pH 7.5], 5 mM Tris-HCl [pH 7.5], 140 mM choline chloride, 2 mM KCl, 1 mM CaCl₂, 1 mM MgCl₂). 100 µL of liquid scintillation cocktail (brand name, Supermix: Perkin-Elmer) was added to each well of the 96-well plate and stirred for 10 min, and then radioactivity was determined with micro β (Perkin-Elmer), a type of liquid scintillation counter. The value as the sugar uptake activity was obtained by deducting the radioactivity in the presence of an excess amount of an SGLT1 inhibiting compound as the background from each measured value, the inhibition rate (%) was calculated from the control sugar uptake activity obtained without using a test compound and the sugar uptake activity obtained using a test compound having a predetermined the concentration, and concentrations of the test compound at which the sugar uptake activity was inhibited by 50% were assessed. The results are shown in Table 3. Based on the following results, the compound of the present invention was found to exhibit excellent SGLT1 inhibiting activity.

**[Table 3]**

| Example number | SGLT1 inhibiting activity IC50 (nM) |
|---|---|
| Example 9 | 54 |
| Example 38 | 207 |
| Example 41 | 87 |
| Example 42 | 63 |
| Example 43 | 212 |
| Example 44 | 29 |
| Example 45 | 118 |
| Example 46 | 56 |
| Example 47 | 66 |
| Example 57 | 217 |
| Example 60 | 81 |
| Example 62 | 155 |
| Example 63 | 68 |
| Example 66 | 214 |
| Example 67 | 491 |
| Example 68 | 123 |
| Example 69 | 231 |
| Example 70 | 199 |
| Example 74 | 281 |
| Example 75 | 269 |
| Example 77 | 252 |
| Example 78 | 234 |
| Example 82 | 219 |
| Example 83 | 142 |
| Example 84 | 31 |
| Example 88 | 51 |
| Example 89 | 81 |
| Example 93 | 430 |
| Example 94 | 37 |
| Example 95 | 82 |
| Example 96 | 77 |
| Example 97 | 52 |
| Example 98 | 180 |
| Example 101 | 168 |

### (Test Example 2) Determination of SGLT2 inhibiting activity using human SGLT2 expressing cell

### 1) Preparation of vector expressing human SGLT2 cDNA in animal cell

Amplification was performed by PCR using human SGLT2 cDNA clone (Origene: Clone Number, TC303267; GenBank accession number, NMR_003041) as a template. The sense oligonucleotide primer for PCR was
5'-ttaagcttaccatggaggagcacacagaggcagg-3' (Primer 3: SEQ ID NO: 3 in the sequence listing),
and the antisense oligonucleotide primer was
5'-ttctcgagttaggcatagaagccccagagg-3' (Primer 4: SEQ ID NO: 4 in the sequence listing).
The PCR product was subjected to agarose electrophoresis, then a target DNA fragment was recovered from a single band corresponding to 2037 bases, digested with restriction enzymes *Hind*III and *Xho*I, and introduced into the *Hind*III/*Xho*I site of vector pCMV-Script (Stratagene) to obtain SGLT2 expressing plasmid pCMV-SGLT2. A *Hind*III/*Xho*I fragment was excised from pCMV-SGLT2 and introduced into the *Bam*HI/*Xho*I site of pENTR1A (Gateway, Invitrogen) to prepare pENTR-SGLT2. Retrovirus vector pLPCX (Clontech) into which Gateway Vector Conversion System Cassette A (Invitrogen) was introduced was used as the destination plasmid to prepare SGLT2 expressing retrovirus vector pLPCX-SGLT2.

### 2) Establishment of human SGLT2 expressing cell

The retrovirus pLPCX-SGLT2 obtained in 1) was transfected into integrin αvβ3 expressing HEK-293 cells, the cells were treated with antibiotic G418 (brand name, Geneticin: Invitrogen) and puromycin (Clontech) to obtain HEK-SGLT2 cells stably expressing the target vector having resistance. The stably expressing cells were cultured and maintained in DMEM medium containing 250 mg/mL G418, 1 mg/mL puromycin, 3 mM KGT-1075, and 10% FBS.

### 3) Determination of SGLT2 inhibiting activity

HEK-SGLT2 cells were suspended at a density of 10⁶ cells/mL in DMEM medium containing 250 mg/ml G418, 1 mg/ml puromycin, and 10% FBS, and 100 µL was seeded in each well of a type I collagen-coated 96-well culture plate (Corning). On the following day, the medium was replaced with a sugar uptake buffer (10 mM HEPES [pH 7.5], 5 mM Tris-HCl [pH 7.5], 140 mM NaCl, 2 mM KCl, 1 mM CaCl₂, 1 mM MgCl₂), and the cells were cultured with 1 mM [¹⁴C]-α-methyl-D-glucopyranoside (0.1 mCi) and a test compound at 37°C for 30 min, and then washed 3 times with a washing buffer (10 mM HEPES (pH 7.5), 5 mM Tris-HCl (pH 7.5), 140 mM choline chloride, 2 mM KCl, 1 mM CaCl₂, 1 mM MgCl₂). 100 µL of liquid scintillation cocktail (brand name, Supermix: Perkin-Elmer) was added to each well of the 96-well plate and stirred for 10 min, and then radioactivity was determined with micro β (Perkin-Elmer), a type of liquid scintillation counter. The value obtained by deducting the radioactivity in the presence of an excess amount of an SGLT2 inhibiting compound as the background from each measured value was taken as the sugar uptake activity, the inhibition rate (%) was calculated from the control sugar uptake activity obtained without using a test compound and the sugar uptake activity obtained using a test compound having a predetermined concentration, and the concentrations of the test compound at which the sugar uptake activity was inhibited by 50% were assessed. The results are shown in Table 4. Based on the following results, the compound of the present invention was found to exhibit excellent SGLT2 inhibiting activity.

**[Table 4]**

| Example number | SGLT2 inhibiting activity IC50 (nM) |
|---|---|
| Example 9 | 9.4 |
| Example 38 | 17 |
| Example 41 | 14 |
| Example 42 | 18 |
| Example 43 | 61 |
| Example 44 | 10 |
| Example 45 | 45 |
| Example 46 | 16 |
| Example 47 | 38 |
| Example 55 | 10 |
| Example 56 | 11 |
| Example 57 | 11 |
| Example 60 | 12 |
| Example 62 | 32 |
| Example 63 | 34 |
| Example 64 | 13 |
| Example 65 | 20 |
| Example 66 | 23 |
| Example 67 | 30 |
| Example 68 | 12 |
| Example 69 | 40 |
| Example 70 | 40 |
| Example 74 | 47 |
| Example 75 | 38 |
| Example 77 | 25 |
| Example 78 | 11 |
| Example 82 | 9.4 |
| Example 83 | 39 |
| Example 84 | 6.3 |
| Example 85 | 20 |
| Example 86 | 19 |
| Example 88 | 19 |
| Example 89 | 35 |
| Example 93 | 13 |
| Example 94 | 11 |
| Example 95 | 49 |
| Example 96 | 36 |
| Example 97 | 11 |
| Example 98 | 17 |
| Example 99 | 272 |
| Example 101 | 136 |

### (Test Example 3) In vivo test

Each test compound is suspended or dissolved in a vehicle (0.5% methylcellulose solution) and is orally given to C57BL/6NCrlCrlj mice (7 to 10-week-old male) at a dosage of 10 mL/kg in two or more doses (preferably within the range of 0.03 to 10 mg/kg) after fasting overnight. 10 mL/kg vehicle is orally given to a control group. At 10 min after dosing, 2 g/10 mL/kg of glucose is orally given. Blood sugar levels are measured over time (before giving glucose, and at 20, 40, 60, and 120 min after dosing), and the area under the blood sugar level curve is calculated. The 50% effective dose, ED50, is obtained using the decreasing rate of the area under the blood sugar level curve in each group compared to those in the control group as an indicator to evaluate the efficacy of the test compound in an organism.

### (Formulation Example)

### Tablet

The compound of Example 9 (5 g), lactose (90 g), corn starch (34 g), crystalline cellulose (20 g), and magnesium stearate (1 g) are mixed with a blender and tableted with a tableting machine to obtain a tablet.

### Industrial Applicability

A compound represented by the general formula (I), (II), or (III) or a pharmacologically acceptable salt thereof of the present invention causes minimal adverse reactions, exhibits excellent human SGLT1 and/or SGLT2 inhibiting activity, and is useful as a therapeutic or preventive drug for type 1 diabetes, type 2 diabetes, gestational diabetes, hyperglycemia due to other causes, impaired glucose tolerance (IGT), a diabetes-related disease (for example, obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout), or a diabetic complication (for example, retinopathy, nephropathy, nervous disorder, cataract, foot gangrene, infections, or ketosis) as well as a pharmaceutical composition for therapeutic or prophylactic treatment of a warm-blooded animal (for example, a human, an equine, a bovine or a swine, preferably a human).

### [Sequence listing free text]

SEQ ID NO: 1: PCR sense primer for human SGLT1
SEQ ID NO: 2: PCR antisense primer for human SGLT1
SEQ ID NO: 3: PCR sense primer for human SGLT2
SEQ ID NO: 4: PCR antisense primer for human SGLT2

## Claims

1. A compound represented by the following general formula (I): 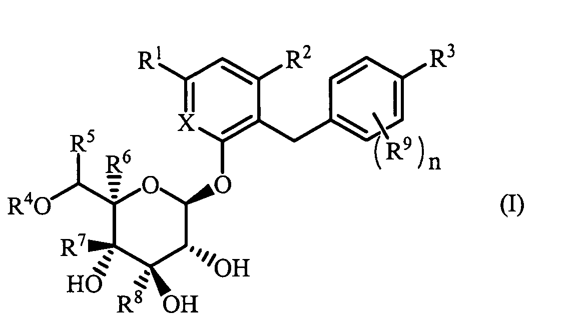 wherein
R¹ represents a hydrogen atom, an amino group, a mono- or di-(C₁-C₆ alkyl)amino group, a C₁-C₆ alkyl group, a hydroxy C₁-C₆ alkyl group, a C₂-C₇ acyloxy C₁-C₆ alkyl group, a hydroxy C₂-C₇ acyloxy C₁-C₆ alkyl group, or an amino C₂-C₇ acylamino group;
R² represents a hydrogen atom, a halogen atom, or a C₁-C₆ alkyl group;
R³ represents a C₁-C₆ alkyl group, a hydroxy C₁-C₆ alkyl group, a C₁-C₆ alkoxy group, a hydroxy C₁-C₆ alkoxy group, a C₁-C₆ alkylthio group, or a halogenated C₁-C₆ alkoxy group;
R⁴ represents a hydrogen atom, a C₁-C₆ alkyl group, a C₂-C₇ acyl group, a C₁-C₆ alkoxycarbonyl group, a hydroxy C₂-C₇ acyl group, a hydroxy C₁-C₆ alkoxycarbonyl group, a hydroxycarbonyl C₂-C₇ acyl group, a C₁-C₆ alkoxy C₂-C₇ acyl group, a C₁-C₆ alkoxy C₁-C₆ alkoxycarbonyl group, or a C₁-C₆ alkoxycarbonyl C₂-C₇ acyl group;
R⁵, R⁶, R⁷ and R⁸ are the same or different and each represents a hydrogen atom or a C₁-C₆ alkyl group, provided that R⁵, R⁶, R⁷, and R⁸ are not hydrogen atoms at the same time;
R⁹ represents a halogen atom;
n is 0 to 4; and
X is CH or N,
or a pharmacologically acceptable salt thereof.

2. The compound or a pharmacologically acceptable salt thereof according to claim 1, wherein R¹ represents an amino group, a mono- or di-(C₁-C₆ alkyl)amino group, a C₁-C₆ alkyl group, a hydroxy C₁-C₆ alkyl group, or a hydroxy C₂-C₇ acyloxy C₁-C₆ alkyl group.

3. The compound or a pharmacologically acceptable salt thereof according to claim 1, wherein R¹ represents an amino group, a hydroxy C₁-C₆ alkyl group, or a hydroxy C₂-C₇ acyloxy C₁-C₆ alkyl group.

4. The compound or a pharmacologically acceptable salt thereof according to claim 1, wherein R¹ represents an amino group, a hydroxymethyl group, a hydroxyethyl group, or a hydroxyacetyloxymethyl group.

5. The compound or a pharmacologically acceptable salt thereof according to any one selected from claims 1 to 4, wherein R² represents a hydrogen atom, a fluorine atom, or a methyl group.

6. The compound or a pharmacologically acceptable salt thereof according to any one selected from claims 1 to 5, wherein R³ represents a C₁-C₆ alkyl group, a C₁-C₆ alkoxy group, or a halogenated C₁-C₆ alkoxy group.

7. The compound or a pharmacologically acceptable salt thereof according to any one selected from claims 1 to 5, wherein R³ represents a C₁-C₃ alkyl group, a C₁-C₃ alkoxy group, or a halogenated C₁-C₃ alkoxy group.

8. The compound or a pharmacologically acceptable salt thereof according to any one selected from claims 1 to 5, wherein R³ represents a methyl group, an ethyl group, a methoxy group, an ethoxy group, an isopropoxy group, or a cyclopropyloxy group.

9. The compound or a pharmacologically acceptable salt thereof according to any one selected from claims 1 to 8, wherein R⁴ represents a hydrogen atom, a C₂-C₇ acyl group, or a hydroxy C₂-C₇ acyl group.

10. The compound or a pharmacologically acceptable salt thereof according to any one selected from claims 1 to 8, wherein R⁴ represents a hydrogen atom, a C₂-C₃ acyl group, or a hydroxy C₂-C₃ acyl group.

11. The compound or a pharmacologically acceptable salt thereof according to any one selected from claims 1 to 8, wherein R⁴ represents a hydrogen atom.

12. The compound or a pharmacologically acceptable salt thereof according to any one selected from claims 1 to 11, wherein R⁵ and R⁷ are the same or different and each represents a hydrogen atom or a C₁-C₆ alkyl group.

13. The compound or a pharmacologically acceptable salt thereof according to any one selected from claims 1 to 11, wherein R⁵ and R⁷ are the same or different and each represents a hydrogen atom or a methyl group.

14. The compound or a pharmacologically acceptable salt thereof according to any one selected from claims 1 to 13, wherein R⁶ and R⁸ represent a hydrogen atom.

15. The compound or a pharmacologically acceptable salt thereof according to any one selected from claims 1 to 14, wherein n is 1.

16. The compound or a pharmacologically acceptable salt thereof according to any one selected from claims 1 to 15, wherein R⁹ represents a fluorine atom.

17. The compound or a pharmacologically acceptable salt thereof according to any one selected from claims 1 to 14, wherein n is 0.

18. The compound or a pharmacologically acceptable salt thereof according to any one selected from claims 1 to 17, wherein X is CH.

19. A compound represented by the following general formula (II): 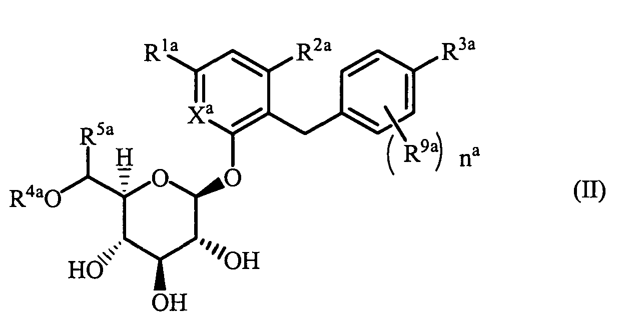 wherein
R^{1a} represents a hydrogen atom, an amino group, a mono- or di-(C₁-C₆ alkyl)amino group, a C₁-C₆ alkyl group, a hydroxy C₁-C₆ alkyl group, a C₂-C₇ acyloxy C₁-C₆ alkyl group, a hydroxy C₂-C₇ acyloxy C₁-C₆ alkyl group, or an amino C₂-C₇ acylamino group;
R^{2a} represents a hydrogen atom, a halogen atom, or a C₁-C₆ alkyl group;
R^{3a} represents a C₁-C₆ alkyl group, a hydroxy C₁-C₆ alkyl group, a C₁-C₆ alkoxy group, a hydroxy C₁-C₆ alkoxy group, a C₁-C₆ alkylthio group, or a halogenated C₁-C₆ alkoxy group;
R^{4a} represents a hydrogen atom, a C₁-C₆ alkyl group, a C₂-C₇ acyl group, a C₁-C₆ alkoxycarbonyl group, a hydroxy C₂-C₇ acyl group, a hydroxy C₁-C₆ alkoxycarbonyl group, a hydroxycarbonyl C₂-C₇ acyl group, a C₁-C₆ alkoxy C₂-C₇ acyl group, a C₁-C₆ alkoxy C₁-C₆ alkoxycarbonyl group, or a C₁-C₆ alkoxycarbonyl C₂-C₇ acyl group;
R^{5a} represents a C₁-C₆ alkyl group;
R^{9a} represents a halogen atom;
n^{a} is 0 to 4; and
X^{a} is CH or N,
or a pharmacologically acceptable salt thereof.

20. A compound represented by the following general formula (III): 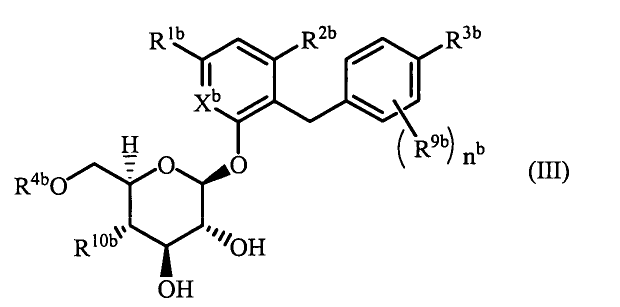 wherein
R^{1b} represents a hydrogen atom, an amino group, a mono- or di-(C₁-C₆ alkyl)amino group, a C₁-C₆ alkyl group, a hydroxy C₁-C₆ alkyl group, a C₂-C₇ acyloxy C₁-C₆ alkyl group, a hydroxy C₂-C₇ acyloxy C₁-C₆ alkyl group, or an amino C₂-C₇ acylamino group;
R^{2b} represents a hydrogen atom, a halogen atom, or a C₁-C₆ alkyl group;
R^{3b} represents a C₁-C₆ alkyl group, a hydroxy C₁-C₆ alkyl group, a C₁-C₆ alkoxy group, a hydroxy C₁-C₆ alkoxy group, a C₁-C₆ alkylthio group, or a halogenated C₁-C₆ alkoxy group;
R^{4b} represents a hydrogen atom, a C₁-C₆ alkyl group, a C₂-C₇ acyl group, a C₁-C₆ alkoxycarbonyl group, a hydroxy C₂-C₇ acyl group, a hydroxy C₁-C₆ alkoxycarbonyl group, a hydroxycarbonyl C₂-C₇ acyl group, a C₁-C₆ alkoxy C₂-C₇ acyl group, a C₁-C₆ alkoxy C₁-C₆ alkoxycarbonyl group, or a C₁-C₆ alkoxycarbonyl C₂-C₇ acyl group;
R^{9b} represents a halogen atom;
n^{b} is 0 to 4;
R^{10b} represents a hydrogen atom, a C₁-C₆ alkyl group, a C₁-C₆ alkoxy group, or a hydroxyl group;
X^{b} is CH or N; and,
provided that when R^{10b} represents a hydroxyl group, R^{4b} represents a C₁-C₆ alkyl group,
or a pharmacologically acceptable salt thereof.

21. A compound or a pharmacologically acceptable salt thereof selected from the following:
2-(4-methoxybenzyl)-5-hydroxymethylphenyl 7-deoxy-D-glycero-β-D-gluco-heptopyranoside, 5-amino-2-(4-ethylbenzyl)phenyl 7-deoxy-L-glycero-β-D-gluco-heptopyranoside, 5-amino-2-(4-ethylbenzyl)phenyl 4-C-methyl-β-D-glucopyranoside,
5-amino-2-(4-ethylbenzyl)phenyl 7-deoxy-D-glycero-β-D-gluco-heptopyranoside, 5-amino-2-(4-methoxybenzyl)phenyl 7-deoxy-L-glycero-β-D-gluco-heptopyranoside,
5-amino-2-(4-methoxybenzyl)phenyl 7-deoxy-D-glycero-β-D-gluco-heptopyranoside,
3-fluoro-5-hydroxymethyl-2-(4-methoxybenzyl)phenyl 7-deoxy-D-glycero-β-D-gluco-heptopyranoside, 2-(4-ethylbenzyl)-3-fluoro-5-hydroxymethyl-phenyl 7-deoxy-D-glycero-β-D-gluco-heptopyranoside, 2-(4-ethoxybenzyl)-5-hydroxymethylphenyl 7-deoxy-D-glycero-β-D-gluco-heptopyranoside, 2-(4-ethylbenzyl)-5-(2-hydroxyethyl)phenyl 7-deoxy-D-glycero-β-D-gluco-heptopyranoside, 3-chloro-5-hydroxymethyl-2-(4-methoxybenzyl)phenyl 7-deoxy-D-glycero-β-D-gluco-heptopyranoside, 2-(2-fluoro-4-methoxybenzyl)-5-hydroxymethylphenyl 7-deoxy-D-glycero-β-D-gluco-heptopyranoside, 5-hydroxymethyl-2-(4-methoxybenzyl)-3-methylphenyl 7-deoxy-D-glycero-β-D-gluco-heptopyranoside, 2-(4-cyclopropyloxybenzyl)-5-hydroxymethylphenyl 7-deoxy-D-glycero-β-D-gluco-heptopyranoside, 2-(4-ethoxybenzyl)-3-fluoro-5-hydroxymethyl-phenyl 7-deoxy-D-glycero-β-D-gluco-heptopyranoside, 3-fluoro-5-hydroxymethyl-2-(4-isopropoxybenzyl)phenyl 7-deoxy-D-glycero-β-D-gluco-heptopyranoside, 5-hydroxyacetyloxymethyl-2-(4-ethoxybenzyl)phenyl 7-deoxy-D-glycero-β-D-gluco-heptopyranoside, 2-(4-cyclopropoxybenzyl)-5-hydroxymethyl-3-methyl-phenyl 7-deoxy-D-glycero-β-D-gluco-heptopyranoside, 2-(4-cyclopropylbenzyl)-5-hydroxymethylphenyl 7-deoxy-D-glycero-β-D-gluco-heptopyranoside, 3-chloro-2-(4-ethoxybenzyl)-5-hydroxymethyl-phenyl 7-deoxy-D-glycero-β-D-gluco-heptopyranoside, 3-fluoro-5-hydroxymethyl-2-(4-methylbenzyl)phenyl 7-deoxy-D-glycero-β-D-gluco-heptopyranoside, 2-(4-ethylbenzyl)-5-hydroxymethyl-3-methylphenyl 7-deoxy-D-glycero-β-D-gluco-heptopyranoside, and 2-(4-cyclopropylbenzyl)-3-fluoro-5-hydroxymethylphenyl 7-deoxy-D-glycero-β-D-gluco-heptopyranoside.

22. A pharmaceutical composition comprising, as an active ingredient, the compound or a pharmacologically acceptable salt thereof according to any one of claims 1 to 21.

23. The pharmaceutical composition according to claim 22 for inhibition of human SGLT1 and/or human SGLT2 activity.

24. The pharmaceutical composition according to claim 23 for therapeutic or prophylactic treatment of type 1 diabetes, type 2 diabetes, gestational diabetes, hyperglycemia due to other causes, or impaired glucose tolerance.

25. The pharmaceutical composition according to claim 23 for therapeutic or prophylactic treatment of type 1 diabetes, type 2 diabetes, or impaired glucose tolerance.

26. The pharmaceutical composition according to claim 23 for therapeutic or prophylactic treatment of a diabetes-related disease.

27. The pharmaceutical composition according to claim 26, wherein the diabetes-related disease is obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout.

28. The pharmaceutical composition according to claim 26, wherein the diabetes-related disease is obesity.

29. The pharmaceutical composition according to claim 23 for therapeutic or prophylactic treatment of a diabetic complication.

30. The pharmaceutical composition according to claim 29, wherein the diabetic complication is retinopathy, nephropathy, nervous disorder, cataract, foot gangrene, infection, or ketosis.

31. Use of the compound or a pharmacologically acceptable salt thereof according to any one selected from claims 1 to 21 for production of a pharmaceutical composition.

32. The use according to claim 31, wherein the pharmaceutical composition is a composition for inhibition of human SGLT1 and/or human SGLT2 activity.

33. The use according to claim 31, wherein the pharmaceutical composition is a composition for therapeutic or prophylactic treatment of type 1 diabetes, type 2 diabetes, gestational diabetes, hyperglycemia due to other causes, or impaired glucose tolerance.

34. The use according to claim 31, wherein the pharmaceutical composition is a composition for therapeutic or prophylactic treatment of type 1 diabetes, type 2 diabetes, or impaired glucose tolerance.

35. The use according to claim 31, wherein the pharmaceutical composition is a composition for therapeutic or prophylactic treatment of a diabetes-related disease.

36. The use according to claim 35, wherein the diabetes-related disease is obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout.

37. The use according to claim 35, wherein the diabetes-related disease is obesity.

38. The use according to claim 31, wherein the pharmaceutical composition is a composition for therapeutic or prophylactic treatment of a diabetic complication.

39. The use according to claim 38, wherein the diabetic complication is retinopathy, nephropathy, nervous disorder, cataract, foot gangrene, infection, or ketosis.

40. A method for inhibiting human SGLT1 and/or human SGLT2 activity comprising administering a pharmacologically effective amount of the compound or a pharmacologically acceptable salt thereof according to any one selected from claims 1 to 21 to a warm-blooded animal.

41. A method for therapeutic or prophylactic treatment of a disease comprising administering a pharmacologically effective amount of the compound or a pharmacologically acceptable salt thereof according to any one selected from claims 1 to 21 to a warm-blooded animal.

42. The method according to claim 41, wherein the disease is type 1 diabetes, type 2 diabetes, gestational diabetes, hyperglycemia due to other causes, or impaired glucose tolerance.

43. The method according to claim 41, wherein the disease is type 1 diabetes, type 2 diabetes, or impaired glucose tolerance.

44. The method according to claim 41, wherein the disease is a diabetes-related disease.

45. The method according to claim 44, wherein the diabetes-related disease is obesity, hyperlipemia, hypercholesterolemia, lipid metabolic abnormality, hypertension, fatty liver, metabolic syndrome, edema, heart failure, angina pectoris, myocardial infarction, arteriosclerosis, hyperuricemia, or gout.

46. The method according to claim 44, wherein the diabetes-related disease is obesity.

47. The method according to claim 41, wherein the disease is a diabetic complication.

48. The method according to claim 47, wherein the diabetic complication is retinopathy, nephropathy, nervous disorder, cataract, foot gangrene, infection, or ketosis.

49. The method according to any one selected from claims 40 to 48, wherein the warm-blooded animal is a human.


### [Sequence Listing]

SEQUENCE LISTING

<110> DAIICHI SANKYO COMPANY, LIMITED

<120> BENZYLPHENYLGLUCOPYRANOSIDE DERIVATIVE

<130> EPP100500

<140> EP 07791904.8 (PCT/JP2007/065231)
<141> 2007-08-03

<150> JP2006-213600
<151> 2006-08-04

<160> 4

<170> PatentIn version 3.4

<210> 1
<211> 34
<212> DNA
<213> Artificial

<220>
<223> PCR sense primer for human SGLT1

<400> 1
ttaagcttac catggacagt agcacctgga gccc 34

<210> 2
<211> 30
<212> DNA
<213> Artificial

<220>
<223> PCR antisense primer for human SGLT1

<400> 2
ttctcgagtc aggcaaaata tgcatggcaa 30

<210> 3
<211> 34
<212> DNA
<213> Artificial

<220>
<223> PCR sense primer for human SGLT2

<400> 3
ttaagcttac catggaggag cacacagagg cagg 34

<210> 4
<211> 30
<212> DNA
<213> Artificial

<220>
<223> PCR antisense primer for human SGLT2

<400> 4
ttctcgagtt aggcatagaa gccccagagg 30